# EUROPEAN PATENT APPLICATION

(11) **EP 3 699 281 A1**
(43) Date of publication of application: **26.08.2020**
(21) Application number: 20167428.0
(22) Date of filing: 27.02.2017
(51) Int. Cl.: C12N 15/113, C12N 9/22, C12N 15/82

(54) **NOVEL CAS9 SYSTEMS AND METHODS OF USE**

(30) Priority: 11.03.2016 US 201662306926 P; 04.08.2016 US 201662370858 P
(62) Divisional of application: 17711832.0
(71) Applicant: Pioneer Hi-Bred International, Inc., Johnston, Iowa 50131-1014 (US)
(72) Inventor: CIGAN, Andrew Mark, Madison, WI 53704 (US); KING, Matthew G., Johnston, IA 50131-0552 (US); LIN, Haining, Clive, IA 50325 (US); YOUNG, Joshua K., Johnston, IA 50131 (US)
(74) Representative: J A Kemp LLP

(57) **Abstract**

Compositions and methods are provided for novel Cas9 systems, including, but not limiting to, novel guide polynucleotide/Cas9 endonucleases complexes, single or dual guide RNAs, guide RNA elements, and Cas9 endonucleases. The novel Cas9 systems described herein are derived from a CRISPR-Cas locus that differs from type II CRISPR-Cas loci, in that the novel CRISPR-Cas locus described herein contains multiple CRISPR arrays flanking a tracrRNA encoding region and cas9 gene and does not contain a cas1 gene and a cas2 gene. The present disclosure also describes methods for genome modification of a target sequence in the genome of a cell, for gene editing, and for inserting a polynucleotide of interest into the genome of a cell.

SEQUENCE LISTING

<110>  Pioneer Hi-Bred International Inc.
       Cigan, Andrew  Mark
       King, Matt
       Lin, Helen
       Young, Joshua K

<120>  NOVEL CAS9 SYSTEMS AND METHODS OF USE

<130>  BB2554 PCT

<150>  62/306926
<151>  2016-03-11

<150>  62/370858
<151>  2016-08-04

<160>  80

<170>  PatentIn version 3.5

<210>  1
<211>  5185
<212>  DNA
<213>  Artificial sequence

<220>
<223>  CRISPR-Cas Locus 1 (Bacillus species)

<400>  1
ataaggtaaa ttaattcata ttgatatgta ggtgaaacaa atagaaaata aagagcctaa      60
acaataatac gatctaagta aaatatacac atataaagag ttccctgata ataaaattag     120
cggtcgttgc gataattgcg gcaatacctc attcaagagt tccgttaaag atttcatctt     180
tttaagagaa tgtcgtcaat gcggtatgaa gaaaatcata tagccctatc cagggctttt     240
ttctttgtgt aaatggaagt aaacttttgg gtaaaagaga aggtgcatgt attgtaactg     300
aattgttacc tctctatcct tcctaaaata ataagcatgg cgccatagta ttgattaagc     360
gatgtagaga ctaaagaaaa ctaaataaaa tcttcatttg aacagagaga gcccccttat      420
aagaaaataa ggggatatca gaatagtaca taaaacttgt agaactccaa tatgggaact     480
ctatattagt ttaacatgta ttaaagggaa ttttgaaaat caacaaaata taggataact     540
cgctctatat aggttgaaaa ataaaaaaca aaaaagcctg taagcgttga tattacagac     600
ctttttgaat acgtatcata atacagcaat ggcattaatg gaactatgac taggagggat     660
tgcatgaaga aagtaacaaa attataatac agcaatggct taaatagaac tatgattaat     720
gattttcgct ctaaatcaca gaggatatta taccagattt ctgttgtttt tctgaaaacc     780
acccttgtct tcatattttt tatttgaaaa atcagatatt ttcgttataa tagagatatc     840
ataatacagc aatggctttg atgtttctat gataagggct taggcccgtg gcgttgggga      900
tcgcctgccc attttaatgg gcttctcccc acctatttaa tgagaatttt acaaccttgg      960
ctattcttaa atagctaagg tttttttgta tacatataaa ttaatagagg tgagattatg    1020
agagagttgg attatcgcat aggattagat attggaacga attctattgg ctgggggatc    1080
attgaattat cttggaacaa agatagagaa caatatgaga aagcaagaat tgtcgacaag    1140
ggtgttcgta tgtttgataa ggctgaaata cctaagactg gtgcttctct tgctgaaccg    1200
aggcgtttag cgcgctcatc acgtagaaga ttaaatcgca aaagccagag aaaaaaagat    1260
atacgtaatt tacttgttca acatgaaatt attagtcaaa gggaattggc ttcgttatat    1320
cccctgtcaa aaagttcaat ggatatttgg gatattcgat tggagggatt agatcgcttg    1380
ttagaccatt ttgaatggac tcgattatta attcatttag cgcaaaggcg cggttttaaa    1440
tcaaatcgga agtctgaatt gaaagatgtg gagacaggaa aagtattatc aagtattcaa    1500
gcaaatgaaa aacgattatc actgtaccgt acagtgggag agatgtggat gaagaatgaa    1560
gattttagta aatatgacaa aaggcgtaat tctcctaatg agtatgtatt ttcggttagc    1620
cgtgcagatt tagaaaaaga aattgtgact ctatttgaag cgcaaagaaa atttcagtca    1680
tcatatgcat cggttgattt acaaaaaaca tacttacaaa tttgggcaca ccaacttcct    1740
tttgcttctg ggaatgcaat tgtaaataaa gtgggatatt gttcattatt aaaaggtaaa    1800
gaaaagagag ttccgaaagc aacatatact tttcaatatt tcagtacact agatcaaata    1860
aatcgaacac gattaggtcc taatttccaa ccatttacga aggaacagag agacattatt    1920

ttagataaaa tgtttaatcg aacagattat tataaaaaaa agacaatacc cgaagttact 1980
tactatgata ttcggaaatg gttagcatta gatgaaacaa ttcaatttaa aggacttacc 2040
tatgacccaa atgaagagct gaaaaaaata gaattgaaat cctttattaa tttaaagcca 2100
ttttatgaaa ttaaacaggt agttactaat tacgccaaga aataaatgaa ggcattctca 2160
acattagact atgatacatt tgcatatgct ttaacagttt ataaaacgga caaagatatt 2220
agatcttatt taaagaaatc taataattta tcaaaatgtt gctacgatga tcaattaata 2280
gaagagctat taactctctc ctatacaaag tttggtcatt tatcatttaa agcaattaat 2340
catgtactgc caattatgca agagggaagg acttatcagg aagcaataca ccaattagga 2400
tatgatacca ctaatctaaa aaaagaaaac agaagtatgt tcttgcccct tattcccgat 2460
gagataacaa atccaattgt taaaagagca ctaactcaag cacgtaaagt tgtaaatgct 2520
attattagaa gatatggttc cccaaattct gttcatattg aactagcccg tgagcttcct 2580
aaaaatcatg atgagagaac gaaaataatg aaagctcatg atgaaaatta taagaaaaat 2640
aaaggagcca tatcaatttt gattgagaat ggaattttaa atccgacagg atatgatatt 2700
gtacgttata agttatggaa agagcaagga gagcgatgtg tttattcgct aaaacagatt 2760
cctgctaata cgttttttaa tgaattgaaa aaagagcgaa gtggcccccc ggttctagag 2820
atagatcaca ttttaccgta tagtcagagt tttattgata gttatcataa taaagtacta 2880
gtttatgagg aggagaatca aaaaaaggga aatcgaattc catatactta ttttttagaa 2940
gaaaataagg actgggaaag cttcgaaagc tacgtacgat tgaatagttt tttgtctaaa 3000
aagaagcgcg gatatttatt gaaaaaagct tacttgccaa gagagagtaa catgattaag 3060
gagcgtcatt taaatgatac tcgatatgct agtagctatc tgaaaaactt cattgagaaa 3120
aatttgaaat ttaaagaagt tgaaggtagt acaagaaaca aacatgtaca gacggttaac 3180
ggtataatta cagcccatct gcgaaaaaga tggggattag aaaaagatag gaaggaaaca 3240
tatttgcatc atgcaatgga cgctattatt gttgcttgta cagaccatca tatggttact 3300
aaagtaacgg agtactatca aataaaagaa agtaataagt caataaagaa accatacttt 3360
cctttgccat gggtgggctt tagagaggaa attttatcac atttagcaag gcaaccaatt 3420
gctagaaaaa ttagtgagga acttaaaatt ggatatcaat catttgatta tatacttgta 3480
tcgcgaatgc caaaaagatc tgtcactgga tcagctcatg aacagacaat catgaaaaaa 3540
ggtggtatcg acaaaaaagg taaaactatt atcataaagc gtgtgtactt aaaggatatt 3600
aagtttgatg agaatggcga ttttaaaatg gttggaaaag aacaagattt agcaacttat 3660
gaagcgataa agcaaagata tatagagtat ggaaaagaat cgaagaaagc atttgaaaca 3720
cctttataca agcctagtaa aaaaggaaaa ggaaacctca ttaaaaagat caaagtagaa 3780
gtgcaaacca aatcttttgt tcgagaagtt aatggaggcg tagctcaaaa tggtgattta 3840
gtaagagtag atttgtttga aaaggataat agatattata tggtacctat ttatgtaatg 3900
gatactgttc attccgaact accaaataaa gctgtaacaa gcagtaaagg ctatgagcaa 3960
tggttaacaa tagataacag ctttacgttc aaattcagtt tatatcctta tgatttagta 4020
cgacttgtaa aaggtgatga agatcgtttc ctatatttta gtacccttga tattaattcg 4080
gatcgtctta atttcaaaga tgtaaacacg ccatcaaagc aggctgaaaa tcgttatagc 4140
cttaaaacaa ttgagaattt agaaaaatat gaggttggtg ttttaggtga tttaaggttt 4200
gtgagacaag aaatacgtaa aaatttttaa tagataaaaa ttagcggaat ttttatacgg 4260
taagcaatat tagcttgttc accgtattat aaaaaacaaa aaagcctgta agcgttgatg 4320
ttataggctt ttttgaatac gtatcataat acagcaatgg cttaaatgga actatgacca 4380
ggccaagaag tggaagggtt gtgaagcgat cataatacag caatggctta aatggaacta 4440
tcacgtccga acaaactacg tcatcaatac ctttatcata atacagcaat agctttaatg 4500
gaactatgac acaaatgtgg acaaccagtc atcatataca atcataatac agaaatggct 4560
ttaatgggac tatgacaaat acgatcaatt acatctagac tttgtcatca taatacagaa 4620
atggcttaaa tggaactatg gcacgccttt tggtatggta aaaataatct ccatcataat 4680
acggaaatgg ccttaatgga attatgactt tatctggaat tatcgttcca aaaaaatatc 4740
ataacacagc taaaaaatac aataataagt tcagtatagc gctatgctac ttttcgccgg 4800
gtttatttct atcatttgaa tttgaaatat atctctatcc tctattccca agacacgcat 4860
ttcttcttta aatgaacgcc agggttcaat cttaatagat ttagtagttt tatctattag 4920
cgtatactca tgtacataat atgatttttc atttttgctc attttaattt ttccaaacct 4980
ccttaaaaca actgaaaaac tttaggtgta taaaaattac atattaagtg ctggttttta 5040
attaatgttt atttggtatt gcttatagta attgtaatat ctttttaagg ataagtgaga 5100
cttaaagccg atttgctgca taatttgttc aggattattt ttctgtttaa ttttacttaa 5160
aataaaggtg tttcggaaat gttga 5185

<210> 2
<211> 5630
<212> DNA
<213> Artificial sequence

<220>
<223> CRISPR-Cas Locus 2 (Bacillus species)

<220>
<221>  misc_feature
<222>  (4480)..(4481)
<223>  n is a, c, g, or t

<400>  2
tacataaaac ttgtagaact ccaatatggg aactctatat tagtttaacg cgtatgaaag      60
agaaggtgga tgtattgtaa ctgaattgtt acttctctat ccttcctaaa aataataagc     120
atggcgctat agcattgatt aagtgatgga aagattaaag acaacaaaac aaaatcttca     180
tttgaaaaga gaaagccccc ttataagaaa ataagggggt tgaggtagaa taaagtaaac     240
gtcgactcga actccaaacc agcggagctt ctaagttagt ttaacacgta tgaaagagaa     300
ggtggatgta ttgtaatcga attgttaccc ctctgtcctt ccttaaaata ataaaaatcc     360
tttatgtata tagaaattat atgtttttga aaaccgataa aatatagggt aactcactct     420
atataggttg aaaagtaaaa atgaaaaacc ctgtaagcgt tgatattaca gggttttttg      480
aatacgtatc ataaaacagc aatggctaaa atggaactat gacaccactg tttgttgtgt     540
tgctccactt gcaatcataa aacagcaatg gctttaatgg gactatgact aggagggatt     600
gcatgaagaa agtaacaaaa ttataaaaca gcaatggcta aaatggaacc ataattaatg     660
attttcgctc taaatcacag aggatattat agcagatctc tgttgttttt ctgaaaacta     720
cccttgtctc catggattta tttgaaaaat tcgacatttt ttgttataat agggatatca     780
taaaacagca atggctttga tgtttctatg ataagggctt aggcccgtgg cgttggggat     840
cgcctgccca tttttatggg cttctcccca tctatttaat gaaaatttta caaccttggc     900
tattcttaaa tagctaaggt ttttttgtat acatataaac taatagaggt gagattatga     960
gagagttgga ttatcgcata ggattagata ttggaacgaa ttctattggc tggggtgttg    1020
ttgaattatc ttggaacaaa gatagagaac aatatgagaa agcaagaatt gtcgacaagg    1080
gtgttcgtat gtttgataag gctgaaatac ctaaaactgg tgcttctctt gctgaaccga    1140
ggcgtacagc gcgttcatca cgtagaaggt taaatcgcaa aagccagaga aaaaaagata    1200
tacgtaattt acttgttcaa catgaaatta ttagtcaaaa ggaattggct tcgttatatc    1260
ccctgtcaaa aagttcaatg gatatttggg atattcgatt ggatgggtta gatcgcttgt    1320
tagaccgtct tgaatggact cgattattaa ttcatttagc acaaaagcgc ggttttaaat    1380
caaatcgaaa gtctgaactg aaagatgtgg agacaggaaa agtattatca agtattcaag    1440
taaatgaaaa acgattatca ctgtaccgta cagtgggaga gatgtggatg aagaatgaag    1500
attttagtaa atatgacaaa aggcgtaatt ctcctaatga gtatgtattt tcggttagcc    1560
gtgcaaattt agaaaaagaa attgtgactc tatttgaagc gcaaagaaaa tttcagtcat    1620
catatgcatc agttgattta caaaaaacat acttacaaat ttgggcacat caacttcctt    1680
ttgcttctgg gaatgcaatt gtaaataaag taggatattg ttcattatta aaagggaaag    1740
aaaagagagt tccgaaagca acatatactt ttcaatattt tagtacacta gatcaaataa    1800
accgaacacg attaggacct aatttccaac catttacgaa ggaacagaga gacataatct    1860
tagataaaat gtttcaacgg acagattatt ataaaaaaaa gacaataccc gaagttactt    1920
actatgatat tcggaaatgg ttagcactag atgaaacaat tcaatttaaa ggacttacct    1980
atgacccaaa cgaagagctg aaaaaaatag aaatgaaact ctttataaat ttaaagccat    2040
tttatgaaat taaaaaggta gttactaatt acgcaaaaaa aacaaatgag gtattctcaa    2100
cattagacta tgatacagtt gcatacgctt taacagttta taaaacggac aaagatatta    2160
tatcttattt gaagagatct aataatttat caaaacgttg ttacgatgat caattaatag    2220
aagagctatt aactctttcc tatacaaagt ttggtcattt atcatttaaa gccattaatc    2280
atgtactgcc aattatgcaa gagggaagga cttatcagga agcaatacac caattaggat    2340
atgataccac taatctaaaa aaagaaaaca gaagtatgtt cttgcccctt attccagatg    2400
agataacaaa tccaattgtt aaaagagcac taactcaagc acgtaaagtt gtaaatgcta    2460
ttattagaag atatggttcc cccaattctg ttcatattga actagctcgt gagctttcta    2520
aaactcatga tgagagaacg aaaataatga aagctcatga tgaaaattat aagaaaaata    2580
aaggagccat atcaattttg attgagaatg gaattttaaa tccgacagga tatgatattg    2640
tacgttataa gttatggaaa gagcaaggag aacgatgtgc ttattcgcta aaacagattc    2700
atgctaatac gttttttaat gaattgaaaa aagagcgaag tggtccccca gttctagaga    2760
tagatcacat tttaccgtat agtcagagtt ttattgatag ttatcataat aaagtactag    2820
tttatgggga tgagaatcaa aaaaagggaa atcgaattcc atatacttat tttttagaag    2880
gaaataagga ctgggaaagc tttgaaagct acgtacgatt gaatagttta ttttctaaaa    2940
agaagcgcgg atatttattg aaaaaagctt acttgccaag agagagtaac atgattaagg    3000
agcgtcattt aaatgatact cgatatgcta gtagctattt gaaaaacttc attgagaaaa    3060
atttgaaatt taaagaagtc gaaggtagta cacgaaaaaa acatgtacag acggttaacg    3120
gtataattac agcccatctg cgaaaaagat ggggattaga aaaagataga caggaaacat    3180
atttgcatca tgcaatggac gctattatcg ttgcttgtac agaccatcat atggtcacta    3240
aagtaacgga gtactatcaa ataaaagaaa gtaataagtc aataaagaaa ccatactttc    3300
ctttgccatg ggtggccttt agagaggaaa ttttatcaca tttagcaagg cagccaattg    3360

ctagaaaaat tagtgaggaa cttaaaattg gatatcaatc atttgattat atacttgtat 3420
cgcgaatgcc aaaaagatct gtcactggag cagctcatga acagacaatc atgaaaaaag 3480
gtggtatcga taaaaaagga aagacaatta tcataaagcg tgtgtactta aaggatatta 3540
agtttgatga gaatggcgat tttaaaatgg ttggaaaaga acaggattta gcaacttatg 3600
aagcgataaa gcaaagatat atagagtatg gaaaagaatc gaaaaaagca tttgaaacac 3660
ctttatacaa gcctagtaaa aaaggaaaag ggaacctcat taaaaagatc aaagtagaag 3720
tgcaaaccaa atcttttgtt cgagaagtta atggaggcgt agctcaaaat ggtgatttag 3780
taagagtaga tttgtttgaa aaagataata gatattatat ggtacctatt tatgtaatgg 3840
atactgttca ttccgaacta ccaaataaag ctgtaacaag cagtaaaggc tatgagaaat 3900
ggttaacaat agataacagc tttacgttca aattcagttt atatccttat gatttagtac 3960
gacttgtaaa aggtgatgaa gatcgtttcc tatattttag taccettgat attaattcgg 4020
atcgtcttaa tttcaaagat gtaaacaagc catcaaagca ggctgaaaat cgttatagcc 4080
ttaaaacaat tgagaattta gaaaaatatg aggttggtgt tttaggtgat ttaaggtttg 4140
tgagacaaga aatacgtaaa aatttttaat agataaaaat tagcttaatt ttaatacggt 4200
aagcaatatt agcttgttca ccgtattata aaaaacaaaa aagcctgtaa gcgttgatga 4260
tataggcttt tttgaatacg tatcataata cagtaatggc ttaaatggaa ctatcacgtc 4320
cgaacaaact acgtcatcaa tacctttatc ataatacagc aatagcttta atggagctat 4380
gacaagtttg cattccgacc taaaacagat ggaatcataa tacagcaatg gcttaaatgg 4440
aactatgact caaaacctca aacttcgatt taccacatgn natcataata cagcaatggc 4500
aataatggaa ctatgacata tgtttatcac aacattcgtg acaatgaatc ataatacagc 4560
aatggcttaa aaggaactat gacgacatgg atattcttcc gacagattta aaatcataat 4620
acagcaacgg cttaaaagga actatgacac ctctggcttt gtttctggct taacttctat 4680
cataatacag taatggctta aatggaacta tgaccgaggt tactagctta ggtgcattct 4740
acttatcata aaacagcaat ggctttagtg gaactatgac tctagactat tatctttatg 4800
tttcacgcaa atcataaaac agcaatggca ttaatggaac tatgaccctt gatgtcaacc 4860
tgttttctac gctcgatcat aaaacagcaa cggcttaaat ggaactatga caacacagaa 4920
gtttataaaa agaaataata atcataaaac agcaatggct taaatggaac tatgaccttc 4980
aggtgcatgt gcatctttta ttaggtatca taatacagca atggcaataa tggaactatg 5040
acgcaaatga tgattacacc gttgaaaaaa tatcataaaa cagcaatggc tttaatggaa 5100
ctatgactgc atgaggaatt gttgttctgt cttctttatc ataaaacagc aatggtaaaa 5160
atggaactat gactaatgca gacttaagtc agaataagcg tagatcataa tacagctgaa 5220
aaatacaata ataagttcat tatagcgcta tgctactttt tccccgggtt tatttttatc 5280
atttgaattt gaaatatatc tctgtcctct attcccagga cacgcatttc ttcttttaaa 5340
gatcgccagg gttcaatctt aatagattta gtagttttat ctagcagagt atactcgtgt 5400
acgtaatatg ttttttcgtt tttgctcatt gtaactcctc tgttcttctt aaaataattg 5460
aaaatctgta ggtatataaa aattacatat taagtgctgg tttttaatta gtgtttgttg 5520
gagattgctt atagtaattg taatatcttt ttaaggataa gtgagactta aagccgattt 5580
gctgcataat ttgttcaggg ttatttttct gtttaatttt acttaaaata 5630

<210> 3
<211> 6416
<212> DNA
<213> Artificial sequence

<220>
<223> CRISPR-Cas Locus 3 (Bacillus species)

<400> 3
ccttaaaaag atacgatgcg tactacgcag acactaacca gaacaagggt taaaaaacta 60
cacttgttat ataaatttta tatacctaca ggtttttgag gtaagttgga gttgttaaga 120
tgaaaaaaaa caaggagtca tattttatac acgaatatac cttgcgagac aagactacta 180
aatcaataaa aattgaatca tggcgttcat taaaggaaga aatgatagcc ctgggcataa 240
aagacagtga catctttcaa attcaaatgc taaaaataga ttcaaaaaaa gagtaacata 300
gcgctatgcc atgttactct tttttatttt aataatataa agtcgttttt ctacgctcaa 360
aagcaactag ttccgcaaac aaaaatataa attactattt caaaaaacaa gagaacttaa 420
ggattaaact gacgagaaat cctctttttt ataagtgaaa tccttatcta aggctatatt 480
agcaaggttt ttgagacatc catcatagca tagcaatttt tcttatggaa ctatagccgt 540
ttttacagca tcttcatctt tttctaaatc atagcattgc gatttttttt tggaactatt 600
gccccttgac cttttctttg ccttgatctt tatcatagca tagcaatttt tcttatggat 660
ctatagcata gttgtggatg tggaggaaat ggctactatc ataacattgc aatatttctt 720
ttggaactat agccatatcg ccagtatcaa cattcacttc tgatcataac atagtaatat 780
ttcttatgga tctatagcct cgttattctt gattaaatat atacgaagat catagcatag 840
cgattttcct tatggaacta tagcaactgg cggggctact agaaaggaga tgttgatcat 900

agcatagcga tttcccttat ggaattatag caccgttact aattgttcaa cacttaaatt 960
aaccatagca ttgcaatatt tcttatggaa ctatagccca atttcgcatg gatgtacttt 1020
ttagtatcat agcgtagcga tgtttcctat ggaactatag caaggttact gatgctaagg 1080
atgtagtgac tatcatagca atttttctta tgaaactata gcagcctgtg tgctagacag 1140
tacctataat acaccatatc atagccttat ttcttatgga actatagcag aatccccgtg 1200
ttagcctatc caggctatat catagcattg cgatgtttct tagagaacta taatattttt 1260
ctatttattt gtggtgaaaa aaattatagc atagtgatat tctctacgga atgatagcat 1320
taatattaac aagttaagat atgttcctct taattaattc atatcactat tattcccaat 1380
ctcattattt ttataattat tcgtttatct aggtataaag tttattttac gggtcaataa 1440
tgaaaccata aggatcatat catagcgatg tttcttaagt tactatagta agggcaatga 1500
cccgtggcgt ttggggatcg ccttatcctg gtatggatat tctccccatg tgaaaagcac 1560
ctaagcatag cgctatggtg cttttatttt gtaaaacatg aaggaggtta tgaaattgaa 1620
atatgtatta gggttggata ttggaatcgc atcatgtggt tgggcagtaa taaatcaaga 1680
aaaacataga attgaagatt taggggttcg tatttttgat aaagctgaga atcccaaaga 1740
tggaaaatct ttagctacac ctagacgtga cgcacgctct accagaagaa ccctacgccg 1800
taaaaaacat agaatgcaac ggataaagat acttttagta aagcacggct tactatccaa 1860
gaccgaaatc gaccatctat atgaaagtgc tactgagatc gatgtttggt acttacgttt 1920
aaatgctcta gagcgcagat taaatccaaa agagtttgct agagttctta ttcaccttgc 1980
taaaagaaga ggatttaaat ctaatagaaa agaaacaact ctctcggaaa acggacaaat 2040
cttagaaaat atttcagaga acctgcagat tatggagcaa aaaaattata gaactgttgg 2100
agaaatgatt ctaaaagata aaagatttga aaatcataaa agaaacaaag atggtactta 2160
tatcgggact gttactcggc aacaacttca agaagaaata caaatgatat tcaatgcaca 2220
acgtttgtac aaaaacaatc atgcaacaga gaaatttgaa agtagctact tagaaatctg 2280
ggcatctcaa cgtccctatg cttctaaaga tcaaattgaa aaaatgattg gttactgtac 2340
cttagagcca aaagaaaaaa gagtgcctaa agcttcttgg tcatttcaat attttgtagc 2400
gctacaaacc ataaacaacc tccgactgat cagtaaagac aggatagaag aactctcttt 2460
tgaagaaaag aaacgaatta tgaatctagc actcgaaaaa tcaattgtta aatacattga 2520
tatccgaaaa ttactatctg ttcctaatga atttcaattt aatagccttt catattcagc 2580
tgatatagta gatacaacgg tggaaaataa aaaatgtata gaactcaaag aatatcactc 2640
tattaacaag gtatataaac aaatttacgg acagaacgct ctaaatttat tacctaaaga 2700
ttacgataca attgcatacg gacttactat ctttaaagat gataaagata tcctagaata 2760
tttacaaaat aaatacgtaa atgcaaaagg aaaacctata agtaatttag ctaacaagac 2820
ctacgatgat acatttattc aggtattatt gactctgaat ttctcaaaaa tgggacattt 2880
atcatttaaa gctttaaaaa atattattcc ttttttagaa gaaggtctat cctacgataa 2940
agcatgtgag aaggcaggat acaactttaa gggagcatcc tacacaaaaa aaacaaagta 3000
cctacctgtt atccctcaga atacaaatcc tgttgtacat cgtgctcttt cacaaacaag 3060
aaaagttata aatgccatta taaaaaaata tggttctcca aacgcaatcc atattgaaac 3120
tgcaagagaa ttatcgaaaa cattccaaga aagaaaagaa atagatagta tgtaccagga 3180
taactctaaa aagaatgaac acgccatttg taagttaaaa gaattgggga atattaaacc 3240
ctcaggaata aatattgtga aatttaagct ttggaacgag caagatggta gatgtatgta 3300
ttctggaaaa tatattgaac cacatcgctt atttgaagaa ggttatacgg aagtagatca 3360
tatactccct tacagccgta gcttagatga cagctataac aataaagtat taacgctagg 3420
aacagaaaat cagcgtaaag gaaacaaaac tccttatgaa tacatgagga ataattcaat 3480
ttgggatgaa tttgaaacaa gagtacagtc aaataaaaaa atcaacaaaa agaaacaaca 3540
aaaattactt ttacaaaact tttcacatgc gagagaacaa gaatttattg aacgcaatct 3600
caatgatacc cgttatgcca caatatatct atcatccctt attcaacaac acctcatatt 3660
ctctgaaagc tctcgtgaga aaaaagttca tacagttagt ggaattataa catctcattt 3720
gcgtagtaga tggggattta acaaagatcg gaaagagggg catatccatc atgctttaga 3780
tgcggtaatc gttgctgtca catccgatca catgattcaa agagtaacaa aatattataa 3840
gttaaaagag ttaaatagaa atattcaagc caaacggatg cagtttcctg aaccttggga 3900
aggatttcgt ttagagttag aagcaagagt ttctccaaat acccagcaat atttaaaggg 3960
tattcgtttt aaaaattacg ccgatgtcga tctatccgaa atcaaaccta tctttgtttc 4020
aagaatgcct aagcgctcta ttactggtga attacatcaa gaaacaatcc ggaaattcct 4080
tggttataac gaaaagggga aagtgctaac cgcgacaaaa acaaaattag aagatattcc 4140
ttttgatgct aatggtgatt ttcccatgtt tggaaaagaa actgatttat atacgtataa 4200
tgccataaaa gaacgttatt taagtcataa aaaagataaa cgcaaatcat tccaagagcc 4260
tttatacaaa cccacaaaat caggaggaat tggaccactt ataaaatcta ttaaaataat 4320
ggatacaaga accattgtta atcctgttaa tcaaggtaaa ggtgtcgtgt ataatagtaa 4380
aattgctcgt actgatgtat tcaaaaaggg tgagaagtac tatttaattc ccatttacac 4440
aattgatctt atgaaaaata tcttacctca aaaagcaatt acagcaggaa aaggatatga 4500
ggattggaca actattgatc ctagctttac ttttttgttt agtctttttc ccaatgatct 4560
aatccaaatt gtcccctcaa aaaacaaaac aattaaagct agaacaactg tatcgaaaaa 4620
agaggtatta ctaccctcac tcacaggata ttttaaaggt gttcatagtg gaactgctgg 4680

gattactgtt gaaacacatg atggcagtat tatagcaaat gtaggctcca agcagttact 4740
atcatttgag aaataccagg tagatgtact tggacattac acaaaaatta agaaggaaaa 4800
acgtattgga atggcaatat agaatggtaa caaaaaacgc agcttgctaa tatagaattt 4860
tgaataggga aacatatgca aacaaagaag atattatctc tgcttaactt actttatttt 4920
aaaaagaaaa aaatcgcatt ctaaacaaat gaaacctctc ctttttcaaa ttaaaaaacc 4980
ttgtctaatc ctctagtgac aaggtttttc agcatctacc agaaaataac gatttttctt 5040
atggaactat agcggtggtg attatggcca aaggtaaggt acaaccatag catagcgatt 5100
tttctcatgg aactttagct tggaaaactc catactggca aaaacattac catagcatgg 5160
cgatttttct tatggaacta tagcaggaga ctcggttttc tgactaggag tcagaccata 5220
gcatagcgat gtttcttacg gaactatagc ctcggtgtat atcgaaggac aaaggaatag 5280
accataccat agcgatgttt cttatggaac tatagcaaca cgttttgaaa cacaatcttg 5340
atcatatcat agcaacgttt tttatggagc tacagctggc ttttgtggtc tgtggtctct 5400
ttttcaccat agcatagcga tgtttcttat gaaactatag cgttaaatta ccatttggtc 5460
caatggattt aatcatacca tagcgacatt tcttatggaa ctatagccgg tttcaactcc 5520
ttttaaggca tttctcaacc atagcatagc gatgtttctt atggaactat agcgttttgg 5580
atcaggtgca tcaggaagag cataccatag catagcgata tttcttatgg aactatagcc 5640
tcttaacgaa tccagtagca gcagaacata ccatagcata gcgatatttc tcatggaact 5700
atagcccaat ccatcatgca tgggtggtag ttgaaccata gcatagcgat gtttcttatg 5760
gaactatagc gttaatacat actcttaatc cttataattc accatagcat agcgatgctt 5820
cttatggaac tacagctcgc cgcccataaa caagcttatt tttctatcat tttatattaa 5880
tattatatat tacaatgcct tatatatatt tttaacaaaa atatatatcc caaacctaaa 5940
agactaaata taacttataa atttacttat caaatagcct ccagcaagga aattgtacac 6000
ttactcttac tttaaaatca gtaagtttta ttagtacatt tacataaata atcccttcgc 6060
ctatacacaa aagaattatg ttataataac tactgaacat atgaatagga taacaactac 6120
ctgctgttgg caagtagaaa tttatctaat aaaaagatca cctgtttcca agacaagtga 6180
tctttttgta tacatttcct gtactttttt gagattccat ttgccaatac tacccataaa 6240
actttataaa aagtgttctc ctgccactaa aaacatgatt aaccaaaaac acactatcat 6300
aagacataca gtcctacatt tccctacaaa ggactcaact ttttctacta tatatttcat 6360
gttagttcat ttccttttcc tcttaattag catattcaac ccaacatttt tcttta 6416

<210> 4
<211> 5667
<212> DNA
<213> Artificial sequence

<220>
<223> CRISPR-Cas Locus 4 (Bacillus species)

<400> 4
taatgaatta aatgaataca gaggtttaat gatgaaaaaa aatgaggaat catattatat 60
caatcaatac actttacgag atggtactac taaatctatt aagattgaat cttggcgctc 120
actaaaagat gaaatgatag ccctgagcat aaaagacagt gacatctttc aaattcaaat 180
gctaaaaata gattcaaaaa aagagtaaca tagcgctatg ccatgttact cttttttatt 240
ttaataatat aaagtcgttt ttctacgctc aaaagcaact agctccgcaa acaaaaatat 300
aaattactat ttcaaaaaac aagagaactt aaggattaaa ctgacgagaa atcctctttt 360
ttataagtga aatccttatc taaggctata ttagcaaggt ttttgagaca tccatcatag 420
catagcaata tttcttatgg aactatagca tagttgtgga tgtggaggaa atggctacta 480
tcatagcata gcaatatttc ttttggaact atagccatat cgccagtatc aacattcgct 540
tctgatcata acatagtaat atttcttatg gatctatagc ctcgttattc ttgattaaat 600
atatacgaag atcatatcat agcgattttc cttatggaat tatagcaccg ttactaattg 660
ttcaacgctt aaattaacca tagcattgca atatttctta tggatctata gcccaatttc 720
gcatggatgt actttttagt atcatagcgt agcgatgttt cctatggaac tatagcaagg 780
ttactgatgc taaggatgta gtgactatca tagcaatttt tcttatgaaa ctatagcagc 840
ctgtgtgcta gacagtacct ataatacacc atatcatagc cttatttctt atggaactat 900
agcagaatcc ctgtgttagc ctatccgggc tatatcatag cattgcgatg tttcttaggg 960
aactatagca tttccgctaa cgttttgttg tctctccata tcatatcata gcgatgtttc 1020
ttagagaact atagtatttt tctgtttatt tgtggtgaaa aaaattatag catagggata 1080
ttctcaacgg aatgatagca ttaatattaa caagttaaga aatgttcctt ttaattaatt 1140
catatcacta ttatttccaa tctctttatt tttataatta tacgtgtatc taagtataaa 1200
gtttattttc tgggtcaata atgaaatcat aaggatcata tcatagcgat gtttcttaag 1260
ttactatagt aagggcaatg acccgtggcg tttggagatc gcctcatcca ttacggatat 1320
tctccccatg tgaaaagcac ctaagcataa ggctaaggtg cttttatttt gaaaaacgtg 1380
aaggaggtcg tgtaattgca atatgtatta ggattggata ttggaatcgc atcttgtggt 1440

tgggctgtaa tcaatcaaga aaaagagaga attgaagatt taggggttcg tatttttgat 1500
aaagctgaaa atcccaaaga cggaaaatct ttagctgcac ctaggcgtga cgcacgctct 1560
actaggagaa ccctacgccg taaaaaacat agaatgctac ggataaagat acttttagta 1620
aaacaaggct tactatctaa gaacgaactt aaccatctat atgaaagtac tactgaagtc 1680
gatgtttgga acttacgttt agatgctcta gaacgcaaat tgaatccaaa agagtttacc 1740
agagttctta ttcatcttgc taaaagaaga ggatttaaat ctaatagaaa agaaaccact 1800
ctctcagaaa acggacaaat cttagaaagc atttcagaga atcaacagat tatggagcaa 1860
caaaattata gaactgttgg agaaatgatt ctaaaagata aaaaatttga aaaccataaa 1920
agaaacaaag atggtactta tatcggaccc gttacccgtc aacaacttca agaagaaata 1980
caaatgatat tcactgcaca acgtttgtac aaaaacgatt atgcaacaga ggaatttgaa 2040
attagctact tagaaatctg ggcatctcaa cgtccctatg cttctaaaga tcaaattgaa 2100
aaaatgattg gttactgtac cttagagcca aaagaaaaaa gagtgcctaa agcttcttgg 2160
tcatttcaat attttgtagc gctacaaacc ataaacaacc tccgactgat cagtaaagac 2220
aggatagaag aactctcttt caaagaaaag aaacaaatta tgaatctagc acttgaaaaa 2280
tcaattgtta aatacattga tatccgaaaa ttattatcca ttcctaatga atttcagttt 2340
aatagcctat catattcagc tgatacagta gatactacgt tggagaataa aagatgtata 2400
gaactcaagg aatattactc tattaacaag gtatataaac aaatttatga gaagaacgcc 2460
ctaaatttat tacctataga ttacgataca attgcatacg gactaactat ctttaaagat 2520
gataaagata tccttgaata tttacaaaat aaatatgtaa atgcaaaagg aaaacctata 2580
aataatttag ctaaaaagac ctacgattac acatttattc aagcattact gactctgaat 2640
ttcacgaaaa tgggacattt atcatttaaa gctttaaaaa atattattcc tttcttagaa 2700
gaagggctat catacgataa agcatgtgag aaggcaggat acaactttaa aggaacatcc 2760
tacacagaaa aaacaaagta cctacctgtt atccctcaaa atacaaatcc tgttgtacat 2820
cgtgctcttt cacaaacaag aaaagttata aatgccatta taaaaaaata tggttctcca 2880
aacgcaatcc atattgaaac tgcaagagaa ttatcgaaaa cattccaaga aagaaaagaa 2940
atagatagta tgtaccagga taactctaaa aagaatgaac acgccattca taagttaaaa 3000
gaattgggga acattaaccc ctcaggaata aatattgtga aatttaagct ttggaatgag 3060
caagatggta gatgtatgta ttctggaaaa tatatcgaac cacatcggtt atttgaagaa 3120
ggttatacgg aagtagatca catactcccc tatagccgta gcttagatga tagctataac 3180
aacaaaacat taacactagg aacagaaaat cagcgtaaag gaaacaaaac tccttatgaa 3240
tacatgggga atacttcaat ttggaatgac tttgaaacaa gggtacattc aaataaaaaa 3300
atcaacaaaa agaaacaaca aaaattactt ttacaacact tttcatatgc gagagaacaa 3360
gaatttattg aacgcaatct gaatgatacc cgttatgcca caatatatct atcatccctt 3420
attcaacaac acctcatatt ctctgaaagc tctcgtaaga aaaaagttca tacagttagt 3480
ggaattataa catctcattt acgtagtaga tggggattta acaaagatcg gaaagaggga 3540
catattcatc atgctttaga tgcggtaatc gtcgctgtca catccgatca catgattcaa 3600
agagtaacaa aatattataa gttaaaagag ttaaatagaa accttcaagc caaacggatg 3660
ccgtttcctg aaccttggga aggatttcgt ttagagttag aagcaaggat ttctccaaat 3720
acccagcaat atttaaaggg atttcgtttt aaaaattacg ccgatgtcaa cctatccgaa 3780
atcaaaccta tctttgtttc aagaatgcct aagcgctcta ttactggtga attacatcaa 3840
gaaacgattc ggaaattcat tggttataac gaaaaaggga aagtattaac cgcaataaaa 3900
acaaaattag aagatatccc ttttgatgcc aatggtgatt ttcccatgta tgggaaagaa 3960
actgatttat atacgtataa tgccataaaa gaacgttttt taagtcataa aaaagataaa 4020
cgcaaatcat tccaagaacc tttatacaaa cccacaaaat caggaggaat tggaccactt 4080
ataaaatcta ttaaaataat tgatacaaga accattgtta atcctgttaa tcaaggtaaa 4140
ggtgtcgtgt ataatagtaa aattgctcgt actgatgtat tcaaaaagga tgagaagtac 4200
tatttaattc ccatttacac aattgatctt atgaaaaata tcttacctca aaaagcaatt 4260
acagcaggaa aaggatatga ggattggaca accattgatc ctagctttac ttttttattt 4320
agtctttttc ccaatgatct aattcaaatt gtcccctcaa aaaataaaac aataaaagct 4380
agaacaactg tatcgaaaaa agaggtatta ctaccctcac tcacaggata ttttaaaggt 4440
gttcatagtg gaactgcggg gattactgtt gaaacacacg atggcagtgt tatagcaaat 4500
gtaggctcaa agcaattact attaattgag aaatatcaag tagatatact tggacattat 4560
acaaaaataa aggaggaaaa acgtattggg atggcgatat agaatggtaa caaaaagagc 4620
gcagcttgct aatatggaat tttcaatagg gaaacatacg caaacaaaga agatactagc 4680
tctgctcaac ttactttatt ttaaaaagaa aaaaatcgca ttctaaacaa atgaaatccc 4740
tcctttttca aattaaaaaa ccttgtctaa tcctctagtg acaaggtttt ttcgacatct 4800
accctatcat agcgttggtt cttttggaac tatagctata ctaaggaata tcaagaacat 4860
ttagcgatca tatcatagcg ttggttcttt tcgaactata gcgcgttcaa ttgctgtttg 4920
acaataccta aaaccacatt atagcggttt ttcttatgga actatagctc tctgttgcga 4980
ttccattaac gttcttagat catagcatag cgacttttct tttggaacca tagcctctca 5040
tcaatataac gttgctgttt catccatcat atcatagtgt tggttccttt cgaactatag 5100
cgcactccat gacgggacac catcaacgta aaccatatca tagcgatggt tcttatggga 5160
ctatagccac aacacactta tcaatattaa ttcctatacc atatcatagc gatggttctt 5220

ttcgaactat agccacgttt tgtgtctggt aatatcgtac cgtaccatat catagtgata 5280
gttcttagcg taatttccgt aaaccatatc ttagcgttgg ttctttcggg actatagacg 5340
tttaaggatt gacacaaaat attcctaaaa acacattacc ttcaaaataa aattggatta 5400
ttttatatat agttttaacc aataaaatat gttccaaaca tagctactct tacatatcat 5460
tgtttttagc atttccttta ctaacctgaa aaatattgta attaaccatt acaaatgcct 5520
tgtatagcgc tatgacaatt taatacatgc aaatgaatga caagtcacac ttcccatatt 5580
cacatataca taacgaaatg tttcaaatca agactaatat agtgttacac aattgttgtt 5640
cttcaaaaaa tattttacac atttaca 5667

<210> 5
<211> 6120
<212> DNA
<213> Artificial sequence

<220>
<223> CRISPR-Cas Locus 5 (unknown origin)

<400> 5
gtaaagcaag taggatttaa aactcacgta tccttaaaaa gatatgatac gtactatgca 60
gacactaacc agaacaaggg ttaaaaaact acacttgtta tataaatttt atatacctac 120
aggtttttga ggtaagttgg agttgttaag atgaagaaaa acaaggattc atatttcata 180
cacgaatata ccttgcgaga caagactact aaatcaataa aaattgaatc gtggcgttca 240
ttgaaggaag aaatgatagc cctgggcata aaagacagtg acatctttca aattcaaatg 300
ctaaaaatag attcaaaaaa agagtaacat tagcgctatg ccatgttact ctttttattt 360
ttaataatat aaagtcgttt ttctacgctc aaaagcaact agttccgcaa acaaaaatat 420
aaattactat ttcaaaaaac aagagaactt aaggattaaa ctgacgagaa atcctctttt 480
ttataagtga aatccttatc taaggctata ttagcaaggt ttttgagaca tccatcatag 540
catagcaatt tttcttatgg aactatagct gtttttacag catcttcatc tttttctaaa 600
tcatagcatt gcgatttttt ttttggatct atagcatagt tgtggatgtg gaggaaatgg 660
ctactatcat agcattgcaa tatttctttt ggaactatag ccatatcgcc agtatcaaca 720
ttcgcttctg accataacat agtaatattt cttatggatc tatagcctcg ttattcttga 780
ttaaatatat acgaagatca tagcatagcg atttccttta tggaactata gcaactggcg 840
gggctactag aaaggagatg ttgatcatag catagcgaat gctcctatgg aactatagcc 900
tactaaacac aaaccctatt ccatcataga tcatatcata gcgatttttc ttagggaact 960
atagcccaaa ttccaaaaca aaataaaata gttgcatcat atcatagcga tgtttccttt 1020
ggaactatag cgacttggga tgattcgtct tatccttgga atcatagcat agcaatattt 1080
cttatggaac tatatcctct tgcgcaaggt ctttaaattc atcatcatag cataacgatt 1140
tttcttttgg aactatagtc tgttatgtgt ccctctaact ctaaaatcca tcatagcata 1200
gcgatattct ctacggaatg atagcattaa tattaacaag ttaagatatg tgcctcttaa 1260
ttaattcata tcactattat tcccaatctc attattttta taattattcg tttatctagg 1320
tataaagttt attttacggg tcaataatga aaccataagg atcatatcat agcgatgttt 1380
cttaagttac tatagtaagg gcaatgaccc gtggcgtttg gggatcgcct tatcctggta 1440
tggatattct ccccatgtga aaagcaccta agcatagcgc tatggtgctt ttattttgta 1500
aaacatgaag gaggttatga aattgaaata tgtattaggg ttggatattg gaattgcatc 1560
atgtggttgg gcagtaataa atcaagaaaa acatagaatt gaagatttag gggttcgtat 1620
ttttgataaa gctgagaatc ccaaagatgg aaaatcttta gctacaccta gacgtgacgc 1680
acgttctacc agaagaaccc tacgccgtaa aaaacataga atgcaacgga taaagatact 1740
tttagtaaag cacggcttac tatccaagac cgaaatcgac catctatatg aaagtgctac 1800
tgagatcgat gtttggtact tacgtttaaa tgctctagag cgcagattaa atccaaaaga 1860
gtttgctaga gttcttattc accttgctaa aagaagagga tttaaatcta atagaaaaga 1920
aacaactctc tcggaaaacg gaaaaatctt agaaaatatt tcagagaacc tgcagattat 1980
ggagcaaaaa aattatagaa ctgttggaga aatgattcta aaagataaaa aatttgaaaa 2040
tcataaaaga aacaaagatg gtacttatat cgggactgtt actcggcaac aacttcaaga 2100
agaaatacaa atgatattca atgcacaacg tttgtacaaa aacaatcatg caacagagaa 2160
atttgaaagt agctacttag aaatctgggc atctcaacgt ccctatgctt ctaaagatca 2220
aattgaaaaa atgattggtt actgtacctt agagccaaaa gaaaaaagag tgcctaaagc 2280
ttcttggtca tttcaatatt ttgtagcgct acaaaccata aacaacctcc gactgatcag 2340
taaagacagg atagaagaac tctcttttga agaaaagaaa cgaattatga atctagcact 2400
cgaaaaatca attgttaaat acattgatat ccgaaaatta ctatctgttc ctaatgaatt 2460
tcaatttaat agcctttcat attcagctga tatagtagat acaacggtgg aaaataaaaa 2520
atgtatagaa ctcaaagaat atcactctat taacaaggta tataaacaaa tttacgaaca 2580
gaacgctcta aatttattac ctaaagatta cgatacaatt gcatacggac ttactatctt 2640
taaagatgat aaagatatcc tagaatattt acaaaataaa tacgtaaatg caaaaggaaa 2700

acctataagt aattttgcta acaagaccta cgatgataca tttattcagg tattattgac 2760
tctgaatttc tcaaaaatgg gacatttatc atttaaagct ttaaaaaata ttattccttt 2820
tttagaagaa ggtctatcct acgataaagc atgtgagaag gcaggataca actttaaggg 2880
aacatcctac acaaaaaaaa caaagtacct acctgttatc cctcagaata caaatcctgt 2940
tgtacatcgt gctctttcac aaacaagaaa agttataaat gccattataa aaaaatatgg 3000
ttctccaaac gcaatccata ttgaaactgc aagagaatta tcgaaaacat ttcaagaaag 3060
aaaagaaata gatagtatgt accaggataa ctctaaaaag aatgaacacg ccattcataa 3120
gttaaaagaa ttggggaaca ttaacccctc aggaataaat attgtgaaat ttaagctttg 3180
gaatgagcaa gatagtagat gtatgtattc tggaaaatat atcgaaccac atcgattatt 3240
tgaagaaggt tatacggaag tagatcatat actcccctat agccggagct tagatgacag 3300
ctataacaat aaagtgttaa cgctagggac agaaaatcag ggtaaaggaa acagaactcc 3360
ttatgaatac atggggaata cttcaatttg ggatgaattt gaaacaagag tacagtcaaa 3420
taaaaaaatc aacaaaaaga aacaacaaaa attactttta caaaactttt cacatgcgag 3480
agaacaagaa tttattgaac gcaatctcaa tgatacccgt tatgccacaa tatatatatc 3540
atcccttatt caacaacacc tcatattctc tgaaagctct cgtgagaaaa aagttcatac 3600
agttagtgga attataacat ctcatttgcg tagtagatgg ggatttaaca aagatcggaa 3660
agaggggcat atccatcatg ctttagatgc ggtaatcgtt gctgtcacat ccgatcacat 3720
gattcaaaga gtaacaaaat attataagtt aaaagaatta aatagaaacc gtcaagccaa 3780
acggatgcag tttcctgaac cttgggaagg atttcgttta gagttagaag caaggatttc 3840
tccaaatacc cagcaatact taaagggaat tcgtttaaaa aattacgccg atgtcaatct 3900
atctgaaatc aaacctatct ttgtttcaag aatgcctaag cgctctatta ctggtgaatt 3960
acatcaagaa acaatccgga aattccttgg ttataacgaa aaagggaaag tgctaaccgc 4020
aataaaaaca aaattagaag atattccttt tgatgctaat ggtgattttc ccatgtatgg 4080
aaaagaaact gatttatata cgtataatgc cataaaagaa cgttatttaa gtcataaaaa 4140
agataaacgc aaatcattcc aagaaccttt atacaaaccc acaaaatcag gaggaattgg 4200
accacttata aaatctatta aaataatgga tacaagaacc attgttaatc ctgttaatca 4260
aggtaaaggt gtcgtgtata atagtaaaat tgctcgcact gatgtattca aaaaggctga 4320
aaagtactat ttaattccca tttacacaat tgatcttatg aaaaatatct tacctcaaaa 4380
agcaattaca gcaggaaaag gatatgagga ttggacaacc attgatccta gctttacttt 4440
tttgtttagt ctttttccca atgatctaat ccaaattgtc ccctcaaaaa ataaaataat 4500
taaagctaga acaactgtat cgaaaaaaga ggtattacta ccctcactca caggatattt 4560
taaaggtgtt catagtggaa ctgctgggat tactgttgaa acacacgatg gcagtattat 4620
agcaaatgta ggctccaagc agttactatc atttgagaaa taccaggtag atgtacttgg 4680
acattacaca aaaattaaga aggaaaaacg tattggaatg gcaatataga atggtaacaa 4740
aaaacgcagc ttgctaatat agaattttga atagggaaac atatgcaaac aaagaagata 4800
ttatctctgc ttaacttact ttattttaaa aagaaaaaaa tcgcattcta aacaaatgaa 4860
acctctcctt tttcaaatta aaaaaccttg tctaatcctc tagtgacaag gtttttcagc 4920
atctaccaga aaataacgat gtttcttatg gaactatagc ggtggtgatt atggccaaag 4980
gtaaggtaca accatagcat agcgattttt ctcatggaac tttagcttgg aaaactccat 5040
actggcaaaa acattaccat agcatggcga tgtttcttat ggaactatag caggagactc 5100
ggttttctga ctaggagtca gaccatagca tagcgatgtt tcttacggaa ctatagcctc 5160
ggtgtatatc gaaggacaaa ggaatagacc ataccatagc gatgcttctt atggaactat 5220
agccggtttc aactcctttt aaggcatttc tcaaccatag catagcgatg tttcttatgg 5280
aactatagcg ttttggatca ggtgcatcag gaagagcata ccatagcata gcgatatttc 5340
ttatggaact atagcctctt aacgaatcca gtagcagcag aacataccat agcatagcga 5400
tatttctcat ggaactatag cccaatccat catgcatggg tggtagttga accatagcat 5460
agcgatgttt cttatggaac tatagcgtta atacatactc ttaatcctta taattcacca 5520
tagcatagcg atgcttctta tggaactaca gctcgccgcc cataaacaag cttatttttc 5580
tatcatttta tattaatatt atatattaca atgccttata tatattttta acaaaaatat 5640
atatcccaaa cctaaaagac taaacataac ttataaattt acttatcaaa tagcctccag 5700
caaggaaatt gtacacttac tcttacttta aaatcagtaa gttttattag tacatttaca 5760
taaaaaatcc cttcgcctat acacaaaaga tttatgttat aataactact gaacatatga 5820
ataggataac aactacctgc tgttggcaag tagaaattta tctaataaaa agatcacctg 5880
tttccaagac aagtgatctt tttgtataca tttcctgtac ttttttgaga ttccatttgc 5940
caatactacc cataaaactt tataaaaagt gttctcctgc cactaaaaac atgattaacc 6000
aaaaacacac tatcataaga catacagtcc tacatttccc tacaaaggac tcaacttttt 6060
ctactatata tttcatgtta gttcatttcc ttttcctctt aattagcata ttcaacccaa 6120

<210> 6
<211> 3213
<212> DNA
<213> Artificial sequence

<220>
<223>  Cas endonuclease of Locus 1 (Cas-Locus 1)-Bacillus species

<400>  6
atgagagagt tggattatcg cataggatta gatattggaa cgaattctat tggctggggg      60
atcattgaat tatcttggaa caaagataga gaacaatatg agaaagcaag aattgtcgac     120
aagggtgttc gtatgtttga taaggctgaa atacctaaga ctggtgcttc tcttgctgaa     180
ccgaggcgtt tagcgcgctc atcacgtaga agattaaatc gcaaaagcca gagaaaaaaa     240
gatatacgta atttacttgt tcaacatgaa attattagtc aaagggaatt ggcttcgtta     300
tatcccctgt caaaaagttc aatggatatt tgggatattc gattggaggg attagatcgc     360
ttgttagacc attttgaatg gactcgatta ttaattcatt tagcgcaaag gcgcggtttt     420
aaatcaaatc ggaagtctga attgaaagat gtggagacag gaaaagtatt atcaagtatt     480
caagcaaatg aaaaacgatt atcactgtac cgtacagtgg gagagatgtg gatgaagaat     540
gaagatttta gtaaatatga caaaaggcgt aattctccta atgagtatgt attttcggtt     600
agccgtgcag atttagaaaa agaaattgtg actctatttg aagcgcaaag aaaatttcag     660
tcatcatatg catcggttga tttacaaaaa acatacttac aaatttgggc acaccaactt     720
ccttttgctt ctgggaatgc aattgtaaat aaagtgggat attgttcatt attaaaaggt     780
aaagaaaaga gagttccgaa agcaacatat acttttcaat atttcagtac actagatcaa     840
ataaatcgaa cacgattagg tcctaatttc caaccattta cgaaggaaca gagagacatt     900
attttagata aaatgtttaa tcgaacagat tattataaaa aaaagacaat acccgaagtt     960
acttactatg atattcggaa atggttagca ttagatgaaa caattcaatt taaaggactt    1020
acctatgacc caaatgaaga gctgaaaaaa atagaattga aatcctttat taatttaaag    1080
ccattttatg aaattaaaca ggtagttact aattacgcca agaaaataaa tgaggcattc    1140
tcaacattag actatgatac atttgcatat gctttaacag tttataaaac ggacaaagat    1200
attagatctt atttaaagaa atctaataat ttatcaaaat gttgctacga tgatcaatta    1260
atagaagagc tattaactct ctcctataca aagtttggtc atttatcatt taaagcaatt    1320
aatcatgtac tgccaattat gcaagaggga aggacttatc aggaagcaat acaccaatta    1380
ggatatgata ccactaatct aaaaaaagaa aacagaagta tgttcttgcc ccttattccc    1440
gatgagataa caaatccaat tgttaaaaga gcactaactc aagcacgtaa agttgtaaat    1500
gctattatta gaagatatgg ttccccaaat tctgttcata ttgaactagc ccgtgagctt    1560
cctaaaaatc atgatgagag aacgaaaata atgaaagctc atgatgaaaa ttataagaaa    1620
aataaaggag ccatatcaat tttgattgag aatggaattt taaatccgac aggatatgat    1680
attgtacgtt ataagttatg gaaagagcaa ggagagcgat gtgtttattc gctaaaacag    1740
attcctgcta atacgttttt taatgaattg aaaaaagagc gaagtggccc cccggttcta    1800
gagatagatc acattttacc gtatagtcag agttttattg atagttatca taataaagta    1860
ctagtttatg aggaggagaa tcaaaaaaag ggaaatcgaa ttccatatac ttatttttta    1920
gaagaaaata aggactggga aagcttcgaa agctacgtac gattgaatag ttttttgtct    1980
aaaaagaagc gcggatattt attgaaaaaa gcttacttgc caagagagag taacatgatt    2040
aaggagcgtc atttaaatga tactcgatat gctagtagct atctgaaaaa cttcattgag    2100
aaaaatttga aatttaaaga agttgaaggt agtacaagaa acaaacatgt acagacggtt    2160
aacggtataa ttacagccca tctgcgaaaa agatggggat tagaaaaaga taggaaggaa    2220
acatatttgc atcatgcaat ggacgctatt attgttgctt gtacagacca tcatatggtt    2280
actaaagtaa cggagtacta tcaaataaaa gaaagtaata agtcaataaa gaaaccatac    2340
tttcctttgc catgggtggg ctttagagag gaaattttat cacatttagc aaggcaacca    2400
attgctagaa aaattagtga ggaacttaaa attggatatc aatcatttga ttatatactt    2460
gtatcgcgaa tgccaaaaag atctgtcact ggatcagctc atgaacagac aatcatgaaa    2520
aaaggtggta tcgacaaaaa aggtaaaact attatcataa agcgtgtgta cttaaaggat    2580
attaagtttg atgagaatgg cgattttaaa atggttggaa aagaacaaga tttagcaact    2640
tatgaagcga taaagcaaag atatatagag tatggaaaag aatcgaagaa agcatttgaa    2700
acacctttat acaagcctag taaaaaagga aaaggaaacc tcattaaaaa gatcaaagta    2760
gaagtgcaaa ccaaatcttt tgttcgagaa gttaatggag gcgtagctca aaatggtgat    2820
ttagtaagag tagatttgtt tgaaaaggat aatagatatt atatggtacc tatttatgta    2880
atggatactg ttcattccga actaccaaat aaagctgtaa caagcagtaa aggctatgag    2940
caatggttaa caatagataa cagctttacg ttcaaattca gtttatatcc ttatgattta    3000
gtacgacttg taaaaggtga tgaagatcgt ttcctatatt ttagtaccct tgatattaat    3060
tcggatcgtc ttaatttcaa agatgtaaac acgccatcaa agcaggctga aaatcgttat    3120
agccttaaaa caattgagaa tttagaaaaa tatgaggttg gtgttttagg tgatttaagg    3180
tttgtgagac aagaaatacg taaaaatttt taa                                 3213

<210>  7
<211>  3213
<212>  DNA
<213>  Artificial sequence

<220>
<223>  Cas endonuclease of Locus 2 (Cas-Locus 2)-Bacillus species

<400>  7
atgagagagt tggattatcg cataggatta gatattggaa cgaattctat tggctggggt      60
gttgttgaat tatcttggaa caaagataga gaacaatatg agaaagcaag aattgtcgac     120
aagggtgttc gtatgtttga taaggctgaa atacctaaaa ctggtgcttc tcttgctgaa     180
ccgaggcgta cagcgcgttc atcacgtaga aggttaaatc gcaaaagcca gagaaaaaaa     240
gatatacgta atttacttgt tcaacatgaa attattagtc aaaaggaatt ggcttcgtta     300
tatcccctgt caaaaagttc aatggatatt tgggatattc gattggatgg gttagatcgc     360
ttgttagacc gtcttgaatg gactcgatta ttaattcatt tagcacaaaa gcgcggtttt     420
aaatcaaatc gaaagtctga actgaaagat gtggagacag gaaaagtatt atcaagtatt     480
caagtaaatg aaaaacgatt atcactgtac cgtacagtgg gagagatgtg gatgaagaat     540
gaagatttta gtaaatatga caaaaggcgt aattctccta atgagtatgt attttcggtt     600
agccgtgcaa atttagaaaa agaaattgtg actctatttg aagcgcaaag aaaatttcag     660
tcatcatatg catcagttga tttacaaaaa acatacttac aaatttgggc acatcaactt     720
ccttttgctt ctgggaatgc aattgtaaat aaagtaggat attgttcatt attaaaaggg     780
aaagaaaaga gagttccgaa agcaacatat acttttcaat attttagtac actagatcaa     840
ataaaccgaa cacgattagg acctaatttc caaccattta cgaaggaaca gagagacata     900
atcttagata aaatgtttca acggacagat tattataaaa aaaagacaat acccgaagtt     960
acttactatg atattcggaa atggttagca ctagatgaaa caattcaatt taaaggactt    1020
acctatgacc caaacgaaga gctgaaaaaa atagaaatga aactctttat aaatttaaag    1080
ccattttatg aaattaaaaa ggtagttact aattacgcaa aaaaaacaaa tgaggtattc    1140
tcaacattag actatgatac agttgcatac gctttaacag tttataaaac ggacaaagat    1200
attatatctt atttgaagag atctaataat ttatcaaaac gttgttacga tgatcaatta    1260
atagaagagc tattaactct ttcctataca aagtttggtc atttatcatt taaagccatt    1320
aatcatgtac tgccaattat gcaagaggga aggacttatc aggaagcaat acaccaatta    1380
ggatatgata ccactaatct aaaaaaagaa aacagaagta tgttcttgcc ccttattcca    1440
gatgagataa caaatccaat tgttaaaaga gcactaactc aagcacgtaa agttgtaaat    1500
gctattatta gaagatatgg ttcccccaat tctgttcata ttgaactagc tcgtgagctt    1560
tctaaaactc atgatgagag aacgaaaata atgaaagctc atgatgaaaa ttataagaaa    1620
aataaaggag ccatatcaat tttgattgag aatggaattt taaatccgac aggatatgat    1680
attgtacgtt ataagttatg gaaagagcaa ggagaacgat gtgcttattc gctaaaacag    1740
attcatgcta atacgttttt taatgaattg aaaaaagagc gaagtggtcc cccagttcta    1800
gagatagatc acattttacc gtatagtcag agttttattg atagttatca taataaagta    1860
ctagtttatg gggatgagaa tcaaaaaaag ggaaatcgaa ttccatatac ttatttttta    1920
gaaggaaata aggactggga aagctttgaa agctacgtac gattgaatag tttattttct    1980
aaaaagaagc gcggatattt attgaaaaaa gcttacttgc caagagagag taacatgatt    2040
aaggagcgtc atttaaatga tactcgatat gctagtagct atttgaaaaa cttcattgag    2100
aaaaatttga aatttaaaga agtcgaaggt agtacacgaa aaaaacatgt acagacggtt    2160
aacggtataa ttacagccca tctgcgaaaa agatggggat tagaaaaaga tagacaggaa    2220
acatatttgc atcatgcaat ggacgctatt atcgttgctt gtacagacca tcatatggtc    2280
actaaagtaa cggagtacta tcaaataaaa gaaagtaata agtcaataaa gaaaccatac    2340
tttcctttgc catgggtggc ctttagagag gaaattttat cacatttagc aaggcagcca    2400
attgctagaa aaattagtga ggaacttaaa attggatatc aatcatttga ttatatactt    2460
gtatcgcgaa tgccaaaaag atctgtcact ggagcagctc atgaacagac aatcatgaaa    2520
aaaggtggta tcgataaaaa aggaaagaca attatcataa agcgtgtgta cttaaaggat    2580
attaagtttg atgagaatgg cgattttaaa atggttggaa aagaacagga tttagcaact    2640
tatgaagcga taaagcaaag atatatagag tatggaaaag aatcgaaaaa agcatttgaa    2700
acacctttat acaagcctag taaaaaagga aaagggaacc tcattaaaaa gatcaaagta    2760
gaagtgcaaa ccaaatcttt tgttcgagaa gttaatggag gcgtagctca aaatggtgat    2820
ttagtaagag tagatttgtt tgaaaaagat aatagatatt atatggtacc tatttatgta    2880
atggatactg ttcattccga actaccaaat aaagctgtaa caagcagtaa aggctatgag    2940
aaatggttaa caatagataa cagctttacg ttcaaattca gtttatatcc ttatgattta    3000
gtacgacttg taaaaggtga tgaagatcgt ttcctatatt ttagtaccct tgatattaat    3060
tcggatcgtc ttaatttcaa agatgtaaac aagccatcaa agcaggctga aaatcgttat    3120
agccttaaaa caattgagaa tttagaaaaa tatgaggttg gtgttttagg tgatttaagg    3180
tttgtgagac aagaaatacg taaaaatttt taa                                 3213

<210>  8
<211>  3207
<212>  DNA

<213>  Artificial sequence

<220>
<223>  Cas endonuclease of Locus 3 (Cas-Locus 3)-Bacillus species

<400>  8
ttgaaatatg tattagggtt ggatattgga atcgcatcat gtggttgggc agtaataaat      60
caagaaaaac atagaattga agatttaggg gttcgtattt ttgataaagc tgagaatccc     120
aaagatggaa aatctttagc tacacctaga cgtgacgcac gctctaccag aagaacccta     180
cgccgtaaaa aacatagaat gcaacggata aagatacttt tagtaaagca cggcttacta     240
tccaagaccg aaatcgacca tctatatgaa agtgctactg agatcgatgt ttggtactta     300
cgtttaaatg ctctagagcg cagattaaat ccaaaagagt ttgctagagt tcttattcac     360
cttgctaaaa gaagaggatt taaatctaat agaaaagaaa caactctctc ggaaaacgga     420
caaatcttag aaaatatttc agagaacctg cagattatgg agcaaaaaaa ttatagaact     480
gttggagaaa tgattctaaa agataaaaga tttgaaaatc ataaaagaaa caaagatggt     540
acttatatcg ggactgttac tcggcaacaa cttcaagaag aaatacaaat gatattcaat     600
gcacaacgtt tgtacaaaaa caatcatgca acagagaaat ttgaaagtag ctacttagaa     660
atctgggcat ctcaacgtcc ctatgcttct aaagatcaaa ttgaaaaaat gattggttac     720
tgtaccttag agccaaaaga aaaaagagtg cctaaagctt cttggtcatt tcaatatttt     780
gtagcgctac aaaccataaa caacctccga ctgatcagta aagacaggat agaagaactc     840
tcttttgaag aaaagaaacg aattatgaat ctagcactcg aaaaatcaat tgttaaatac     900
attgatatcc gaaaattact atctgttcct aatgaatttc aatttaatag cctttcatat     960
tcagctgata tagtagatac aacggtggaa aataaaaaat gtatagaact caaagaatat    1020
cactctatta acaaggtata taaacaaatt tacggacaga acgctctaaa tttattacct    1080
aaagattacg atacaattgc atacggactt actatcttta aagatgataa agatatccta    1140
gaatatttac aaaataaata cgtaaatgca aaaggaaaac ctataagtaa tttagctaac    1200
aagacctacg atgatacatt tattcaggta ttattgactc tgaatttctc aaaaatggga    1260
catttatcat ttaaagcttt aaaaaatatt attccttttt tagaagaagg tctatcctac    1320
gataaagcat gtgagaaggc aggatacaac tttaagggag catcctacac aaaaaaaaca    1380
aagtacctac ctgttatccc tcagaataca aatcctgttg tacatcgtgc tctttcacaa    1440
acaagaaaag ttataaatgc cattataaaa aaatatggtt ctccaaacgc aatccatatt    1500
gaaactgcaa gagaattatc gaaaacattc caagaaagaa aagaaataga tagtatgtac    1560
caggataact ctaaaaagaa tgaacacgcc atttgtaagt taaaagaatt ggggaatatt    1620
aaaccctcag gaataaatat tgtgaaattt aagctttgga acgagcaaga tggtagatgt    1680
atgtattctg gaaaatatat tgaaccacat cgcttatttg aagaaggtta tacggaagta    1740
gatcatatac tcccttacag ccgtagctta gatgacagct ataacaataa agtattaacg    1800
ctaggaacag aaaatcagcg taaaggaaac aaaactcctt atgaatacat gaggaataat    1860
tcaatttggg atgaatttga aacaagagta cagtcaaata aaaaaatcaa caaaaagaaa    1920
caacaaaaat tacttttaca aaacttttca catgcgagag aacaagaatt tattgaacgc    1980
aatctcaatg atacccgtta tgccacaata tatctatcat cccttattca acaacacctc    2040
atattctctg aaagctctcg tgagaaaaaa gttcatacag ttagtggaat tataacatct    2100
catttgcgta gtagatgggg atttaacaaa gatcggaaag aggggcatat ccatcatgct    2160
ttagatgcgg taatcgttgc tgtcacatcc gatcacatga ttcaaagagt aacaaaatat    2220
tataagttaa aagagttaaa tagaaatatt caagccaaac ggatgcagtt tcctgaacct    2280
tgggaaggat ttcgtttaga gttagaagca agagtttctc caaataccca gcaatattta    2340
aagggtattc gttttaaaaa ttacgccgat gtcgatctat ccgaaatcaa acctatcttt    2400
gtttcaagaa tgcctaagcg ctctattact ggtgaattac atcaagaaac aatccggaaa    2460
ttccttggtt ataacgaaaa ggggaaagtg ctaaccgcga caaaaacaaa attagaagat    2520
attccttttg atgctaatgg tgattttccc atgtttggaa aagaaactga tttatatacg    2580
tataatgcca taaaagaacg ttatttaagt cataaaaaag ataaacgcaa atcattccaa    2640
gagcctttat acaaacccac aaaatcagga ggaattggac cacttataaa atctattaaa    2700
ataatggata caagaaccat tgttaatcct gttaatcaag gtaaaggtgt cgtgtataat    2760
agtaaaattg ctcgtactga tgtattcaaa aagggtgaga agtactattt aattcccatt    2820
tacacaattg atcttatgaa aaatatctta cctcaaaaag caattacagc aggaaaagga    2880
tatgaggatt ggacaactat tgatcctagc tttacttttt tgtttagtct ttttcccaat     2940
gatctaatcc aaattgtccc ctcaaaaaac aaaacaatta aagctagaac aactgtatcg    3000
aaaaaagagg tattactacc ctcactcaca ggatatttta aaggtgttca tagtggaact    3060
gctgggatta ctgttgaaac acatgatggc agtattatag caaatgtagg ctccaagcag    3120
ttactatcat ttgagaaata ccaggtagat gtacttggac attacacaaa aattaagaag    3180
gaaaaacgta ttggaatggc aatatag                                        3207

<210>  9
<211>  3207

<212>  DNA
<213>  Artificial sequence

<220>
<223>  Cas endonuclease of Locus 4 (Cas-Locus 4)-Bacillus species

<400>  9
ttgcaatatg tattaggatt ggatattgga atcgcatctt gtggttgggc tgtaatcaat      60
caagaaaaag agagaattga agatttaggg gttcgtattt ttgataaagc tgaaaatccc     120
aaagacggaa aatctttagc tgcacctagg cgtgacgcac gctctactag gagaacccta     180
cgccgtaaaa aacatagaat gctacggata aagatacttt tagtaaaaca aggcttacta     240
tctaagaacg aacttaacca tctatatgaa agtactactg aagtcgatgt ttggaactta     300
cgtttagatg ctctagaacg caaattgaat ccaaaagagt ttaccagagt tcttattcat     360
cttgctaaaa gaagaggatt taaatctaat agaaaagaaa ccactctctc agaaaacgga     420
caaatcttag aaagcatttc agagaatcaa cagattatgg agcaacaaaa ttatagaact     480
gttggagaaa tgattctaaa agataaaaaa tttgaaaacc ataaaagaaa caaagatggt     540
acttatatcg ggaccgttac ccgtcaacaa cttcaagaag aaatacaaat gatattcact     600
gcacaacgtt tgtacaaaaa cgattatgca acagaggaat ttgaaattag ctacttagaa     660
atctgggcat ctcaacgtcc ctatgcttct aaagatcaaa ttgaaaaaat gattggttac     720
tgtaccttag agccaaaaga aaaaagagtg cctaaagctt cttggtcatt tcaatatttt     780
gtagcgctac aaaccataaa caacctccga ctgatcagta aagacaggat agaagaactc     840
tctttcaaag aaaagaaaca aattatgaat ctagcacttg aaaaatcaat tgttaaatac     900
attgatatcc gaaaattatt atccattcct aatgaatttc agtttaatag cctatcatat     960
tcagctgata cagtagatac tacgttggag aataaaagat gtatagaact caaggaatat    1020
tactctatta acaaggtata taaacaaatt tatgagaaga acgccctaaa tttattacct    1080
atagattacg atacaattgc atacggacta actatcttta aagatgataa agatatcctt    1140
gaatatttac aaaataaata tgtaaatgca aaaggaaaac ctataaataa tttagctaaa    1200
aagacctacg attacacatt tattcaagca ttactgactc tgaatttcac gaaaatggga    1260
catttatcat ttaaagcttt aaaaaatatt attcctttct tagaagaagg gctatcatac    1320
gataaagcat gtgagaaggc aggatacaac tttaaaggaa catcctacac agaaaaaaca    1380
aagtacctac ctgttatccc tcaaaataca aatcctgttg tacatcgtgc tctttcacaa    1440
acaagaaaag ttataaatgc cattataaaa aaatatggtt ctccaaacgc aatccatatt    1500
gaaactgcaa gagaattatc gaaaacattc caagaaagaa aagaaataga tagtatgtac    1560
caggataact ctaaaaagaa tgaacacgcc attcataagt taaaagaatt ggggaacatt    1620
aacccctcag gaataaatat tgtgaaattt aagctttgga atgagcaaga tggtagatgt    1680
atgtattctg gaaaatatat cgaaccacat cggttatttg aagaaggtta tacggaagta    1740
gatcacatac tcccctatag ccgtagctta gatgatagct ataacaacaa aacattaaca    1800
ctaggaacag aaaatcagcg taaaggaaac aaaactcctt atgaatacat ggggaatact    1860
tcaatttgga atgactttga aacaagggta cattcaaata aaaaaatcaa caaaaagaaa    1920
caacaaaaat tacttttaca acacttttca tatgcgagag aacaagaatt tattgaacgc    1980
aatctgaatg atacccgtta tgccacaata tatctatcat cccttattca acaacacctc    2040
atattctctg aaagctctcg taagaaaaaa gttcatacag ttagtggaat tataacatct    2100
catttacgta gtagatgggg atttaacaaa gatcggaaag agggacatat tcatcatgct    2160
ttagatgcgg taatcgtcgc tgtcacatcc gatcacatga ttcaaagagt aacaaaatat    2220
tataagttaa aagagttaaa tagaaacctt caagccaaac ggatgccgtt tcctgaacct    2280
tgggaaggat ttcgtttaga gttagaagca aggatttctc caaataccca gcaatattta    2340
aagggatttc gttttaaaaa ttacgccgat gtcaacctat ccgaaatcaa acctatcttt    2400
gtttcaagaa tgcctaagcg ctctattact ggtgaattac atcaagaaac gattcggaaa    2460
ttcattggtt ataacgaaaa agggaaagta ttaaccgcaa taaaaacaaa attagaagat    2520
atcccttttg atgccaatgg tgattttccc atgtatggga aagaaactga tttatatacg    2580
tataatgcca taaaagaacg ttttttaagt cataaaaaag ataaacgcaa atcattccaa    2640
gaacctttat acaaacccac aaaatcagga ggaattggac cacttataaa atctattaaa    2700
ataattgata caagaaccat tgttaatcct gttaatcaag gtaaaggtgt cgtgtataat    2760
agtaaaattg ctcgtactga tgtattcaaa aaggatgaga agtactattt aattcccatt    2820
tacacaattg atcttatgaa aaatatctta cctcaaaaag caattacagc aggaaaagga    2880
tatgaggatt ggacaaccat tgatcctagc tttacttttt tatttagtct ttttcccaat    2940
gatctaattc aaattgtccc ctcaaaaaat aaaacaataa aagctagaac aactgtatcg    3000
aaaaaagagg tattactacc ctcactcaca ggatatttta aaggtgttca tagtggaact    3060
gcggggatta ctgttgaaac acacgatggc agtgttatag caaatgtagg ctcaaagcaa    3120
ttactattaa ttgagaaata tcaagtagat atacttggac attatacaaa aataaaggag    3180
gaaaaacgta ttgggatggc gatatag                                        3207

<210>  10

<211>  3207
<212>  DNA
<213>  Artificial sequence

<220>
<223>  Cas endonuclease of Locus 5 (Cas-Locus 5)

<400>  10
ttgaaatatg tattagggtt ggatattgga attgcatcat gtggttgggc agtaataaat      60
caagaaaaac atagaattga agatttaggg gttcgtattt ttgataaagc tgagaatccc     120
aaagatggaa aatctttagc tacacctaga cgtgacgcac gttctaccag aagaacccta     180
cgccgtaaaa aacatagaat gcaacggata aagatacttt tagtaaagca cggcttacta     240
tccaagaccg aaatcgacca tctatatgaa agtgctactg agatcgatgt ttggtactta     300
cgtttaaatg ctctagagcg cagattaaat ccaaaagagt ttgctagagt tcttattcac     360
cttgctaaaa gaagaggatt taaatctaat agaaaagaaa caactctctc ggaaaacgga     420
aaaatcttag aaaatatttc agagaacctg cagattatgg agcaaaaaaa ttatagaact     480
gttggagaaa tgattctaaa agataaaaaa tttgaaaatc ataaaagaaa caaagatggt     540
acttatatcg ggactgttac tcggcaacaa cttcaagaag aaatacaaat gatattcaat     600
gcacaacgtt tgtacaaaaa caatcatgca acagagaaat ttgaaagtag ctacttagaa     660
atctgggcat ctcaacgtcc ctatgcttct aaagatcaaa ttgaaaaaat gattggttac     720
tgtaccttag agccaaaaga aaaaagagtg cctaaagctt cttggtcatt tcaatatttt     780
gtagcgctac aaaccataaa caacctccga ctgatcagta aagacaggat agaagaactc     840
tcttttgaag aaaagaaacg aattatgaat ctagcactcg aaaaatcaat tgttaaatac     900
attgatatcc gaaaattact atctgttcct aatgaatttc aatttaatag cctttcatat     960
tcagctgata tagtagatac aacggtggaa aataaaaaat gtatagaact caaagaatat    1020
cactctatta acaaggtata taaacaaatt tacgaacaga acgctctaaa tttattacct    1080
aaagattacg atacaattgc atacggactt actatcttta aagatgataa agatatccta    1140
gaatatttac aaaataaata cgtaaatgca aaaggaaaac ctataagtaa ttttgctaac    1200
aagacctacg atgatacatt tattcaggta ttattgactc tgaatttctc aaaaatggga    1260
catttatcat ttaaagcttt aaaaaatatt attccttttt tagaagaagg tctatcctac    1320
gataaagcat gtgagaaggc aggatacaac tttaagggaa catcctacac aaaaaaaaca    1380
aagtacctac ctgttatccc tcagaataca aatcctgttg tacatcgtgc tctttcacaa    1440
acaagaaaag ttataaatgc cattataaaa aaatatggtt ctccaaacgc aatccatatt    1500
gaaactgcaa gagaattatc gaaaacattt caagaaagaa aagaaataga tagtatgtac    1560
caggataact ctaaaaagaa tgaacacgcc attcataagt taaaagaatt ggggaacatt    1620
aacccctcag gaataaatat tgtgaaattt aagctttgga atgagcaaga tagtagatgt    1680
atgtattctg gaaaatatat cgaaccacat cgattatttg aagaaggtta tacggaagta    1740
gatcatatac tcccctatag ccggagctta gatgacagct ataacaataa agtgttaacg    1800
ctagggacag aaaatcaggg taaaggaaac agaactcctt atgaatacat ggggaatact    1860
tcaatttggg atgaatttga aacaagagta cagtcaaata aaaaaatcaa caaaaagaaa    1920
caacaaaaat tacttttaca aaacttttca catgcgagag aacaagaatt tattgaacgc    1980
aatctcaatg atacccgtta tgccacaata tatatatcat cccttattca acaacacctc    2040
atattctctg aaagctctcg tgagaaaaaa gttcatacag ttagtggaat tataacatct    2100
catttgcgta gtagatgggg atttaacaaa gatcggaaag aggggcatat ccatcatgct    2160
ttagatgcgg taatcgttgc tgtcacatcc gatcacatga ttcaaagagt aacaaaatat    2220
tataagttaa aagaattaaa tagaaaccgt caagccaaac ggatgcagtt tcctgaacct    2280
tgggaaggat ttcgtttaga gttagaagca aggatttctc caaataccca gcaatactta    2340
aagggaattc gtttaaaaaa ttacgccgat gtcaatctat ctgaaatcaa acctatcttt    2400
gtttcaagaa tgcctaagcg ctctattact ggtgaattac atcaagaaac aatccggaaa    2460
ttccttggtt ataacgaaaa agggaaagtg ctaaccgcaa taaaaacaaa attagaagat    2520
attccttttg atgctaatgg tgattttccc atgtatggaa aagaaactga tttatatacg    2580
tataatgcca taaaagaacg ttatttaagt cataaaaaag ataaacgcaa atcattccaa    2640
gaacctttat acaaacccac aaaatcagga ggaattggac cacttataaa atctattaaa    2700
ataatggata caagaaccat tgttaatcct gttaatcaag gtaaaggtgt cgtgtataat    2760
agtaaaattg ctcgcactga tgtattcaaa aaggctgaaa agtactattt aattcccatt    2820
tacacaattg atcttatgaa aaatatctta cctcaaaaag caattacagc aggaaaagga    2880
tatgaggatt ggacaaccat tgatcctagc tttacttttt tgtttagtct ttttcccaat     2940
gatctaatcc aaattgtccc ctcaaaaaat aaaataatta aagctagaac aactgtatcg    3000
aaaaaagagg tattactacc ctcactcaca ggatatttta aaggtgttca tagtggaact    3060
gctgggatta ctgttgaaac acacgatggc agtattatag caaatgtagg ctccaagcag    3120
ttactatcat ttgagaaata ccaggtagat gtacttggac attacacaaa aattaagaag    3180
gaaaaacgta ttggaatggc aatatag                                        3207

<210>  11
<211>  1070
<212>  PRT
<213>  Artificial sequence

<220>
<223>  Cas-Locus 1 (Bacillus species)

<400>  11
Met Arg Glu Leu Asp Tyr Arg Ile Gly Leu Asp Ile Gly Thr Asn Ser
1               5                   10                  15
Ile Gly Trp Gly Ile Ile Glu Leu Ser Trp Asn Lys Asp Arg Glu Gln
            20                  25                  30
Tyr Glu Lys Ala Arg Ile Val Asp Lys Gly Val Arg Met Phe Asp Lys
        35                  40                  45
Ala Glu Ile Pro Lys Thr Gly Ala Ser Leu Ala Glu Pro Arg Arg Leu
    50                  55                  60
Ala Arg Ser Ser Arg Arg Arg Leu Asn Arg Lys Ser Gln Arg Lys Lys
65                  70                  75                  80
Asp Ile Arg Asn Leu Leu Val Gln His Glu Ile Ile Ser Gln Arg Glu
                85                  90                  95
Leu Ala Ser Leu Tyr Pro Leu Ser Lys Ser Ser Met Asp Ile Trp Asp
            100                 105                 110
Ile Arg Leu Glu Gly Leu Asp Arg Leu Leu Asp His Phe Glu Trp Thr
        115                 120                 125
Arg Leu Leu Ile His Leu Ala Gln Arg Arg Gly Phe Lys Ser Asn Arg
    130                 135                 140
Lys Ser Glu Leu Lys Asp Val Glu Thr Gly Lys Val Leu Ser Ser Ile
145                 150                 155                 160
Gln Ala Asn Glu Lys Arg Leu Ser Leu Tyr Arg Thr Val Gly Glu Met
                165                 170                 175
Trp Met Lys Asn Glu Asp Phe Ser Lys Tyr Asp Lys Arg Arg Asn Ser
            180                 185                 190
Pro Asn Glu Tyr Val Phe Ser Val Ser Arg Ala Asp Leu Glu Lys Glu
        195                 200                 205
Ile Val Thr Leu Phe Glu Ala Gln Arg Lys Phe Gln Ser Ser Tyr Ala
    210                 215                 220
Ser Val Asp Leu Gln Lys Thr Tyr Leu Gln Ile Trp Ala His Gln Leu
225                 230                 235                 240
Pro Phe Ala Ser Gly Asn Ala Ile Val Asn Lys Val Gly Tyr Cys Ser
                245                 250                 255
Leu Leu Lys Gly Lys Glu Lys Arg Val Pro Lys Ala Thr Tyr Thr Phe
            260                 265                 270
Gln Tyr Phe Ser Thr Leu Asp Gln Ile Asn Arg Thr Arg Leu Gly Pro
        275                 280                 285
Asn Phe Gln Pro Phe Thr Lys Glu Gln Arg Asp Ile Ile Leu Asp Lys
    290                 295                 300
Met Phe Asn Arg Thr Asp Tyr Tyr Lys Lys Lys Thr Ile Pro Glu Val
305                 310                 315                 320
Thr Tyr Tyr Asp Ile Arg Lys Trp Leu Ala Leu Asp Glu Thr Ile Gln
                325                 330                 335
Phe Lys Gly Leu Thr Tyr Asp Pro Asn Glu Glu Leu Lys Lys Ile Glu
            340                 345                 350
Leu Lys Ser Phe Ile Asn Leu Lys Pro Phe Tyr Glu Ile Lys Gln Val
        355                 360                 365
Val Thr Asn Tyr Ala Lys Lys Ile Asn Glu Ala Phe Ser Thr Leu Asp
    370                 375                 380
Tyr Asp Thr Phe Ala Tyr Ala Leu Thr Val Tyr Lys Thr Asp Lys Asp
385                 390                 395                 400
Ile Arg Ser Tyr Leu Lys Lys Ser Asn Asn Leu Ser Lys Cys Cys Tyr
                405                 410                 415
Asp Asp Gln Leu Ile Glu Glu Leu Leu Thr Leu Ser Tyr Thr Lys Phe
            420                 425                 430

Gly His Leu Ser Phe Lys Ala Ile Asn His Val Leu Pro Ile Met Gln
        435                 440                 445
Glu Gly Arg Thr Tyr Gln Glu Ala Ile His Gln Leu Gly Tyr Asp Thr
    450                 455                 460
Thr Asn Leu Lys Lys Glu Asn Arg Ser Met Phe Leu Pro Leu Ile Pro
465                 470                 475                 480
Asp Glu Ile Thr Asn Pro Ile Val Lys Arg Ala Leu Thr Gln Ala Arg
                485                 490                 495
Lys Val Val Asn Ala Ile Ile Arg Arg Tyr Gly Ser Pro Asn Ser Val
            500                 505                 510
His Ile Glu Leu Ala Arg Glu Leu Pro Lys Asn His Asp Glu Arg Thr
        515                 520                 525
Lys Ile Met Lys Ala His Asp Glu Asn Tyr Lys Lys Asn Lys Gly Ala
    530                 535                 540
Ile Ser Ile Leu Ile Glu Asn Gly Ile Leu Asn Pro Thr Gly Tyr Asp
545                 550                 555                 560
Ile Val Arg Tyr Lys Leu Trp Lys Glu Gln Gly Glu Arg Cys Val Tyr
                565                 570                 575
Ser Leu Lys Gln Ile Pro Ala Asn Thr Phe Phe Asn Glu Leu Lys Lys
            580                 585                 590
Glu Arg Ser Gly Pro Pro Val Leu Glu Ile Asp His Ile Leu Pro Tyr
        595                 600                 605
Ser Gln Ser Phe Ile Asp Ser Tyr His Asn Lys Val Leu Val Tyr Glu
    610                 615                 620
Glu Glu Asn Gln Lys Lys Gly Asn Arg Ile Pro Tyr Thr Tyr Phe Leu
625                 630                 635                 640
Glu Glu Asn Lys Asp Trp Glu Ser Phe Glu Ser Tyr Val Arg Leu Asn
                645                 650                 655
Ser Phe Leu Ser Lys Lys Lys Arg Gly Tyr Leu Leu Lys Lys Ala Tyr
            660                 665                 670
Leu Pro Arg Glu Ser Asn Met Ile Lys Glu Arg His Leu Asn Asp Thr
        675                 680                 685
Arg Tyr Ala Ser Ser Tyr Leu Lys Asn Phe Ile Glu Lys Asn Leu Lys
    690                 695                 700
Phe Lys Glu Val Glu Gly Ser Thr Arg Asn Lys His Val Gln Thr Val
705                 710                 715                 720
Asn Gly Ile Ile Thr Ala His Leu Arg Lys Arg Trp Gly Leu Glu Lys
                725                 730                 735
Asp Arg Lys Glu Thr Tyr Leu His His Ala Met Asp Ala Ile Ile Val
            740                 745                 750
Ala Cys Thr Asp His His Met Val Thr Lys Val Thr Glu Tyr Tyr Gln
        755                 760                 765
Ile Lys Glu Ser Asn Lys Ser Ile Lys Lys Pro Tyr Phe Pro Leu Pro
    770                 775                 780
Trp Val Gly Phe Arg Glu Glu Ile Leu Ser His Leu Ala Arg Gln Pro
785                 790                 795                 800
Ile Ala Arg Lys Ile Ser Glu Glu Leu Lys Ile Gly Tyr Gln Ser Phe
                805                 810                 815
Asp Tyr Ile Leu Val Ser Arg Met Pro Lys Arg Ser Val Thr Gly Ser
            820                 825                 830
Ala His Glu Gln Thr Ile Met Lys Lys Gly Gly Ile Asp Lys Lys Gly
        835                 840                 845
Lys Thr Ile Ile Ile Lys Arg Val Tyr Leu Lys Asp Ile Lys Phe Asp
    850                 855                 860
Glu Asn Gly Asp Phe Lys Met Val Gly Lys Glu Gln Asp Leu Ala Thr
865                 870                 875                 880
Tyr Glu Ala Ile Lys Gln Arg Tyr Ile Glu Tyr Gly Lys Glu Ser Lys
                885                 890                 895
Lys Ala Phe Glu Thr Pro Leu Tyr Lys Pro Ser Lys Lys Gly Lys Gly
            900                 905                 910
Asn Leu Ile Lys Lys Ile Lys Val Glu Val Gln Thr Lys Ser Phe Val
        915                 920                 925
Arg Glu Val Asn Gly Gly Val Ala Gln Asn Gly Asp Leu Val Arg Val

930                 935                 940
Asp Leu Phe Glu Lys Asp Asn Arg Tyr Tyr Met Val Pro Ile Tyr Val
945                 950                 955                 960
Met Asp Thr Val His Ser Glu Leu Pro Asn Lys Ala Val Thr Ser Ser
                965                 970                 975
Lys Gly Tyr Glu Gln Trp Leu Thr Ile Asp Asn Ser Phe Thr Phe Lys
            980                 985                 990
Phe Ser Leu Tyr Pro Tyr Asp Leu  Val Arg Leu Val Lys  Gly Asp Glu
        995                 1000                1005
Asp Arg  Phe Leu Tyr Phe Ser  Thr Leu Asp Ile Asn  Ser Asp Arg
    1010                1015                1020
Leu Asn  Phe Lys Asp Val Asn  Thr Pro Ser Lys Gln  Ala Glu Asn
    1025                1030                1035
Arg Tyr  Ser Leu Lys Thr Ile  Glu Asn Leu Glu Lys  Tyr Glu Val
    1040                1045                1050
Gly Val  Leu Gly Asp Leu Arg  Phe Val Arg Gln Glu  Ile Arg Lys
    1055                1060                1065
Asn Phe
    1070

<210>  12
<211>  1070
<212>  PRT
<213>  Artificial sequence

<220>
<223>  Cas-Locus 2 (Bacillus species)

<400>  12
Met Arg Glu Leu Asp Tyr Arg Ile Gly Leu Asp Ile Gly Thr Asn Ser
1               5                   10                  15
Ile Gly Trp Gly Val Val Glu Leu Ser Trp Asn Lys Asp Arg Glu Gln
            20                  25                  30
Tyr Glu Lys Ala Arg Ile Val Asp Lys Gly Val Arg Met Phe Asp Lys
        35                  40                  45
Ala Glu Ile Pro Lys Thr Gly Ala Ser Leu Ala Glu Pro Arg Arg Thr
    50                  55                  60
Ala Arg Ser Ser Arg Arg Arg Leu Asn Arg Lys Ser Gln Arg Lys Lys
65                  70                  75                  80
Asp Ile Arg Asn Leu Leu Val Gln His Glu Ile Ile Ser Gln Lys Glu
                85                  90                  95
Leu Ala Ser Leu Tyr Pro Leu Ser Lys Ser Ser Met Asp Ile Trp Asp
            100                 105                 110
Ile Arg Leu Asp Gly Leu Asp Arg Leu Leu Asp Arg Leu Glu Trp Thr
        115                 120                 125
Arg Leu Leu Ile His Leu Ala Gln Lys Arg Gly Phe Lys Ser Asn Arg
    130                 135                 140
Lys Ser Glu Leu Lys Asp Val Glu Thr Gly Lys Val Leu Ser Ser Ile
145                 150                 155                 160
Gln Val Asn Glu Lys Arg Leu Ser Leu Tyr Arg Thr Val Gly Glu Met
                165                 170                 175
Trp Met Lys Asn Glu Asp Phe Ser Lys Tyr Asp Lys Arg Arg Asn Ser
            180                 185                 190
Pro Asn Glu Tyr Val Phe Ser Val Ser Arg Ala Asn Leu Glu Lys Glu
        195                 200                 205
Ile Val Thr Leu Phe Glu Ala Gln Arg Lys Phe Gln Ser Ser Tyr Ala
    210                 215                 220
Ser Val Asp Leu Gln Lys Thr Tyr Leu Gln Ile Trp Ala His Gln Leu
225                 230                 235                 240
Pro Phe Ala Ser Gly Asn Ala Ile Val Asn Lys Val Gly Tyr Cys Ser
                245                 250                 255
Leu Leu Lys Gly Lys Glu Lys Arg Val Pro Lys Ala Thr Tyr Thr Phe
            260                 265                 270

Gln Tyr Phe Ser Thr Leu Asp Gln Ile Asn Arg Thr Arg Leu Gly Pro
        275                 280                 285
Asn Phe Gln Pro Phe Thr Lys Glu Gln Arg Asp Ile Ile Leu Asp Lys
    290                 295                 300
Met Phe Gln Arg Thr Asp Tyr Tyr Lys Lys Lys Thr Ile Pro Glu Val
305                 310                 315                 320
Thr Tyr Tyr Asp Ile Arg Lys Trp Leu Ala Leu Asp Glu Thr Ile Gln
                325                 330                 335
Phe Lys Gly Leu Thr Tyr Asp Pro Asn Glu Glu Leu Lys Lys Ile Glu
            340                 345                 350
Met Lys Leu Phe Ile Asn Leu Lys Pro Phe Tyr Glu Ile Lys Lys Val
        355                 360                 365
Val Thr Asn Tyr Ala Lys Lys Thr Asn Glu Val Phe Ser Thr Leu Asp
    370                 375                 380
Tyr Asp Thr Val Ala Tyr Ala Leu Thr Val Tyr Lys Thr Asp Lys Asp
385                 390                 395                 400
Ile Ile Ser Tyr Leu Lys Arg Ser Asn Asn Leu Ser Lys Arg Cys Tyr
                405                 410                 415
Asp Asp Gln Leu Ile Glu Glu Leu Leu Thr Leu Ser Tyr Thr Lys Phe
            420                 425                 430
Gly His Leu Ser Phe Lys Ala Ile Asn His Val Leu Pro Ile Met Gln
        435                 440                 445
Glu Gly Arg Thr Tyr Gln Glu Ala Ile His Gln Leu Gly Tyr Asp Thr
    450                 455                 460
Thr Asn Leu Lys Lys Glu Asn Arg Ser Met Phe Leu Pro Leu Ile Pro
465                 470                 475                 480
Asp Glu Ile Thr Asn Pro Ile Val Lys Arg Ala Leu Thr Gln Ala Arg
                485                 490                 495
Lys Val Val Asn Ala Ile Ile Arg Arg Tyr Gly Ser Pro Asn Ser Val
            500                 505                 510
His Ile Glu Leu Ala Arg Glu Leu Ser Lys Thr His Asp Glu Arg Thr
        515                 520                 525
Lys Ile Met Lys Ala His Asp Glu Asn Tyr Lys Lys Asn Lys Gly Ala
    530                 535                 540
Ile Ser Ile Leu Ile Glu Asn Gly Ile Leu Asn Pro Thr Gly Tyr Asp
545                 550                 555                 560
Ile Val Arg Tyr Lys Leu Trp Lys Glu Gln Gly Glu Arg Cys Ala Tyr
                565                 570                 575
Ser Leu Lys Gln Ile His Ala Asn Thr Phe Phe Asn Glu Leu Lys Lys
            580                 585                 590
Glu Arg Ser Gly Pro Pro Val Leu Glu Ile Asp His Ile Leu Pro Tyr
        595                 600                 605
Ser Gln Ser Phe Ile Asp Ser Tyr His Asn Lys Val Leu Val Tyr Gly
    610                 615                 620
Asp Glu Asn Gln Lys Lys Gly Asn Arg Ile Pro Tyr Thr Tyr Phe Leu
625                 630                 635                 640
Glu Gly Asn Lys Asp Trp Glu Ser Phe Glu Ser Tyr Val Arg Leu Asn
                645                 650                 655
Ser Leu Phe Ser Lys Lys Lys Arg Gly Tyr Leu Leu Lys Lys Ala Tyr
            660                 665                 670
Leu Pro Arg Glu Ser Asn Met Ile Lys Glu Arg His Leu Asn Asp Thr
        675                 680                 685
Arg Tyr Ala Ser Ser Tyr Leu Lys Asn Phe Ile Glu Lys Asn Leu Lys
    690                 695                 700
Phe Lys Glu Val Glu Gly Ser Thr Arg Lys Lys His Val Gln Thr Val
705                 710                 715                 720
Asn Gly Ile Ile Thr Ala His Leu Arg Lys Arg Trp Gly Leu Glu Lys
                725                 730                 735
Asp Arg Gln Glu Thr Tyr Leu His His Ala Met Asp Ala Ile Ile Val
            740                 745                 750
Ala Cys Thr Asp His His Met Val Thr Lys Val Thr Glu Tyr Tyr Gln
        755                 760                 765
Ile Lys Glu Ser Asn Lys Ser Ile Lys Lys Pro Tyr Phe Pro Leu Pro

770                 775                 780
Trp Val Ala Phe Arg Glu Glu Ile Leu Ser His Leu Ala Arg Gln Pro
785                 790                 795                 800
Ile Ala Arg Lys Ile Ser Glu Glu Leu Lys Ile Gly Tyr Gln Ser Phe
                805                 810                 815
Asp Tyr Ile Leu Val Ser Arg Met Pro Lys Arg Ser Val Thr Gly Ala
            820                 825                 830
Ala His Glu Gln Thr Ile Met Lys Lys Gly Gly Ile Asp Lys Lys Gly
        835                 840                 845
Lys Thr Ile Ile Ile Lys Arg Val Tyr Leu Lys Asp Ile Lys Phe Asp
    850                 855                 860
Glu Asn Gly Asp Phe Lys Met Val Gly Lys Glu Gln Asp Leu Ala Thr
865                 870                 875                 880
Tyr Glu Ala Ile Lys Gln Arg Tyr Ile Glu Tyr Gly Lys Glu Ser Lys
                885                 890                 895
Lys Ala Phe Glu Thr Pro Leu Tyr Lys Pro Ser Lys Lys Gly Lys Gly
            900                 905                 910
Asn Leu Ile Lys Lys Ile Lys Val Glu Val Gln Thr Lys Ser Phe Val
        915                 920                 925
Arg Glu Val Asn Gly Gly Val Ala Gln Asn Gly Asp Leu Val Arg Val
    930                 935                 940
Asp Leu Phe Glu Lys Asp Asn Arg Tyr Tyr Met Val Pro Ile Tyr Val
945                 950                 955                 960
Met Asp Thr Val His Ser Glu Leu Pro Asn Lys Ala Val Thr Ser Ser
                965                 970                 975
Lys Gly Tyr Glu Lys Trp Leu Thr Ile Asp Asn Ser Phe Thr Phe Lys
            980                 985                 990
Phe Ser Leu Tyr Pro Tyr Asp Leu  Val Arg Leu Val Lys  Gly Asp Glu
        995                 1000                1005
Asp Arg  Phe Leu Tyr Phe Ser  Thr Leu Asp Ile Asn  Ser Asp Arg
    1010                1015                1020
Leu Asn  Phe Lys Asp Val Asn  Lys Pro Ser Lys Gln  Ala Glu Asn
    1025                1030                1035
Arg Tyr  Ser Leu Lys Thr Ile  Glu Asn Leu Glu Lys  Tyr Glu Val
    1040                1045                1050
Gly Val  Leu Gly Asp Leu Arg  Phe Val Arg Gln Glu  Ile Arg Lys
    1055                1060                1065
Asn Phe
    1070

<210>  13
<211>  1068
<212>  PRT
<213>  Artificial sequence

<220>
<223>  Cas-Locus 3 (Bacillus species)

<400>  13
Met Lys Tyr Val Leu Gly Leu Asp Ile Gly Ile Ala Ser Cys Gly Trp
1               5                   10                  15
Ala Val Ile Asn Gln Glu Lys His Arg Ile Glu Asp Leu Gly Val Arg
            20                  25                  30
Ile Phe Asp Lys Ala Glu Asn Pro Lys Asp Gly Lys Ser Leu Ala Thr
        35                  40                  45
Pro Arg Arg Asp Ala Arg Ser Thr Arg Arg Thr Leu Arg Arg Lys Lys
    50                  55                  60
His Arg Met Gln Arg Ile Lys Ile Leu Leu Val Lys His Gly Leu Leu
65                  70                  75                  80
Ser Lys Thr Glu Ile Asp His Leu Tyr Glu Ser Ala Thr Glu Ile Asp
                85                  90                  95
Val Trp Tyr Leu Arg Leu Asn Ala Leu Glu Arg Arg Leu Asn Pro Lys
            100                 105                 110

Glu Phe Ala Arg Val Leu Ile His Leu Ala Lys Arg Arg Gly Phe Lys
        115                 120                 125
Ser Asn Arg Lys Glu Thr Thr Leu Ser Glu Asn Gly Gln Ile Leu Glu
    130                 135                 140
Asn Ile Ser Glu Asn Leu Gln Ile Met Glu Gln Lys Asn Tyr Arg Thr
145                 150                 155                 160
Val Gly Glu Met Ile Leu Lys Asp Lys Arg Phe Glu Asn His Lys Arg
                165                 170                 175
Asn Lys Asp Gly Thr Tyr Ile Gly Thr Val Thr Arg Gln Gln Leu Gln
            180                 185                 190
Glu Glu Ile Gln Met Ile Phe Asn Ala Gln Arg Leu Tyr Lys Asn Asn
        195                 200                 205
His Ala Thr Glu Lys Phe Glu Ser Ser Tyr Leu Glu Ile Trp Ala Ser
    210                 215                 220
Gln Arg Pro Tyr Ala Ser Lys Asp Gln Ile Glu Lys Met Ile Gly Tyr
225                 230                 235                 240
Cys Thr Leu Glu Pro Lys Glu Lys Arg Val Pro Lys Ala Ser Trp Ser
                245                 250                 255
Phe Gln Tyr Phe Val Ala Leu Gln Thr Ile Asn Asn Leu Arg Leu Ile
            260                 265                 270
Ser Lys Asp Arg Ile Glu Glu Leu Ser Phe Glu Glu Lys Lys Arg Ile
        275                 280                 285
Met Asn Leu Ala Leu Glu Lys Ser Ile Val Lys Tyr Ile Asp Ile Arg
    290                 295                 300
Lys Leu Leu Ser Val Pro Asn Glu Phe Gln Phe Asn Ser Leu Ser Tyr
305                 310                 315                 320
Ser Ala Asp Ile Val Asp Thr Thr Val Glu Asn Lys Lys Cys Ile Glu
                325                 330                 335
Leu Lys Glu Tyr His Ser Ile Asn Lys Val Tyr Lys Gln Ile Tyr Gly
            340                 345                 350
Gln Asn Ala Leu Asn Leu Leu Pro Lys Asp Tyr Asp Thr Ile Ala Tyr
        355                 360                 365
Gly Leu Thr Ile Phe Lys Asp Asp Lys Asp Ile Leu Glu Tyr Leu Gln
    370                 375                 380
Asn Lys Tyr Val Asn Ala Lys Gly Lys Pro Ile Ser Asn Leu Ala Asn
385                 390                 395                 400
Lys Thr Tyr Asp Asp Thr Phe Ile Gln Val Leu Leu Thr Leu Asn Phe
                405                 410                 415
Ser Lys Met Gly His Leu Ser Phe Lys Ala Leu Lys Asn Ile Ile Pro
            420                 425                 430
Phe Leu Glu Glu Gly Leu Ser Tyr Asp Lys Ala Cys Glu Lys Ala Gly
        435                 440                 445
Tyr Asn Phe Lys Gly Ala Ser Tyr Thr Lys Lys Thr Lys Tyr Leu Pro
    450                 455                 460
Val Ile Pro Gln Asn Thr Asn Pro Val Val His Arg Ala Leu Ser Gln
465                 470                 475                 480
Thr Arg Lys Val Ile Asn Ala Ile Ile Lys Lys Tyr Gly Ser Pro Asn
                485                 490                 495
Ala Ile His Ile Glu Thr Ala Arg Glu Leu Ser Lys Thr Phe Gln Glu
            500                 505                 510
Arg Lys Glu Ile Asp Ser Met Tyr Gln Asp Asn Ser Lys Lys Asn Glu
        515                 520                 525
His Ala Ile Cys Lys Leu Lys Glu Leu Gly Asn Ile Lys Pro Ser Gly
    530                 535                 540
Ile Asn Ile Val Lys Phe Lys Leu Trp Asn Glu Gln Asp Gly Arg Cys
545                 550                 555                 560
Met Tyr Ser Gly Lys Tyr Ile Glu Pro His Arg Leu Phe Glu Glu Gly
                565                 570                 575
Tyr Thr Glu Val Asp His Ile Leu Pro Tyr Ser Arg Ser Leu Asp Asp
            580                 585                 590
Ser Tyr Asn Asn Lys Val Leu Thr Leu Gly Thr Glu Asn Gln Arg Lys
        595                 600                 605
Gly Asn Lys Thr Pro Tyr Glu Tyr Met Arg Asn Asn Ser Ile Trp Asp

610                 615                 620
Glu Phe Glu Thr Arg Val Gln Ser Asn Lys Lys Ile Asn Lys Lys Lys
625                 630                 635                 640
Gln Gln Lys Leu Leu Leu Gln Asn Phe Ser His Ala Arg Glu Gln Glu
                645                 650                 655
Phe Ile Glu Arg Asn Leu Asn Asp Thr Arg Tyr Ala Thr Ile Tyr Leu
            660                 665                 670
Ser Ser Leu Ile Gln Gln His Leu Ile Phe Ser Glu Ser Ser Arg Glu
        675                 680                 685
Lys Lys Val His Thr Val Ser Gly Ile Ile Thr Ser His Leu Arg Ser
    690                 695                 700
Arg Trp Gly Phe Asn Lys Asp Arg Lys Glu Gly His Ile His His Ala
705                 710                 715                 720
Leu Asp Ala Val Ile Val Ala Val Thr Ser Asp His Met Ile Gln Arg
                725                 730                 735
Val Thr Lys Tyr Tyr Lys Leu Lys Glu Leu Asn Arg Asn Ile Gln Ala
            740                 745                 750
Lys Arg Met Gln Phe Pro Glu Pro Trp Glu Gly Phe Arg Leu Glu Leu
        755                 760                 765
Glu Ala Arg Val Ser Pro Asn Thr Gln Gln Tyr Leu Lys Gly Ile Arg
    770                 775                 780
Phe Lys Asn Tyr Ala Asp Val Asp Leu Ser Glu Ile Lys Pro Ile Phe
785                 790                 795                 800
Val Ser Arg Met Pro Lys Arg Ser Ile Thr Gly Glu Leu His Gln Glu
                805                 810                 815
Thr Ile Arg Lys Phe Leu Gly Tyr Asn Glu Lys Gly Lys Val Leu Thr
            820                 825                 830
Ala Thr Lys Thr Lys Leu Glu Asp Ile Pro Phe Asp Ala Asn Gly Asp
        835                 840                 845
Phe Pro Met Phe Gly Lys Glu Thr Asp Leu Tyr Thr Tyr Asn Ala Ile
    850                 855                 860
Lys Glu Arg Tyr Leu Ser His Lys Lys Asp Lys Arg Lys Ser Phe Gln
865                 870                 875                 880
Glu Pro Leu Tyr Lys Pro Thr Lys Ser Gly Gly Ile Gly Pro Leu Ile
                885                 890                 895
Lys Ser Ile Lys Ile Met Asp Thr Arg Thr Ile Val Asn Pro Val Asn
            900                 905                 910
Gln Gly Lys Gly Val Val Tyr Asn Ser Lys Ile Ala Arg Thr Asp Val
        915                 920                 925
Phe Lys Lys Gly Glu Lys Tyr Tyr Leu Ile Pro Ile Tyr Thr Ile Asp
    930                 935                 940
Leu Met Lys Asn Ile Leu Pro Gln Lys Ala Ile Thr Ala Gly Lys Gly
945                 950                 955                 960
Tyr Glu Asp Trp Thr Thr Ile Asp Pro Ser Phe Thr Phe Leu Phe Ser
                965                 970                 975
Leu Phe Pro Asn Asp Leu Ile Gln Ile Val Pro Ser Lys Asn Lys Thr
            980                 985                 990
Ile Lys Ala Arg Thr Thr Val Ser  Lys Lys Glu Val Leu  Leu Pro Ser
        995                 1000                1005
Leu Thr  Gly Tyr Phe Lys Gly  Val His Ser Gly Thr  Ala Gly Ile
    1010                1015                1020
Thr Val  Glu Thr His Asp Gly  Ser Ile Ile Ala Asn  Val Gly Ser
    1025                1030                1035
Lys Gln  Leu Leu Ser Phe Glu  Lys Tyr Gln Val Asp  Val Leu Gly
    1040                1045                1050
His Tyr  Thr Lys Ile Lys Lys  Glu Lys Arg Ile Gly  Met Ala Ile
    1055                1060                1065

<210>  14
<211>  1068
<212>  PRT
<213>  Artificial sequence

<220>
<223>  Cas-Locus 4 (Bacillus species)

<400>  14
Met Gln Tyr Val Leu Gly Leu Asp Ile Gly Ile Ala Ser Cys Gly Trp
1               5                   10                  15
Ala Val Ile Asn Gln Glu Lys Glu Arg Ile Glu Asp Leu Gly Val Arg
            20                  25                  30
Ile Phe Asp Lys Ala Glu Asn Pro Lys Asp Gly Lys Ser Leu Ala Ala
        35                  40                  45
Pro Arg Arg Asp Ala Arg Ser Thr Arg Arg Thr Leu Arg Arg Lys Lys
    50                  55                  60
His Arg Met Leu Arg Ile Lys Ile Leu Leu Val Lys Gln Gly Leu Leu
65                  70                  75                  80
Ser Lys Asn Glu Leu Asn His Leu Tyr Glu Ser Thr Thr Glu Val Asp
                85                  90                  95
Val Trp Asn Leu Arg Leu Asp Ala Leu Glu Arg Lys Leu Asn Pro Lys
            100                 105                 110
Glu Phe Thr Arg Val Leu Ile His Leu Ala Lys Arg Arg Gly Phe Lys
        115                 120                 125
Ser Asn Arg Lys Glu Thr Thr Leu Ser Glu Asn Gly Gln Ile Leu Glu
    130                 135                 140
Ser Ile Ser Glu Asn Gln Gln Ile Met Glu Gln Gln Asn Tyr Arg Thr
145                 150                 155                 160
Val Gly Glu Met Ile Leu Lys Asp Lys Lys Phe Glu Asn His Lys Arg
                165                 170                 175
Asn Lys Asp Gly Thr Tyr Ile Gly Thr Val Thr Arg Gln Gln Leu Gln
            180                 185                 190
Glu Glu Ile Gln Met Ile Phe Thr Ala Gln Arg Leu Tyr Lys Asn Asp
        195                 200                 205
Tyr Ala Thr Glu Glu Phe Glu Ile Ser Tyr Leu Glu Ile Trp Ala Ser
    210                 215                 220
Gln Arg Pro Tyr Ala Ser Lys Asp Gln Ile Glu Lys Met Ile Gly Tyr
225                 230                 235                 240
Cys Thr Leu Glu Pro Lys Glu Lys Arg Val Pro Lys Ala Ser Trp Ser
                245                 250                 255
Phe Gln Tyr Phe Val Ala Leu Gln Thr Ile Asn Asn Leu Arg Leu Ile
            260                 265                 270
Ser Lys Asp Arg Ile Glu Glu Leu Ser Phe Lys Glu Lys Lys Gln Ile
        275                 280                 285
Met Asn Leu Ala Leu Glu Lys Ser Ile Val Lys Tyr Ile Asp Ile Arg
    290                 295                 300
Lys Leu Leu Ser Ile Pro Asn Glu Phe Gln Phe Asn Ser Leu Ser Tyr
305                 310                 315                 320
Ser Ala Asp Thr Val Asp Thr Thr Leu Glu Asn Lys Arg Cys Ile Glu
                325                 330                 335
Leu Lys Glu Tyr Tyr Ser Ile Asn Lys Val Tyr Lys Gln Ile Tyr Glu
            340                 345                 350
Lys Asn Ala Leu Asn Leu Leu Pro Ile Asp Tyr Asp Thr Ile Ala Tyr
        355                 360                 365
Gly Leu Thr Ile Phe Lys Asp Asp Lys Asp Ile Leu Glu Tyr Leu Gln
    370                 375                 380
Asn Lys Tyr Val Asn Ala Lys Gly Lys Pro Ile Asn Asn Leu Ala Lys
385                 390                 395                 400
Lys Thr Tyr Asp Tyr Thr Phe Ile Gln Ala Leu Leu Thr Leu Asn Phe
                405                 410                 415
Thr Lys Met Gly His Leu Ser Phe Lys Ala Leu Lys Asn Ile Ile Pro
            420                 425                 430
Phe Leu Glu Glu Gly Leu Ser Tyr Asp Lys Ala Cys Glu Lys Ala Gly
        435                 440                 445
Tyr Asn Phe Lys Gly Thr Ser Tyr Thr Glu Lys Thr Lys Tyr Leu Pro
    450                 455                 460
Val Ile Pro Gln Asn Thr Asn Pro Val Val His Arg Ala Leu Ser Gln

465                 470                 475                 480
Thr Arg Lys Val Ile Asn Ala Ile Ile Lys Lys Tyr Gly Ser Pro Asn
                485                 490                 495
Ala Ile His Ile Glu Thr Ala Arg Glu Leu Ser Lys Thr Phe Gln Glu
            500                 505                 510
Arg Lys Glu Ile Asp Ser Met Tyr Gln Asp Asn Ser Lys Lys Asn Glu
        515                 520                 525
His Ala Ile His Lys Leu Lys Glu Leu Gly Asn Ile Asn Pro Ser Gly
    530                 535                 540
Ile Asn Ile Val Lys Phe Lys Leu Trp Asn Glu Gln Asp Gly Arg Cys
545                 550                 555                 560
Met Tyr Ser Gly Lys Tyr Ile Glu Pro His Arg Leu Phe Glu Glu Gly
                565                 570                 575
Tyr Thr Glu Val Asp His Ile Leu Pro Tyr Ser Arg Ser Leu Asp Asp
            580                 585                 590
Ser Tyr Asn Asn Lys Thr Leu Thr Leu Gly Thr Glu Asn Gln Arg Lys
        595                 600                 605
Gly Asn Lys Thr Pro Tyr Glu Tyr Met Gly Asn Thr Ser Ile Trp Asn
    610                 615                 620
Asp Phe Glu Thr Arg Val His Ser Asn Lys Lys Ile Asn Lys Lys Lys
625                 630                 635                 640
Gln Gln Lys Leu Leu Leu Gln His Phe Ser Tyr Ala Arg Glu Gln Glu
                645                 650                 655
Phe Ile Glu Arg Asn Leu Asn Asp Thr Arg Tyr Ala Thr Ile Tyr Leu
            660                 665                 670
Ser Ser Leu Ile Gln Gln His Leu Ile Phe Ser Glu Ser Ser Arg Lys
        675                 680                 685
Lys Lys Val His Thr Val Ser Gly Ile Ile Thr Ser His Leu Arg Ser
    690                 695                 700
Arg Trp Gly Phe Asn Lys Asp Arg Lys Glu Gly His Ile His His Ala
705                 710                 715                 720
Leu Asp Ala Val Ile Val Ala Val Thr Ser Asp His Met Ile Gln Arg
                725                 730                 735
Val Thr Lys Tyr Tyr Lys Leu Lys Glu Leu Asn Arg Asn Leu Gln Ala
            740                 745                 750
Lys Arg Met Pro Phe Pro Glu Pro Trp Glu Gly Phe Arg Leu Glu Leu
        755                 760                 765
Glu Ala Arg Ile Ser Pro Asn Thr Gln Gln Tyr Leu Lys Gly Phe Arg
    770                 775                 780
Phe Lys Asn Tyr Ala Asp Val Asn Leu Ser Glu Ile Lys Pro Ile Phe
785                 790                 795                 800
Val Ser Arg Met Pro Lys Arg Ser Ile Thr Gly Glu Leu His Gln Glu
                805                 810                 815
Thr Ile Arg Lys Phe Ile Gly Tyr Asn Glu Lys Gly Lys Val Leu Thr
            820                 825                 830
Ala Ile Lys Thr Lys Leu Glu Asp Ile Pro Phe Asp Ala Asn Gly Asp
        835                 840                 845
Phe Pro Met Tyr Gly Lys Glu Thr Asp Leu Tyr Thr Tyr Asn Ala Ile
    850                 855                 860
Lys Glu Arg Phe Leu Ser His Lys Lys Asp Lys Arg Lys Ser Phe Gln
865                 870                 875                 880
Glu Pro Leu Tyr Lys Pro Thr Lys Ser Gly Gly Ile Gly Pro Leu Ile
                885                 890                 895
Lys Ser Ile Lys Ile Ile Asp Thr Arg Thr Ile Val Asn Pro Val Asn
            900                 905                 910
Gln Gly Lys Gly Val Val Tyr Asn Ser Lys Ile Ala Arg Thr Asp Val
        915                 920                 925
Phe Lys Lys Asp Glu Lys Tyr Tyr Leu Ile Pro Ile Tyr Thr Ile Asp
    930                 935                 940
Leu Met Lys Asn Ile Leu Pro Gln Lys Ala Ile Thr Ala Gly Lys Gly
945                 950                 955                 960
Tyr Glu Asp Trp Thr Thr Ile Asp Pro Ser Phe Thr Phe Leu Phe Ser
                965                 970                 975

Leu Phe Pro Asn Asp Leu Ile Gln Ile Val Pro Ser Lys Asn Lys Thr
        980                 985                 990
Ile Lys Ala Arg Thr Thr Val Ser  Lys Lys Glu Val Leu  Leu Pro Ser
        995                 1000                1005
Leu Thr  Gly Tyr Phe Lys Gly  Val His Ser Gly Thr  Ala Gly Ile
    1010                1015                1020
Thr Val  Glu Thr His Asp Gly  Ser Val Ile Ala Asn  Val Gly Ser
    1025                1030                1035
Lys Gln  Leu Leu Leu Ile Glu  Lys Tyr Gln Val Asp  Ile Leu Gly
    1040                1045                1050
His Tyr  Thr Lys Ile Lys Glu  Glu Lys Arg Ile Gly  Met Ala Ile
    1055                1060                1065

<210>  15
<211>  1068
<212>  PRT
<213>  Artificial sequence

<220>
<223>  Cas-Locus 5

<400>  15
Met Lys Tyr Val Leu Gly Leu Asp Ile Gly Ile Ala Ser Cys Gly Trp
1               5                   10                  15
Ala Val Ile Asn Gln Glu Lys His Arg Ile Glu Asp Leu Gly Val Arg
            20                  25                  30
Ile Phe Asp Lys Ala Glu Asn Pro Lys Asp Gly Lys Ser Leu Ala Thr
        35                  40                  45
Pro Arg Arg Asp Ala Arg Ser Thr Arg Arg Thr Leu Arg Arg Lys Lys
    50                  55                  60
His Arg Met Gln Arg Ile Lys Ile Leu Leu Val Lys His Gly Leu Leu
65                  70                  75                  80
Ser Lys Thr Glu Ile Asp His Leu Tyr Glu Ser Ala Thr Glu Ile Asp
                85                  90                  95
Val Trp Tyr Leu Arg Leu Asn Ala Leu Glu Arg Arg Leu Asn Pro Lys
            100                 105                 110
Glu Phe Ala Arg Val Leu Ile His Leu Ala Lys Arg Arg Gly Phe Lys
        115                 120                 125
Ser Asn Arg Lys Glu Thr Thr Leu Ser Glu Asn Gly Lys Ile Leu Glu
    130                 135                 140
Asn Ile Ser Glu Asn Leu Gln Ile Met Glu Gln Lys Asn Tyr Arg Thr
145                 150                 155                 160
Val Gly Glu Met Ile Leu Lys Asp Lys Lys Phe Glu Asn His Lys Arg
                165                 170                 175
Asn Lys Asp Gly Thr Tyr Ile Gly Thr Val Thr Arg Gln Gln Leu Gln
            180                 185                 190
Glu Glu Ile Gln Met Ile Phe Asn Ala Gln Arg Leu Tyr Lys Asn Asn
        195                 200                 205
His Ala Thr Glu Lys Phe Glu Ser Ser Tyr Leu Glu Ile Trp Ala Ser
    210                 215                 220
Gln Arg Pro Tyr Ala Ser Lys Asp Gln Ile Glu Lys Met Ile Gly Tyr
225                 230                 235                 240
Cys Thr Leu Glu Pro Lys Glu Lys Arg Val Pro Lys Ala Ser Trp Ser
                245                 250                 255
Phe Gln Tyr Phe Val Ala Leu Gln Thr Ile Asn Asn Leu Arg Leu Ile
            260                 265                 270
Ser Lys Asp Arg Ile Glu Glu Leu Ser Phe Glu Glu Lys Lys Arg Ile
        275                 280                 285
Met Asn Leu Ala Leu Glu Lys Ser Ile Val Lys Tyr Ile Asp Ile Arg
    290                 295                 300
Lys Leu Leu Ser Val Pro Asn Glu Phe Gln Phe Asn Ser Leu Ser Tyr
305                 310                 315                 320
Ser Ala Asp Ile Val Asp Thr Thr Val Glu Asn Lys Lys Cys Ile Glu

                325                 330                 335
Leu Lys Glu Tyr His Ser Ile Asn Lys Val Tyr Lys Gln Ile Tyr Glu
            340                 345                 350
Gln Asn Ala Leu Asn Leu Leu Pro Lys Asp Tyr Asp Thr Ile Ala Tyr
        355                 360                 365
Gly Leu Thr Ile Phe Lys Asp Asp Lys Asp Ile Leu Glu Tyr Leu Gln
    370                 375                 380
Asn Lys Tyr Val Asn Ala Lys Gly Lys Pro Ile Ser Asn Phe Ala Asn
385                 390                 395                 400
Lys Thr Tyr Asp Asp Thr Phe Ile Gln Val Leu Leu Thr Leu Asn Phe
                405                 410                 415
Ser Lys Met Gly His Leu Ser Phe Lys Ala Leu Lys Asn Ile Ile Pro
            420                 425                 430
Phe Leu Glu Glu Gly Leu Ser Tyr Asp Lys Ala Cys Glu Lys Ala Gly
        435                 440                 445
Tyr Asn Phe Lys Gly Thr Ser Tyr Thr Lys Lys Thr Lys Tyr Leu Pro
    450                 455                 460
Val Ile Pro Gln Asn Thr Asn Pro Val Val His Arg Ala Leu Ser Gln
465                 470                 475                 480
Thr Arg Lys Val Ile Asn Ala Ile Ile Lys Lys Tyr Gly Ser Pro Asn
                485                 490                 495
Ala Ile His Ile Glu Thr Ala Arg Glu Leu Ser Lys Thr Phe Gln Glu
            500                 505                 510
Arg Lys Glu Ile Asp Ser Met Tyr Gln Asp Asn Ser Lys Lys Asn Glu
        515                 520                 525
His Ala Ile His Lys Leu Lys Glu Leu Gly Asn Ile Asn Pro Ser Gly
    530                 535                 540
Ile Asn Ile Val Lys Phe Lys Leu Trp Asn Glu Gln Asp Ser Arg Cys
545                 550                 555                 560
Met Tyr Ser Gly Lys Tyr Ile Glu Pro His Arg Leu Phe Glu Glu Gly
                565                 570                 575
Tyr Thr Glu Val Asp His Ile Leu Pro Tyr Ser Arg Ser Leu Asp Asp
            580                 585                 590
Ser Tyr Asn Asn Lys Val Leu Thr Leu Gly Thr Glu Asn Gln Gly Lys
        595                 600                 605
Gly Asn Arg Thr Pro Tyr Glu Tyr Met Gly Asn Thr Ser Ile Trp Asp
    610                 615                 620
Glu Phe Glu Thr Arg Val Gln Ser Asn Lys Lys Ile Asn Lys Lys Lys
625                 630                 635                 640
Gln Gln Lys Leu Leu Leu Gln Asn Phe Ser His Ala Arg Glu Gln Glu
                645                 650                 655
Phe Ile Glu Arg Asn Leu Asn Asp Thr Arg Tyr Ala Thr Ile Tyr Ile
            660                 665                 670
Ser Ser Leu Ile Gln Gln His Leu Ile Phe Ser Glu Ser Ser Arg Glu
        675                 680                 685
Lys Lys Val His Thr Val Ser Gly Ile Ile Thr Ser His Leu Arg Ser
    690                 695                 700
Arg Trp Gly Phe Asn Lys Asp Arg Lys Glu Gly His Ile His His Ala
705                 710                 715                 720
Leu Asp Ala Val Ile Val Ala Val Thr Ser Asp His Met Ile Gln Arg
                725                 730                 735
Val Thr Lys Tyr Tyr Lys Leu Lys Glu Leu Asn Arg Asn Arg Gln Ala
            740                 745                 750
Lys Arg Met Gln Phe Pro Glu Pro Trp Glu Gly Phe Arg Leu Glu Leu
        755                 760                 765
Glu Ala Arg Ile Ser Pro Asn Thr Gln Gln Tyr Leu Lys Gly Ile Arg
    770                 775                 780
Leu Lys Asn Tyr Ala Asp Val Asn Leu Ser Glu Ile Lys Pro Ile Phe
785                 790                 795                 800
Val Ser Arg Met Pro Lys Arg Ser Ile Thr Gly Glu Leu His Gln Glu
                805                 810                 815
Thr Ile Arg Lys Phe Leu Gly Tyr Asn Glu Lys Gly Lys Val Leu Thr
            820                 825                 830

Ala Ile Lys Thr Lys Leu Glu Asp Ile Pro Phe Asp Ala Asn Gly Asp
        835                 840                 845
Phe Pro Met Tyr Gly Lys Glu Thr Asp Leu Tyr Thr Tyr Asn Ala Ile
    850                 855                 860
Lys Glu Arg Tyr Leu Ser His Lys Lys Asp Lys Arg Lys Ser Phe Gln
865                 870                 875                 880
Glu Pro Leu Tyr Lys Pro Thr Lys Ser Gly Gly Ile Gly Pro Leu Ile
                885                 890                 895
Lys Ser Ile Lys Ile Met Asp Thr Arg Thr Ile Val Asn Pro Val Asn
            900                 905                 910
Gln Gly Lys Gly Val Val Tyr Asn Ser Lys Ile Ala Arg Thr Asp Val
        915                 920                 925
Phe Lys Lys Ala Glu Lys Tyr Tyr Leu Ile Pro Ile Tyr Thr Ile Asp
    930                 935                 940
Leu Met Lys Asn Ile Leu Pro Gln Lys Ala Ile Thr Ala Gly Lys Gly
945                 950                 955                 960
Tyr Glu Asp Trp Thr Thr Ile Asp Pro Ser Phe Thr Phe Leu Phe Ser
                965                 970                 975
Leu Phe Pro Asn Asp Leu Ile Gln Ile Val Pro Ser Lys Asn Lys Ile
            980                 985                 990
Ile Lys Ala Arg Thr Thr Val Ser  Lys Lys Glu Val Leu  Leu Pro Ser
        995                 1000                1005
Leu Thr  Gly Tyr Phe Lys Gly  Val His Ser Gly Thr  Ala Gly Ile
    1010                1015                1020
Thr Val  Glu Thr His Asp Gly  Ser Ile Ile Ala Asn  Val Gly Ser
    1025                1030                1035
Lys Gln  Leu Leu Ser Phe Glu  Lys Tyr Gln Val Asp  Val Leu Gly
    1040                1045                1050
His Tyr  Thr Lys Ile Lys Lys  Glu Lys Arg Ile Gly  Met Ala Ile
    1055                1060                1065

<210>  16
<211>  36
<212>  DNA
<213>  Artificial sequence

<220>
<223>  CRISPR repeat consensus Locus 1 (Bacillus species)

<400>  16
atcataatac agcaatggct twaatggaac tatgac                                36

<210>  17
<211>  36
<212>  DNA
<213>  Artificial sequence

<220>
<223>  CRISPR repeat consensus Locus 2 (Bacillus species)

<400>  17
atcataaaac agcaatggct taaatggaac tatgac                                36

<210>  18
<211>  36
<212>  DNA
<213>  Artificial sequence

<220>
<223>  CRISPR repeat consensus Locus 3 (Bacillus species)

<400>  18
accatagcat agcgatgttt cttatggaac tatagc                                36

<210>  19
<211>  36
<212>  DNA
<213>  Artificial sequence

<220>
<223>  CRISPR repeat consensus Locus 4 (Bacillus species)

<400>  19
atcatatcat agcgatrttt cttatggaac tatagc                                36

<210>  20
<211>  36
<212>  DNA
<213>  Artificial sequence

<220>
<223>  CRISPR repeat consensus Locus 5

<400>  20
accatagcat agcgatgttt cttatggaac tatagc                                36

<210>  21
<211>  36
<212>  DNA
<213>  Artificial sequence

<220>
<223>  Anti repeat Locus 1

<400>  21
atcataatac agcaatggct ttgatgtttc tatgat                                36

<210>  22
<211>  36
<212>  DNA
<213>  Artificial sequence

<220>
<223>  Anti repeat Locus 2

<400>  22
atcataaaac agcaatggct ttgatgtttc tatgat                                36

<210>  23
<211>  36
<212>  DNA
<213>  Artificial sequence

<220>
<223>  Anti repeat Locus 3

<400>  23
atcatatcat agcgatgttt cttaagttac tatagt                                36

<210>  24
<211>  36
<212>  DNA
<213>  Artificial sequence

<220>
<223>  Anti repeat Locus 4

<400>  24
atcatatcat agcgatgttt cttaagttac tatagt                                36

<210>  25
<211>  36
<212>  DNA
<213>  Artificial sequence

<220>
<223>  Anti repeat Locus 5

<400>  25
atcatatcat agcgatgttt cttaagttac tatagt                                36

<210>  26
<211>  19
<212>  RNA
<213>  Artificial sequence

<220>
<223>  sgRNA repeat region for Cas-Locus 1

<400>  26
gucauaguuc cauuwaagc                                                   19

<210>  27
<211>  19
<212>  RNA
<213>  Artificial sequence

<220>
<223>  sgRNA repeat region for Cas-Locus 2

<400>  27
gucauaguuc cauuuaagc                                                   19

<210>  28
<211>  20
<212>  RNA
<213>  Artificial sequence

<220>
<223>  sgRNA repeat region for Cas-Locus 3

<400>  28
gcuauaguuc cauaagaaac                                                  20

<210>  29
<211>  20
<212>  RNA
<213>  Artificial sequence

<220>
<223>  sgRNA repeat region for Cas-Locus 4

<400>  29
gcuauaguuc cauaagaaay                                                  20

<210>  30
<211>  20
<212>  RNA
<213>  Artificial sequence

<220>
<223>  sgRNA repeat region for Cas-Locus 5

<400>  30
gcuauaguuc cauaagaaac                                                   20

<210>  31
<211>  19
<212>  RNA
<213>  Artificial sequence

<220>
<223>  sgRNA anti-repeat region for Cas-Locus 1

<400>  31
gcuuugaugu uucuaugau                                                    19

<210>  32
<211>  19
<212>  RNA
<213>  Artificial sequence

<220>
<223>  sgRNA anti-repeat region for Cas-Locus 2

<400>  32
gcuuugaugu uucuaugau                                                    19

<210>  33
<211>  20
<212>  RNA
<213>  Artificial sequence

<220>
<223>  sgRNA anti-repeat region for Cas-Locus 3

<400>  33
guuucuuaag uuacuauagu                                                   20

<210>  34
<211>  20
<212>  RNA
<213>  Artificial sequence

<220>
<223>  sgRNA anti-repeat region for Cas-Locus 4

<400>  34
guuucuuaag uuacuauagu                                                   20

<210>  35
<211>  20
<212>  RNA
<213>  Artificial sequence

<220>
<223>  sgRNA anti-repeat region for Cas-Locus 5

<400>  35
guuucuuaag uuacuauagu                                                   20

<210>  36

<211>  114
<212>  RNA
<213>  Artificial sequence

<220>
<223>   3 prime tracrRNA for Cas-Locus 1

<400>  36
aagggcuuag gcccguggcg uuggggaucg ccugcccauu uuaaugggcu ucuccccacc      60
uauuuaauga gaauuuuaca accuuggcua uucuuaaaua gcuaagguuu uuuu           114

<210>  37
<211>  114
<212>  RNA
<213>  Artificial sequence

<220>
<223>   3 prime tracrRNA for Cas-Locus 2

<400>  37
aagggcuuag gcccguggcg uuggggaucg ccugcccauu uuuaugggcu ucuccccauc      60
uauuuaauga aaauuuuaca accuuggcua uucuuaaaua gcuaagguuu uuuu           114

<210>  38
<211>  101
<212>  RNA
<213>  Artificial sequence

<220>
<223>   3 prime tracrRNA for Cas-Locus 3

<400>  38
aagggcaaug acccguggcg uuuggggauc gccuuauccu gguauggaua uucuccccau      60
gugaaaagca ccuaagcaua gcgcuauggu gcuuuuauuu u                         101

<210>  39
<211>  100
<212>  RNA
<213>  Artificial sequence

<220>
<223>   3 prime tracrRNA for Cas-Locus 4

<400>  39
aagggcaaug acccguggcg uuuggagauc gccucaucca uuacggauau ucuccccaug      60
ugaaaagcac cuaagcauaa ggcuaaggug cuuuuauuuu                           100

<210>  40
<211>  101
<212>  RNA
<213>  Artificial sequence

<220>
<223>   3 prime tracrRNA for Cas-Locus 5

<400>  40
aagggcaaug acccguggcg uuuggggauc gccuuauccu gguauggaua uucuccccau      60
gugaaaagca ccuaagcaua gcgcuauggu gcuuuuauuu u                         101

<210>  41
<211>  176
<212>  RNA
<213>  Artificial Sequence

<220>
<223>  sgRNA for Cas-Locus 1

<220>
<221>  misc_feature
<222>  (1)..(20)
<223>  n is a, c, g, or u

<400>  41
nnnnnnnnnn nnnnnnnnnn gucauaguuc cauuwaagcg aaagcuuuga uguuucuaug      60
auaagggcuu aggcccgugg cguuggggau cgccugccca uuuuaauggg cuucucccca     120
ccuauuuaau gagaauuuua caaccuuggc uauucuuaaa uagcuaaggu uuuuuu         176

<210>  42
<211>  176
<212>  RNA
<213>  Artificial Sequence

<220>
<223>  sgRNA for Cas-Locus 2

<220>
<221>  misc_feature
<222>  (1)..(20)
<223>  n is a, c, g, or u

<400>  42
nnnnnnnnnn nnnnnnnnnn gucauaguuc cauuuaagcg aaagcuuuga uguuucuaug      60
auaagggcuu aggcccgugg cguuggggau cgccugccca uuuuuauggg cuucucccca     120
ucuauuuaau gaaaauuuua caaccuuggc uauucuuaaa uagcuaaggu uuuuuu         176

<210>  43
<211>  165
<212>  RNA
<213>  Artificial Sequence

<220>
<223>  sgRNA for Cas-Locus 3

<220>
<221>  misc_feature
<222>  (1)..(20)
<223>  n is a, c, g, or u

<400>  43
nnnnnnnnnn nnnnnnnnnn gcuauaguuc cauaagaaac gaaaguuucu uaaguuacua      60
uaguaagggc aaugacccgu ggcguuuggg gaucgccuua uccugguaug gauauucucc     120
ccaugugaaa agcaccuaag cauagcgcua uggugcuuuu auuuu                     165

<210>  44
<211>  164
<212>  RNA
<213>  Artificial Sequence

<220>
<223>  sgRNA for Cas-Locus 4

<220>
<221>  misc_feature
<222>  (1)..(20)
<223>  n is a, c, g, or u

<400>  44
nnnnnnnnnn nnnnnnnnnn gcuauaguuc cauaagaaay gaaaguuucu uaaguuacua      60
uaguaagggc aaugacccgu ggcguuugga gaucgccuca uccauuacgg auauucuccc     120
caugugaaaa gcaccuaagc auaaggcuaa ggugcuuuua uuuu                      164

<210>  45
<211>  165
<212>  RNA
<213>  Artificial Sequence

<220>
<223>  sgRNA for Cas-Locus 5

<220>
<221>  misc_feature
<222>  (1)..(20)
<223>  n is a, c, g, or u

<400>  45
nnnnnnnnnn nnnnnnnnnn gcuauaguuc cauaagaaac gaaaguuucu uaaguuacua      60
uaguaagggc aaugacccgu ggcguuuggg gaucgccuua uccugguaug gauauucucc     120
ccaugugaaa agcaccuaag cauagcgcua uggugcuuuu auuuu                     165

<210>  46
<211>  3213
<212>  DNA
<213>  Unknown

<220>
<223>  Cas9 endonuclease of Locus 3 (Cas-Locus 3)  open reading frame
       (ORF)-2 from Bacillus species

<400>  46
atgaaattga aatatgtatt agggttggat attggaatcg catcatgtgg ttgggcagta      60
ataaatcaag aaaaacatag aattgaagat ttaggggttc gtatttttga taaagctgag     120
aatcccaaag atggaaaatc tttagctaca cctagacgtg acgcacgctc taccagaaga     180
accctacgcc gtaaaaaaca tagaatgcaa cggataaaga tacttttagt aaagcacggc     240
ttactatcca agaccgaaat cgaccatcta tatgaaagtg ctactgagat cgatgtttgg     300
tacttacgtt taaatgctct agagcgcaga ttaaatccaa aagagtttgc tagagttctt     360
attcaccttg ctaaaagaag aggatttaaa tctaatagaa aagaaacaac tctctcggaa     420
aacggacaaa tcttagaaaa tatttcagag aacctgcaga ttatggagca aaaaaattat     480
agaactgttg gagaaatgat tctaaaagat aaaagatttg aaaatcataa aagaaacaaa     540
gatggtactt atatcgggac tgttactcgg caacaacttc aagaagaaat acaaatgata     600
ttcaatgcac aacgtttgta caaaaacaat catgcaacag agaaatttga aagtagctac     660
ttagaaatct gggcatctca acgtccctat gcttctaaag atcaaattga aaaaatgatt     720
ggttactgta ccttagagcc aaaagaaaaa agagtgccta aagcttcttg gtcatttcaa     780
tattttgtag cgctacaaac cataaacaac ctccgactga tcagtaaaga caggatagaa     840
gaactctctt ttgaagaaaa gaaacgaatt atgaatctag cactcgaaaa atcaattgtt     900
aaatacattg atatccgaaa attactatct gttcctaatg aatttcaatt taatagcctt     960
tcatattcag ctgatatagt agatacaacg gtggaaaata aaaaatgtat agaactcaaa    1020
gaatatcact ctattaacaa ggtatataaa caaatttacg gacagaacgc tctaaattta    1080
ttacctaaag attacgatac aattgcatac ggacttacta tctttaaaga tgataaagat    1140
atcctagaat atttacaaaa taaatacgta aatgcaaaag gaaaacctat aagtaattta    1200
gctaacaaga cctacgatga tacatttatt caggtattat tgactctgaa tttctcaaaa    1260
atgggacatt tatcatttaa agctttaaaa aatattattc cttttttaga agaaggtcta    1320
tcctacgata aagcatgtga gaaggcagga tacaacttta agggagcatc ctacacaaaa    1380
aaaacaaagt acctacctgt tatccctcag aatacaaatc ctgttgtaca tcgtgctctt    1440
tcacaaacaa gaaaagttat aaatgccatt ataaaaaaat atggttctcc aaacgcaatc    1500
catattgaaa ctgcaagaga attatcgaaa acattccaag aaagaaaaga aatagatagt    1560
atgtaccagg ataactctaa aaagaatgaa cacgccattt gtaagttaaa agaattgggg    1620
aatattaaac cctcaggaat aaatattgtg aaatttaagc tttggaacga gcaagatggt    1680
agatgtatgt attctggaaa atatattgaa ccacatcgct tatttgaaga aggttatacg    1740
gaagtagatc atatactccc ttacagccgt agcttagatg acagctataa caataaagta    1800

ttaacgctag gaacagaaaa tcagcgtaaa ggaaacaaaa ctccttatga atacatgagg 1860
aataattcaa tttgggatga atttgaaaca agagtacagt caaataaaaa aatcaacaaa 1920
aagaaacaac aaaaattact tttacaaaac ttttcacatg cgagagaaca agaatttatt 1980
gaacgcaatc tcaatgatac ccgttatgcc acaatatatc tatcatccct tattcaacaa 2040
cacctcatat tctctgaaag ctctcgtgag aaaaaagttc atacagttag tggaattata 2100
acatctcatt tgcgtagtag atggggattt aacaaagatc ggaaagaggg gcatatccat 2160
catgctttag atgcggtaat cgttgctgtc acatccgatc acatgattca aagagtaaca 2220
aaatattata agttaaaaga gttaaataga aatattcaag ccaaacggat gcagtttcct 2280
gaaccttggg aaggatttcg tttagagtta gaagcaagag tttctccaaa tacccagcaa 2340
tatttaaagg gtattcgttt taaaaattac gccgatgtcg atctatccga aatcaaacct 2400
atctttgttt caagaatgcc taagcgctct attactggtg aattacatca agaaacaatc 2460
cggaaattcc ttggttataa cgaaaagggg aaagtgctaa ccgcgacaaa aacaaaatta 2520
gaagatattc cttttgatgc taatggtgat tttcccatgt ttggaaaaga aactgattta 2580
tatacgtata atgccataaa agaacgttat ttaagtcata aaaaagataa acgcaaatca 2640
ttccaagagc ctttatacaa acccacaaaa tcaggaggaa ttggaccact tataaaatct 2700
attaaaataa tggatacaag aaccattgtt aatcctgtta atcaaggtaa aggtgtcgtg 2760
tataatagta aaattgctcg tactgatgta ttcaaaaagg gtgagaagta ctatttaatt 2820
cccatttaca caattgatct tatgaaaaat atcttacctc aaaaagcaat tacagcagga 2880
aaaggatatg aggattggac aactattgat cctagcttta cttttttgtt tagtcttttt 2940
cccaatgatc taatccaaat tgtcccctca aaaaacaaaa caattaaagc tagaacaact 3000
gtatcgaaaa aagaggtatt actaccctca ctcacaggat attttaaagg tgttcatagt 3060
ggaactgctg ggattactgt tgaaacacat gatggcagta ttatagcaaa tgtaggctcc 3120
aagcagttac tatcatttga gaaataccag gtagatgtac ttggacatta cacaaaaatt 3180
aagaaggaaa aacgtattgg aatggcaata tag 3213

<210> 47
<211> 3225
<212> DNA
<213> Unknown

<220>
<223> Cas9 endonuclease of Locus 3 (Cas-Locus 3) ORF3, from Bacillus
      species

<400> 47
atgaaggagg ttatgaaatt gaaatatgta ttagggttgg atattggaat cgcatcatgt 60
ggttgggcag taataaatca agaaaaacat agaattgaag atttaggggt tcgtattttt 120
gataaagctg agaatcccaa agatggaaaa tctttagcta cacctagacg tgacgcacgc 180
tctaccagaa gaaccctacg ccgtaaaaaa catagaatgc aacggataaa gatactttta 240
gtaaagcacg gcttactatc caagaccgaa atcgaccatc tatatgaaag tgctactgag 300
atcgatgttt ggtacttacg tttaaatgct ctagagcgca gattaaatcc aaaagagttt 360
gctagagttc ttattcacct tgctaaaaga agaggattta aatctaatag aaaagaaaca 420
actctctcgg aaaacggaca aatcttagaa aatatttcag agaacctgca gattatggag 480
caaaaaaatt atagaactgt tggagaaatg attctaaaag ataaaagatt tgaaaatcat 540
aaaagaaaca aagatggtac ttatatcggg actgttactc ggcaacaact tcaagaagaa 600
atacaaatga tattcaatgc acaacgtttg tacaaaaaca atcatgcaac agagaaattt 660
gaaagtagct acttagaaat ctgggcatct caacgtccct atgcttctaa agatcaaatt 720
gaaaaaatga ttggttactg taccttagag ccaaaagaaa aaagagtgcc taaagcttct 780
tggtcatttc aatattttgt agcgctacaa accataaaca acctccgact gatcagtaaa 840
gacaggatag aagaactctc ttttgaagaa aagaaacgaa ttatgaatct agcactcgaa 900
aaatcaattg ttaaatacat tgatatccga aaattactat ctgttcctaa tgaatttcaa 960
tttaatagcc tttcatattc agctgatata gtagatacaa cggtggaaaa taaaaaatgt 1020
atagaactca aagaatatca ctctattaac aaggtatata aacaaattta cggacagaac 1080
gctctaaatt tattacctaa agattacgat acaattgcat acggacttac tatctttaaa 1140
gatgataaag atatcctaga atatttacaa aataaatacg taaatgcaaa aggaaaacct 1200
ataagtaatt tagctaacaa gacctacgat gatacattta ttcaggtatt attgactctg 1260
aatttctcaa aaatgggaca tttatcattt aaagctttaa aaaatattat tcctttttta 1320
gaagaaggtc tatcctacga taaagcatgt gagaaggcag gatacaactt taagggagca 1380
tcctacacaa aaaaaacaaa gtacctacct gttatccctc agaatacaaa tcctgttgta 1440
catcgtgctc tttcacaaac aagaaaagtt ataaatgcca ttataaaaaa atatggttct 1500
ccaaacgcaa tccatattga aactgcaaga gaattatcga aaacattcca agaaagaaaa 1560
gaaatagata gtatgtacca ggataactct aaaaagaatg aacacgccat ttgtaagtta 1620
aaagaattgg ggaatattaa accctcagga ataaatattg tgaaatttaa gctttggaac 1680

gagcaagatg gtagatgtat gtattctgga aaatatattg aaccacatcg cttatttgaa 1740
gaaggttata cggaagtaga tcatatactc ccttacagcc gtagcttaga tgacagctat 1800
aacaataaag tattaacgct aggaacagaa aatcagcgta aaggaaacaa aactccttat 1860
gaatacatga ggaataattc aatttgggat gaatttgaaa caagagtaca gtcaaataaa 1920
aaaatcaaca aaaagaaaca acaaaaatta cttttacaaa acttttcaca tgcgagagaa 1980
caagaattta ttgaacgcaa tctcaatgat acccgttatg ccacaatata tctatcatcc 2040
cttattcaac aacacctcat attctctgaa agctctcgtg agaaaaaagt tcatacagtt 2100
agtggaatta taacatctca tttgcgtagt agatggggat ttaacaaaga tcggaaagag 2160
gggcatatcc atcatgcttt agatgcggta atcgttgctg tcacatccga tcacatgatt 2220
caaagagtaa caaaatatta taagttaaaa gagttaaata gaaatattca agccaaacgg 2280
atgcagtttc ctgaaccttg ggaaggattt cgtttagagt tagaagcaag agtttctcca 2340
aatacccagc aatatttaaa gggtattcgt tttaaaaatt acgccgatgt cgatctatcc 2400
gaaatcaaac ctatctttgt ttcaagaatg cctaagcgct ctattactgg tgaattacat 2460
caagaaacaa tccggaaatt ccttggttat aacgaaaagg ggaaagtgct aaccgcgaca 2520
aaaacaaaat tagaagatat tccttttgat gctaatggtg attttcccat gtttggaaaa 2580
gaaactgatt tatatacgta taatgccata aaagaacgtt atttaagtca taaaaaagat 2640
aaacgcaaat cattccaaga gcctttatac aaacccacaa aatcaggagg aattggacca 2700
cttataaaat ctattaaaat aatggataca agaaccattg ttaatcctgt taatcaaggt 2760
aaaggtgtcg tgtataatag taaaattgct cgtactgatg tattcaaaaa gggtgagaag 2820
tactatttaa ttcccattta cacaattgat cttatgaaaa atatcttacc tcaaaaagca 2880
attacagcag gaaaaggata tgaggattgg acaactattg atcctagctt tacttttttg 2940
tttagtcttt ttcccaatga tctaatccaa attgtcccct caaaaaacaa aacaattaaa 3000
gctagaacaa ctgtatcgaa aaaagaggta ttactaccct cactcacagg atattttaaa 3060
ggtgttcata gtggaactgc tgggattact gttgaaacac atgatggcag tattatagca 3120
aatgtaggct ccaagcagtt actatcattt gagaaatacc aggtagatgt acttggacat 3180
tacacaaaaa ttaagaagga aaaacgtatt ggaatggcaa tatag 3225

<210> 48
<211> 3213
<212> DNA
<213> Unknown

<220>
<223> Cas9 endonuclease of Locus 5 (Cas-Locus 5) ORF2, from Bacillus
 species

<400> 48
atgaaattga aatatgtatt agggttggat attggaattg catcatgtgg ttgggcagta 60
ataaatcaag aaaaacatag aattgaagat ttaggggttc gtatttttga taaagctgag 120
aatcccaaag atggaaaatc tttagctaca cctagacgtg acgcacgttc taccagaaga 180
accctacgcc gtaaaaaaca tagaatgcaa cggataaaga tacttttagt aaagcacggc 240
ttactatcca agaccgaaat cgaccatcta tatgaaagtg ctactgagat cgatgtttgg 300
tacttacgtt taaatgctct agagcgcaga ttaaatccaa aagagtttgc tagagttctt 360
attcaccttg ctaaaagaag aggatttaaa tctaatagaa aagaaacaac tctctcggaa 420
aacggaaaaa tcttagaaaa tatttcagag aacctgcaga ttatggagca aaaaaattat 480
agaactgttg gagaaatgat tctaaaagat aaaaaatttg aaaatcataa aagaaacaaa 540
gatggtactt atatcgggac tgttactcgg caacaacttc aagaagaaat acaaatgata 600
ttcaatgcac aacgtttgta caaaaacaat catgcaacag agaaatttga aagtagctac 660
ttagaaatct gggcatctca acgtccctat gcttctaaag atcaaattga aaaaatgatt 720
ggttactgta ccttagagcc aaaagaaaaa agagtgccta aagcttcttg gtcatttcaa 780
tattttgtag cgctacaaac cataaacaac ctccgactga tcagtaaaga caggatagaa 840
gaactctctt ttgaagaaaa gaaacgaatt atgaatctag cactcgaaaa atcaattgtt 900
aaatacattg atatccgaaa attactatct gttcctaatg aatttcaatt taatagcctt 960
tcatattcag ctgatatagt agatacaacg gtggaaaata aaaaatgtat agaactcaaa 1020
gaatatcact ctattaacaa ggtatataaa caaatttacg aacagaacgc tctaaattta 1080
ttacctaaag attacgatac aattgcatac ggacttacta tctttaaaga tgataaagat 1140
atcctagaat atttacaaaa taaatacgta aatgcaaaag gaaaacctat aagtaatttt 1200
gctaacaaga cctacgatga tacatttatt caggtattat tgactctgaa tttctcaaaa 1260
atgggacatt tatcatttaa agctttaaaa aatattattc cttttttaga agaaggtcta 1320
tcctacgata aagcatgtga gaaggcagga tacaacttta agggaacatc ctacacaaaa 1380
aaaacaaagt acctacctgt tatccctcag aatacaaatc ctgttgtaca tcgtgctctt 1440
tcacaaacaa gaaaagttat aaatgccatt ataaaaaaat atggttctcc aaacgcaatc 1500
catattgaaa ctgcaagaga attatcgaaa acatttcaag aaagaaaaga aatagatagt 1560

atgtaccagg ataactctaa aaagaatgaa cacgccattc ataagttaaa agaattgggg 1620
aacattaacc cctcaggaat aaatattgtg aaatttaagc tttggaatga gcaagatagt 1680
agatgtatgt attctggaaa atatatcgaa ccacatcgat tatttgaaga aggttatacg 1740
gaagtagatc atatactccc ctatagccgg agcttagatg acagctataa caataaagtg 1800
ttaacgctag ggacagaaaa tcagggtaaa ggaaacagaa ctccttatga atacatgggg 1860
aatacttcaa tttgggatga atttgaaaca agagtacagt caaataaaaa aatcaacaaa 1920
aagaaacaac aaaaattact tttacaaaac ttttcacatg cgagagaaca agaatttatt 1980
gaacgcaatc tcaatgatac ccgttatgcc acaatatata tatcatccct tattcaacaa 2040
cacctcatat tctctgaaag ctctcgtgag aaaaaagttc atacagttag tggaattata 2100
acatctcatt tgcgtagtag atggggattt aacaaagatc ggaaagaggg gcatatccat 2160
catgctttag atgcggtaat cgttgctgtc acatccgatc acatgattca aagagtaaca 2220
aaatattata agttaaaaga attaaataga aaccgtcaag ccaaacggat gcagtttcct 2280
gaaccttggg aaggatttcg tttagagtta gaagcaagga tttctccaaa tacccagcaa 2340
tacttaaagg gaattcgttt aaaaaattac gccgatgtca atctatctga aatcaaacct 2400
atctttgttt caagaatgcc taagcgctct attactggtg aattacatca agaaacaatc 2460
cggaaattcc ttggttataa cgaaaaaggg aaagtgctaa ccgcaataaa aacaaaatta 2520
gaagatattc cttttgatgc taatggtgat tttcccatgt atggaaaaga aactgattta 2580
tatacgtata atgccataaa agaacgttat ttaagtcata aaaaagataa acgcaaatca 2640
ttccaagaac ctttatacaa acccacaaaa tcaggaggaa ttggaccact tataaaatct 2700
attaaaataa tggatacaag aaccattgtt aatcctgtta atcaaggtaa aggtgtcgtg 2760
tataatagta aaattgctcg cactgatgta ttcaaaaagg ctgaaaagta ctatttaatt 2820
cccatttaca caattgatct tatgaaaaat atcttacctc aaaaagcaat tacagcagga 2880
aaaggatatg aggattggac aaccattgat cctagcttta cttttttgtt tagtcttttt 2940
cccaatgatc taatccaaat tgtcccctca aaaaataaaa taattaaagc tagaacaact 3000
gtatcgaaaa aagaggtatt actaccctca ctcacaggat attttaaagg tgttcatagt 3060
ggaactgctg ggattactgt tgaaacacac gatggcagta ttatagcaaa tgtaggctcc 3120
aagcagttac tatcatttga gaaataccag gtagatgtac ttggacatta cacaaaaatt 3180
aagaaggaaa aacgtattgg aatggcaata tag 3213

<210> 49
<211> 3225
<212> DNA
<213> Unknown

<220>
<223> Cas9 endonuclease of Locus 5 (Cas-Locus 5) ORF3, from Bacillus
species

<400> 49
atgaaggagg ttatgaaatt gaaatatgta ttagggttgg atattggaat tgcatcatgt 60
ggttgggcag taataaatca agaaaaacat agaattgaag atttaggggt tcgtattttt 120
gataaagctg agaatcccaa agatggaaaa tctttagcta cacctagacg tgacgcacgt 180
tctaccagaa gaaccctacg ccgtaaaaaa catagaatgc aacggataaa gatactttta 240
gtaaagcacg gcttactatc caagaccgaa atcgaccatc tatatgaaag tgctactgag 300
atcgatgttt ggtacttacg tttaaatgct ctagagcgca gattaaatcc aaaagagttt 360
gctagagttc ttattcacct tgctaaaaga agaggattta aatctaatag aaaagaaaca 420
actctctcgg aaaacggaaa aatcttagaa aatatttcag agaacctgca gattatggag 480
caaaaaaatt atagaactgt tggagaaatg attctaaaag ataaaaaatt tgaaaatcat 540
aaaagaaaca aagatggtac ttatatcggg actgttactc ggcaacaact tcaagaagaa 600
atacaaatga tattcaatgc acaacgtttg tacaaaaaca atcatgcaac agagaaattt 660
gaaagtagct acttagaaat ctgggcatct caacgtccct atgcttctaa agatcaaatt 720
gaaaaaatga ttggttactg taccttagag ccaaaagaaa aaagagtgcc taaagcttct 780
tggtcatttc aatattttgt agcgctacaa accataaaca acctccgact gatcagtaaa 840
gacaggatag aagaactctc ttttgaagaa aagaaacgaa ttatgaatct agcactcgaa 900
aaatcaattg ttaaatacat tgatatccga aaattactat ctgttcctaa tgaatttcaa 960
tttaatagcc tttcatattc agctgatata gtagatacaa cggtggaaaa taaaaaatgt 1020
atagaactca aagaatatca ctctattaac aaggtatata aacaaattta cgaacagaac 1080
gctctaaatt tattacctaa agattacgat acaattgcat acggacttac tatctttaaa 1140
gatgataaag atatcctaga atatttacaa aataaatacg taaatgcaaa aggaaaacct 1200
ataagtaatt ttgctaacaa gacctacgat gatacattta ttcaggtatt attgactctg 1260
aatttctcaa aaatgggaca tttatcattt aaagctttaa aaaatattat tcctttttta 1320
gaagaaggtc tatcctacga taaagcatgt gagaaggcag gatacaactt taagggaaca 1380
tcctacacaa aaaaaacaaa gtacctacct gttatccctc agaatacaaa tcctgttgta 1440

catcgtgctc tttcacaaac aagaaaagtt ataaatgcca ttataaaaaa atatggttct 1500
ccaaacgcaa tccatattga aactgcaaga gaattatcga aaacatttca agaaagaaaa 1560
gaaatagata gtatgtacca ggataactct aaaaagaatg aacacgccat tcataagtta 1620
aaagaattgg ggaacattaa cccctcagga ataaatattg tgaaatttaa gctttggaat 1680
gagcaagata gtagatgtat gtattctgga aaatatatcg aaccacatcg attatttgaa 1740
gaaggttata cggaagtaga tcatatactc ccctatagcc ggagcttaga tgacagctat 1800
aacaataaag tgttaacgct agggacagaa aatcagggta aaggaaacag aactccttat 1860
gaatacatgg ggaatacttc aatttgggat gaatttgaaa caagagtaca gtcaaataaa 1920
aaaatcaaca aaaagaaaca acaaaaatta cttttacaaa acttttcaca tgcgagagaa 1980
caagaattta ttgaacgcaa tctcaatgat acccgttatg ccacaatata tatatcatcc 2040
cttattcaac aacacctcat attctctgaa agctctcgtg agaaaaaagt tcatacagtt 2100
agtggaatta taacatctca tttgcgtagt agatggggat ttaacaaaga tcggaaagag 2160
gggcatatcc atcatgcttt agatgcggta atcgttgctg tcacatccga tcacatgatt 2220
caaagagtaa caaaatatta taagttaaaa gaattaaata gaaaccgtca agccaaacgg 2280
atgcagtttc ctgaaccttg ggaaggattt cgtttagagt tagaagcaag gatttctcca 2340
aatacccagc aatacttaaa gggaattcgt ttaaaaaatt acgccgatgt caatctatct 2400
gaaatcaaac ctatctttgt ttcaagaatg cctaagcgct ctattactgg tgaattacat 2460
caagaaacaa tccggaaatt ccttggttat aacgaaaaag ggaaagtgct aaccgcaata 2520
aaaacaaaat tagaagatat tccttttgat gctaatggtg attttcccat gtatggaaaa 2580
gaaactgatt tatatacgta taatgccata aaagaacgtt atttaagtca taaaaaagat 2640
aaacgcaaat cattccaaga acctttatac aaacccacaa aatcaggagg aattggacca 2700
cttataaaat ctattaaaat aatggataca agaaccattg ttaatcctgt taatcaaggt 2760
aaaggtgtcg tgtataatag taaaattgct cgcactgatg tattcaaaaa ggctgaaaag 2820
tactatttaa ttcccattta cacaattgat cttatgaaaa atatcttacc tcaaaaagca 2880
attacagcag gaaaaggata tgaggattgg acaaccattg atcctagctt tacttttttg 2940
tttagtcttt ttcccaatga tctaatccaa attgtcccct caaaaaataa aataattaaa 3000
gctagaacaa ctgtatcgaa aaaagaggta ttactaccct cactcacagg atattttaaa 3060
ggtgttcata gtggaactgc tgggattact gttgaaacac acgatggcag tattatagca 3120
aatgtaggct ccaagcagtt actatcattt gagaaatacc aggtagatgt acttggacat 3180
tacacaaaaa ttaagaagga aaaacgtatt ggaatggcaa tatag 3225

<210> 50
<211> 1070
<212> PRT
<213> Unknown

<220>
<223> Cas9 endonuclease of Locus 3 (Cas-Locus 3) open reading frame
(ORF)-2 from Bacillus species

<400> 50
Met Lys Leu Lys Tyr Val Leu Gly Leu Asp Ile Gly Ile Ala Ser Cys
1               5                   10                  15
Gly Trp Ala Val Ile Asn Gln Glu Lys His Arg Ile Glu Asp Leu Gly
            20                  25                  30
Val Arg Ile Phe Asp Lys Ala Glu Asn Pro Lys Asp Gly Lys Ser Leu
        35                  40                  45
Ala Thr Pro Arg Arg Asp Ala Arg Ser Thr Arg Arg Thr Leu Arg Arg
    50                  55                  60
Lys Lys His Arg Met Gln Arg Ile Lys Ile Leu Leu Val Lys His Gly
65                  70                  75                  80
Leu Leu Ser Lys Thr Glu Ile Asp His Leu Tyr Glu Ser Ala Thr Glu
                85                  90                  95
Ile Asp Val Trp Tyr Leu Arg Leu Asn Ala Leu Glu Arg Arg Leu Asn
            100                 105                 110
Pro Lys Glu Phe Ala Arg Val Leu Ile His Leu Ala Lys Arg Arg Gly
        115                 120                 125
Phe Lys Ser Asn Arg Lys Glu Thr Thr Leu Ser Glu Asn Gly Gln Ile
    130                 135                 140
Leu Glu Asn Ile Ser Glu Asn Leu Gln Ile Met Glu Gln Lys Asn Tyr
145                 150                 155                 160
Arg Thr Val Gly Glu Met Ile Leu Lys Asp Lys Arg Phe Glu Asn His
                165                 170                 175

Lys Arg Asn Lys Asp Gly Thr Tyr Ile Gly Thr Val Thr Arg Gln Gln
            180                 185                 190
Leu Gln Glu Glu Ile Gln Met Ile Phe Asn Ala Gln Arg Leu Tyr Lys
        195                 200                 205
Asn Asn His Ala Thr Glu Lys Phe Glu Ser Ser Tyr Leu Glu Ile Trp
    210                 215                 220
Ala Ser Gln Arg Pro Tyr Ala Ser Lys Asp Gln Ile Glu Lys Met Ile
225                 230                 235                 240
Gly Tyr Cys Thr Leu Glu Pro Lys Glu Lys Arg Val Pro Lys Ala Ser
                245                 250                 255
Trp Ser Phe Gln Tyr Phe Val Ala Leu Gln Thr Ile Asn Asn Leu Arg
            260                 265                 270
Leu Ile Ser Lys Asp Arg Ile Glu Glu Leu Ser Phe Glu Glu Lys Lys
        275                 280                 285
Arg Ile Met Asn Leu Ala Leu Glu Lys Ser Ile Val Lys Tyr Ile Asp
    290                 295                 300
Ile Arg Lys Leu Leu Ser Val Pro Asn Glu Phe Gln Phe Asn Ser Leu
305                 310                 315                 320
Ser Tyr Ser Ala Asp Ile Val Asp Thr Thr Val Glu Asn Lys Lys Cys
                325                 330                 335
Ile Glu Leu Lys Glu Tyr His Ser Ile Asn Lys Val Tyr Lys Gln Ile
            340                 345                 350
Tyr Gly Gln Asn Ala Leu Asn Leu Leu Pro Lys Asp Tyr Asp Thr Ile
        355                 360                 365
Ala Tyr Gly Leu Thr Ile Phe Lys Asp Asp Lys Asp Ile Leu Glu Tyr
    370                 375                 380
Leu Gln Asn Lys Tyr Val Asn Ala Lys Gly Lys Pro Ile Ser Asn Leu
385                 390                 395                 400
Ala Asn Lys Thr Tyr Asp Asp Thr Phe Ile Gln Val Leu Leu Thr Leu
                405                 410                 415
Asn Phe Ser Lys Met Gly His Leu Ser Phe Lys Ala Leu Lys Asn Ile
            420                 425                 430
Ile Pro Phe Leu Glu Glu Gly Leu Ser Tyr Asp Lys Ala Cys Glu Lys
        435                 440                 445
Ala Gly Tyr Asn Phe Lys Gly Ala Ser Tyr Thr Lys Lys Thr Lys Tyr
    450                 455                 460
Leu Pro Val Ile Pro Gln Asn Thr Asn Pro Val Val His Arg Ala Leu
465                 470                 475                 480
Ser Gln Thr Arg Lys Val Ile Asn Ala Ile Ile Lys Lys Tyr Gly Ser
                485                 490                 495
Pro Asn Ala Ile His Ile Glu Thr Ala Arg Glu Leu Ser Lys Thr Phe
            500                 505                 510
Gln Glu Arg Lys Glu Ile Asp Ser Met Tyr Gln Asp Asn Ser Lys Lys
        515                 520                 525
Asn Glu His Ala Ile Cys Lys Leu Lys Glu Leu Gly Asn Ile Lys Pro
    530                 535                 540
Ser Gly Ile Asn Ile Val Lys Phe Lys Leu Trp Asn Glu Gln Asp Gly
545                 550                 555                 560
Arg Cys Met Tyr Ser Gly Lys Tyr Ile Glu Pro His Arg Leu Phe Glu
                565                 570                 575
Glu Gly Tyr Thr Glu Val Asp His Ile Leu Pro Tyr Ser Arg Ser Leu
            580                 585                 590
Asp Asp Ser Tyr Asn Asn Lys Val Leu Thr Leu Gly Thr Glu Asn Gln
        595                 600                 605
Arg Lys Gly Asn Lys Thr Pro Tyr Glu Tyr Met Arg Asn Asn Ser Ile
    610                 615                 620
Trp Asp Glu Phe Glu Thr Arg Val Gln Ser Asn Lys Lys Ile Asn Lys
625                 630                 635                 640
Lys Lys Gln Gln Lys Leu Leu Leu Gln Asn Phe Ser His Ala Arg Glu
                645                 650                 655
Gln Glu Phe Ile Glu Arg Asn Leu Asn Asp Thr Arg Tyr Ala Thr Ile
            660                 665                 670
Tyr Leu Ser Ser Leu Ile Gln Gln His Leu Ile Phe Ser Glu Ser Ser

675                 680                 685
Arg Glu Lys Lys Val His Thr Val Ser Gly Ile Ile Thr Ser His Leu
    690                 695                 700
Arg Ser Arg Trp Gly Phe Asn Lys Asp Arg Lys Glu Gly His Ile His
705                 710                 715                 720
His Ala Leu Asp Ala Val Ile Val Ala Val Thr Ser Asp His Met Ile
                725                 730                 735
Gln Arg Val Thr Lys Tyr Tyr Lys Leu Lys Glu Leu Asn Arg Asn Ile
            740                 745                 750
Gln Ala Lys Arg Met Gln Phe Pro Glu Pro Trp Glu Gly Phe Arg Leu
        755                 760                 765
Glu Leu Glu Ala Arg Val Ser Pro Asn Thr Gln Gln Tyr Leu Lys Gly
    770                 775                 780
Ile Arg Phe Lys Asn Tyr Ala Asp Val Asp Leu Ser Glu Ile Lys Pro
785                 790                 795                 800
Ile Phe Val Ser Arg Met Pro Lys Arg Ser Ile Thr Gly Glu Leu His
                805                 810                 815
Gln Glu Thr Ile Arg Lys Phe Leu Gly Tyr Asn Glu Lys Gly Lys Val
            820                 825                 830
Leu Thr Ala Thr Lys Thr Lys Leu Glu Asp Ile Pro Phe Asp Ala Asn
        835                 840                 845
Gly Asp Phe Pro Met Phe Gly Lys Glu Thr Asp Leu Tyr Thr Tyr Asn
    850                 855                 860
Ala Ile Lys Glu Arg Tyr Leu Ser His Lys Lys Asp Lys Arg Lys Ser
865                 870                 875                 880
Phe Gln Glu Pro Leu Tyr Lys Pro Thr Lys Ser Gly Gly Ile Gly Pro
                885                 890                 895
Leu Ile Lys Ser Ile Lys Ile Met Asp Thr Arg Thr Ile Val Asn Pro
            900                 905                 910
Val Asn Gln Gly Lys Gly Val Val Tyr Asn Ser Lys Ile Ala Arg Thr
        915                 920                 925
Asp Val Phe Lys Lys Gly Glu Lys Tyr Tyr Leu Ile Pro Ile Tyr Thr
    930                 935                 940
Ile Asp Leu Met Lys Asn Ile Leu Pro Gln Lys Ala Ile Thr Ala Gly
945                 950                 955                 960
Lys Gly Tyr Glu Asp Trp Thr Thr Ile Asp Pro Ser Phe Thr Phe Leu
                965                 970                 975
Phe Ser Leu Phe Pro Asn Asp Leu Ile Gln Ile Val Pro Ser Lys Asn
            980                 985                 990
Lys Thr Ile Lys Ala Arg Thr Thr  Val Ser Lys Lys Glu  Val Leu Leu
        995                 1000                1005
Pro Ser  Leu Thr Gly Tyr Phe  Lys Gly Val His Ser  Gly Thr Ala
    1010                1015                1020
Gly Ile  Thr Val Glu Thr His  Asp Gly Ser Ile Ile  Ala Asn Val
    1025                1030                1035
Gly Ser  Lys Gln Leu Leu Ser  Phe Glu Lys Tyr Gln  Val Asp Val
    1040                1045                1050
Leu Gly  His Tyr Thr Lys Ile  Lys Lys Glu Lys Arg  Ile Gly Met
    1055                1060                1065
Ala Ile
    1070

<210>  51
<211>  1074
<212>  PRT
<213>  Unknown

<220>
<223>  Cas9 endonuclease of Locus 3 (Cas-Locus 3) ORF3, from Bacillus
       species

<400>  51
Met Lys Glu Val Met Lys Leu Lys Tyr Val Leu Gly Leu Asp Ile Gly

1                   5                   10                  15
Ile Ala Ser Cys Gly Trp Ala Val Ile Asn Gln Glu Lys His Arg Ile
                20                  25                  30
Glu Asp Leu Gly Val Arg Ile Phe Asp Lys Ala Glu Asn Pro Lys Asp
            35                  40                  45
Gly Lys Ser Leu Ala Thr Pro Arg Arg Asp Ala Arg Ser Thr Arg Arg
    50                  55                  60
Thr Leu Arg Arg Lys Lys His Arg Met Gln Arg Ile Lys Ile Leu Leu
65                  70                  75                  80
Val Lys His Gly Leu Leu Ser Lys Thr Glu Ile Asp His Leu Tyr Glu
                85                  90                  95
Ser Ala Thr Glu Ile Asp Val Trp Tyr Leu Arg Leu Asn Ala Leu Glu
            100                 105                 110
Arg Arg Leu Asn Pro Lys Glu Phe Ala Arg Val Leu Ile His Leu Ala
        115                 120                 125
Lys Arg Arg Gly Phe Lys Ser Asn Arg Lys Glu Thr Thr Leu Ser Glu
    130                 135                 140
Asn Gly Gln Ile Leu Glu Asn Ile Ser Glu Asn Leu Gln Ile Met Glu
145                 150                 155                 160
Gln Lys Asn Tyr Arg Thr Val Gly Glu Met Ile Leu Lys Asp Lys Arg
                165                 170                 175
Phe Glu Asn His Lys Arg Asn Lys Asp Gly Thr Tyr Ile Gly Thr Val
            180                 185                 190
Thr Arg Gln Gln Leu Gln Glu Glu Ile Gln Met Ile Phe Asn Ala Gln
        195                 200                 205
Arg Leu Tyr Lys Asn Asn His Ala Thr Glu Lys Phe Glu Ser Ser Tyr
    210                 215                 220
Leu Glu Ile Trp Ala Ser Gln Arg Pro Tyr Ala Ser Lys Asp Gln Ile
225                 230                 235                 240
Glu Lys Met Ile Gly Tyr Cys Thr Leu Glu Pro Lys Glu Lys Arg Val
                245                 250                 255
Pro Lys Ala Ser Trp Ser Phe Gln Tyr Phe Val Ala Leu Gln Thr Ile
            260                 265                 270
Asn Asn Leu Arg Leu Ile Ser Lys Asp Arg Ile Glu Glu Leu Ser Phe
        275                 280                 285
Glu Glu Lys Lys Arg Ile Met Asn Leu Ala Leu Glu Lys Ser Ile Val
    290                 295                 300
Lys Tyr Ile Asp Ile Arg Lys Leu Leu Ser Val Pro Asn Glu Phe Gln
305                 310                 315                 320
Phe Asn Ser Leu Ser Tyr Ser Ala Asp Ile Val Asp Thr Thr Val Glu
                325                 330                 335
Asn Lys Lys Cys Ile Glu Leu Lys Glu Tyr His Ser Ile Asn Lys Val
            340                 345                 350
Tyr Lys Gln Ile Tyr Gly Gln Asn Ala Leu Asn Leu Leu Pro Lys Asp
        355                 360                 365
Tyr Asp Thr Ile Ala Tyr Gly Leu Thr Ile Phe Lys Asp Asp Lys Asp
    370                 375                 380
Ile Leu Glu Tyr Leu Gln Asn Lys Tyr Val Asn Ala Lys Gly Lys Pro
385                 390                 395                 400
Ile Ser Asn Leu Ala Asn Lys Thr Tyr Asp Asp Thr Phe Ile Gln Val
                405                 410                 415
Leu Leu Thr Leu Asn Phe Ser Lys Met Gly His Leu Ser Phe Lys Ala
            420                 425                 430
Leu Lys Asn Ile Ile Pro Phe Leu Glu Glu Gly Leu Ser Tyr Asp Lys
        435                 440                 445
Ala Cys Glu Lys Ala Gly Tyr Asn Phe Lys Gly Ala Ser Tyr Thr Lys
    450                 455                 460
Lys Thr Lys Tyr Leu Pro Val Ile Pro Gln Asn Thr Asn Pro Val Val
465                 470                 475                 480
His Arg Ala Leu Ser Gln Thr Arg Lys Val Ile Asn Ala Ile Ile Lys
                485                 490                 495
Lys Tyr Gly Ser Pro Asn Ala Ile His Ile Glu Thr Ala Arg Glu Leu
            500                 505                 510

Ser Lys Thr Phe Gln Glu Arg Lys Glu Ile Asp Ser Met Tyr Gln Asp
        515                 520                 525
Asn Ser Lys Lys Asn Glu His Ala Ile Cys Lys Leu Lys Glu Leu Gly
    530                 535                 540
Asn Ile Lys Pro Ser Gly Ile Asn Ile Val Lys Phe Lys Leu Trp Asn
545                 550                 555                 560
Glu Gln Asp Gly Arg Cys Met Tyr Ser Gly Lys Tyr Ile Glu Pro His
                565                 570                 575
Arg Leu Phe Glu Glu Gly Tyr Thr Glu Val Asp His Ile Leu Pro Tyr
            580                 585                 590
Ser Arg Ser Leu Asp Asp Ser Tyr Asn Asn Lys Val Leu Thr Leu Gly
        595                 600                 605
Thr Glu Asn Gln Arg Lys Gly Asn Lys Thr Pro Tyr Glu Tyr Met Arg
    610                 615                 620
Asn Asn Ser Ile Trp Asp Glu Phe Glu Thr Arg Val Gln Ser Asn Lys
625                 630                 635                 640
Lys Ile Asn Lys Lys Lys Gln Gln Lys Leu Leu Leu Gln Asn Phe Ser
                645                 650                 655
His Ala Arg Glu Gln Glu Phe Ile Glu Arg Asn Leu Asn Asp Thr Arg
            660                 665                 670
Tyr Ala Thr Ile Tyr Leu Ser Ser Leu Ile Gln Gln His Leu Ile Phe
        675                 680                 685
Ser Glu Ser Ser Arg Glu Lys Lys Val His Thr Val Ser Gly Ile Ile
    690                 695                 700
Thr Ser His Leu Arg Ser Arg Trp Gly Phe Asn Lys Asp Arg Lys Glu
705                 710                 715                 720
Gly His Ile His His Ala Leu Asp Ala Val Ile Val Ala Val Thr Ser
                725                 730                 735
Asp His Met Ile Gln Arg Val Thr Lys Tyr Tyr Lys Leu Lys Glu Leu
            740                 745                 750
Asn Arg Asn Ile Gln Ala Lys Arg Met Gln Phe Pro Glu Pro Trp Glu
        755                 760                 765
Gly Phe Arg Leu Glu Leu Glu Ala Arg Val Ser Pro Asn Thr Gln Gln
    770                 775                 780
Tyr Leu Lys Gly Ile Arg Phe Lys Asn Tyr Ala Asp Val Asp Leu Ser
785                 790                 795                 800
Glu Ile Lys Pro Ile Phe Val Ser Arg Met Pro Lys Arg Ser Ile Thr
                805                 810                 815
Gly Glu Leu His Gln Glu Thr Ile Arg Lys Phe Leu Gly Tyr Asn Glu
            820                 825                 830
Lys Gly Lys Val Leu Thr Ala Thr Lys Thr Lys Leu Glu Asp Ile Pro
        835                 840                 845
Phe Asp Ala Asn Gly Asp Phe Pro Met Phe Gly Lys Glu Thr Asp Leu
    850                 855                 860
Tyr Thr Tyr Asn Ala Ile Lys Glu Arg Tyr Leu Ser His Lys Lys Asp
865                 870                 875                 880
Lys Arg Lys Ser Phe Gln Glu Pro Leu Tyr Lys Pro Thr Lys Ser Gly
                885                 890                 895
Gly Ile Gly Pro Leu Ile Lys Ser Ile Lys Ile Met Asp Thr Arg Thr
            900                 905                 910
Ile Val Asn Pro Val Asn Gln Gly Lys Gly Val Val Tyr Asn Ser Lys
        915                 920                 925
Ile Ala Arg Thr Asp Val Phe Lys Lys Gly Glu Lys Tyr Tyr Leu Ile
    930                 935                 940
Pro Ile Tyr Thr Ile Asp Leu Met Lys Asn Ile Leu Pro Gln Lys Ala
945                 950                 955                 960
Ile Thr Ala Gly Lys Gly Tyr Glu Asp Trp Thr Thr Ile Asp Pro Ser
                965                 970                 975
Phe Thr Phe Leu Phe Ser Leu Phe Pro Asn Asp Leu Ile Gln Ile Val
            980                 985                 990
Pro Ser Lys Asn Lys Thr Ile Lys  Ala Arg Thr Thr Val  Ser Lys Lys
        995                 1000                 1005
Glu Val  Leu Leu Pro Ser Leu  Thr Gly Tyr Phe Lys  Gly Val His

1010                1015                1020
Ser Gly Thr Ala Gly Ile Thr Val Glu Thr His Asp Gly Ser Ile
    1025                1030                1035
Ile Ala Asn Val Gly Ser Lys Gln Leu Leu Ser Phe Glu Lys Tyr
    1040                1045                1050
Gln Val Asp Val Leu Gly His Tyr Thr Lys Ile Lys Lys Glu Lys
    1055                1060                1065
Arg Ile Gly Met Ala Ile
    1070

<210>  52
<211>  1070
<212>  PRT
<213>  Unknown

<220>
<223>  Cas9 endonuclease of Locus 5 (Cas-Locus 5) ORF2, from Bacillus
       species

<400>  52
Met Lys Leu Lys Tyr Val Leu Gly Leu Asp Ile Gly Ile Ala Ser Cys
1               5                   10                  15
Gly Trp Ala Val Ile Asn Gln Glu Lys His Arg Ile Glu Asp Leu Gly
            20                  25                  30
Val Arg Ile Phe Asp Lys Ala Glu Asn Pro Lys Asp Gly Lys Ser Leu
        35                  40                  45
Ala Thr Pro Arg Arg Asp Ala Arg Ser Thr Arg Arg Thr Leu Arg Arg
    50                  55                  60
Lys Lys His Arg Met Gln Arg Ile Lys Ile Leu Leu Val Lys His Gly
65                  70                  75                  80
Leu Leu Ser Lys Thr Glu Ile Asp His Leu Tyr Glu Ser Ala Thr Glu
                85                  90                  95
Ile Asp Val Trp Tyr Leu Arg Leu Asn Ala Leu Glu Arg Arg Leu Asn
            100                 105                 110
Pro Lys Glu Phe Ala Arg Val Leu Ile His Leu Ala Lys Arg Arg Gly
        115                 120                 125
Phe Lys Ser Asn Arg Lys Glu Thr Thr Leu Ser Glu Asn Gly Lys Ile
    130                 135                 140
Leu Glu Asn Ile Ser Glu Asn Leu Gln Ile Met Glu Gln Lys Asn Tyr
145                 150                 155                 160
Arg Thr Val Gly Glu Met Ile Leu Lys Asp Lys Lys Phe Glu Asn His
                165                 170                 175
Lys Arg Asn Lys Asp Gly Thr Tyr Ile Gly Thr Val Thr Arg Gln Gln
            180                 185                 190
Leu Gln Glu Glu Ile Gln Met Ile Phe Asn Ala Gln Arg Leu Tyr Lys
        195                 200                 205
Asn Asn His Ala Thr Glu Lys Phe Glu Ser Ser Tyr Leu Glu Ile Trp
    210                 215                 220
Ala Ser Gln Arg Pro Tyr Ala Ser Lys Asp Gln Ile Glu Lys Met Ile
225                 230                 235                 240
Gly Tyr Cys Thr Leu Glu Pro Lys Glu Lys Arg Val Pro Lys Ala Ser
                245                 250                 255
Trp Ser Phe Gln Tyr Phe Val Ala Leu Gln Thr Ile Asn Asn Leu Arg
            260                 265                 270
Leu Ile Ser Lys Asp Arg Ile Glu Glu Leu Ser Phe Glu Glu Lys Lys
        275                 280                 285
Arg Ile Met Asn Leu Ala Leu Glu Lys Ser Ile Val Lys Tyr Ile Asp
    290                 295                 300
Ile Arg Lys Leu Leu Ser Val Pro Asn Glu Phe Gln Phe Asn Ser Leu
305                 310                 315                 320
Ser Tyr Ser Ala Asp Ile Val Asp Thr Thr Val Glu Asn Lys Lys Cys
                325                 330                 335
Ile Glu Leu Lys Glu Tyr His Ser Ile Asn Lys Val Tyr Lys Gln Ile

340                 345                 350
Tyr Glu Gln Asn Ala Leu Asn Leu Leu Pro Lys Asp Tyr Asp Thr Ile
        355                 360                 365
Ala Tyr Gly Leu Thr Ile Phe Lys Asp Asp Lys Asp Ile Leu Glu Tyr
    370                 375                 380
Leu Gln Asn Lys Tyr Val Asn Ala Lys Gly Lys Pro Ile Ser Asn Phe
385                 390                 395                 400
Ala Asn Lys Thr Tyr Asp Asp Thr Phe Ile Gln Val Leu Leu Thr Leu
                405                 410                 415
Asn Phe Ser Lys Met Gly His Leu Ser Phe Lys Ala Leu Lys Asn Ile
            420                 425                 430
Ile Pro Phe Leu Glu Glu Gly Leu Ser Tyr Asp Lys Ala Cys Glu Lys
        435                 440                 445
Ala Gly Tyr Asn Phe Lys Gly Thr Ser Tyr Thr Lys Lys Thr Lys Tyr
    450                 455                 460
Leu Pro Val Ile Pro Gln Asn Thr Asn Pro Val Val His Arg Ala Leu
465                 470                 475                 480
Ser Gln Thr Arg Lys Val Ile Asn Ala Ile Ile Lys Lys Tyr Gly Ser
                485                 490                 495
Pro Asn Ala Ile His Ile Glu Thr Ala Arg Glu Leu Ser Lys Thr Phe
            500                 505                 510
Gln Glu Arg Lys Glu Ile Asp Ser Met Tyr Gln Asp Asn Ser Lys Lys
        515                 520                 525
Asn Glu His Ala Ile His Lys Leu Lys Glu Leu Gly Asn Ile Asn Pro
    530                 535                 540
Ser Gly Ile Asn Ile Val Lys Phe Lys Leu Trp Asn Glu Gln Asp Ser
545                 550                 555                 560
Arg Cys Met Tyr Ser Gly Lys Tyr Ile Glu Pro His Arg Leu Phe Glu
                565                 570                 575
Glu Gly Tyr Thr Glu Val Asp His Ile Leu Pro Tyr Ser Arg Ser Leu
            580                 585                 590
Asp Asp Ser Tyr Asn Asn Lys Val Leu Thr Leu Gly Thr Glu Asn Gln
        595                 600                 605
Gly Lys Gly Asn Arg Thr Pro Tyr Glu Tyr Met Gly Asn Thr Ser Ile
    610                 615                 620
Trp Asp Glu Phe Glu Thr Arg Val Gln Ser Asn Lys Lys Ile Asn Lys
625                 630                 635                 640
Lys Lys Gln Gln Lys Leu Leu Leu Gln Asn Phe Ser His Ala Arg Glu
                645                 650                 655
Gln Glu Phe Ile Glu Arg Asn Leu Asn Asp Thr Arg Tyr Ala Thr Ile
            660                 665                 670
Tyr Ile Ser Ser Leu Ile Gln Gln His Leu Ile Phe Ser Glu Ser Ser
        675                 680                 685
Arg Glu Lys Lys Val His Thr Val Ser Gly Ile Ile Thr Ser His Leu
    690                 695                 700
Arg Ser Arg Trp Gly Phe Asn Lys Asp Arg Lys Glu Gly His Ile His
705                 710                 715                 720
His Ala Leu Asp Ala Val Ile Val Ala Val Thr Ser Asp His Met Ile
                725                 730                 735
Gln Arg Val Thr Lys Tyr Tyr Lys Leu Lys Glu Leu Asn Arg Asn Arg
            740                 745                 750
Gln Ala Lys Arg Met Gln Phe Pro Glu Pro Trp Glu Gly Phe Arg Leu
        755                 760                 765
Glu Leu Glu Ala Arg Ile Ser Pro Asn Thr Gln Gln Tyr Leu Lys Gly
    770                 775                 780
Ile Arg Leu Lys Asn Tyr Ala Asp Val Asn Leu Ser Glu Ile Lys Pro
785                 790                 795                 800
Ile Phe Val Ser Arg Met Pro Lys Arg Ser Ile Thr Gly Glu Leu His
                805                 810                 815
Gln Glu Thr Ile Arg Lys Phe Leu Gly Tyr Asn Glu Lys Gly Lys Val
            820                 825                 830
Leu Thr Ala Ile Lys Thr Lys Leu Glu Asp Ile Pro Phe Asp Ala Asn
        835                 840                 845

Gly Asp Phe Pro Met Tyr Gly Lys Glu Thr Asp Leu Tyr Thr Tyr Asn
    850                 855                 860
Ala Ile Lys Glu Arg Tyr Leu Ser His Lys Lys Asp Lys Arg Lys Ser
865                 870                 875                 880
Phe Gln Glu Pro Leu Tyr Lys Pro Thr Lys Ser Gly Gly Ile Gly Pro
                885                 890                 895
Leu Ile Lys Ser Ile Lys Ile Met Asp Thr Arg Thr Ile Val Asn Pro
            900                 905                 910
Val Asn Gln Gly Lys Gly Val Val Tyr Asn Ser Lys Ile Ala Arg Thr
        915                 920                 925
Asp Val Phe Lys Lys Ala Glu Lys Tyr Tyr Leu Ile Pro Ile Tyr Thr
    930                 935                 940
Ile Asp Leu Met Lys Asn Ile Leu Pro Gln Lys Ala Ile Thr Ala Gly
945                 950                 955                 960
Lys Gly Tyr Glu Asp Trp Thr Thr Ile Asp Pro Ser Phe Thr Phe Leu
                965                 970                 975
Phe Ser Leu Phe Pro Asn Asp Leu Ile Gln Ile Val Pro Ser Lys Asn
            980                 985                 990
Lys Ile Ile Lys Ala Arg Thr Thr  Val Ser Lys Lys Glu  Val Leu Leu
        995                 1000                1005
Pro Ser  Leu Thr Gly Tyr Phe  Lys Gly Val His Ser  Gly Thr Ala
    1010                1015                1020
Gly Ile  Thr Val Glu Thr His  Asp Gly Ser Ile Ile  Ala Asn Val
    1025                1030                1035
Gly Ser  Lys Gln Leu Leu Ser  Phe Glu Lys Tyr Gln  Val Asp Val
    1040                1045                1050
Leu Gly  His Tyr Thr Lys Ile  Lys Lys Glu Lys Arg  Ile Gly Met
    1055                1060                1065
Ala Ile
    1070

<210>  53
<211>  1074
<212>  PRT
<213>  Unknown

<220>
<223>  Cas9 endonuclease of Locus 5 (Cas-Locus 5) ORF3, from Bacillus
       species

<400>  53
Met Lys Glu Val Met Lys Leu Lys Tyr Val Leu Gly Leu Asp Ile Gly
1               5                   10                  15
Ile Ala Ser Cys Gly Trp Ala Val Ile Asn Gln Glu Lys His Arg Ile
            20                  25                  30
Glu Asp Leu Gly Val Arg Ile Phe Asp Lys Ala Glu Asn Pro Lys Asp
        35                  40                  45
Gly Lys Ser Leu Ala Thr Pro Arg Arg Asp Ala Arg Ser Thr Arg Arg
    50                  55                  60
Thr Leu Arg Arg Lys Lys His Arg Met Gln Arg Ile Lys Ile Leu Leu
65                  70                  75                  80
Val Lys His Gly Leu Leu Ser Lys Thr Glu Ile Asp His Leu Tyr Glu
                85                  90                  95
Ser Ala Thr Glu Ile Asp Val Trp Tyr Leu Arg Leu Asn Ala Leu Glu
            100                 105                 110
Arg Arg Leu Asn Pro Lys Glu Phe Ala Arg Val Leu Ile His Leu Ala
        115                 120                 125
Lys Arg Arg Gly Phe Lys Ser Asn Arg Lys Glu Thr Thr Leu Ser Glu
    130                 135                 140
Asn Gly Lys Ile Leu Glu Asn Ile Ser Glu Asn Leu Gln Ile Met Glu
145                 150                 155                 160
Gln Lys Asn Tyr Arg Thr Val Gly Glu Met Ile Leu Lys Asp Lys Lys
                165                 170                 175

Phe Glu Asn His Lys Arg Asn Lys Asp Gly Thr Tyr Ile Gly Thr Val
            180                 185                 190
Thr Arg Gln Gln Leu Gln Glu Glu Ile Gln Met Ile Phe Asn Ala Gln
        195                 200                 205
Arg Leu Tyr Lys Asn Asn His Ala Thr Glu Lys Phe Glu Ser Ser Tyr
    210                 215                 220
Leu Glu Ile Trp Ala Ser Gln Arg Pro Tyr Ala Ser Lys Asp Gln Ile
225                 230                 235                 240
Glu Lys Met Ile Gly Tyr Cys Thr Leu Glu Pro Lys Glu Lys Arg Val
                245                 250                 255
Pro Lys Ala Ser Trp Ser Phe Gln Tyr Phe Val Ala Leu Gln Thr Ile
            260                 265                 270
Asn Asn Leu Arg Leu Ile Ser Lys Asp Arg Ile Glu Glu Leu Ser Phe
        275                 280                 285
Glu Glu Lys Lys Arg Ile Met Asn Leu Ala Leu Glu Lys Ser Ile Val
    290                 295                 300
Lys Tyr Ile Asp Ile Arg Lys Leu Leu Ser Val Pro Asn Glu Phe Gln
305                 310                 315                 320
Phe Asn Ser Leu Ser Tyr Ser Ala Asp Ile Val Asp Thr Thr Val Glu
                325                 330                 335
Asn Lys Lys Cys Ile Glu Leu Lys Glu Tyr His Ser Ile Asn Lys Val
            340                 345                 350
Tyr Lys Gln Ile Tyr Glu Gln Asn Ala Leu Asn Leu Leu Pro Lys Asp
        355                 360                 365
Tyr Asp Thr Ile Ala Tyr Gly Leu Thr Ile Phe Lys Asp Asp Lys Asp
    370                 375                 380
Ile Leu Glu Tyr Leu Gln Asn Lys Tyr Val Asn Ala Lys Gly Lys Pro
385                 390                 395                 400
Ile Ser Asn Phe Ala Asn Lys Thr Tyr Asp Asp Thr Phe Ile Gln Val
                405                 410                 415
Leu Leu Thr Leu Asn Phe Ser Lys Met Gly His Leu Ser Phe Lys Ala
            420                 425                 430
Leu Lys Asn Ile Ile Pro Phe Leu Glu Glu Gly Leu Ser Tyr Asp Lys
        435                 440                 445
Ala Cys Glu Lys Ala Gly Tyr Asn Phe Lys Gly Thr Ser Tyr Thr Lys
    450                 455                 460
Lys Thr Lys Tyr Leu Pro Val Ile Pro Gln Asn Thr Asn Pro Val Val
465                 470                 475                 480
His Arg Ala Leu Ser Gln Thr Arg Lys Val Ile Asn Ala Ile Ile Lys
                485                 490                 495
Lys Tyr Gly Ser Pro Asn Ala Ile His Ile Glu Thr Ala Arg Glu Leu
            500                 505                 510
Ser Lys Thr Phe Gln Glu Arg Lys Glu Ile Asp Ser Met Tyr Gln Asp
        515                 520                 525
Asn Ser Lys Lys Asn Glu His Ala Ile His Lys Leu Lys Glu Leu Gly
    530                 535                 540
Asn Ile Asn Pro Ser Gly Ile Asn Ile Val Lys Phe Lys Leu Trp Asn
545                 550                 555                 560
Glu Gln Asp Ser Arg Cys Met Tyr Ser Gly Lys Tyr Ile Glu Pro His
                565                 570                 575
Arg Leu Phe Glu Glu Gly Tyr Thr Glu Val Asp His Ile Leu Pro Tyr
            580                 585                 590
Ser Arg Ser Leu Asp Asp Ser Tyr Asn Asn Lys Val Leu Thr Leu Gly
        595                 600                 605
Thr Glu Asn Gln Gly Lys Gly Asn Arg Thr Pro Tyr Glu Tyr Met Gly
    610                 615                 620
Asn Thr Ser Ile Trp Asp Glu Phe Glu Thr Arg Val Gln Ser Asn Lys
625                 630                 635                 640
Lys Ile Asn Lys Lys Lys Gln Gln Lys Leu Leu Leu Gln Asn Phe Ser
                645                 650                 655
His Ala Arg Glu Gln Glu Phe Ile Glu Arg Asn Leu Asn Asp Thr Arg
            660                 665                 670
Tyr Ala Thr Ile Tyr Ile Ser Ser Leu Ile Gln Gln His Leu Ile Phe

675                 680                 685
Ser Glu Ser Ser Arg Glu Lys Lys Val His Thr Val Ser Gly Ile Ile
    690                 695                 700
Thr Ser His Leu Arg Ser Arg Trp Gly Phe Asn Lys Asp Arg Lys Glu
705                 710                 715                 720
Gly His Ile His His Ala Leu Asp Ala Val Ile Val Ala Val Thr Ser
                725                 730                 735
Asp His Met Ile Gln Arg Val Thr Lys Tyr Tyr Lys Leu Lys Glu Leu
            740                 745                 750
Asn Arg Asn Arg Gln Ala Lys Arg Met Gln Phe Pro Glu Pro Trp Glu
        755                 760                 765
Gly Phe Arg Leu Glu Leu Glu Ala Arg Ile Ser Pro Asn Thr Gln Gln
    770                 775                 780
Tyr Leu Lys Gly Ile Arg Leu Lys Asn Tyr Ala Asp Val Asn Leu Ser
785                 790                 795                 800
Glu Ile Lys Pro Ile Phe Val Ser Arg Met Pro Lys Arg Ser Ile Thr
                805                 810                 815
Gly Glu Leu His Gln Glu Thr Ile Arg Lys Phe Leu Gly Tyr Asn Glu
            820                 825                 830
Lys Gly Lys Val Leu Thr Ala Ile Lys Thr Lys Leu Glu Asp Ile Pro
        835                 840                 845
Phe Asp Ala Asn Gly Asp Phe Pro Met Tyr Gly Lys Glu Thr Asp Leu
    850                 855                 860
Tyr Thr Tyr Asn Ala Ile Lys Glu Arg Tyr Leu Ser His Lys Lys Asp
865                 870                 875                 880
Lys Arg Lys Ser Phe Gln Glu Pro Leu Tyr Lys Pro Thr Lys Ser Gly
                885                 890                 895
Gly Ile Gly Pro Leu Ile Lys Ser Ile Lys Ile Met Asp Thr Arg Thr
            900                 905                 910
Ile Val Asn Pro Val Asn Gln Gly Lys Gly Val Val Tyr Asn Ser Lys
        915                 920                 925
Ile Ala Arg Thr Asp Val Phe Lys Lys Ala Glu Lys Tyr Tyr Leu Ile
    930                 935                 940
Pro Ile Tyr Thr Ile Asp Leu Met Lys Asn Ile Leu Pro Gln Lys Ala
945                 950                 955                 960
Ile Thr Ala Gly Lys Gly Tyr Glu Asp Trp Thr Thr Ile Asp Pro Ser
                965                 970                 975
Phe Thr Phe Leu Phe Ser Leu Phe Pro Asn Asp Leu Ile Gln Ile Val
            980                 985                 990
Pro Ser Lys Asn Lys Ile Ile Lys  Ala Arg Thr Thr Val  Ser Lys Lys
        995                 1000                1005
Glu Val  Leu Leu Pro Ser Leu  Thr Gly Tyr Phe Lys  Gly Val His
    1010                1015                1020
Ser Gly  Thr Ala Gly Ile Thr  Val Glu Thr His Asp  Gly Ser Ile
    1025                1030                1035
Ile Ala  Asn Val Gly Ser Lys  Gln Leu Leu Ser Phe  Glu Lys Tyr
    1040                1045                1050
Gln Val  Asp Val Leu Gly His  Tyr Thr Lys Ile Lys  Lys Glu Lys
    1055                1060                1065
Arg Ile  Gly Met Ala Ile
    1070

<210>  54
<211>  189
<212>  DNA
<213>  Solanum tuberosum

<400>  54
gtaagtttct gcttctacct ttgatatata tataataatt atcattaatt agtagtaata      60
taatatttca aatatttttt tcaaaataaa agaatgtagt atatagcaat tgcttttctg     120
tagtttataa gtgtgtatat tttaatttat aacttttcta atatatgacc aaaacatggt     180
gatgtgcag                                                             189

<210>  55
<211>  9
<212>  PRT
<213>  Simian virus

<400>  55
Met Ala Pro Lys Lys Lys Arg Lys Val
1               5

<210>  56
<211>  18
<212>  PRT
<213>  Agrobacterium tumefaciens

<400>  56
Lys Arg Pro Arg Asp Arg His Asp Gly Glu Leu Gly Gly Arg Lys Arg
1               5                   10                  15
Ala Arg

<210>  57
<211>  1101
<212>  PRT
<213>  Artificial Sequence

<220>
<223>  Maize Optimized Cas-Locus 3 Endonuclease with N- and C-terminal
       NLSs

<400>  57
Met Ala Pro Lys Lys Lys Arg Lys Val Met Lys Glu Val Met Lys Leu
1               5                   10                  15
Lys Tyr Val Leu Gly Leu Asp Ile Gly Ile Ala Ser Cys Gly Trp Ala
            20                  25                  30
Val Ile Asn Gln Glu Lys His Arg Ile Glu Asp Leu Gly Val Arg Ile
        35                  40                  45
Phe Asp Lys Ala Glu Asn Pro Lys Asp Gly Lys Ser Leu Ala Thr Pro
    50                  55                  60
Arg Arg Asp Ala Arg Ser Thr Arg Arg Thr Leu Arg Arg Lys Lys His
65                  70                  75                  80
Arg Met Gln Arg Ile Lys Ile Leu Leu Val Lys His Gly Leu Leu Ser
                85                  90                  95
Lys Thr Glu Ile Asp His Leu Tyr Glu Ser Ala Thr Glu Ile Asp Val
            100                 105                 110
Trp Tyr Leu Arg Leu Asn Ala Leu Glu Arg Arg Leu Asn Pro Lys Glu
        115                 120                 125
Phe Ala Arg Val Leu Ile His Leu Ala Lys Arg Arg Gly Phe Lys Ser
    130                 135                 140
Asn Arg Lys Glu Thr Thr Leu Ser Glu Asn Gly Gln Ile Leu Glu Asn
145                 150                 155                 160
Ile Ser Glu Asn Leu Gln Ile Met Glu Gln Lys Asn Tyr Arg Thr Val
                165                 170                 175
Gly Glu Met Ile Leu Lys Asp Lys Arg Phe Glu Asn His Lys Arg Asn
            180                 185                 190
Lys Asp Gly Thr Tyr Ile Gly Thr Val Thr Arg Gln Gln Leu Gln Glu
        195                 200                 205
Glu Ile Gln Met Ile Phe Asn Ala Gln Arg Leu Tyr Lys Asn Asn His
    210                 215                 220
Ala Thr Glu Lys Phe Glu Ser Ser Tyr Leu Glu Ile Trp Ala Ser Gln
225                 230                 235                 240
Arg Pro Tyr Ala Ser Lys Asp Gln Ile Glu Lys Met Ile Gly Tyr Cys
                245                 250                 255
Thr Leu Glu Pro Lys Glu Lys Arg Val Pro Lys Ala Ser Trp Ser Phe

            260                 265                 270
Gln Tyr Phe Val Ala Leu Gln Thr Ile Asn Asn Leu Arg Leu Ile Ser
        275                 280                 285
Lys Asp Arg Ile Glu Glu Leu Ser Phe Glu Glu Lys Lys Arg Ile Met
    290                 295                 300
Asn Leu Ala Leu Glu Lys Ser Ile Val Lys Tyr Ile Asp Ile Arg Lys
305                 310                 315                 320
Leu Leu Ser Val Pro Asn Glu Phe Gln Phe Asn Ser Leu Ser Tyr Ser
                325                 330                 335
Ala Asp Ile Val Asp Thr Thr Val Glu Asn Lys Lys Cys Ile Glu Leu
            340                 345                 350
Lys Glu Tyr His Ser Ile Asn Lys Val Tyr Lys Gln Ile Tyr Gly Gln
        355                 360                 365
Asn Ala Leu Asn Leu Leu Pro Lys Asp Tyr Asp Thr Ile Ala Tyr Gly
    370                 375                 380
Leu Thr Ile Phe Lys Asp Asp Lys Asp Ile Leu Glu Tyr Leu Gln Asn
385                 390                 395                 400
Lys Tyr Val Asn Ala Lys Gly Lys Pro Ile Ser Asn Leu Ala Asn Lys
                405                 410                 415
Thr Tyr Asp Asp Thr Phe Ile Gln Val Leu Leu Thr Leu Asn Phe Ser
            420                 425                 430
Lys Met Gly His Leu Ser Phe Lys Ala Leu Lys Asn Ile Ile Pro Phe
        435                 440                 445
Leu Glu Glu Gly Leu Ser Tyr Asp Lys Ala Cys Glu Lys Ala Gly Tyr
    450                 455                 460
Asn Phe Lys Gly Ala Ser Tyr Thr Lys Lys Thr Lys Tyr Leu Pro Val
465                 470                 475                 480
Ile Pro Gln Asn Thr Asn Pro Val Val His Arg Ala Leu Ser Gln Thr
                485                 490                 495
Arg Lys Val Ile Asn Ala Ile Ile Lys Lys Tyr Gly Ser Pro Asn Ala
            500                 505                 510
Ile His Ile Glu Thr Ala Arg Glu Leu Ser Lys Thr Phe Gln Glu Arg
        515                 520                 525
Lys Glu Ile Asp Ser Met Tyr Gln Asp Asn Ser Lys Lys Asn Glu His
    530                 535                 540
Ala Ile Cys Lys Leu Lys Glu Leu Gly Asn Ile Lys Pro Ser Gly Ile
545                 550                 555                 560
Asn Ile Val Lys Phe Lys Leu Trp Asn Glu Gln Asp Gly Arg Cys Met
                565                 570                 575
Tyr Ser Gly Lys Tyr Ile Glu Pro His Arg Leu Phe Glu Glu Gly Tyr
            580                 585                 590
Thr Glu Val Asp His Ile Leu Pro Tyr Ser Arg Ser Leu Asp Asp Ser
        595                 600                 605
Tyr Asn Asn Lys Val Leu Thr Leu Gly Thr Glu Asn Gln Arg Lys Gly
    610                 615                 620
Asn Lys Thr Pro Tyr Glu Tyr Met Arg Asn Asn Ser Ile Trp Asp Glu
625                 630                 635                 640
Phe Glu Thr Arg Val Gln Ser Asn Lys Lys Ile Asn Lys Lys Lys Gln
                645                 650                 655
Gln Lys Leu Leu Leu Gln Asn Phe Ser His Ala Arg Glu Gln Glu Phe
            660                 665                 670
Ile Glu Arg Asn Leu Asn Asp Thr Arg Tyr Ala Thr Ile Tyr Leu Ser
        675                 680                 685
Ser Leu Ile Gln Gln His Leu Ile Phe Ser Glu Ser Ser Arg Glu Lys
    690                 695                 700
Lys Val His Thr Val Ser Gly Ile Ile Thr Ser His Leu Arg Ser Arg
705                 710                 715                 720
Trp Gly Phe Asn Lys Asp Arg Lys Glu Gly His Ile His His Ala Leu
                725                 730                 735
Asp Ala Val Ile Val Ala Val Thr Ser Asp His Met Ile Gln Arg Val
            740                 745                 750
Thr Lys Tyr Tyr Lys Leu Lys Glu Leu Asn Arg Asn Ile Gln Ala Lys
        755                 760                 765

Arg Met Gln Phe Pro Glu Pro Trp Glu Gly Phe Arg Leu Glu Leu Glu
    770                 775                 780
Ala Arg Val Ser Pro Asn Thr Gln Gln Tyr Leu Lys Gly Ile Arg Phe
785                 790                 795                 800
Lys Asn Tyr Ala Asp Val Asp Leu Ser Glu Ile Lys Pro Ile Phe Val
                805                 810                 815
Ser Arg Met Pro Lys Arg Ser Ile Thr Gly Glu Leu His Gln Glu Thr
            820                 825                 830
Ile Arg Lys Phe Leu Gly Tyr Asn Glu Lys Gly Lys Val Leu Thr Ala
        835                 840                 845
Thr Lys Thr Lys Leu Glu Asp Ile Pro Phe Asp Ala Asn Gly Asp Phe
    850                 855                 860
Pro Met Phe Gly Lys Glu Thr Asp Leu Tyr Thr Tyr Asn Ala Ile Lys
865                 870                 875                 880
Glu Arg Tyr Leu Ser His Lys Lys Asp Lys Arg Lys Ser Phe Gln Glu
                885                 890                 895
Pro Leu Tyr Lys Pro Thr Lys Ser Gly Gly Ile Gly Pro Leu Ile Lys
            900                 905                 910
Ser Ile Lys Ile Met Asp Thr Arg Thr Ile Val Asn Pro Val Asn Gln
        915                 920                 925
Gly Lys Gly Val Val Tyr Asn Ser Lys Ile Ala Arg Thr Asp Val Phe
    930                 935                 940
Lys Lys Gly Glu Lys Tyr Tyr Leu Ile Pro Ile Tyr Thr Ile Asp Leu
945                 950                 955                 960
Met Lys Asn Ile Leu Pro Gln Lys Ala Ile Thr Ala Gly Lys Gly Tyr
                965                 970                 975
Glu Asp Trp Thr Thr Ile Asp Pro Ser Phe Thr Phe Leu Phe Ser Leu
            980                 985                 990
Phe Pro Asn Asp Leu Ile Gln Ile  Val Pro Ser Lys Asn  Lys Thr Ile
        995                 1000                1005
Lys Ala  Arg Thr Thr Val Ser  Lys Lys Glu Val Leu  Leu Pro Ser
    1010                1015                1020
Leu Thr  Gly Tyr Phe Lys Gly  Val His Ser Gly Thr  Ala Gly Ile
    1025                1030                1035
Thr Val  Glu Thr His Asp Gly  Ser Ile Ile Ala Asn  Val Gly Ser
    1040                1045                1050
Lys Gln  Leu Leu Ser Phe Glu  Lys Tyr Gln Val Asp  Val Leu Gly
    1055                1060                1065
His Tyr  Thr Lys Ile Lys Lys  Glu Lys Arg Ile Gly  Met Ala Ile
    1070                1075                1080
Lys Arg  Pro Arg Asp Arg His  Asp Gly Glu Leu Gly  Gly Arg Lys
    1085                1090                1095
Arg Ala  Arg
    1100

<210>  58
<211>  896
<212>  DNA
<213>  Zea mays

<400>  58
gtgcagcgtg acccggtcgt gcccctctct agagataatg agcattgcat gtctaagtta      60
taaaaaatta ccacatattt tttttgtcac acttgtttga agtgcagttt atctatcttt     120
atacatatat ttaaacttta ctctacgaat aatataatct atagtactac aataatatca     180
gtgttttaga gaatcatata aatgaacagt tagacatggt ctaaaggaca attgagtatt     240
ttgacaacag gactctacag ttttatcttt ttagtgtgca tgtgttctcc tttttttttg     300
caaatagctt cacctatata atacttcatc cattttatta gtacatccat ttagggttta     360
gggttaatgg tttttataga ctaatttttt tagtacatct attttattct attttagcct     420
ctaaattaag aaaactaaaa ctctatttta gtttttttat ttaataattt agatataaaa     480
tagaataaaa taaagtgact aaaaattaaa caaataccct ttaagaaatt aaaaaaacta     540
aggaaacatt tttcttgttt cgagtagata atgccagcct gttaaacgcc gtcgacgagt     600
ctaacggaca ccaaccagcg aaccagcagc gtcgcgtcgg gccaagcgaa gcagacggca     660
cggcatctct gtcgctgcct ctggacccct ctcgagagtt ccgctccacc gttggacttg     720

ctccgctgtc ggcatccaga aattgcgtgg cggagcggca gacgtgagcc ggcacggcag 780
gcggcctcct cctcctctca cggcaccggc agctacgggg gattcctttc ccaccgctcc 840
ttcgctttcc cttcctcgcc cgccgtaata aatagacacc ccctccacac cctctt 896

<210> 59
<211> 82
<212> DNA
<213> Zea mays

<400> 59
tccccaacct cgtgttgttc ggagcgcaca cacacacaac cagatctccc ccaaatccac 60
ccgtcggcac ctccgcttca ag 82

<210> 60
<211> 1013
<212> DNA
<213> Zea mays

<400> 60
gtacgccgct cgtcctcccc cccccccctc tctaccttct ctagatcggc gttccggtcc 60
atgcatggtt agggcccggt agttctactt ctgttcatgt ttgtgttaga tccgtgtttg 120
tgttagatcc gtgctgctag cgttcgtaca cggatgcgac ctgtacgtca gacacgttct 180
gattgctaac ttgccagtgt ttctctttgg ggaatcctgg gatggctcta gccgttccgc 240
agacgggatc gatttcatga ttttttttgt ttcgttgcat agggtttggt ttgccctttt 300
cctttatttc aatatatgcc gtgcacttgt ttgtcgggtc atcttttcat gctttttttt 360
gtcttggttg tgatgatgtg gtctggttgg gcggtcgttc tagatcggag tagaattctg 420
tttcaaacta cctggtggat ttattaattt tggatctgta tgtgtgtgcc atacatattc 480
atagttacga attgaagatg atggatggaa atatcgatct aggataggta tacatgttga 540
tgcgggtttt actgatgcat atacagagat gctttttgtt cgcttggttg tgatgatgtg 600
gtgtggttgg gcggtcgttc attcgttcta gatcggagta gaatactgtt tcaaactacc 660
tggtgtattt attaattttg gaactgtatg tgtgtgtcat acatcttcat agttacgagt 720
ttaagatgga tggaaatatc gatctaggat aggtatacat gttgatgtgg gttttactga 780
tgcatataca tgatggcata tgcagcatct attcatatgc tctaaccttg agtacctatc 840
tattataata aacaagtatg ttttataatt attttgatct tgatatactt ggatgatggc 900
atatgcagca gctatatgtg gattttttta gccctgcctt catacgctat ttatttgctt 960
ggtactgttt cttttgtcga tgctcaccct gttgtttggt gttacttctg cag 1013

<210> 61
<211> 5829
<212> DNA
<213> Artificial Sequence

<220>
<223> Maize Optimized Cas-Locus 3 Expression Cassette, synthesized

<400> 61
gtgcagcgtg acccggtcgt gcccctctct agagataatg agcattgcat gtctaagtta 60
taaaaaatta ccacatattt tttttgtcac acttgtttga agtgcagttt atctatcttt 120
atacatatat ttaaacttta ctctacgaat aatataatct atagtactac aataatatca 180
gtgttttaga gaatcatata aatgaacagt tagacatggt ctaaaggaca attgagtatt 240
ttgacaacag gactctacag ttttatcttt ttagtgtgca tgtgttctcc tttttttttg 300
caaatagctt cacctatata atacttcatc cattttatta gtacatccat ttagggttta 360
gggttaatgg tttttataga ctaatttttt tagtacatct attttattct attttagcct 420
ctaaattaag aaaactaaaa ctctatttta gtttttttat ttaataattt agatataaaa 480
tagaataaaa taaagtgact aaaaattaaa caaataccct ttaagaaatt aaaaaaacta 540
aggaaacatt tttcttgttt cgagtagata atgccagcct gttaaacgcc gtcgacgagt 600
ctaacggaca ccaaccagcg aaccagcagc gtcgcgtcgg gccaagcgaa gcagacggca 660
cggcatctct gtcgctgcct ctggacccct ctcgagagtt ccgctccacc gttggacttg 720
ctccgctgtc ggcatccaga aattgcgtgg cggagcggca gacgtgagcc ggcacggcag 780
gcggcctcct cctcctctca cggcaccggc agctacgggg gattcctttc ccaccgctcc 840
ttcgctttcc cttcctcgcc cgccgtaata aatagacacc ccctccacac cctctttccc 900
caacctcgtg ttgttcggag cgcacacaca cacaaccaga tctcccccaa atccacccgt 960
cggcacctcc gcttcaaggt acgccgctcg tcctcccccc cccccctctc taccttctct 1020

agatcggcgt tccggtccat gcatggttag ggcccggtag ttctacttct gttcatgttt 1080
gtgttagatc cgtgtttgtg ttagatccgt gctgctagcg ttcgtacacg gatgcgacct 1140
gtacgtcaga cacgttctga ttgctaactt gccagtgttt ctctttgggg aatcctggga 1200
tggctctagc cgttccgcag acgggatcga tttcatgatt ttttttgttt cgttgcatag 1260
ggtttggttt gcccttttcc tttatttcaa tatatgccgt gcacttgttt gtcgggtcat 1320
cttttcatgc ttttttttgt cttggttgtg atgatgtggt ctggttgggc ggtcgttcta 1380
gatcggagta gaattctgtt tcaaactacc tggtggattt attaattttg gatctgtatg 1440
tgtgtgccat acatattcat agttacgaat tgaagatgat ggatggaaat atcgatctag 1500
gataggtata catgttgatg cgggttttac tgatgcatat acagagatgc tttttgttcg 1560
cttggttgtg atgatgtggt gtggttgggc ggtcgttcat tcgttctaga tcggagtaga 1620
atactgtttc aaactacctg gtgtatttat taattttgga actgtatgtg tgtgtcatac 1680
atcttcatag ttacgagttt aagatggatg gaaatatcga tctaggatag gtatacatgt 1740
tgatgtgggt tttactgatg catatacatg atggcatatg cagcatctat tcatatgctc 1800
taaccttgag tacctatcta ttataataaa caagtatgtt ttataattat tttgatcttg 1860
atatacttgg atgatggcat atgcagcagc tatatgtgga tttttttagc cctgccttca 1920
tacgctattt atttgcttgg tactgtttct tttgtcgatg ctcaccctgt tgtttggtgt 1980
tacttctgca ggtcgactct agaggatcca tggcaccgaa gaagaagcgc aaggtgatga 2040
aggaggtcat gaaactcaag tatgtcctcg gcttggacat cggcatcgcc agctgcggct 2100
gggccgtaat aaatcaagag aagcaccgca tcgaggacct cggcgtcaga atcttcgaca 2160
aagccgagaa ccccaaggta agtttctgct tctacctttg atatatatat aataattatc 2220
attaattagt agtaatataa tatttcaaat atttttttca aaataaaaga atgtagtata 2280
tagcaattgc ttttctgtag tttataagtg tgtatatttt aatttataac ttttctaata 2340
tatgaccaaa acatggtgat gtgcaggacg gcaaaagcct cgcgaccccg aggagagacg 2400
cgcgctccac gaggaggaca ctgaggcgca agaagcacag gatgcagaga atcaagatcc 2460
tcctcgtcaa acacgggctc ctctccaaga cagagatcga ccacctctat gaaagtgcca 2520
cagagatcga cgtgtggtac ctcagactca acgccctcga gcgcagatta aatcccaagg 2580
agttcgccag ggtcctcatc cacctggcca aacggcgggg cttcaagagc aacagaaagg 2640
agaccacgct gtccgagaac ggccagatcc tcgaaaatat ttccgagaac cttcagatca 2700
tggagcagaa aaactaccgc accgtcggcg agatgatcct caaggataaa agattcgaga 2760
accataaaag aaacaaggac ggcacgtaca ttggcacagt gaccagacag cagctgcagg 2820
aggaaataca aatgatcttc aacgcccagc gcctctacaa gaacaaccac gccacagaga 2880
agttcgagag cagctatctg gagatatggg ccagccagag accatacgcc agcaaggacc 2940
agatcgagaa gatgatcggc tactgcaccc tcgagcccaa agagaagagg gtcccaaagg 3000
cgtcgtggag ctttcagtat tttgtcgcgc tgcagaccat aaacaacctc agactgatct 3060
cgaaggacag gatcgaggag ctcagcttcg aggagaaaaa gcggatcatg aacctcgccc 3120
ttgagaaatc aattgtcaaa tacattgaca tccgcaagct cctcagcgtg cccaacgagt 3180
ttcagttcaa cagcctcagc tacagcgccg acatcgtcga caccaccgtc gaaaataaaa 3240
aatgcatcga gctcaaggag taccacagca tcaacaaggt atataaacag atctacggcc 3300
agaacgccct caaccttctt cccaaggact acgacacaat cgcctacggc ctcaccatat 3360
tcaaggacga caaggacatc ctcgagtacc tccaaaataa atacgtcaac gccaagggca 3420
agccaatctc caaccttgcc aacaagacat acgacgatac atttatccag gtcctcctca 3480
cactcaactt tagcaagatg ggccacctct ccttcaaagc cctcaaaaat attatcccct 3540
tcctcgagga gggcctcagc tacgacaaag catgtgagaa ggccggctac aacttcaagg 3600
gcgccagcta caccaagaaa acaaagtacc tgcccgtgat cccccagaat acaaaccccg 3660
tcgtccacag agccctcagc cagacgcgga aggtcatcaa cgccatcata aaaaaatatg 3720
gctcgccaaa tgcgatccac atcgagaccg ctagggagct tagcaagacc ttccaggagc 3780
gcaaggagat cgacagcatg taccaggaca acagcaagaa gaacgagcac gccatctgca 3840
agctcaagga gctcggcaac attaaaccca gcggaataaa tatcgtcaag ttcaagctct 3900
ggaacgagca ggacggcaga tgtatgtaca gcggaaaata tatagagccg cacaggctct 3960
tcgaggaggg ctacacagag gtcgaccaca tactgccgta cagcaggagc cttgacgact 4020
cgtacaacaa taaagtcctc acgcttggca cagagaacca gcgcaagggc aacaaaacgc 4080
cctacgaata catgaggaat aatagcatct gggacgagtt cgagacaagg gtgcagtcca 4140
ataaaaaaat caacaagaag aagcagcaga agctcctcct ccagaacttc agccacgcca 4200
gggagcagga gttcattgag agaaacctca acgacacgcg ctacgccacc atctacctca 4260
gcagcctgat tcagcagcac ctgattttca gcgagagcag cagggagaag aaggtccaca 4320
cagtctcggg gatcatcaca tcccacctgc gctcccgctg gggcttcaac aaggaccgca 4380
aggagggcca catccaccac gccctcgacg ccgtcatcgt cgccgtcacc agcgaccaca 4440
tgatccagcg cgtcacaaaa tattacaagc tcaaggagct caacaggaac atccaggcca 4500
agcgcatgca gtttccggag ccttgggagg gcttcaggct cgagctcgag gcccgcgtca 4560
gcccgaacac ccagcagtac ctcaagggca tcaggtttaa gaactacgcc gacgtggacc 4620
ttagcgaaat caaacccatc ttcgtctcaa gaatgcccaa gcggtccatc acgggcgagc 4680
tccaccagga gacaatccgc aagttcctcg gctacaacga gaaagggaaa gtcctgacgg 4740
ccaccaagac caagctcgag gacatcccct tcgacgccaa tggcgacttt cccatgttcg 4800

gcaaggagac cgacctctac acctacaacg ccataaaaga gcgctacctc agccataaaa 4860
aagacaaacg caagtccttc caggagccgc tctacaaacc caccaagagc gggggcatcg 4920
gccccctcat aaaaagcatt aaaataatgg acacccgcac catcgtcaac cccgtcaatc 4980
aaggcaaggg cgtcgtctac aacagcaaga tagcccgcac cgacgtgttc aagaaaggcg 5040
agaagtacta cctgatcccg atctacacta tagacctgat gaaaaatatc ctcccacaga 5100
aggccataac ggcgggcaaa ggctacgagg actggacgac catagaccct tccttcacat 5160
tcctcttctc cctgttcccc aacgacctca tccagatcgt ccccagcaag aacaagacaa 5220
ttaaagccag aacgacggtg tccaagaagg aggtcctcct cccctccctg accggctact 5280
ttaagggcgt gcacagcggc acagccggca tcacggtgga gacccacgac ggctccatca 5340
ttgctaacgt cggcagcaaa cagctgctca gcttcgagaa gtaccaggtc gacgtcctcg 5400
gccactatac caaaattaag aaggagaagc gcatcggcat ggccataaag aggcccaggg 5460
accgccacga cggcgagctc ggcggcagga agagggcccg ctagttaacc tagacttgtc 5520
catcttctgg attggccaac ttaattaatg tatgaaataa aaggatgcac acatagtgac 5580
atgctaatca ctataatgtg ggcatcaaag ttgtgtgtta tgtgtaatta ctagttatct 5640
gaataaaaga gaaagagatc atccatattt cttatcctaa atgaatgtca cgtgtcttta 5700
taattctttg atgaaccaga tgcatttcat taaccaaatc catatacata taaatattaa 5760
tcatatataa ttaatatcaa ttgggttagc aaaacaaatc tagtctaggt gtgttttgcg 5820
aatgcggcc 5829

<210> 62
<211> 16
<212> RNA
<213> Unknown

<220>
<223> sgRNA Repeat Region for Cas-Locus 3 Version 2 from Bacillus
species

<400> 62
gcuauaguuc cauaag 16

<210> 63
<211> 26
<212> RNA
<213> Unknown

<220>
<223> sgRNA Repeat Region for Cas-Locus 3 Version 3 from Bacillus
species

<400> 63
gcuauaguuc cauaagaaac aucgcu 26

<210> 64
<211> 16
<212> RNA
<213> Unknown

<220>
<223> sgRNA Anti-Repeat Region for Cas-Locus 3 Version 2 from Bacillus
species

<400> 64
cuuaaguuac uauagu 16

<210> 65
<211> 26
<212> RNA
<213> Unknown

<220>
<223> sgRNA Anti-Repeat Region for Cas-Locus 3 Version 3 Bacillus
species

<400>  65
agcgauguuu cuuaaguuac uauagu                                            26

<210>  66
<211>  157
<212>  RNA
<213>  Artificial sequence

<220>
<223>  synthesized sgRNA for Cas-Locus 3 Version 2

<220>
<221>  misc_feature
<222>  (1)..(20)
<223>  n is a, c, g, or u

<400>  66
nnnnnnnnnn nnnnnnnnnn gcuauaguuc cauaaggaaa cuuaaguuac uauaguaagg      60
gcaaugaccc guggcguuug gggaucgccu uauccuggua uggauauucu ccccauguga      120
aaagcaccua agcauagcgc uauggugcuu uuauuuu                              157

<210>  67
<211>  177
<212>  RNA
<213>  Artificial sequence

<220>
<223>  synthesized sgRNA for Cas-Locus 3 Version 3

<220>
<221>  misc_feature
<222>  (1)..(20)
<223>  n is a, c, g, or u

<400>  67
nnnnnnnnnn nnnnnnnnnn gcuauaguuc cauaagaaac aucgcugaaa agcgauguuu      60
cuuaaguuac uauaguaagg gcaaugaccc guggcguuug gggaucgccu uauccuggua      120
uggauauucu ccccauguga aaagcaccua agcauagcgc uauggugcuu uuauuuu        177

<210>  68
<211>  1000
<212>  DNA
<213>  Zea mays

<400>  68
tgagagtaca atgatgaacc tagattaatc aatgccaaag tctgaaaaat gcaccctcag      60
tctatgatcc agaaaatcaa gattgcttga ggccctgttc ggttgttccg gattagagcc      120
ccggattaat tcctagccgg attacttctc taatttatat agattttgat gagctggaat      180
gaatcctggc ttattccggt acaaccgaac aggccctgaa ggataccagt aatcgctgag      240
ctaaattggc atgctgtcag agtgtcagta ttgcagcaag gtagtgagat aaccggcatc      300
atggtgccag tttgatggca ccattagggt tagagatggt ggccatgggc gcatgtcctg      360
gccaactttg tatgatatat ggcagggtga ataggaaagt aaaattgtat tgtaaaaagg      420
gatttcttct gtttgttagc gcatgtacaa ggaatgcaag ttttgagcga gggggcatca      480
aagatctggc tgtgtttcca gctgtttttg ttagccccat cgaatccttg acataatgat      540
cccgcttaaa taagcaacct cgcttgtata gttccttgtg ctctaacaca cgatgatgat      600
aagtcgtaaa atagtggtgt ccaaagaatt tccaggccca gttgtaaaag ctaaaatgct      660
attcgaattt ctactagcag taagtcgtgt ttagaaatta tttttttata tacctttttt      720
ccttctatgt acagtaggac acagtgtcag cgccgcgttg acggagaata tttgcaaaaa      780
agtaaaagag aaagtcatag cggcgtatgt gccaaaaact tcgtcacaga gagggccata      840
agaaacatgg cccacggccc aatacgaagc accgcgacga agcccaaaca gcagtccgta      900
ggtggagcaa agcgctgggt aatacgcaaa cgttttgtcc caccttgact aatcacaaga      960
gtggagcgta ccttataaac cgagccgcaa gcaccgaatt                          1000

<210> 69
<211> 29
<212> DNA
<213> Zea mays

<400> 69
ggtcagggac accatatgaa tgaaagaaa 29

<210> 70
<211> 30
<212> DNA
<213> Zea mays

<400> 70
gatcccacat aggtgatgag ctaagagtaa 30

<210> 71
<211> 30
<212> DNA
<213> Zea mays

<400> 71
gaccacacga atctggcctt gaccaggata 30

<210> 72
<211> 6065
<212> DNA
<213> Unknown

<220>
<223> Locus 42 from Bacillus species

<400> 72
ccttaaaaag atacgatacg tactacgcag acactaacca gaacaagggt taaaaaacta 60
cacttgatat ataaatttta tatacctaca ggtttttgag gtaagttgga gttgttaaga 120
tgaagaaaaa caaggagtca tattttatac acgaatatac cttgcgagac aagactacta 180
aatcaataaa aattgaatcg tggcgttcat taaaggaaga aatgcaagtc ctgctcataa 240
aagacagtgc catctttcaa attcaaatgc taaaagtaga ttcaaaaaag agtaacatag 300
cgctatgcca tgctactctt ttttatttta ataatatagc gtgatttttc tacacttaaa 360
tgcaactagt tccgcaaaca aaaaataaat tactatttca aaaaacaaga aaccttaagg 420
attaaactga cgaaagatgc tcttttttga taaggaaaat ccttatctaa ggctataata 480
ataaggtttt taaagcatcc atcatagaat agcgatgttt cttttggaac tatagccaca 540
acacacttat caatatctat tccgatacca tagcatagcg atttttctta gggaactata 600
gctaaatcgt tcgttccacc tgcaatacgt gcatcatggc attacaatat ttcttatgga 660
actatagcca taatcaacgc cctctaaatg tcgactatat catagcgatg tttctttcag 720
aactatagcc caaattccaa aacaaaataa aatagttgca tcatatcata gcgattttcc 780
ttatggaact atagctgtgt agacaatgat aatgcaggta gagccatcat agcatagcgg 840
ttttcctttt ggaactatag cctcttcaaa cgcccaaatt acttttttgg aatcatagca 900
tagcaatgtt tcttagggaa ttatagttat ccatatgcca taacttgata gggttcaatc 960
atagcatagc gatgtttctc atggaacttt agcttgtatt tacttattga atcaataatg 1020
tttaccatag catattaata tttcttatgg aactatagcc acttcttcag gcgtctgatt 1080
cgtcgcctcg atcatagcat agcaatattt cttatggaac tatagcatgt cttgatacat 1140
aaaggaattt acaaagatca tagcataacg atttttcttt tcgaactata gctgatagcc 1200
tcgtttggca gccagaccaa gaatcatagt ggtgtcaatg tcaagtgtga caagtttaaa 1260
atcatagcat agcttttttc tcctagaatt atagctcaga tggcttatcc ctttcagaat 1320
tctttatcaa atcatagcat agcgattttt cttttggaac tatagcgact tgggatgatt 1380
tgtcttattc ttggaatcat agcttagcga tggttcttag gaaactatag ctcgtttctt 1440
ctaagtaaat aaggaggaat gatcataaca tagcgatatt ctccacggaa tgatagcatt 1500
aatattaaca agttaagaaa tgttcctctt aattaattta tatcacaatt attcccaatt 1560
tcattatttt tataattata cgtttatcta ggtataaagt ttatttttcg ggtcaataat 1620
taaatcataa ggatcatatc atagcgatgt ttcttaagtt actatagtaa gggcaatgac 1680
ccgtggcgtt tggggatcgc cttatcctgg tttggatatt ctccccatgt taaaagcacc 1740

taagcatagc gctatggtgc ttttattatg taaaacatga aggaggttat aaaattgaaa 1800
tatgtattag ggttggatat tggaatcgca tcctgtggtt gggcagtaat aaatcaagaa 1860
aaacatagaa tagaagattt aggggttcgt atttttgata aagctgaaaa ccccaaagat 1920
ggaaaatctt tagctacacc tagacgtgac gcacgctcta ccaggagaat cctacgccgt 1980
aaaaaacata gaatgcaacg gataaagata cttttagtaa agcatggctt actatccaag 2040
accgaactcg accatctata tgaaagtgct actgagaaca atgtttggga cttacgttta 2100
aatgctctag atcacagatt aaatccaaaa gagtttgcca gagttcttat tcaccttgct 2160
aaaagaagag gatttaaatc taatagaaaa gaaacaactc tttcggaaaa cggacaaatc 2220
ttagaaaaca tttcagagaa cctgcagatt atggagcaaa aaaattatag aactgttgga 2280
gaaatgattc taaaagataa aaaatttgaa aaccataaaa gaaacaaaga tggtacttat 2340
atcgggactg ttactcggca acaacttcaa gaagaaatac aaatgatatt ccatgcacaa 2400
cgtttgtaca aaaatgatca tgcaacagag aaatttgaaa gtagctactt agaaatctgg 2460
gcatctcaac gtccctatgc ttctaaagat caaattgaaa aaatgattgg ctactgtacc 2520
ttagagccaa aagaaaaaag agtgcctaaa gcttcttggt catttcaata ttttgtagcg 2580
ctacaaacca taaacaacct ccgactgatc agtaaagaca gaatagaaga actctccttt 2640
gaagaaaaga aacgaattat gaatttagca ctcgaaaaat caattgttaa atacattgat 2700
atccgaaaat tattatctgt tcctaatgaa tttcaattta atagcctatc atattcagct 2760
gatacagtag atacaacggt ggaaagtaga aaatgtatag aactcaaaga atatcattct 2820
attaacaagg tatataaaca aatttacgga cagaacgccc taaatttatt acctaaagat 2880
tacgatacaa ttgcatacgg acttactatc tttaaagatg ataaagatat cctagcatat 2940
ttacaaaata aatacgtaaa tgcaaaagga aaatctataa gtaatttagc taacaagacc 3000
tacgatgata catttattca ggtattattg actctgaatt tctcaaaaat gggacattta 3060
tcatttaaag ctttaaaaaa tattattcct tttttagaag aaggtctatc ctacgataaa 3120
gcatgtgaga aggcaggata caactttaag ggaacatcct acacaaaaaa aacaaagtac 3180
ctaccagtta tccctcagaa tacaaatcct gttgtacatc gtgcactttc acagacaaga 3240
aaagttataa atgccattat taaaaaatat ggttctccaa acgcaatcca tattgaaact 3300
gctagagaat tatcgaaaac atttcaagaa agaaaagaaa tagatagtat gtaccaggat 3360
aactctaaaa agaatgaaca cgccattcat aagttaaaag aattggggaa cattaacccc 3420
tcaggaataa atattgtgaa atttaagctt tggaatgaac aagatagtag atgtatgtat 3480
tctggaaaat atatcgaacc acatcgatta tttgaagaag gttatacgga agtagatcat 3540
atactcccct atagccggag cttagatgac agctataaca ataaagcatt aacgctagga 3600
acagaaaatc agggtaaagg aaacaaaact ccttatgaat acatggggaa tacttcaatt 3660
tgggatgaat ttgaaacaag agtacagtca aataaaaaaa tcaacaaaaa gaaacaacaa 3720
aaattacttt tacaaaactt ttcacatgcg agagaacaag agtttattga acgcaatctc 3780
aatgataccc gttatgccac aatatatcta tcatccctta ttcaacaaca cctcatattc 3840
tctgaaagct ctcgtgagaa aaaagttcat acagttagtg gaattataac atctcatttt 3900
cgtagtagat ggggatttaa caaagatagg aaagaggggc atatccatca tgctttagat 3960
gcggtaatcg ttgctgtcac atccgatcac atgattaaaa gagtaacaaa atattataag 4020
ttaaaagagt taaatagaaa ctttcaagcc aaacggatgc agtttcctga accttgggaa 4080
ggatttcgtt tagagttaga agcaagggtt tctccaaatc cccagcaata tttaaagggt 4140
attcgtttta aaaattacgc cgatatcaat ctatccgaaa tcaaacctat ctttgtttca 4200
agaatgccta agcgctctat tactggtgaa ttacatcaag aaacgatccg gaaattcctt 4260
ggttataacg aaaaggggaa agtgctaacc gcaataaaaa caaaattaga agatattcct 4320
tttgattcca atggtgattt ttccatgtat gggaaagaaa ctgatttata cacgtataat 4380
gccataaaag aacgttattt aagtcataaa aaagataaac gcaaatcatt ccaagaacct 4440
ttatacaaac ccacaaaatc aggaggaatt ggaccactta taaaatctat taaaataatt 4500
gatacaagaa ccattgttaa tcctgttaat aaaggtaaag gtgtcgtgta taatagtaaa 4560
attgctcgta ctgatgtatt caaaaaggat gagaagtact atttaattcc catttacaca 4620
attgatctta tgaaaaatat cttacctcaa aaagcaatta cagcaggaaa aggatataag 4680
gattggacaa ccattaatcc cagctttact tttttattta gtctttttcc caatgatcta 4740
atcaaaattg tccccgcaaa aaataaaaca attaaagcta gaacaactgt attgaaaaaa 4800
gaggtattac taccctcact cacaggatat tttaaaggtg ttcatagtgg aactgctggg 4860
attactgttg aaacacacga tggcagtatt atagcaaatg taggttccaa gcaattacta 4920
tcatttgaga aatatcaagt agatgtactt ggacattata caaaaataaa gaaggaaaaa 4980
cgtattggca tggcgatata gaatggtaac aaaaagagcg cagcttgcta atatgaaact 5040
ttcaataggg aaacatacgc aaacaaagaa gatactatct ctgcacaact taatttattt 5100
aaaaaagaaa aaaatcgcat tctaaacaaa tgaaatccct cattttttaa attaaaaaac 5160
cttgtctaat cctctagtga caaggttttt tcagcatcta tcagaacata gcgatgtttc 5220
ttatggaact atagcgtccc ataaacctta aattcatata cagttatcat agcatagcga 5280
tttctcttat ggaactatag ccccgacgat cgtccccatt cttccctgaa caccatagca 5340
tagcgatgct tcttatggaa ctatagcaag agactcggtt ttctgactag gattcagacc 5400
atagcatagc gatgtttctt atggaactat agcactacct agtgcatgaa ttgcttgcaa 5460
tcgaccatag tatagcgatg tttcttatgg aactatagct caccgaacac aaagcgtgta 5520

gccatactaa ccatatcata gcgatgcttc ttatggaact acagctcgcc gcccataaac 5580
aagcttattt tctatcatct tatattaatt tttcttatga aacagtatcc tattggcctt 5640
acttcatgaa gactcaaatg tcataaccgt atttccagat aaaattacat cttatcccat 5700
caagacacac cattctcatt atatactgca atgctttata tatattttta acaaaaatat 5760
atattccaaa cctaaaggac taaatataac ttataaattt acttatcaaa tagcctccag 5820
caaggaattt gtacacttac tcttactttt aaatcagtaa gttttagtaa tacatttaca 5880
taaataatcc tttcgcctat aaacaaaaga attatgttat aatatctact gaacatatga 5940
ataggataac aactacctgc tgttggcaag tagaaattta tctggttctg gtgcaaaaaa 6000
ctaaatgagt tgaaaatgtt ccttcaaact tggctatact ggtattacta ctttaaaaag 6060
gtgtg 6065

<210> 73
<211> 3204
<212> DNA
<213> Unknown

<220>
<223> cas9 Gene ORF in Locus 42 from Bacillus species

<400> 73
ttgaaatatg tattagggtt ggatattgga atcgcatcct gtggttgggc agtaataaat 60
caagaaaaac atagaataga agatttaggg gttcgtattt ttgataaagc tgaaaacccc 120
aaagatggaa aatctttagc tacacctaga cgtgacgcac gctctaccag gagaatccta 180
cgccgtaaaa aacatagaat gcaacggata aagatacttt tagtaaagca tggcttacta 240
tccaagaccg aactcgacca tctatatgaa agtgctactg agaacaatgt ttgggactta 300
cgtttaaatg ctctagatca cagattaaat ccaaaagagt ttgccagagt tcttattcac 360
cttgctaaaa gaagaggatt taaatctaat agaaaagaaa caactctttc ggaaaacgga 420
caaatcttag aaaacatttc agagaacctg cagattatgg agcaaaaaaa ttatagaact 480
gttggagaaa tgattctaaa agataaaaaa tttgaaaacc ataaaagaaa caaagatggt 540
acttatatcg ggactgttac tcggcaacaa cttcaagaag aaatacaaat gatattccat 600
gcacaacgtt tgtacaaaaa tgatcatgca acagagaaat ttgaaagtag ctacttagaa 660
atctgggcat ctcaacgtcc ctatgcttct aaagatcaaa ttgaaaaaat gattggctac 720
tgtaccttag agccaaaaga aaaaagagtg cctaaagctt cttggtcatt tcaatatttt 780
gtagcgctac aaaccataaa caacctccga ctgatcagta aagacagaat agaagaactc 840
tcctttgaag aaaagaaacg aattatgaat ttagcactcg aaaaatcaat tgttaaatac 900
attgatatcc gaaaattatt atctgttcct aatgaatttc aatttaatag cctatcatat 960
tcagctgata cagtagatac aacggtggaa agtagaaaat gtatagaact caaagaatat 1020
cattctatta acaaggtata taaacaaatt tacggacaga acgccctaaa tttattacct 1080
aaagattacg atacaattgc atacggactt actatcttta aagatgataa agatatccta 1140
gcatatttac aaaataaata cgtaaatgca aaaggaaaat ctataagtaa tttagctaac 1200
aagacctacg atgatacatt tattcaggta ttattgactc tgaatttctc aaaaatggga 1260
catttatcat ttaaagcttt aaaaaatatt attccttttt tagaagaagg tctatcctac 1320
gataaagcat gtgagaaggc aggatacaac tttaagggaa catcctacac aaaaaaaaca 1380
aagtacctac cagttatccc tcagaataca aatcctgttg tacatcgtgc actttcacag 1440
acaagaaaag ttataaatgc cattattaaa aaatatggtt ctccaaacgc aatccatatt 1500
gaaactgcta gagaattatc gaaaacattt caagaaagaa aagaaataga tagtatgtac 1560
caggataact ctaaaaagaa tgaacacgcc attcataagt taaaagaatt ggggaacatt 1620
aacccctcag gaataaatat tgtgaaattt aagctttgga atgaacaaga tagtagatgt 1680
atgtattctg gaaaatatat cgaaccacat cgattatttg aagaaggtta tacggaagta 1740
gatcatatac tcccctatag ccggagctta gatgacagct ataacaataa agcattaacg 1800
ctaggaacag aaaatcaggg taaaggaaac aaaactcctt atgaatacat ggggaatact 1860
tcaatttggg atgaatttga aacaagagta cagtcaaata aaaaaatcaa caaaaagaaa 1920
caacaaaaat tacttttaca aaacttttca catgcgagag aacaagagtt tattgaacgc 1980
aatctcaatg atacccgtta tgccacaata tatctatcat cccttattca acaacacctc 2040
atattctctg aaagctctcg tgagaaaaaa gttcatacag ttagtggaat tataacatct 2100
cattttcgta gtagatgggg atttaacaaa gataggaaag aggggcatat ccatcatgct 2160
ttagatgcgg taatcgttgc tgtcacatcc gatcacatga ttaaaagagt aacaaaatat 2220
tataagttaa aagagttaaa tagaaacttt caagccaaac ggatgcagtt tcctgaacct 2280
tgggaaggat ttcgtttaga gttagaagca agggtttctc caaatcccca gcaatattta 2340
aagggtattc gttttaaaaa ttacgccgat atcaatctat ccgaaatcaa acctatcttt 2400
gtttcaagaa tgcctaagcg ctctattact ggtgaattac atcaagaaac gatccggaaa 2460
ttccttggtt ataacgaaaa ggggaaagtg ctaaccgcaa taaaaacaaa attagaagat 2520
attccttttg attccaatgg tgatttttcc atgtatggga aagaaactga tttatacacg 2580

tataatgcca taaaagaacg ttatttaagt cataaaaaag ataaacgcaa atcattccaa 2640
gaacctttat acaaacccac aaaatcagga ggaattggac cacttataaa atctattaaa 2700
ataattgata caagaaccat tgttaatcct gttaataaag gtaaaggtgt cgtgtataat 2760
agtaaaattg ctcgtactga tgtattcaaa aaggatgaga agtactattt aattcccatt 2820
tacacaattg atcttatgaa aaatatctta cctcaaaaag caattacagc aggaaaagga 2880
tataaggatt ggacaaccat taatcccagc tttacttttt tatttagtct ttttcccaat 2940
gatctaatca aaattgtccc cgcaaaaaat aaaacaatta aagctagaac aactgtattg 3000
aaaaaagagg tattactacc ctcactcaca ggatatttta aaggtgttca tagtggaact 3060
gctgggatta ctgttgaaac acacgatggc agtattatag caaatgtagg ttccaagcaa 3120
ttactatcat ttgagaaata tcaagtagat gtacttggac attatacaaa aataaagaag 3180
gaaaaacgta ttggcatggc gata 3204

<210> 74
<211> 1068
<212> PRT
<213> Unknown

<220>
<223> Translation of cas9 Gene ORF in Locus 42 from Bacillus species

<400> 74
Met Lys Tyr Val Leu Gly Leu Asp Ile Gly Ile Ala Ser Cys Gly Trp
1               5                   10                  15
Ala Val Ile Asn Gln Glu Lys His Arg Ile Glu Asp Leu Gly Val Arg
            20                  25                  30
Ile Phe Asp Lys Ala Glu Asn Pro Lys Asp Gly Lys Ser Leu Ala Thr
        35                  40                  45
Pro Arg Arg Asp Ala Arg Ser Thr Arg Arg Ile Leu Arg Arg Lys Lys
    50                  55                  60
His Arg Met Gln Arg Ile Lys Ile Leu Leu Val Lys His Gly Leu Leu
65                  70                  75                  80
Ser Lys Thr Glu Leu Asp His Leu Tyr Glu Ser Ala Thr Glu Asn Asn
                85                  90                  95
Val Trp Asp Leu Arg Leu Asn Ala Leu Asp His Arg Leu Asn Pro Lys
            100                 105                 110
Glu Phe Ala Arg Val Leu Ile His Leu Ala Lys Arg Arg Gly Phe Lys
        115                 120                 125
Ser Asn Arg Lys Glu Thr Thr Leu Ser Glu Asn Gly Gln Ile Leu Glu
    130                 135                 140
Asn Ile Ser Glu Asn Leu Gln Ile Met Glu Gln Lys Asn Tyr Arg Thr
145                 150                 155                 160
Val Gly Glu Met Ile Leu Lys Asp Lys Lys Phe Glu Asn His Lys Arg
                165                 170                 175
Asn Lys Asp Gly Thr Tyr Ile Gly Thr Val Thr Arg Gln Gln Leu Gln
            180                 185                 190
Glu Glu Ile Gln Met Ile Phe His Ala Gln Arg Leu Tyr Lys Asn Asp
        195                 200                 205
His Ala Thr Glu Lys Phe Glu Ser Ser Tyr Leu Glu Ile Trp Ala Ser
    210                 215                 220
Gln Arg Pro Tyr Ala Ser Lys Asp Gln Ile Glu Lys Met Ile Gly Tyr
225                 230                 235                 240
Cys Thr Leu Glu Pro Lys Glu Lys Arg Val Pro Lys Ala Ser Trp Ser
                245                 250                 255
Phe Gln Tyr Phe Val Ala Leu Gln Thr Ile Asn Asn Leu Arg Leu Ile
            260                 265                 270
Ser Lys Asp Arg Ile Glu Glu Leu Ser Phe Glu Glu Lys Lys Arg Ile
        275                 280                 285
Met Asn Leu Ala Leu Glu Lys Ser Ile Val Lys Tyr Ile Asp Ile Arg
    290                 295                 300
Lys Leu Leu Ser Val Pro Asn Glu Phe Gln Phe Asn Ser Leu Ser Tyr
305                 310                 315                 320
Ser Ala Asp Thr Val Asp Thr Thr Val Glu Ser Arg Lys Cys Ile Glu
                325                 330                 335

Leu Lys Glu Tyr His Ser Ile Asn Lys Val Tyr Lys Gln Ile Tyr Gly
                340                 345                 350
Gln Asn Ala Leu Asn Leu Leu Pro Lys Asp Tyr Asp Thr Ile Ala Tyr
        355                 360                 365
Gly Leu Thr Ile Phe Lys Asp Asp Lys Asp Ile Leu Ala Tyr Leu Gln
    370                 375                 380
Asn Lys Tyr Val Asn Ala Lys Gly Lys Ser Ile Ser Asn Leu Ala Asn
385                 390                 395                 400
Lys Thr Tyr Asp Asp Thr Phe Ile Gln Val Leu Leu Thr Leu Asn Phe
                405                 410                 415
Ser Lys Met Gly His Leu Ser Phe Lys Ala Leu Lys Asn Ile Ile Pro
            420                 425                 430
Phe Leu Glu Glu Gly Leu Ser Tyr Asp Lys Ala Cys Glu Lys Ala Gly
        435                 440                 445
Tyr Asn Phe Lys Gly Thr Ser Tyr Thr Lys Lys Thr Lys Tyr Leu Pro
    450                 455                 460
Val Ile Pro Gln Asn Thr Asn Pro Val Val His Arg Ala Leu Ser Gln
465                 470                 475                 480
Thr Arg Lys Val Ile Asn Ala Ile Ile Lys Lys Tyr Gly Ser Pro Asn
                485                 490                 495
Ala Ile His Ile Glu Thr Ala Arg Glu Leu Ser Lys Thr Phe Gln Glu
            500                 505                 510
Arg Lys Glu Ile Asp Ser Met Tyr Gln Asp Asn Ser Lys Lys Asn Glu
        515                 520                 525
His Ala Ile His Lys Leu Lys Glu Leu Gly Asn Ile Asn Pro Ser Gly
    530                 535                 540
Ile Asn Ile Val Lys Phe Lys Leu Trp Asn Glu Gln Asp Ser Arg Cys
545                 550                 555                 560
Met Tyr Ser Gly Lys Tyr Ile Glu Pro His Arg Leu Phe Glu Glu Gly
                565                 570                 575
Tyr Thr Glu Val Asp His Ile Leu Pro Tyr Ser Arg Ser Leu Asp Asp
            580                 585                 590
Ser Tyr Asn Asn Lys Ala Leu Thr Leu Gly Thr Glu Asn Gln Gly Lys
        595                 600                 605
Gly Asn Lys Thr Pro Tyr Glu Tyr Met Gly Asn Thr Ser Ile Trp Asp
    610                 615                 620
Glu Phe Glu Thr Arg Val Gln Ser Asn Lys Lys Ile Asn Lys Lys Lys
625                 630                 635                 640
Gln Gln Lys Leu Leu Leu Gln Asn Phe Ser His Ala Arg Glu Gln Glu
                645                 650                 655
Phe Ile Glu Arg Asn Leu Asn Asp Thr Arg Tyr Ala Thr Ile Tyr Leu
            660                 665                 670
Ser Ser Leu Ile Gln Gln His Leu Ile Phe Ser Glu Ser Ser Arg Glu
        675                 680                 685
Lys Lys Val His Thr Val Ser Gly Ile Ile Thr Ser His Phe Arg Ser
    690                 695                 700
Arg Trp Gly Phe Asn Lys Asp Arg Lys Glu Gly His Ile His His Ala
705                 710                 715                 720
Leu Asp Ala Val Ile Val Ala Val Thr Ser Asp His Met Ile Lys Arg
                725                 730                 735
Val Thr Lys Tyr Tyr Lys Leu Lys Glu Leu Asn Arg Asn Phe Gln Ala
            740                 745                 750
Lys Arg Met Gln Phe Pro Glu Pro Trp Glu Gly Phe Arg Leu Glu Leu
        755                 760                 765
Glu Ala Arg Val Ser Pro Asn Pro Gln Gln Tyr Leu Lys Gly Ile Arg
    770                 775                 780
Phe Lys Asn Tyr Ala Asp Ile Asn Leu Ser Glu Ile Lys Pro Ile Phe
785                 790                 795                 800
Val Ser Arg Met Pro Lys Arg Ser Ile Thr Gly Glu Leu His Gln Glu
                805                 810                 815
Thr Ile Arg Lys Phe Leu Gly Tyr Asn Glu Lys Gly Lys Val Leu Thr
            820                 825                 830
Ala Ile Lys Thr Lys Leu Glu Asp Ile Pro Phe Asp Ser Asn Gly Asp

835                 840                 845
Phe Ser Met Tyr Gly Lys Glu Thr Asp Leu Tyr Thr Tyr Asn Ala Ile
    850                 855                 860
Lys Glu Arg Tyr Leu Ser His Lys Lys Asp Lys Arg Lys Ser Phe Gln
865                 870                 875                 880
Glu Pro Leu Tyr Lys Pro Thr Lys Ser Gly Gly Ile Gly Pro Leu Ile
                885                 890                 895
Lys Ser Ile Lys Ile Ile Asp Thr Arg Thr Ile Val Asn Pro Val Asn
            900                 905                 910
Lys Gly Lys Gly Val Val Tyr Asn Ser Lys Ile Ala Arg Thr Asp Val
        915                 920                 925
Phe Lys Lys Asp Glu Lys Tyr Tyr Leu Ile Pro Ile Tyr Thr Ile Asp
    930                 935                 940
Leu Met Lys Asn Ile Leu Pro Gln Lys Ala Ile Thr Ala Gly Lys Gly
945                 950                 955                 960
Tyr Lys Asp Trp Thr Thr Ile Asn Pro Ser Phe Thr Phe Leu Phe Ser
                965                 970                 975
Leu Phe Pro Asn Asp Leu Ile Lys Ile Val Pro Ala Lys Asn Lys Thr
            980                 985                 990
Ile Lys Ala Arg Thr Thr Val Leu  Lys Lys Glu Val Leu  Leu Pro Ser
        995                 1000                1005
Leu Thr  Gly Tyr Phe Lys Gly  Val His Ser Gly Thr  Ala Gly Ile
    1010                1015                1020
Thr Val  Glu Thr His Asp Gly  Ser Ile Ile Ala Asn  Val Gly Ser
    1025                1030                1035
Lys Gln  Leu Leu Ser Phe Glu  Lys Tyr Gln Val Asp  Val Leu Gly
    1040                1045                1050
His Tyr  Thr Lys Ile Lys Lys  Glu Lys Arg Ile Gly  Met Ala Ile
    1055                1060                1065

<210>  75
<211>  36
<212>  DNA
<213>  Unknown

<220>
<223>  CRISPR Array Repeat Consensus for Locus 42  from Bacillus species

<400>  75
atcatagcat agcgatgttt cttatggaac tatagc                              36

<210>  76
<211>  36
<212>  DNA
<213>  unknown

<220>
<223>  CRISPR Array Anti-Repeat for Locus 42  from Bacillus species

<400>  76
atcatatcat agcgatgttt cttaagttac tatagt                              36

<210>  77
<211>  18
<212>  RNA
<213>  unknown

<220>
<223>   Repeat Region in Single Guide RNA for Locus 42 from Bacillus
       species

<400>  77
gcuauaguuc cauaagaa                                                  18

<210>  78
<211>  18
<212>  RNA
<213>  unknown

<220>
<223>  Anti-Repeat Region in sgRNA for Locus 42 from Bacillus species

<400>  78
uucuuaaguu acuauagu                                                    18

<210>  79
<211>  99
<212>  RNA
<213>  unknown

<220>
<223>  Prime tracrRNA in sgRNA for Locus 42 from Bacillus species

<400>  79
aagggcaaug acccguggcg uuuggggauc gccuuauccu gguuuggaua uucuccccau       60
guuaaaagca ccuaagcaua gcgcuauggu gcuuuuauu                             99

<210>  80
<211>  159
<212>  RNA
<213>  Artificial Sequence

<220>
<223>  synthesized sgRNA Solution for Locus 42

<220>
<221>  misc_feature
<222>  (1)..(20)
<223>  n is a, c, g, or u

<400>  80
nnnnnnnnnn nnnnnnnnnn gcuauaguuc cauaagaaga aauucuuaag uuacuauagu      60
aagggcaaug acccguggcg uuuggggauc gccuuauccu gguuuggaua uucuccccau      120
guuaaaagca ccuaagcaua gcgcuauggu gcuuuuauu                            159

## Description

This application claims the benefit of U.S. Provisional Application No. 62/306926, filed March 11, 2016, and U.S. Provisional Application No. 62/370858, filed August 04, 2016, both of which are hereby incorporated herein in their entirety by reference.

### FIELD

The disclosure relates to the field of plant molecular biology, in particular, to compositions for novel guided Cas9 endonuclease systems and compositions and methods for altering the genome of a cell.

### REFERENCE TO SEQUENCE LISTING SUBMITTED ELECTRONICALLY

The official copy of the sequence listing is submitted electronically via EFS-Web as an ASCII formatted sequence listing with a file named 20170222_BB2554PCT_ST25.txt created on February 22, 2017 and having a size of 207 kilobytes and is filed concurrently with the specification. The sequence listing contained in this ASCII formatted document is part of the specification and is herein incorporated by reference in its entirety.

### BACKGROUND

Recombinant DNA technology has made it possible to modify (edit) specific endogenous chromosomal sequences and/or insert DNA sequences at targeted genomic locations, thus altering the organism's phenotype. Site-specific integration techniques, which employ site-specific recombination systems, as well as other types of recombination technologies, have been used to generate targeted insertions of genes of interest in a variety of organism. Genome-editing techniques such as designer zinc finger nucleases (ZFNs) or transcription activator-like effector nucleases (TALENs), or homing meganucleases, are available for producing targeted genome perturbations, but these systems tends to have a low specificity and employ designed nucleases that need to be redesigned for each target site, which renders them costly and time-consuming to prepare.

Although several approaches have been developed to target a specific site for modification in the genome of an organism, there still remains a need for new

genome engineering technologies that are affordable, easy to set up, scalable, and amenable to targeting multiple positions within the genome of an organism.

### BRIEF SUMMARY

Compositions and methods are provided for novel Cas9 systems and elements comprising such systems, including, but not limiting to, novel guide polynucleotide/Cas9 endonucleases complexes, single guide RNAs, guide RNA elements, and Cas9 endonucleases. The novel Cas9 systems described herein are derived from CRISPR-Cas loci that differ from type II CRISPR-Cas loci, in that the novel CRISPR-Cas loci described herein contain multiple CRISPR arrays flanking a tracrRNA encoding region and cas9 gene and does not contain a cas1 gene and a cas2 gene.

In one embodiment of the disclosure, the guide RNA is a single guide RNA capable of forming a guide RNA/Cas9 endonuclease complex, wherein said guide RNA/Cas9 endonuclease complex can recognize, bind to, and optionally nick or cleave a target sequence, said single guide RNA is selected from the group consisting of SEQ ID NOs: 41, 42, 43, 44, 45, 66, 67, 80, a functional fragment of SEQ ID NOs: 41, 42, 43, 44, 45, 66, 67, 80, and a functional variant of SEQ ID NOs: 41, 42, 43, 44, 45, 66, 67, 80.

In one embodiment of the disclosure, the guide RNA is single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79.

In one embodiment of the disclosure, the guide RNA is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77.

The guide RNA can also be a duplex molecule comprising a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77, wherein said tracrRNA comprises a nucleotide sequence selected from the group consisting of SEQ ID NOs : 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79, wherein said chimeric non-naturally occurring crRNA comprises a variable targeting domain capable of hybridizing to said target sequence.

In one embodiment of the disclosure, the guide RNA/Cas9 endonuclease complex is a guide RNA/Cas9 endonuclease comprising at least one guide RNA and a Cas9 endonuclease , wherein said Cas9 endonuclease is selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA/Cas9 endonuclease complex is capable of recognizing, binding to, and optionally nicking or cleaving all or part of a target sequence..

In one embodiment of the disclosure, the method is a method for modifying a target site in the genome of a cell, the method comprising introducing into said cell at least one guide RNA and at least one Cas9 endonuclease protein selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA and Cas9 endonuclease can form a complex that is capable of recognizing, binding to, and optionally nicking or cleaving all or part of said target site. The method can further comprise identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).

In one embodiment of the disclosure, the method is a method for editing a nucleotide sequence in the genome of a cell, the method comprising introducing into said cell a polynucleotide modification template, at least one guide RNA, and at least one Cas9 endonuclease protein selected from the group consisting of SEQ ID NOs: consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.

In one embodiment of the disclosure, the method is a method for modifying a target site in the genome of a cell, the method comprising introducing into said cell at least one guide RNA, at least one donor DNA, and at least one Cas9 endonuclease selected from the group consisting of SEQ ID NOs: SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said at least one guide RNA and at least one Cas9 endonuclease can form a complex that is capable of recognizing, binding to, and optionally nicking or cleaving all or part of said target site, wherein said donor DNA comprises a polynucleotide of interest.

Also provided are nucleic acid constructs, plants, plant cells, explants, seeds and grain having an altered target site or altered polynucleotide of interest produced by the methods described herein. Additional embodiments of the methods and compositions of the present disclosure are shown herein.

### BRIEF DESCRIPTION OF THE DRAWINGS AND THE SEQUENCE LISTING

The disclosure can be more fully understood from the following detailed description and the accompanying drawings and Sequence Listing, which form a part of this application. The sequence descriptions and sequence listing attached hereto comply with the rules governing nucleotide and amino acid sequence disclosures in patent applications as set forth in 37 C.F.R. §§1.821-1.825. The sequence descriptions contain the three letter codes for amino acids as defined in 37 C.F.R. §§ 1.821-1.825, which are incorporated herein by reference.

### Figures

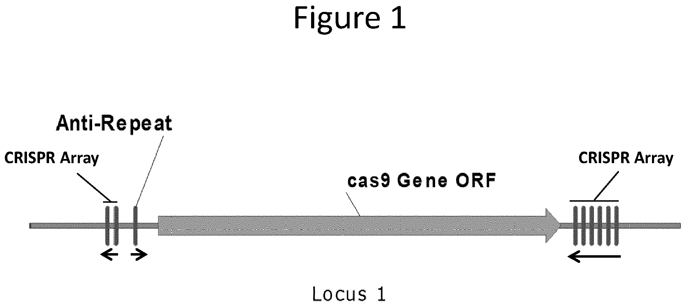

Figure 1 shows a diagram of a genomic DNA region from *Bacillus species* representing a CRISPR-Cas locus (referred to as Locus 1) described herein. Arrows indicate the transcriptional directional of the anti-repeat and the CRISPR repeats within the CRISPR array (CRISPR array). Cas9 Gene ORF refers to the open reading frame of the Cas9 endonuclease. Locus 1 differs from a type II CRISPR-Cas locus, in that it contains two CRISPR arrays flanking a tracrRNA encoding region and *cas9* gene and does not contain a *cas1* gene and a *cas2* gene.

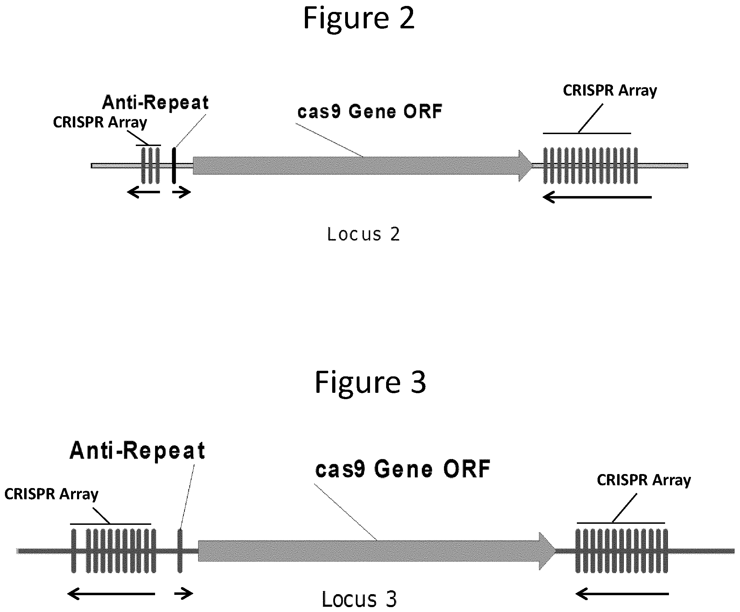

Figure 2 shows a diagram of a genomic DNA region from *Bacillus species* representing a CRISPR-Cas locus (referred to as Locus 2) described herein. Arrows indicate the transcriptional directional of the anti-repeat and the CRISPR repeats within the CRISPR array (CRISPR array). Cas9 Gene ORF refers to the open reading frame of the Cas9 endonuclease. Locus 2 differs from a type II CRISPR-Cas locus, in that it contains two CRISPR arrays flanking a tracrRNA encoding region and *cas9* gene and does not contain a *cas1* gene and a *cas2* gene.
Figure 3 shows a diagram of a genomic DNA region from *Bacillus* species representing a CRISPR-Cas locus (referred to as Locus 3) described herein. Arrows indicate the transcriptional directional of the anti-repeat and the CRISPR repeats within the CRISPR array (CRISPR array). Cas9 Gene ORF refers to the open reading frame of the Cas9 endonuclease. Locus 3 differs from a type II CRISPR-Cas locus, in that it contains two CRISPR arrays flanking a tracrRNA encoding region and *cas9* gene and does not contain a *cas1* gene and a *cas2* gene.

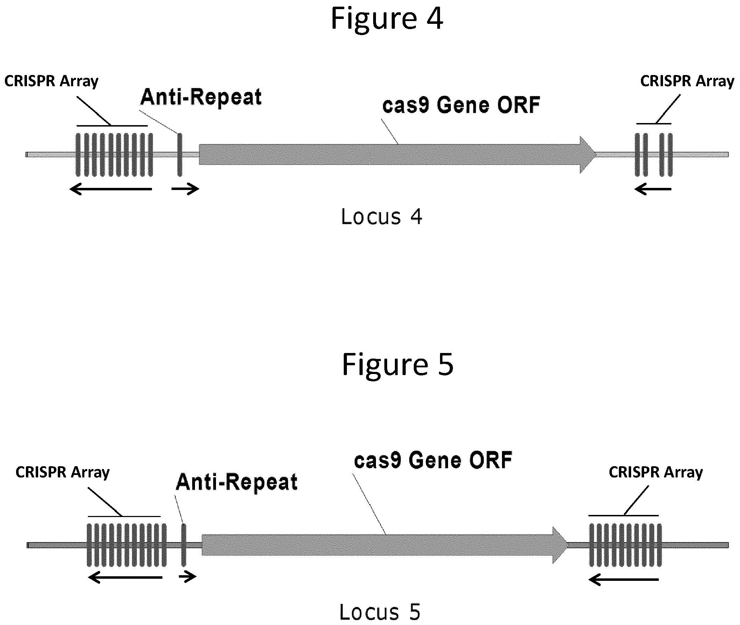

Figure 4 shows a diagram of a genomic DNA region from *Bacillus species* representing a CRISPR-Cas locus (referred to as Locus 4) described herein. Arrows indicate the transcriptional directional of the anti-repeat and the CRISPR repeats within the CRISPR array (CRISPR array). Cas9 Gene ORF refers to the open reading frame of the Cas9 endonuclease. Locus 4 differs from a type II CRISPR-Cas locus, in that it contains two CRISPR arrays flanking a tracrRNA encoding region and *cas9* gene and does not contain a *cas1* gene and a *cas2* gene.
Figure 5 shows a diagram of a genomic DNA region from unknown origin representing a CRISPR-Cas locus (referred to as Locus 5) described herein. Arrows indicate the transcriptional directional of the anti-repeat and the CRISPR repeats within the CRISPR array (CRISPR array). Cas9 Gene ORF refers to the open reading frame of the Cas9 endonuclease. Locus 5 differs from a type II CRISPR-Cas locus, in that it contains two CRISPR arrays flanking a tracrRNA encoding region and *cas9* gene and does not contain a *cas1* gene and a *cas2* gene.

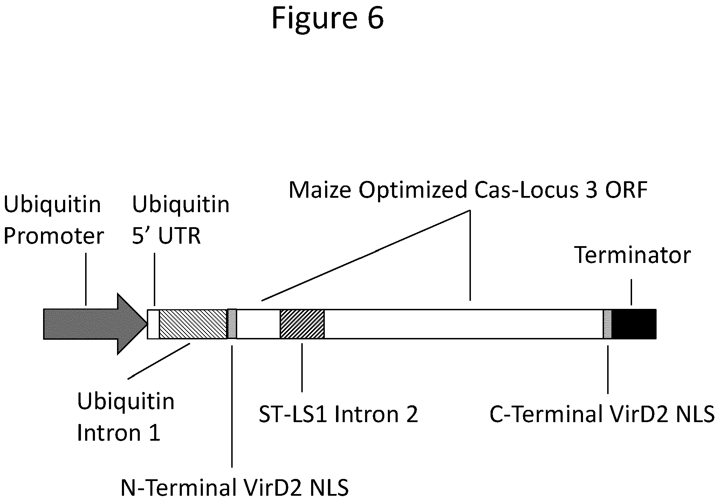

Figure 6 depicts an expression cassette comprising a maize optimized Cas-Locus 3 Open reading Frame (ORF) endonuclease gene (Maize Optimized Cas-Locus 3 ORF) encoding a Cas-Locus-3 endonuclease protein. Components include a Ubiquitin Promoter operably linked to a Ubiquitin 5 prime untranslated region (5'UTR), Ubiquitin intron 1, a N-Terminal VirD2 nuclear Localization signal (NLS), a maize optimized Cas-Locus 3 Open reading Frame (ORF) endonuclease gene comprising a ST-LS1 intron-2 , a C-terminal VirD2 nuclear localization signal (NLS) and a terminator.

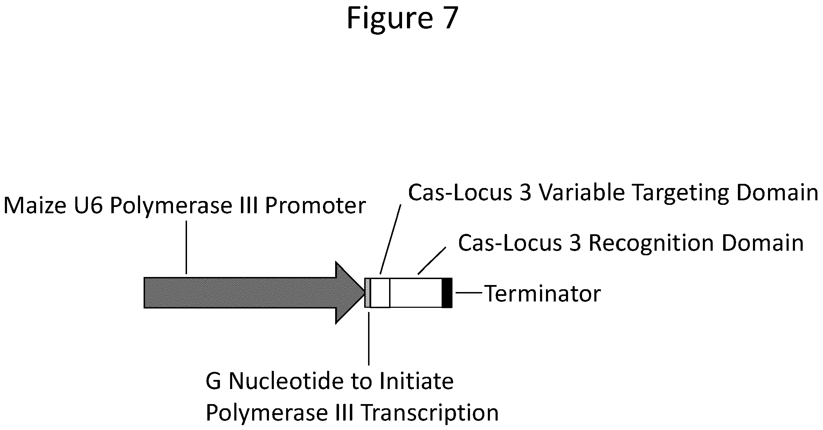

Figure 7 depicts an expression cassette comprising a maize U6 Polymerase III Promoter operably linked to a DNA sequence encoding a guide polynucleotide that can form a functional complex with a Cas-Locus 3 endonuclease capable of binding, nicking or cleaving a single stranded or double stranded target site. Components include a maize U6 Polymerase III Promoter operably linked to a G nucleotide to initiate Pol-III transcription, a Cas-Locus 3 variable targeting domain, a Cas-Locus 3 recognition domain, and a terminator.

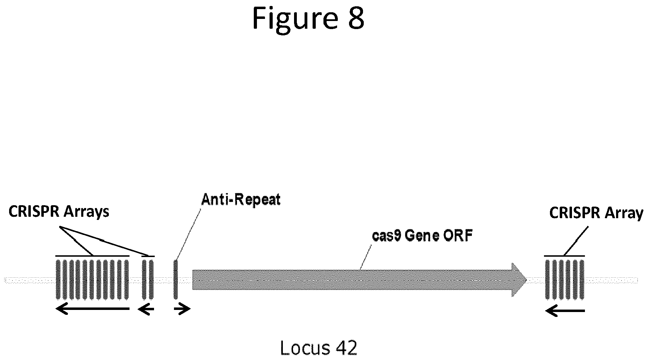

Figure 8 shows a diagram of a genomic DNA region from *Bacillus species* representing a CRISPR-Cas locus (referred to as Locus 42) described herein. Arrows indicate the transcriptional directional of the anti-repeat and the CRISPR repeats within the CRISPR array (CRISPR array). *Cas9* Gene ORF refers to the open reading frame of the Cas9 endonuclease. Locus 42 differs from a type II CRISPR-Cas locus, in that it contains three CRISPR arrays flanking a tracrRNA encoding region and *cas9* gene and does not contain a *cas1* gene and a *cas2* gene.

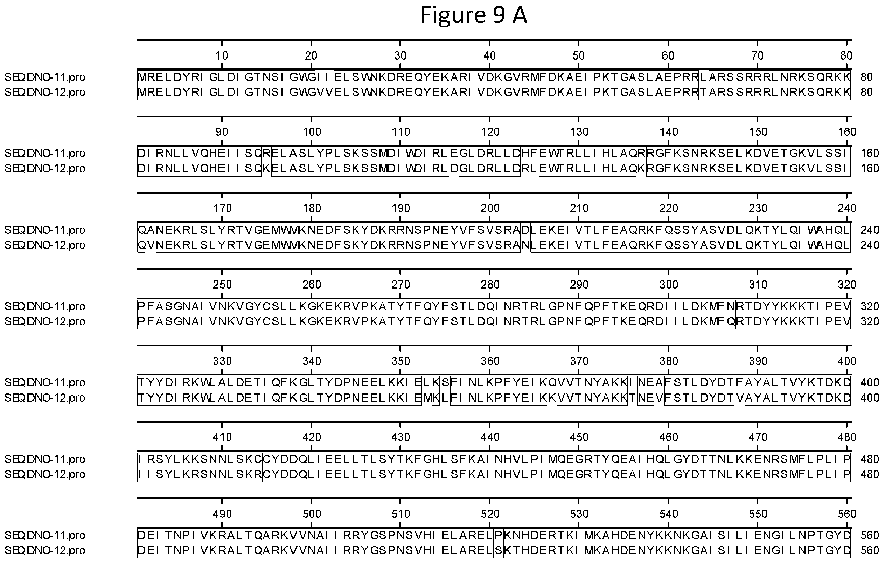

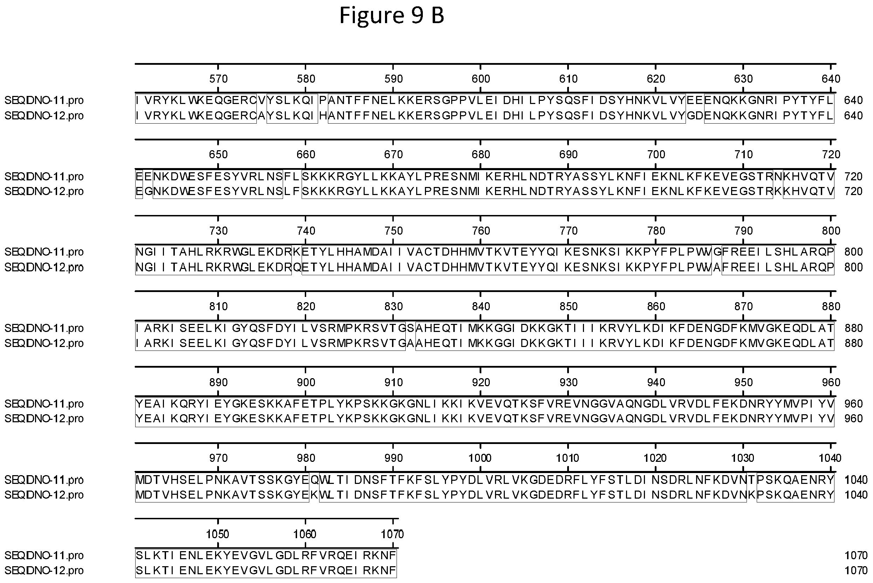

Figure 9A-9B shows an amino acid alignment of the Cas9 endonuclease from Locus 1 (SEQ ID NO: 11) and the Cas9 endonuclease from Locus 2 (SEQ ID NO: 12).

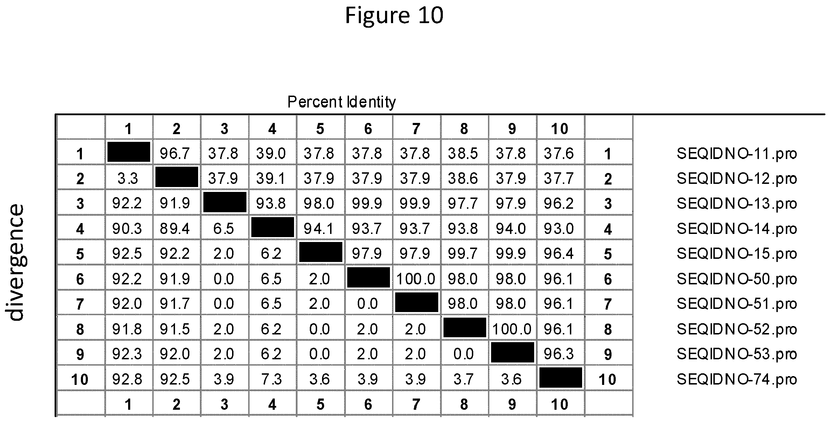

Figure 10 shows the % identity between the Cas9 endonucleases of SEQ ID NOs: 11-15, 50-53 and 74.

### Sequences

**Table 1. Summary of Nucleic Acid and Protein SEQ ID Numbers**

| Description | Nucleic acid SEQ ID NO: | Protein SEQ ID NO: |
|---|---|---|
| Locus 1 | 1 | |
| Locus 2 | 2 | |
| Locus 3 | 3 | |
| Locus 4 | 4 | |
| Locus 5 | 5 | |
| Cas9 endonuclease from Locus 1 | 6 | 11 |
| Cas9 endonuclease from Locus 2 | 7 | 12 |
| Cas9 endonuclease from Locus 3 (ORF1) | 8 | 13 |
| Cas9 endonuclease from Locus 4 | 9 | 14 |
| Cas9 endonuclease from Locus 5 (ORF1) | 10 | 15 |
| CRISPR repeat consensus from Locus 1 to Locus 5, respectively | 16-20 | |
| Anti Repeat from Locus 1 to Locus 5, respectively | 21-25 | |
| sgRNA repeat region ( Locus 1 to Locus 5, respectively) | 26-30 | |
| sgRNA anti Repeat region (Locus 1 to Locus 5, respectively) | 31-35 | |
| 3' tracrRNA in gRNA ( Locus 1 to Locus 5, respectively) | 36-40 | |
| sgRNAs | 41-45 | |
| Cas9 endonuclease of Locus 3 (Cas-Locus 3) ORF 2-Bacillus species | 46 | |
| Cas9 endonuclease of Locus 3 (Cas-Locus 3) ORF 3-Bacillus species | 47 | |
| Cas9 endonuclease of Locus 5 (Cas-Locus 5) ORF 2-Bacillus species | 48 | |
| Cas9 endonuclease of Locus 5 (Cas-Locus 5) ORF 3-Bacillus species | 49 | |
| Cas9 endonuclease (Cas-Locus 3, Variant 2) | | 50 |
| Cas9 endonuclease (Cas-Locus 3, Variant 3) | | 51 |
| Cas9 endonuclease (Cas-Locus 5, Variant 2) | | 52 |
| Cas9 endonuclease (Cas-Locus 5, Variant 3) | | 53 |
| ST-LS1 Intron 2 | 54 | |
| SV40 NLS | 55 | |
| VirD2 NLS | 56 | |
| Maize Optimized Cas-Locus 3 Endonuclease with N- and C-terminal NLSs | 57 | |
| Maize Ubiquitin Promoter | 58 | |
| Maize Ubiquitin 5 Prime UTR | 59 | |
| Maize Ubiquitin Intron 1 | 60 | |
| Maize Optimized Cas-Locus 3 Expression Cassette | 61 | |
| sgRNA Repeat Region for Cas-Locus 3 Version 2 | 62 | |
| sgRNA Repeat Region for Cas-Locus 3 Version 3 | 63 | |
| sgRNA Anti-Repeat Region for Cas-Locus 3 Version 2 | 64 | |
| sgRNA Anti-Repeat Region for Cas-Locus 3 Version 3 | 65 | |
| sgRNA for Cas-Locus 3 Version 2 | 66 | |
| sgRNA for Cas-Locus 3 Version 3 | 67 | |
| Maize U6 Polymerase III Promoter | 68 | |
| MS26 Maize Genomic Target Sequence | 69 | |
| LIGULELESS Maize Genomic Target Sequence | 70 | |
| MS45 Maize Genomic Target Sequence | 71 | |
| Locus 42 | 72 | |
| Cas9 endonuclease (from Locus 42) | 73 | 74 |
| CRISPR repeat consensus of Locus 42 | 75 | |
| Anti-Repeat of Locus 42 | 76 | |
| sgRNA repeat region of Locus 42 | 77 | |
| sgRNA anti-repeat region of Locus 42 | 78 | |
| 3' tracrRNA in gRNA (Locus 42) | 79 | |
| sgRNA | 80 | |

### DETAILED DESCRIPTION

Compositions are provided for novel Cas9 systems and elements comprising such systems, including, but not limiting to, novel guide polynucleotide/Cas9 endonuclease complexes, single guide polynucleotides, guide RNA elements, and Cas9 endonucleases. The present disclosure further includes compositions and methods for genome modification of a target sequence in the genome of a cell, for gene editing, and for inserting a polynucleotide of interest into the genome of a cell.

The term "cas gene" herein refers to one or more genes that are generally coupled, associated or close to, or in the vicinity of flanking CRISPR loci. The terms "Cas gene", "Cas endonuclease gene", "CRISPR-associated (Cas) gene" and "Clustered Regularly Interspaced Short Palindromic Repeats-associated gene" are used interchangeably herein.

CRISPR (clustered regularly interspaced short palindromic repeats) loci refers to certain genetic loci encoding components of DNA cleavage systems, for example, used by bacterial and archaeal cells to destroy foreign DNA (Horvath and Barrangou, 2010, Science 327:167-170; WO2007/025097, published March 1, 2007). A CRISPR locus can consist of a CRISPR array, comprising short direct repeats (CRISPR repeats) separated by short variable DNA sequences (called 'spacers'), which can be flanked by diverse Cas (CRISPR-associated) genes. The number of CRISPR-associated genes at a given CRISPR locus can vary between species. Multiple CRISPR/Cas systems have been described including Class 1 systems, with multisubunit effector complexes (comprising type I, type III and type IV subtypes), and Class 2 systems, with single protein effectors (comprising type II and type V subtypes, such as but not limiting to Cas9, Cpf1 ,C2c1 ,C2c2, C2c3). Class 1 systems (Makarova et al. 2015, Nature Reviews; Microbiology Vol. 13:1-15; Zetsche et al., 2015, Cell 163, 1-13; Shmakov et al., 2015, Molecular_Cell 60, 1-13; Haft et al., 2005, Computational Biology, PLoS Comput Biol 1(6): e60. doi:10.1371 /journal .pcbi. 0010060 and WO 2013/176772 A1 published on November 23, 2013 incorporated by reference herein).

The type II CRISPR/Cas system from bacteria employs a crRNA (CRISPR RNA) and tracrRNA (trans-activating CRISPR RNA) to guide the Cas endonuclease to its DNA target. The crRNA contains a spacer region complementary to one strand of the double strand DNA target and a region that base pairs with the tracrRNA (trans-activating CRISPR RNA) forming a RNA duplex that directs the Cas endonuclease to cleave the DNA target. Spacers are acquired through a not fully understood process involving Cas1 and Cas2 proteins. All type II CRISPR/Cas loci contain cas1 and cas2 genes in addition to the cas9 gene (Chylinski et al., 2013, RNA Biology 10:726-737; Makarova et al. 2015, Nature Reviews Microbiology Vol. 13:1-15). Type II CRISPR-Cas loci can encode a tracrRNA, which is partially complementary to the repeats within the respective CRISPR array, and can comprise other proteins such as Csn1 and Csn2. The presence of cas9 in the vicinity of Cas 1 and cas2 genes is the hallmark of type II loci (Makarova et al. 2015, Nature Reviews Microbiology Vol. 13:1-15).

The novel CRISPR-Cas loci described herein differ from type II CRISPR-Cas loci, in that the CRISPR-Cas loci described herein do not contain a *cas1* gene, a *cas2* gene or any other known CRISPR-Cas associated gene or gene fragment, except cas9 and are flanked by multiple CRISPR arrays.

The term "Cas9 endonuclease" and "Cas9 protein" are used interchangeably herein and refer to a protein encoded by a Cas9 gene. "Cas9" (formerly referred to as Cas5, Csn1, or Csx12) herein refers to a Cas endonuclease that when in complex with a suitable polynucleotide component (such as crNucleotide and a tracrNucleotide, or a single guide polynucleotide) is capable of recognizing , binding to, and optionally nicking or cleaving all or part of a DNA target sequence. A Cas9 protein comprises a RuvC nuclease domain and an HNH (H-N-H) nuclease domain, each of which can cleave a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al. , 2013, Cell 157:1262-1278). Cas9 endonucleases are typically derived from a type II CRISPR system, which includes a DNA cleavage system utilizing a Cas9 endonuclease in complex with at least one polynucleotide component. For example, a Cas9 can be in complex with a CRISPR RNA (crRNA) and a trans-activating CRISPR RNA (tracrRNA). In another example, a Cas9 can be in complex with a single guide RNA (Makarova et al. 2015, Nature Reviews Microbiology Vol. 13:1-15).

The novel Cas9 endonucleases described herein are derived from CRISPR-Cas loci that differ from type II CRISPR-Cas loci, in that the novel CRISPR-Cas loci described herein contain multiple CRISPR arrays flanking a tracrRNA encoding region and *cas9* gene and do not contain a *cas1* gene and a *cas2* gene in the vicinity of a *cas9* gene.

The term "*cas9* gene" herein refers to a gene encoding a Cas9 endonuclease.

In one embodiment of the disclosure, the composition comprises at least one Cas9 endonuclease selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, or comprises at least one Cas9 endonuclease encoded by a DNA sequence selected from the group consisting of SEQ ID NOs: 6, 7, 8, 9, 10, 46, 47, 48, 49, 73, a functional fragment of SEQ ID NOs: 6, 7, 8, 9, 10, 46, 47, 48, 49, 73, and a functional variant of SEQ ID NOs: 6, 7, 8, 9, 10, 46, 47, 48, 49, 73.

In one embodiment of the disclosure, the composition comprises at least one recombinant DNA (such as a vector) encoding a Cas9 endonuclease selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74 (such as a recombinant DNA comprising the DNA sequence selected from the group consisting of SEQ ID NOs: 6, 7, 8, 9, 10, 46, 47, 48, 49, 73, a functional fragment of SEQ ID NOs: 6, 7, 8, 9, 10, 46, 47, 48, 49, 73, and a functional variant of SEQ ID NOs: 6, 7, 8, 9, 10, 46, 47, 48, 49, 73), or mRNA encoding a Cas9 endonuclease selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74. The Cas9 endonucleases described herein can form a (Ribonucleotide Protein -RNP) complex with at least one guide RNA, wherein said complex is capable of recognizing, binding to, and optionally nicking or cleaving all or part of a target site.

Recombinant DNA expressing the Cas9 endonucleases described herein (including functional fragments thereof, plant -, microbe -,or mammalian-codon optimized Cas9 endonuclease) can be stably integrated into the genome of an organism. For example, plants can be produced that comprise a cas9 gene stably integrated in the plant's genome. Plants expressing a stably integrated Cas9 endonuclease can be exposed to at least one guide RNA and/or a polynucleotide modification templates and/or donor DNAs to enable genome modifications such as gene knockout, gene editing, gene knock-down or DNA insertions.

The terms "functional fragment ", "fragment that is functionally equivalent" and "functionally equivalent fragment" of a Cas9 endonuclease are used interchangeably herein, and refer to a portion or subsequence of the Cas9 endonuclease sequence of the present disclosure in which the ability to recognize, bind to, and optionally nick or cleave (introduce a single or double strand break in) the target site is retained.

Functional fragments of Cas9 endonucleases of the present disclosure include fragments comprising 50-100, 100-200, 100-300, 100-400, 100-500, 100-600, 100-700, 100-800, 100-900, 100-1000, 200-300, 200-400, 200-500, 200-600, 200-700, 200-800, 200-900, 200-1000, 300-400, 300-500, 300-600, 300-700, 300-800, 300-900, 300-1000, 400-500, 400-600, 400-700, 400-800, 400-900, 400-1000, 500-600, 500-700, 500-800, 500-900, 500-1000, 600-700, 600-800, 600-900, 600-1000, 700-800, 700-900, 700-1000, 800-900, 800-1000, or 900-1000 amino acids of a reference Cas9 protein, such as the reference Cas9 endonucleases of the present disclosure of SEQ ID NOs: 11, 12, 13, 14, 15, 50, 51, 52, 53, 74.

Functional fragments of a Cas9 endonuclease of the present disclosure include proteins comprising at least one domain selected from the group consisting of a guide polynucleotide binding domain (an amino acid domain that can bind to or hybridize to a guide RNA), a crRNA binding domain (an amino acid domain that can bind to or hybridize to a crRNA), a tracrRNA binding domain (an amino acid domain that can bind to or hybridize to a tracrRNA), a DNA binding domain (an amino acid domain that can bind to DNA target sequence), a DNA cleavage domain (such as an HNH or RuvC domain) and any combination thereof.

Multiple functional domains and conserved elements were determined for each of the novel Cas9 endonuclease protein of the present disclosure (see Example 8, Table 8). The novel Cas9 endonucleases of the present disclosure comprised an HNH domain, an RuvC domain that included three subdomains (RuvC-I, Ruvc-II and RuvC-II), a Brige Helix domain a PAM interacting domain and DNA/RNA recognition regions including REC1 and REC1'. The REC1 binds to repeat::anti-repeat RNA duplex of the guide RNA while REC1' mainly interacts with targetDNA::guide RNA hybrid duplex.

In some aspects the RuvC-I domain of a Cas9 endonuclease can be 41, 42, 43, 44, 45, 46, 47, 47 , 48, 49, 50, 51, 52 or 53 amino acids in length. The RuvC-I domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the RuvC-I domain of each of the Cas9 endonucleases of the present disclosure and based on this information one can design novel Cas9 endonucleases comprising any one of the RuvC-I domain selected from the group consisting of the RuvC-I domain of Cas-Locus-1, the RuvC-I domain of Cas-Locus 2, the RuvC-I domain of Cas-locus-3, the RuvC-I domain of Cas-Locus-4, the RuvC-I domain of Cas-locus-5, the RuvC-I domain of Cas-Locus-42, a function fragment thereof, and a functional variant thereof. (A functional fragment or functional variant of a RuvC-I domain is a fragment or variant in which the ability to function as a RuvC-I domain is retained).

In some aspects the Bridge Helix (BH) domain of a Cas9 endonuclease can be 28, 29, 30, 31, 32, 33, 34, 35, 36, 37, 38, 39 or 40 amino acids in length. The BH domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the BH domain of each of the Cas9 endonucleases of the present disclosure and based on this information one can design novel Cas9 endonucleases comprising any one of the BH domain selected from the group consisting of the BH domain of Cas-Locus-1, the BH domain of Cas-Locus 2, the BH domain of Cas-locus-3, the BH domain of Cas-Locus-4, the BH domain of Cas-locus-5, the BH domain of Cas-Locus-42, a function fragment thereof, and a functional variant thereof. (A functional fragment or functional variant of a BH domain is a fragment or variant in which the ability to function as a BH domain is retained).

In some aspects the REC1 domain of a Cas9 endonuclease can be 150, 151, 152, 153, 154, 155, 156, 157, 158, 159, 160, 161, 162, 163 or 164 amino acids in length. The REC1 domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the REC1 domain of each of the Cas9 endonucleases of the present disclosure and based on this information one can design novel Cas9 endonucleases comprising any one of the REC1 domain selected from the group consisting of the REC1 domain of Cas-Locus-1, the REC1 domain of Cas-Locus 2, the REC1 domain of Cas-locus-3, the REC1 domain of Cas-Locus-4, the REC1 domain of Cas-locus-5, the REC1 domain of Cas-Locus-42, a function fragment thereof, and a functional variant thereof. (A functional fragment or functional variant of a REC1 domain is a fragment or variant in which the ability to function as a REC1 domain is retained).

In some aspects the REC1' domain of a Cas9 endonuclease can be 228 or 229 amino acids in length. The REC1' domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the REC1' domain of each of the Cas9 endonucleases of the present disclosure and based on this information one can design novel Cas9 endonucleases comprising any one of the REC1' domain selected from the group consisting of the REC1' domain of Cas-Locus-1, the REC1' domain of Cas-Locus 2, the REC1' domain of Cas-locus-3, the REC1' domain of Cas-Locus-4, the REC1' domain of Cas-locus-5, the REC1' domain of Cas-Locus-42, a function fragment thereof, and a functional variant thereof. (A functional fragment or functional variant of a REC1' domain is a fragment or variant in which the ability to function as a REC1' domain is retained).

In some aspects the RuvC-II domain of a Cas9 endonuclease can be 45 or 46 amino acids in length. The RuvC-II domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the RuvC-II domain of each of the Cas9 endonucleases of the present disclosure and based on this information one can design novel Cas9 endonucleases comprising any one of the RuvC-11 domain selected from the group consisting of the REC1' domain of Cas-Locus-1, the RuvC-II domain of Cas-Locus 2, the RuvC-II domain of Cas-locus-3, the RuvC-II domain of Cas-Locus-4, the RuvC-II domain of Cas-locus-5, the RuvC-II domain of Cas-Locus-42, a function fragment thereof, and a functional variant thereof. (A functional fragment or functional variant of a RuvC-II domain is a fragment or variant in which the ability to function as a RuvC-II domain is retained).

In some aspects the HNH domain of a Cas9 endonuclease can be 154, 155, 156, 157, 158, 159, 160, 161, 162, 163 or 164 amino acids in length. The HNH domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the HNH domain of each of the Cas9 endonucleases of the present disclosure and based on this information one can design novel Cas9 endonucleases comprising any one of the HNH domain selected from the group consisting of the REC1' domain of Cas-Locus-1, the HNH domain of Cas-Locus 2, the HNH domain of Cas-locus-3, the HNH domain of Cas-Locus-4, the HNH domain of Cas-locus-5, the HNH domain of Cas-Locus-42, a function fragment thereof, and a functional variant thereof. (A functional fragment or functional variant of a HNH domain is a fragment or variant in which the ability to function as a HNH domain is retained).

In some aspects the RuvC-III domain of a Cas9 endonuclease can be 145, 146, 147 or 148 amino acids in length. The RuvC-III domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the RuvC-III domain of each of the Cas9 endonucleases of the present disclosure and based on this information one can design novel Cas9 endonucleases comprising any one of the RuvC-III domain selected from the group consisting of the REC1' domain of Cas-Locus-1, the RuvC-III domain of Cas-Locus 2, the RuvC-III domain of Cas-locus-3, the RuvC-III domain of Cas-Locus-4, the RuvC-III domain of Cas-locus-5, the RuvC-III domain of Cas-Locus-42, a function fragment thereof, and a functional variant thereof. (A functional fragment or functional variant of a RuvC-III domain is a fragment or variant in which the ability to function as a RuvC-III domain is retained).

In some aspects the PAM interacting (PI) domain of a Cas9 endonuclease can be 242, 243, 244, 245, 246, 247, 248, 249, 250, 251, 252, 253, 254,256, 257, 258, 259 or 260 amino acids in length. The PI domain can be located next to anyone of the amino acid domains selected from the group consisting of HNH, RuvC-I, RuvC-II, RuvC-III, REC1, REC1', Bridge Helix (BH) and PAM interacting (PI) domain. Table 18 described herein the location of the PI domain of each of the Cas9 endonucleases of the present disclosure and based on this information one can design novel Cas9 endonucleases comprising any one of the PI domain selected from the group consisting of the REC1' domain of Cas-Locus-1, the PI domain of Cas-Locus 2, the PI domain of Cas-locus-3, the PI domain of Cas-Locus-4, the PI domain of Cas-locus-5, the PI domain of Cas-Locus-42, a function fragment thereof, and a functional variant thereof. (A functional fragment or functional variant of a PI domain is a fragment or variant in which the ability to function as a PI domain is retained).

Functional fragments of the Cas9 endonucleases of the present disclosure include a protein comprising one or more protein domains of the Cas9 endonuclease of SEQ ID NOs: 11, 12, 13, 14, 15, 50, 51, 52, 53, 74, wherein said protein retains specific binding activity, and optionally endonucleolytic activity, towards a target DNA when associated with a polynucleotide component.

The terms "functional variant", "Variant that is functionally equivalent" and "functionally equivalent variant" of a Cas9 endonuclease are used interchangeably herein, and refer to a variant of the Cas9 endonuclease of the present disclosure in which the ability to recognize, bind to, and optionally nick or cleave (introduce a single or double strand break in) the target site is retained.

A functional variant of a Cas9 protein sequence may be used, but should have specific binding activity, and optionally endonucleolytic activity, toward DNA when associated with a polynucleotide component herein. Such a functional variant of Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9 protein, such as the reference Cas9 endonucleases of the present disclosure, including the Cas9 endonucleases of SEQ ID NOs: 11, 12, 13, 14, 15, 50, 51, 52, 53, 74. Such a functional variant Cas9 protein can have specific binding activity, and optionally cleavage or nicking activity, toward DNA when associated with an RNA component herein. Functional variants of Cas9 include Cas9 endonuclease proteins containing an amino acid change (e.g., deletion, insertion, or substitution) in its HNH and/or RuvC nuclease domain (Jinek et al. 2012)). In some aspects, the amino acid variation resulting in at least about 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of the reference Cas9 protein, is located inside the HNH domain, or inside the RuvC domain, or inside both the HNH and RuvC domain. In some embodiments, the amino acid variation resulting in at least about 80%, 81%, 82%, 83%, 84%, 85%, 86%, 87%, 88%, 89%, 90%, 91%, 92%, 93%, 94%, 95%, 96%, 97%, 98%, or 99% identity to the amino acid sequence of the reference Cas9 protein, is located outside of the HNH domain, or outside the RuvC domain, or outside both the HNH and RuvC domain.

A functional variant of the Cas9 endonuclease described herein can comprise a modified form of the Cas9 polypeptide. The modified form of the Cas9 polypeptide can include an amino acid change (e.g., deletion, insertion, or substitution) that reduces the naturally-occurring nuclease activity of the Cas9 protein. For example, in some instances, the modified form of the Cas9 protein has less than 50%, less than 40%, less than 30%, less than 20%, less than 10%, less than 5%, or less than 1% of the nuclease activity of the corresponding wild-type Cas9 polypeptide (US patent application US20140068797 A1, published on March 6, 2014). In some cases, the modified form of the Cas9 polypeptide has no substantial nuclease activity and is referred to as catalytically "inactivated Cas9" or "deactivated Cas9 (dCas9)." An inactivated Cas9/deactivated Cas9 includes a deactivated Cas9 endonuclease (dCas9).

Cas9 endonuclease fragments and variants can be obtained via methods such as site-directed mutagenesis and synthetic construction.

Methods for determining if fragments and/or variants of a Cas9 endonuclease of the present disclosure are functional include methods that measure the endonuclease activity of the fragment or variant when in complex with a suitable polynucleotide. Methods that measure Cas9 endonuclease activity are well known in the art such as, but not limiting to, PCT/US13/39011, filed May 1, 2013, PCT/US16/32073 filed May 12, 2016, PCT/US16/32028 filed May 12, 2016, incorporated by reference herein). Methods for measuring Cas9 endonuclease activity include methods that measure the mutation frequency at a target site after a double strand break has occurred (see also, Example 3).

Methods for measuring Cas9 endonuclease activity include methods that measure the mutation frequency at a target site after a double strand break has occurred (see also, Example 3). Methods for measuring if a functional fragment or functional variant of a Cas9 endonuclease of the present disclosure can make a double strand break include the following method: The cellular repair of chromosomal double-strand breaks (DSBs) induced by CRISPR-Cas9 in plant cells results in the production of insertion or deletion (indel) mutagenesis (Svitashev *et al.* et al. (2015)). This outcome can be used to detect and monitor the production of DSBs generated by functional fragments of functional variant of the Cas9 endonucleases of the present disclosure (see also Karvelis et al. (2015). Briefly, appropriate CRISPR-Cas9 maize genomic DNA target sites can be selected, a guide RNA transcriptional cassette (recombinant DNA that expresses a guide RNA) and a DNA recombinant construct expressing the Cas9 endonuclease of the present disclosure (or a functional fragment of the Cas9 endonuclease of the present disclosure, or a functional variant of the Cas9 endonuclease variant of the present disclosure endonuclease (such as an expression cassette described in Example 2) can be constructed and can be co-delivered by biolistic transformation into Hi-Type II 10-day-old immature maize embryos (IMEs) in the presence of BBM and WUS2 genes as described in Svitashev *et al.* (2015). A visual marker DNA expression cassette encoding a yellow fluorescent protein can also be co-delivered with the guide RNA transcriptional cassette and the Cas9 endonuclease expression cassette (recombinant DNA construct) to aid in the selection of evenly transformed IMEs . After 2 days, the 20-30 most evenly transformed IMEs can be harvested based on their fluorescence. Total genomic DNA is extracted and the DNA region surrounding the intended target site is PCR amplified with Phusion® HighFidelity PCR Master Mix (New England Biolabs, M0531 L) adding on the sequences necessary for amplicon-specific barcodes and Illumnia sequencing and deep sequenced. The resulting reads are then examined for the presence of mutations at the expected site of cleavage by comparison to control experiments where the guide RNA transcriptional cassette was omitted from the transformation. If mutations are observed at the intended target sites when using a fragment or variant of the Cas9 endonuclease of the present disclosure, in complex with a suitable guide polynucleotide, the fragments or variants are functional.

Methods for measuring if a functional fragment of functional variant of a Cas9 endonuclease of the present disclosure can make a single strand break (also referred to as a nick; hence acts as a nickase) in the double stranded DNA target site include the following method: The cellular repair of chromosomal single-strand breaks (SSBs) in a double-stranded DNA target may be typically repaired seamlessly in plant cells such as maize. Therefore to examine a functional Cas9 fragment or functional variant of a Cas9 for nicking activity, two chromosomal DNA target sites in close proximity (0-200 bp), each targeting a different strand (sense and anti-sense DNA strands) of the double-stranded DNA, can be targeted. If SSB activity is present, the SSB activity from both target sites will result in a DNA double-strand break (DSB) that will result in the production of insertion or deletion (indel) mutagenesis in maize cells. This outcome can then be used to detect and monitor the activity of the Cas9 nickase similar to that described in Karvelis et al. (2015). Briefly, appropriate CRISPR-Cas9 maize genomic DNA target sites are selected, guide RNA transcription cassettes and functional fragment Cas9 nicking expression cassettes are constructed and co-delivered by biolistic transformation into Hi-Type II 10-day-old immature maize embryos (IMEs) in the presence of BBM and WUS2 genes as described in Svitashev et al. (2015). Since particle gun transformation can be highly variable, a visual marker DNA expression cassette encoding a yellow fluorescent protein can also be co-delivered to aid in the selection of evenly transformed IMEs [immature maize embryos]. After 2 days, the 20-30 most evenly transformed IMEs are harvested based on their fluorescence, total genomic DNA extracted, the region surrounding the intended target site PCR amplified with Phusion® HighFidelity PCR Master Mix (New England Biolabs, M0531 L) adding on the sequences necessary for amplicon-specific barcodes and Illumnia sequencing and deep sequenced. The resulting reads are then examined for the presence of mutations at the expected site of cleavage by comparison to control experiments where the small RNA transcriptional cassette was omitted from the transformation.

Methods for measuring if a functional fragment of functional variant of a Cas9 endonuclease of the present disclosure can bind to the intended DNA target site include the following method: The binding of a maize chromosomal DNA target site does not result in either a single-stranded break (SSB) or a double-stranded break (DSB) in the double-stranded DNA target site. Therefore to examine a functional Cas9 fragment for binding activity in maize cells, another nuclease domain (e.g. Fokl) may be attached to the functional Cas9 fragment with binding activity. If binding activity is present, the added nuclease domain may be used to produce a DSB that will result in the production of insertion or deletion (indel) mutagenesis in maize cells. This outcome may then be used to detect and monitor the binding activity of a Cas9 similar to that described in Karvelis et al. (2015). Briefly, appropriate CRISPR-Cas9 maize genomic DNA target sites can be selected, guide RNA transcription cassettes and functional fragment Cas9 binding and nuclease attached expression cassettes can be constructed and co-delivered by biolistic transformation into Hi-Type II 10-day-old immature maize embryos (IMEs) in the presence of BBM and WUS2 genes as described in Svitashev et al. (2015). A visual marker DNA expression cassette encoding a yellow fluorescent protein can also be co-delivered to aid in the selection of evenly transformed IMEs [immature maize embryos]. After 2 days, the 20-30 most evenly transformed IMEs can be harvested based on their fluorescence, total genomic DNA extracted, the region surrounding the intended target site PCR amplified with Phusion® HighFidelity PCR Master Mix (New England Biolabs, M0531 L) adding on the sequences necessary for amplicon-specific barcodes and Illumnia sequencing and deep sequenced. The resulting reads can then be examined for the presence of mutations at the expected site of cleavage by comparison to control experiments where the small RNA transcriptional cassette was omitted from the transformation.

Alternatively, the binding activity of maize chromosomal DNA target sites can be monitored by the transcriptional induction or repression of a gene. This can be accomplished by attaching a transcriptional activation or repression domain to the functional Cas9 binding fragment and targeting it to the promoter region of a gene and binding monitored through an increase in accumulation of the gene transcript or protein. The gene targeted for either activation or repression can be any naturally occurring maize gene or engineered gene (e.g. a gene encoded a red fluorescent protein) introduced into the maize genome by methods known in the art (e.g. particle gun or agrobacterium transformation).

A Cas9 endonuclease protein can comprise a heterologous nuclear localization sequence (NLS). A heterologous NLS amino acid sequence herein may be of sufficient strength to drive accumulation of a Cas9 protein in a detectable amount in the nucleus of a yeast cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas9 amino acid sequence but such that it is exposed on the protein surface. An NLS may be operably linked to the N-terminus or C-terminus of a Cas9 protein herein, for example. Two or more NLS sequences can be linked to a Cas9 protein, for example, such as on both the N- and C-termini of a Cas9 protein. The Cas9 endonuclease gene can be operably linked to a SV40 nuclear targeting signal upstream of the Cas9 codon region and a bipartite VirD2 nuclear localization signal (Tinland et al. (1992) Proc. Natl. Acad. Sci. USA 89:7442-6) downstream of the Cas9 codon region. Non-limiting examples of suitable NLS sequences herein include those disclosed in U.S. Patent Nos. 6660830 and 7309576, which are both incorporated by reference herein.

Cas9 endonucleases described herein, can be used for targeted genome editing (via simplex and multiplex double-strand breaks and nicks) and targeted genome regulation (via tethering of epigenetic effector domains to either the Cas9 protein or sgRNA. A Cas9 endonuclease can also be engineered to function as an RNA-guided recombinase, and via RNA tethers could serve as a scaffold for the assembly of multiprotein and nucleic acid complexes (Mali et al., 2013, Nature Methods Vol. 10: 957-963).

The Cas9 protein, or functional fragment or variant thereof, for use in the disclosed methods, can be isolated from a recombinant source where the genetically modified host cell (e.g. an insect cell or a yeast cell or human-derived cell line) is modified to express the nucleic acid sequence encoding the Cas9 protein. Alternatively, the Cas9 protein can be produced using cell free protein expression systems or be synthetically produced.

The term "plant-optimized Cas9 endonuclease" herein refers to a Cas9 protein encoded by a nucleotide sequence that has been optimized for expression in a plant cell or plant. A "plant-optimized nucleotide sequence encoding a Cas9 endonuclease", "plant-optimized construct encoding a Cas9 endonuclease" and a "plant-optimized polynucleotide encoding a Cas9" are used interchangeably herein and refer to a nucleotide sequence encoding a Cas9 protein, or a variant or functional fragment thereof, that has been optimized for expression in a plant cell or plant. A plant comprising a plant-optimized Cas9 endonuclease includes a plant comprising the nucleotide sequence encoding for the Cas9 sequence *and*/*or a plant comprising the Cas9 endonuclease protein.* In one aspect, the plant-optimized Cas9 endonuclease nucleotide sequence is a maize-optimized, canola-optimized, sorghum-optimized, rice-optimized, wheat-optimized or soybean-optimized Cas9 endonuclease. A plant-optimized nucleotide sequence includes a codon-optimized gene. A plant-optimized nucleotide sequence can be synthesized by modifying a nucleotide sequence encoding a protein such as, for example, a Cas9 endonuclease as disclosed herein, using one or more plant-preferred codons for improved expression. *See*, for example, Campbell and Gowri (1990) Plant Physiol. 92:1-11 for a discussion of host-preferred codon usage.

The term "mammalian-optimized Cas9 endonuclease sequence" herein refers to a nucleotide sequence encoding a Cas9 endonuclease that has been optimized for expression in mammalian cells, particularly for increased expression in mammalian cells.

A Cas9 protein, including the Cas9 endonucleases of the present disclosure, can be part of a fusion protein comprising one or more heterologous protein domains (e.g., 1, 2, 3, or more domains in addition to the Cas9 protein). Such a fusion protein may comprise any additional protein sequence, and optionally a linker sequence between any two domains, such as between Cas9 and a first heterologous domain. Examples of protein domains that may be fused to a Cas9 protein herein include, without limitation, epitope tags (e.g., histidine [His], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas9 protein can also be in fusion with a protein that binds DNA molecules or other molecules, such as maltose binding protein (MBP), S-tag, Lex A DNA binding domain (DBD), GAL4A DNA binding domain, and herpes simplex virus (HSV) VP16.

A catalytically inactive Cas9 can be fused to a heterologous sequence (US patent application US20140068797 A1, published on March 6, 2014). Suitable fusion partners include, but are not limited to, a polypeptide that provides an activity that indirectly increases transcription by acting directly on the target DNA or on a polypeptide (e.g., a histone or other DNA-binding protein) associated with the target DNA. Additional suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity. Further suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.). A catalytically inactive Cas9 can also be fused to a Fokl nuclease to generate double-strand breaks (Guilinger et al. Nature biotechnology, volume 32, number 6, June 2014).

As used herein, the term "guide polynucleotide", relates to a polynucleotide sequence that can form a complex with a Cas9 endonuclease, including the Cas9 endonuclease of the present disclosure, and enables the Cas9 endonuclease to recognize, bind to, and optionally cleave a DNA target site. The guide polynucleotide can be a single molecule or a double molecule. The guide polynucleotide sequence can be a RNA sequence, a DNA sequence, or a combination thereof (a RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization. A guide polynucleotide that solely comprises ribonucleic acids is also referred to as a "guide RNA" or "gRNA" (See also U.S. Patent Application US20150082478, published on March 19, 2015 and US20150059010, published on February 26, 2015, both are incorporated by reference herein).

The guide polynucleotide includes a chimeric non-naturally occurring guide RNA comprising regions that are not found together in nature (i.e., they are heterologous with each other). For example, a chimeric non-naturally occurring guide RNA comprising a first nucleotide sequence domain (referred to as Variable Targeting domain or VT domain) that can hybridize to a nucleotide sequence in a target DNA, linked to a second nucleotide sequence that can recognize the Cas9 endonuclease, such that the first and second nucleotide sequence are not found linked together in nature.

The guide polynucleotide can be a double molecule (also referred to as duplex or dual guide polynucleotide) comprising a crNucleotide sequence and a tracrNucleotide sequence. The crNucleotide includes a first nucleotide sequence domain (referred to as Variable Targeting domain or VT domain) that can hybridize to a nucleotide sequence in a target DNA and a second nucleotide sequence (also referred to as a tracr mate sequence) that is part of a Cas endonuclease recognition (CER) domain. The tracr mate sequence can hybridized to a tracrNucleotide along a region of complementarity and together form the Cas endonuclease recognition domain or CER domain. The CER domain is capable of interacting with a Cas9 endonuclease polypeptide. The crNucleotide and the tracrNucleotide of the duplex guide polynucleotide can be RNA, DNA, and/or RNA-DNA- combination sequences. In some embodiments, the crNucleotide molecule of the duplex guide polynucleotide is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). The crNucleotide can comprise a fragment of the crRNA naturally occurring in Bacteria and Archaea. The size of the fragment of the crRNA naturally occurring in Bacteria and Archaea that can be present in a crNucleotide disclosed herein can range from, but is not limited to, 2, 3, 4, 5, 6, 7, 8, 9,10, 11, 12, 13, 14, 15, 16, 17, 18, 19, 20 or more nucleotides. In some embodiments the tracrNucleotide is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides. In one embodiment, the RNA that guides the RNA/ Cas9 endonuclease complex is a duplexed RNA comprising a duplex crRNA-tracrRNA.

The tracrRNA (trans-activating CRISPR RNA) contains, in the 5'-to-3' direction, (i) a sequence that anneals with the repeat region of CRISPR type II crRNA (described herein the anti-repeat region, such as but not limiting to SEQ ID NOs: 31-35 and 78), and (ii) a stem loop-containing portion (described herein as a 3' tracrRNA such as but not limiting to SEQ ID NOs: 31-35 and 78.

The tracrRNA component of the single or duplex guide RNA for the Cas9 endonuclease systems described herein can comprise an anti-repeat fragment (including any one of SEQ ID NOs: 31, 32, 33, 34, 35 and 78) and a 3' tracrRNA component (including any one of SEQ ID NO: 36, 37, 38, 39, 40 and 79). For example the tracrRNA can comprise SEQ ID NOs: 31 and 36, or SEQ ID NOs: 32 and 37, or SEQ ID NOs: 33 and 38, or SEQ ID NOs: 34 and 39, or SEQ ID NOs: 35 and 40, or SEQ ID NOs: 78 and 79.

Based on the description of the Locus-1 and Locus-2 herein and the % identity between the Cas9 endonucleases Cas-Locus 1 (SEQ ID NO: 11) and Cas9 endonuclease Cas-Locus 2 (SEQ ID NO:12) (Figure 9A-C, 96.7 % identity) one can envision that the tracrRNA components from both systems can be interchanged and still function as a tracrRNA for either Cas-Locus 1 system or Cas1-Locus 2 system. For example, the tracrRNA can be a combination of anyone antirepeat sequence (SEQ ID NOs: 31, 32) with any one 3' tracrRNA sequence (SEQ ID NOs: 36, 37), such as a combination of SEQ ID NO: 31 and 37 or a combination of SEQ ID NO: 32 and 36. Similarly, based on the description of the Locus 3, Locus-4, Locus-5 and Locus-42 herein, and the homology between the Cas9 endonucleases Cas-Locus 3 (SEQ ID NO:13, 65), Cas-Locus 4 (SEQ ID NO:14), Cas-Locus 5 (SEQ ID NO:15), and Cas-Locus 42 (SEQ ID NO:68), (Cas-Locus 4, Cas-Locus5 and Cas-Locus 42 being 93.8%, 98% and 96.2 % identical to Cas-Locus 3)one can envision that the tracrRNA components from both systems can be interchanged and still function as a tracrRNA for either Cas-Locus 1 system or Cas1-Locus 2 system. For example, the tracrRNA can be a combination of anyone anti-repeat sequence (33, 34, 35, and 78) with any one 3' tracrRNA sequence (SEQ ID NO: 38, 39, 40 and 79).

The duplex guide polynucleotide can form a complex with a Cas9 endonuclease, wherein said guide polynucleotide/Cas9 endonuclease complex (also referred to as a guide polynucleotide/Cas9 endonuclease system) can direct the Cas9 endonuclease to a genomic target site, enabling the Cas9 endonuclease to recognize, bind to, and optionally nick or cleave (introduce a single or double-strand break) into the target site. (See also U.S. Patent Application US20150082478, published on March 19, 2015 and US20150059010, published on February 26, 2015, both are herein incorporated by reference).

The guide RNA includes a dual molecule comprising a chimeric non-naturally occurring crRNA linked to at least one tracrRNA. A chimeric non-naturally occurring crRNA includes a crRNA that comprises regions that are not found together in nature (i.e., they are heterologous with each other. For example, a crRNA comprising a first nucleotide sequence domain (referred to as Variable Targeting domain or VT domain) that can hybridize to a nucleotide sequence in a target DNA, linked to a second nucleotide sequence (also referred to as a tracr mate sequence) such that the first and second sequence are not found linked together in nature.

The guide polynucleotide can also be a single molecule (also referred to as single guide polynucleotide) comprising a crNucleotide sequence linked to a tracrNucleotide sequence. The single guide polynucleotide comprises a first nucleotide sequence domain (referred to as Variable Targeting domain or VT domain) that can hybridize to a nucleotide sequence in a target DNA and a Cas endonuclease recognition domain (CER domain), that interacts with a Cas9 endonuclease polypeptide. By "domain" it is meant a contiguous stretch of nucleotides that can be RNA, DNA, and/or RNA-DNA-combination sequence. The VT domain and /or the CER domain of a single guide polynucleotide can comprise a RNA sequence, a DNA sequence, or a RNA-DNA-combination sequence. The single guide polynucleotide being comprised of sequences from the crNucleotide and the tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides). The single guide polynucleotide can form a complex with a Cas9 endonuclease, wherein said guide polynucleotide/Cas9 endonuclease complex (also referred to as a guide polynucleotide/Cas9 endonuclease system) can direct the Cas9 endonuclease to a genomic target site, enabling the Cas9 endonuclease to recognize, bind to, and optionally nick or cleave (introduce a single or double-strand break) the target site. (See also U.S. Patent Application US20150082478, published on March 19, 2015 and US20150059010, published on February 26, 2015, both are herein incorporated by reference).

A chimeric non-naturally occurring single guide RNA (sgRNA) includes a sgRNA that comprises regions that are not found together in nature (i.e., they are heterologous with each other. For example, a sgRNA comprising a first nucleotide sequence domain (referred to as Variable Targeting domain or VT domain) that can hybridize to a nucleotide sequence in a target DNA linked to a second nucleotide sequence (also referred to as a tracr mate sequence) that are not found linked together in nature.

The term "variable targeting domain" or "VT domain" is used interchangeably herein and includes a nucleotide sequence that can hybridize (is complementary) to one strand (nucleotide sequence) of a double strand DNA target site. The % complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable targeting domain can be at least 12, 13, 14, 15, 16, 17, 18, 19, 20, 21, 22, 23, 24, 25, 26, 27, 28, 29 or 30 nucleotides in length.

In some embodiments, the variable targeting domain comprises a contiguous stretch of 12 to 30, 12 to 29, 12 to 28, 12 to 27, 12 to 26, 12 to 25, 12 to 26, 12 to 25, 12 to 24, 12 to 23, 12 to 22, 12 to 21, 12 to 20, 12 to 19, 12 to 18, 12 to 17, 12 to 16, 12 to 15, 12 to 14, 12 to 13, 13 to 30, 13 to 29, 13 to 28, 13 to 27, 13 to 26, 13 to 25, 13 to 26, 13 to 25, 13 to 24, 13 to 23, 13 to 22, 13 to 21, 13 to 20, 13 to 19, 13 to 18, 13 to 17, 13 to 16, 13 to 15, 13 to 14, 14 to 30, 14 to 29, 14 to 28, 14 to 27, 14 to 26, 14 to 25, 14 to 26, 14 to 25, 14 to 24, 14 to 23, 14 to 22, 14 to 21, 14 to 20, 14 to 19, 14 to 18, 14 to 17, 14 to 16, 14 to 15, 15 to 30, 15 to 29, 15 to 28, 15 to 27, 15 to 26, 15 to 25, 15 to 26, 15 to 25, 15 to 24, 15 to 23, 15 to 22, 15 to 21, 15 to 20, 15 to 19, 15 to 18, 15 to 17, 15 to 16, 16 to 30, 16 to 29, 16 to 28, 16 to 27, 16 to 26, 16 to 25, 16 to 24, 16 to 23, 16 to 22, 16 to 21, 16 to 20, 16 to 19, 16 to 18, 16 to 17, 17 to 30, 17 to 29, 17 to 28, 17 to 27, 17 to 26, 17 to 25, 17 to 24, 17 to 23, 17 to 22, 17 to 21, 17 to 20, 17 to 19, 17 to 18, 18 to 30, 18 to 29, 18 to 28, 18 to 27, 18 to 26, 18 to 25, 18 to 24, 18 to 23, 18 to 22, 18 to 21, 18 to 20, 18 to 19, 19 to 30, 19 to 29, 19 to 28, 19 to 27, 19 to 26, 19 to 25, 19 to 24, 19 to 23, 19 to 22, 19 to 21, 19 to 20, 20 to 30, 20 to 29, 20 to 28, 20 to 27, 20 to 26, 20 to 25, 20 to 24, 20 to 23, 20 to 22, 20 to 21, 21 to 30, 21 to 29, 21 to 28, 21 to 27, 21 to 26, 21 to 25, 21 to 24, 21 to 23, 21 to 22, 22 to 30, 22 to 29, 22 to 28, 22 to 27, 22 to 26, 22 to 25, 22 to 24, 22 to 23, 23 to 30, 23 to 29, 23 to 28, 23 to 27, 23 to 26, 23 to 25, 23 to 24, 24 to 30, 24 to 29, 24 to 28, 24 to 27, 24 to 26, 24 to 25, 25 to 30, 25 to 29, 25 to 28, 25 to 27, 25 to 26, 26 to 30, 26 to 29, 26 to 28, 26 to 27, 27 to 30, 27 to 29, 27 to 28, 28 to 30, 28 to 29, or 29 to 30 nucleotides.

The variable targeting domain can be composed of a DNA sequence, a RNA sequence, a modified DNA sequence, a modified RNA sequence, or any combination thereof.

The term "Cas endonuclease recognition domain" or "CER domain" (of a guide polynucleotide) is used interchangeably herein and includes a nucleotide sequence that interacts with a Cas9 endonuclease polypeptide. A CER domain comprises a tracrNucleotide mate sequence followed by a tracrNucleotide sequence. The CER domain can be composed of a DNA sequence, a RNA sequence, a modified DNA sequence, a modified RNA sequence (see for example US 2015-0059010 A1, published on February 26, 2015, incorporated in its entirety by reference herein), or any combination thereof.

The nucleotide sequence linking the crNucleotide and the tracrNucleotide of a single guide polynucleotide can comprise a RNA sequence, a DNA sequence, or a RNA-DNA combination sequence. In one embodiment, the nucleotide sequence linking the crNucleotide and the tracrNucleotide of a single guide polynucleotide (also referred to as "loop") can be at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 78, 79, 80, 81, 82, 83, 84, 85, 86, 87, 88, 89, 90, 91, 92, 93, 94, 95, 96, 97, 98, 99 or 100 nucleotides in length. In some embodiments, the loop can be 3-4, 3-5, 3-6, 3-7, 3-8, 3-9, 3-10, 3-11, 3-12, 3-13, 3-14, 3-15, 3-20, 3-30, 3-40, 3-50, 3-60, 3-70, 3-80, 3-90, 3-100, 4-5, 4-6, 4-7, 4-8, 4-9, 4-10, 4-11, 4-12, 4-13, 4-14, 4-15, 4-20, 4-30, 4-40, 4-50, 4-60, 4-70, 4-80, 4-90, 4-100, 5-6, 5-7, 5-8, 5-9, 5-10, 5-11, 5-12, 5-13, 5-14, 5-15, 5-20, 5-30, 5-40, 5-50, 5-60, 5-70, 5-80, 5-90, 5-100, 6-7, 6-8, 6-9, 6-10, 6-11, 6-12, 6-13, 6-14, 6-15, 6-20, 6-30, 6-40, 6-50, 6-60, 6-70, 6-80, 6-90, 6-100, 7-8, 7-9, 7-10, 7-11, 7-12, 7-13, 7-14, 7-15, 7-20, 7-30, 7-40, 7-50, 7-60, 7-70, 7-80, 7-90, 7-100, 8-9, 8-10, 8-11, 8-12, 8-13, 8-14, 8-15, 8-20, 8-30, 8-40, 8-50, 8-60, 8-70, 8-80, 8-90, 8-100, 9-10, 9-11, 9-12, 9-13, 9-14, 9-15, 9-20, 9-30, 9-40, 9-50, 9-60, 9-70, 9-80, 9-90, 9-100, 10-20, 20-30, 30-40, 40-50, 50-60, 70-80, 80-90 or 90-100 nucleotides in length.

In another embodiment, the nucleotide sequence linking the crNucleotide and the tracrNucleotide of a single guide polynucleotide can comprise a tetraloop sequence, such as, but not limiting to a GAAA tetraloop sequence.

The terms "single guide RNA" and "sgRNA" are used interchangeably herein and relate to a synthetic fusion of two RNA molecules, a crRNA (CRISPR RNA) comprising a variable targeting domain (linked to a tracr mate sequence that hybridizes to a tracrRNA), fused to a tracrRNA (trans-activating CRISPR RNA). The single guide RNA can comprise a crRNA or crRNA fragment/variant and a tracrRNA or tracrRNA fragment/variant that can form a complex with a Cas9 endonuclease, wherein said guide RNA/Cas9 endonuclease complex can direct the Cas9 endonuclease to a DNA target site, enabling the Cas9 endonuclease to recognize, bind to, and optionally nick or cleave (introduce a single or double-strand break) the DNA target site.

Single guide RNAs targeting a target site in the genome of an organism can be designed by changing the Variable Targeting Domain (VT) of anyone of SEQ ID NOs: 41, 42, 43, 44, 45, 66, 67, 80 (or a functional fragment or functional variant of SEQ ID NOs: 41, 42, 43, 44, 45, 66, 67, 80) with any random nucleotide that can hybridize to any desired target sequence. In SEQ ID NOs: 41-45, 66, 67 and 80 the sgRNA comprises a VT domain of 20 Ns.

The terms "functional fragment ", "fragment that is functionally equivalent" and "functionally equivalent fragment" of a single guide RNA, crRNA or tracrRNA are used interchangeably herein, and refer to a portion or subsequence of the single guide RNA, crRNA or tracrRNA, respectively, of the present disclosure in which the ability to function as a guide RNA, crRNA or tracrRNA, respectively, is retained.

A functional fragments of a guide RNA (guide polynucleotide) of the present disclosure include a fragment of 20-40, 20-45, 20-50, 20-55, 20-60, 20-65, 20-70, 20-75, 20-80, 25-40, 25-45, 25-50, 25-55, 25-60, 25-65, 25-70, 25-75, 25-80, 30-40, 30-45, 30-50, 30-55, 30-60, 30-65, 30-70, 30-75, 30-80, 35-40, 35-45, 35-50, 35-55, 35-60, 35-65, 35-70, 35-75, 35-80, 40-45, 40-50, 40-55, 40-60, 40-65, 40-70, 40-75, 40-80, 45-50, 45-55, 45-60, 45-65, 45-70, 45-75, 45-80, 50-55, 50-60, 50-65, 50-70, 50-75, 50-80, 55-55, 55-60, 55-65, 55-70, 55-75, 55-80, 60-65, 60-70, 60-75, 60-80, 65-70, 65-75, 65-80, 70-75, 70-80 or 75-80 nucleotides of a reference guide RNA, such as the reference guide RNAs of SEQ ID NOs: 41, 42, 43, 44, 45, 66, 67 and 80.

Functional fragments of a crRNA of the present disclosure include a fragment of 5-30, 10-30, 15-30, 20-30, 25-30, 5-25, 10-25, 15-25, 20-25, 5-20, 10-20, 15-20, 5-15, 10-15, nucleotides of a reference crRNA, such as the reference crRNAs of SEQ ID NOs: 26, 27, 28, 29, 30, 62, 63 and 77.

The terms "functional variant", "Variant that is functionally equivalent" and "functionally equivalent variant" of a guide RNA, crRNA or tracrRNA (respectively) are used interchangeably herein, and refer to a variant of the guide RNA, crRNA or tracrRNA, respectively, of the present disclosure in which the ability to function as a guide RNA, crRNA or tracrRNA, respectively, is retained.

A functional variant of a single guide RNA may comprise a nucleotide sequence that is at least about 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, or 99% identical to reference single guide RNA, such as the reference single guide RNA of SEQ ID NOs: 41, 42, 43, 44, 45, 66, 67 and 80, described herein. In some embodiments, a functional variant of a single guide RNA comprises a nucleotide sequence having at least about 60%, at least about 65%, at least about 70%, at least about 75%, at least about 80%, at least about 85%, at least about 90%, at least about 95%, at least about 98%, at least about 99%, or 100% nucleotide sequence identity over a stretch of at least 8, 9, 10, 11, 12, 13, 14, 15, 16, 17, 18, 19, 20, 21, 22, 23, 24, 25, 26, 27, 28, 29, 30 contiguous nucleotides to any one of the nucleotide sequences set forth in SEQ ID NOs: 41, 42, 43, 44, 45, 66, 67 and 80.

Functional variants of a guide polynucleotide of the present disclosure can comprise a modified guide polynucleotide wherein the modification comprises an engineered secondary structure, and/or an artificial loop, and/or a reduction in the length and/or degree of complementation in a region of hybridization compared to a region of hybridization of a reference guide polynucleotide, including the guide polynucleotides of SEQ ID NOs: 41, 42, 43, 44, 45, 66, 67 and 80, and/or a reduction in the length and/or degree of complementation in the portion of the protein-binding segment that forms a double stranded RNA duplex.

Functional variants of a guide polynucleotide of the present disclosure can comprise a modified guide polynucleotide wherein the modification comprises adding, removing, or otherwise altering loops and/or hairpins in the single guide RNA.

Functional variants of a guide polynucleotide of the present disclosure can comprise a modified guide polynucleotide wherein the modification comprises one or more modified nucleotides in the nucleotide sequence, wherein the one or more modified nucleotides comprises at least one non-naturally-occurring nucleotide, nucleotide mimetic (as described in US application US2014/0068797, published March 6, 2014), or analog thereof, or wherein the one or more modified nucleotides are selected from the group consisting of 2'-0-methylanalogs, 2'-fluoro analogs 2-aminopurine, 5-bromo-uridine, pseudouridine, and 7 -methylguanosine.

In one aspect, the functional variant of the guide RNA can form a guide RNA/Cas9 endonuclease complex that can recognize, bind to, and optionally nick or cleave a target sequence.

A functional variant of a crRNA may comprise a nucleotide sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to a reference crRNA, such as the reference crRNA of SEQ ID NOs : 26, 27, 28, 29, 30, 62, 63 and 77 described herein. In one aspect, the functional variant of the crRNA can bind to a Cas9 endonuclease described herein and together with a tracrRNA, or as part of a guide RNA, can form a guide RNA/Cas9 endonuclease complex that can recognize, bind to, and optionally nick or cleave a target sequence.

A functional variant of a tracrRNA may comprise an nucleotide sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to a reference tracrRNA, such as the reference tracrRNA comprising a nucleotide sequence selected from the group consisting of SEQ ID NOs: 31, 32, 33, 34, 35, 36, 37, 38, 39, 40, 78 and 79 described herein. In one aspect, the functional variant of the tracrRNA can bind to a Cas9 endonuclease described herein and together with a crRNA, or as part of a guide RNA, can form a guide RNA/Cas9 endonuclease complex that can recognize, bind to, and optionally nick or cleave a target sequence.

Methods for determining if fragments or variants of a guide RNA, crRNA or tracrRNA are functional include methods that measure the Cas9 endonuclease activity when in complex with said fragment/variant guide RNA, crRNA and/or tracrRNA, as described herein. Methods for measuring Cas9 endonuclease activity (either double strand breaks or single strand breaks) include methods that measure the mutation frequency at a target site, as described herein. If mutations are observed at the intended target sites when using a fragment or variant of guide RNA, crRNA and/or tracrRNA of the present disclosure, in complex with a cas9 endonuclease of the present disclosure, the fragments or variants are functional.

In one embodiment of the disclosure, the composition comprises at least one single guide RNA capable of forming a guide RNA/Cas9 endonuclease complex, wherein said guide RNA/Cas9 endonuclease complex can recognize, bind to, and optionally nick or cleave a target sequence, wherein said single guide wherein said single guide RNA is selected from the group consisting of SEQ ID NOs: 41, 42, 43, 44, 45, 66, 67, 80, a functional fragment of SEQ ID NOs: 41, 42, 43, 44, 45, 66, 67, 80, and a functional variant of SEQ ID NOs: 41, 42, 43, 44, 45, 66, 67, 80.

In one embodiment of the disclosure, the guide RNA is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77.

In one embodiment of the disclosure, the composition is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79.

The guide RNA can also be a duplex molecule comprising a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77, wherein said tracrRNA comprises a nucleotide sequence selected from the group consisting of SEQ ID NOs : 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79, wherein said chimeric non-naturally occurring crRNA comprises a variable targeting domain capable of hybridizing to said target sequence.

In one embodiment of the disclosure, the composition is a single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77.

The guide polynucleotide can be produced by any method known in the art, including chemically synthesizing guide polynucleotides (such as but not limiting to Hendel et al. 2015, Nature Biotechnology 33, 985-989), in vitro generated guide polynucleotides, and/or self-splicing guide RNAs (such as but not limiting to Xie et al. 2015, PNAS 112:3570-3575).

A method of expressing RNA components such as gRNA in eukaryotic cells for performing Cas9-mediated DNA targeting has been to use RNA polymerase III (Pol III) promoters, which allow for transcription of RNA with precisely defined, unmodified, 5'- and 3'-ends (DiCarlo et al., Nucleic Acids Res. 41: 4336-4343; Ma et al., Mol. Ther. Nucleic Acids 3:e161). This strategy has been successfully applied in cells of several different species including maize and soybean (US 20150082478, published on March 19, 2015). Methods for expressing RNA components that do not have a 5' cap have been described (WO 2016/025131, published on February 18, 2016).

In some embodiments, a subject nucleic acid (e.g., a guide polynucleotide, a nucleic acid comprising a nucleotide sequence encoding a guide polynucleotide; a nucleic acid encoding Cas9 endonuclease of the present disclosure; a crRNA or a nucleotide encoding a crRNA, a tracrRNA or a nucleotide encoding a tracrRNA, a nucleotide encoding a VT domain, a nucleotide encoding a CER domain, etc.) comprises a modification or sequence that provides for an additional desirable feature (e.g., modified or regulated stability; subcellular targeting; tracking, e.g., a fluorescent label; a binding site for a protein or protein complex; etc.). Nucleotide sequence modification of the guide polynucleotide, VT domain and/or CER domain can be selected from, but not limited to, the group consisting of a 5' cap, a 3' polyadenylated tail, a riboswitch sequence, a stability control sequence, a sequence that forms a dsRNA duplex, a modification or sequence that targets the guide polynucleotide to a subcellular location, a modification or sequence that provides for tracking , a modification or sequence that provides a binding site for proteins , a Locked Nucleic Acid (LNA), a 5-methyl dC nucleotide, a 2,6-Diaminopurine nucleotide, a 2'-Fluoro A nucleotide, a 2'-Fluoro U nucleotide; a 2'-O-Methyl RNA nucleotide, a phosphorothioate bond, linkage to a cholesterol molecule, linkage to a polyethylene glycol molecule, linkage to a spacer 18 molecule, a 5' to 3' covalent linkage, or any combination thereof. These modifications can result in at least one additional beneficial feature, wherein the additional beneficial feature is selected from the group of a modified or regulated stability, a subcellular targeting, tracking, a fluorescent label, a binding site for a protein or protein complex, modified binding affinity to complementary target sequence, modified resistance to cellular degradation, and increased cellular permeability.

The terms "5'-cap" and "7-methylguanylate (m⁷G) cap" are used interchangeably herein. A 7-methylguanylate residue is located on the 5' terminus of messenger RNA (mRNA) in eukaryotes. RNA polymerase II (Pol II) transcribes mRNA in eukaryotes. Messenger RNA capping occurs generally as follows: The most terminal 5' phosphate group of the mRNA transcript is removed by RNA terminal phosphatase, leaving two terminal phosphates. A guanosine monophosphate (GMP) is added to the terminal phosphate of the transcript by a guanylyl transferase, leaving a 5'-5' triphosphate-linked guanine at the transcript terminus. Finally, the 7-nitrogen of this terminal guanine is methylated by a methyl transferase.

As used herein, the terms "guide polynucleotide/Cas9 endonuclease complex", "guide polynucleotide/Cas9 endonuclease system", " guide polynucleotide/Cas9 complex", "guide polynucleotide/Cas9 system" and "guided Cas9 system" "Polynucleotide-guided endonuclease" , "PGEN" are used interchangeably herein and refer to at least one guide polynucleotide and at least one Cas9 endonuclease that are capable of forming a complex, wherein said guide polynucleotide/Cas9 endonuclease complex can direct the Cas9 endonuclease to a DNA target site, enabling the Cas9 endonuclease to recognize, bind to, and optionally nick or cleave (introduce a single or double-strand break) the DNA target site. A guide polynucleotide/Cas9 endonuclease complex herein can comprise Cas9 protein(s) and suitable polynucleotide component(s) of any of the known CRISPR systems (Horvath and Barrangou, 2010, Science 327:167-170; Makarova et al. 2015, Nature Reviews Microbiology Vol. 13:1-15; Zetsche et al., 2015, Cell 163, 1-13; Shmakov et al., 2015, Molecular_Cell 60, 1-13). A Cas9 endonuclease unwinds the DNA duplex at the target sequence and optionally cleaves at least one DNA strand, as mediated by recognition of the target sequence by a polynucleotide (such as, but not limited to, a crRNA or guide RNA) that is in complex with the Cas9 protein. Such recognition and cutting of a target sequence by a Cas9 endonuclease typically occurs if the correct protospacer-adjacent motif (PAM) is located at or adjacent to the 3' end of the DNA target sequence. Alternatively, a Cas9 protein herein may lack DNA cleavage or nicking activity, but can still specifically bind to a DNA target sequence when complexed with a suitable RNA component. (See also U.S. Patent Application US20150082478, published on March 19, 2015 and US20150059010, published on February 26, 2015, both are incorporated by reference herein).

A guide polynucleotide/Cas9 endonuclease complex that can cleave both strands of a DNA target sequence typically comprises a Cas9 protein that has all of its endonuclease domains in a functional state (e.g., wild type endonuclease domains or variants thereof retaining some or all activity in each endonuclease domain). Thus, a wild type Cas9 protein (e.g., a Cas9 protein disclosed herein), or a variant thereof retaining some or all activity in each endonuclease domain of the Cas9 protein, is a suitable example of a Cas9 endonuclease that can cleave both strands of a DNA target sequence. A Cas9 protein comprising functional RuvC and HNH nuclease domains is an example of a Cas9 protein that can cleave both strands of a DNA target sequence.

A guide polynucleotide/Cas9 endonuclease complex that can cleave one strand of a DNA target sequence can be characterized herein as having nickase activity (e.g., partial cleaving capability). A Cas9 nickase typically comprises one functional endonuclease domain that allows the Cas9 to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain. Non-limiting examples of Cas9 nickases suitable for use herein are disclosed in U.S. Patent Appl. Publ. No. 2014/0189896, which is incorporated by reference herein.

A pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a double-strand break (i.e., a DSB with single-stranded overhangs), which is then recognized as a substrate for non-homologous-end-joining, NHEJ (prone to imperfect repair leading to mutations) or homologous recombination, HR. Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair. For example, a Cas9 nickase with a mutant RuvC domain, but functioning HNH domain (i.e., Cas9 HNH+/RuvC-), can be used (e.g., *Streptococcus pyogenes* Cas9 HNH+/RuvC-). Each Cas9 nickase (e.g., Cas9 HNH+/RuvC-) can be directed to specific DNA sites nearby each other (up to 100 base pairs apart) by using suitable RNA components herein with guide RNA sequences targeting each nickase to each specific DNA site.

A guide polynucleotide/Cas9 endonuclease complex in certain embodiments can bind to a DNA target site sequence, but does not cleave any strand at the target site sequence. Such a complex may comprise a Cas9 protein in which all of its nuclease domains are mutant, dysfunctional. For example, a Cas9 protein herein that can bind to a DNA target site sequence, but does not cleave any strand at the target site sequence, may comprise both a mutant, dysfunctional RuvC domain and a mutant, dysfunctional HNH domain. A Cas9 protein herein that binds, but does not cleave, a target DNA sequence can be used to modulate gene expression, for example, in which case the Cas9 protein could be fused with a transcription factor (or portion thereof) (e.g., a repressor or activator, such as any of those disclosed herein).

The terms "guide RNA/Cas9 endonuclease complex", "guide RNA/Cas9 endonuclease system", " guide RNA/Cas9 complex", "guide RNA/Cas9 system", "gRNA/Cas9 complex", "gRNA/Cas9 system", "RNA-guided endonuclease" , "RGEN" are used interchangeably herein and refer to at least one RNA component and at least one Cas9 endonuclease that are capable of forming a complex , wherein said guide RNA/Cas9 endonuclease complex can direct the Cas9 endonuclease to a DNA target site, enabling the Cas9 endonuclease to recognize, bind to, and optionally nick or cleave (introduce a single or double-strand break) the DNA target site.

In one aspect, the guide polynucleotide/Cas9 endonuclease complex comprises a guide RNA of the present disclosure (such as a single guide RNA selected from the group consisting of SEQ ID NOs: 41, 42, 43, 44, 45, 66, 67, 80, a functional fragment of SEQ ID NOs: 41, 42, 43, 44, 45, 66, 67, 80, and a functional variant of SEQ ID NOs: 41, 42, 43, 44, 45, 66, 67, 80) in complex with a cas9 endonuclease of the present disclosure (such as a Cas9 endonuclease selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74).

The present disclosure further provides expression constructs for expressing in a prokaryotic or eukaryotic cell/organism a guide RNA/Cas9 system that is capable of binding to and creating a double-strand break in a target site. In one embodiment, the expression constructs of the disclosure comprise a promoter operably linked to a nucleotide sequence encoding a Cas9 gene (or plant optimized Cas9 gene) and a promoter operably linked to a guide RNA of the present disclosure. The promoter is capable of driving expression of an operably linked nucleotide sequence in a prokaryotic or eukaryotic cell/organism.

The terms "target site", "target sequence", "target site sequence, "target DNA", "target locus", "genomic target site", "genomic target sequence", "genomic target locus" and "protospacer", are used interchangeably herein and refer to a polynucleotide sequence such as, but not limited to, a nucleotide sequence on a chromosome, episome, a transgenic locus, or any other DNA molecule in the genome (including chromosomal, choloroplastic, mitochondrial DNA, plasmid DNA) of a cell, at which a guide polynucleotide/Cas9 endonuclease complex can recognize, bind to, and optionally nick or cleave . The target site can be an endogenous site in the genome of a cell, or alternatively, the target site can be heterologous to the cell and thereby not be naturally occurring in the genome of the cell, or the target site can be found in a heterologous genomic location compared to where it occurs in nature. As used herein, terms "endogenous target sequence" and "native target sequence" are used interchangeable herein to refer to a target sequence that is endogenous or native to the genome of a cell and is at the endogenous or native position of that target sequence in the genome of the cell. An "artificial target site" or "artificial target sequence" are used interchangeably herein and refer to a target sequence that has been introduced into the genome of a cell. Such an artificial target sequence can be identical in sequence to an endogenous or native target sequence in the genome of a cell but be located in a different position (*i.e.*, a non-endogenous or non-native position) in the genome of a cell.

An "altered target site", "altered target sequence", "modified target site", "modified target sequence" are used interchangeably herein and refer to a target sequence as disclosed herein that comprises at least one alteration when compared to non-altered target sequence. Such "alterations" include, for example:
(i) replacement of at least one nucleotide, (ii) a deletion of at least one nucleotide, (iii) an insertion of at least one nucleotide, or (iv) any combination of (i) - (iii).

Methods for "modifying a target site" and "altering a target site" are used interchangeably herein and refer to methods for producing an altered target site.

The length of the target DNA sequence (target site) can vary, and includes, for example, target sites that are at least 12, 13, 14, 15, 16, 17, 18, 19, 20, 21, 22, 23, 24, 25, 26, 27, 28, 29, 30 or more nucleotides in length. It is further possible that the target site can be palindromic, that is, the sequence on one strand reads the same in the opposite direction on the complementary strand. The nick/cleavage site can be within the target sequence or the nick/cleavage site could be outside of the target sequence. In another variation, the cleavage could occur at nucleotide positions immediately opposite each other to produce a blunt end cut or, in other Cases, the incisions could be staggered to produce single-stranded overhangs, also called "sticky ends", which can be either 5' overhangs, or 3' overhangs. Active variants of genomic target sites can also be used. Such active variants can comprise at least 65%, 70%, 75%, 80%, 85%, 90%, 91%, 92%, 93%, 94%, 95%, 96%, 97%, 98%, 99% or more sequence identity to the given target site, wherein the active variants retain biological activity and hence are capable of being recognized and cleaved by a Cas9 endonuclease.

Assays to measure the single or double-strand break of a target site by an endonuclease are known in the art and generally measure the overall activity and specificity of the agent on DNA substrates containing recognition sites.

A "protospacer adjacent motif" (PAM) herein refers to a short nucleotide sequence adjacent to a target sequence (protospacer) that is recognized (targeted) by a guide polynucleotide/Cas9 endonuclease system described herein. The Cas9 endonuclease may not successfully recognize a target DNA sequence if the target DNA sequence is not followed by a PAM sequence. The sequence and length of a PAM herein can differ depending on the Cas9 protein or Cas9 protein complex used. The PAM sequence can be of any length but is typically 1, 2, 3, 4, 5, 6, 7, 8, 9, 10, 11, 12, 13, 14, 15, 16, 17, 18, 19 or 20 nucleotides long.

A "randomized PAM" and "randomized protospacer adjacent motif" are used interchangeably herein, and refer to a random DNA sequence adjacent to a target sequence (protospacer) that is recognized (targeted) by a guide polynucleotide/Cas9 endonuclease system described herein. The randomized PAM sequence can be of any length but is typically 1, 2, 3, 4, 5, 6, 7, 8, 9, 10, 11, 12, 13, 14, 15, 16, 17, 18, 19 or 20 nucleotides long. A randomized nucleotide includes anyone of the nucleotides A, C, G or T.

Given the diversity of Type II CRISPR-Cas systems (Fonfara et al. (2014) Nucleic Acids Res. 42:2577-2590), it is plausible that many of the Cas9 endonucleases and cognate guide RNAs may have unique sequence recognition and enzymatic properties different from those previously described or characterized. For example, cleavage activity and specificity may be enhanced or proto-spacer adjacent motif (PAM) sequence may be different leading to increased genomic target site density. To tap into this vast unexplored diversity and expand the repertoire of Cas9 endonucleases and cognate guide RNAs available for genome targeting, the components of Cas9 target site recognition, the PAM sequence and the guide RNA (either duplexed CRISPR RNA (crRNA) and trans-activating CRISPR RNA (tracrRNA) or chimeric fusion of crRNA and tracrRNA (single guide RNA (sgRNA), need to be established for each new system. As described herein, CRISPR-Cas loci (including Cas9 genes and open reading frames, CRISPR array and anti-repeats) from uncharacterized CRISPR-Cas systems were identified by searching internal Pioneer-DuPont databases consisting of microbial genomes. The Cas9 endonuclease described herein can be expressed and purified by methods known in the art. As described herein, the transcriptional direction of the tracrRNA for all the CRISPR-Cas systems can be deduced (as described in PCT/US16/32028 filed May 12, 2016, and PCT/US16/32073 filed May 12, 2016), and examples of sgRNAs (described herein, see SEQ ID NOs:41-45, 66, 67 and 80) and its components (VT, crRNA repeat, loop, anti-repeat and 3'tracrRNA) were identified for each new CRISPR-Cas endonuclease described herein.

The guide polynucleotide/Cas9 systems described herein can be used for gene targeting.

The terms "gene targeting", "targeting", and "DNA targeting" are used interchangeably herein. DNA targeting herein may be the specific introduction of a knock-out, edit, or knock-in at a particular DNA sequence, such as in a chromosome or plasmid of a cell. In general, DNA targeting can be performed herein by cleaving one or both strands at a specific DNA sequence in a cell with a Cas9 protein associated with a suitable polynucleotide component. Once a single or double-strand break is induced in the DNA, the cell's DNA repair mechanism is activated to repair the break via nonhomologous end-joining (NHEJ) or Homology-Directed Repair (HDR) processes which can lead to modifications at the target site.

The terms "knock-out", "gene knock-out" and "genetic knock-out" are used interchangeably herein. A knock-out represents a DNA sequence of a cell that has been rendered partially or completely inoperative by targeting with a Cas9 protein; such a DNA sequence prior to knock-out could have encoded an amino acid sequence, or could have had a regulatory function (e.g., promoter), for example.

As described herein, a guided Cas9 endonuclease can recognize, bind to a DNA target sequence and introduce a single strand (nick) or double-strand break. Once a single or double-strand break is induced in the DNA, the cell's DNA repair mechanism is activated to repair the break. Error-prone DNA repair mechanisms can produce mutations at double-strand break sites. The most common repair mechanism to bring the broken ends together is the nonhomologous end-joining (NHEJ) pathway (Bleuyard et al., (2006) DNA Repair 5:1-12). The structural integrity of chromosomes is typically preserved by the repair, but deletions, insertions, or other rearrangements (such as chromosomal translocations) are possible (Siebert and Puchta, 2002, Plant Cell 14:1121-31; Pacher et al., 2007, Genetics 175:21-9).

A knock-out may be produced by an indel (insertion or deletion of nucleotide bases in a target DNA sequence through NHEJ), or by specific removal of sequence that reduces or completely destroys the function of sequence at or near the targeting site.

In one embodiment of the disclosure, the method comprises a method for modifying a target site in the genome of a cell, the method comprising introducing into said cell at least one Cas9 endonuclease selected from the group consisting of SEQ ID NOs: 11, 12, 13, 14, 15, 50-53 and 74, or a functional fragment thereof, and at least one guide RNA, wherein said guide RNA and Cas9 endonuclease can form a complex that is capable of recognizing, binding to, and optionally nicking or cleaving all or part of said target site. The method can further comprise identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).

The guide polynucleotide/Cas9 endonuclease system can be used in combination with at least one polynucleotide modification template to allow for editing (modification) of a genomic nucleotide sequence of interest. (See also U.S. Patent Application US20150082478, published on March 19, 2015 and WO2015/026886, published on February 26, 2015, both are incorporated by reference herein.)

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" includes a polynucleotide that comprises at least one nucleotide modification when compared to the nucleotide sequence to be edited. A nucleotide modification can be at least one nucleotide substitution, addition or deletion. Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

In one embodiment, the disclosure describes a method for editing a nucleotide sequence in the genome of a cell, the method comprising introducing into said cell at least one Cas9 endonuclease selected from the group consisting of SEQ ID NOs: 11, 12, 13, 14, 15, 50-53 and 74 or a functional fragment thereof, a polynucleotide modification template, and at least one guide RNA, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.

The nucleotide to be edited can be located within or outside a target site recognized and cleaved by a Cas9 endonuclease. In one embodiment, the at least one nucleotide modification is not a modification at a target site recognized and cleaved by a Cas9 endonuclease. In another embodiment, there are at least 1, 2, 3, 4, 5, 6, 7, 8, 9, 10, 11, 12, 13, 14, 15, 16, 17, 18, 19, 20, 21, 22, 23, 24, 25, 26, 27, 30, 40, 50, 100, 200, 300, 400, 500, 600, 700, 900 or 1000 nucleotides between the at least one nucleotide to be edited and the genomic target site.

The method for editing a nucleotide sequence in the genome of a cell can be a method without the use of an exogenous selectable marker by restoring function to a non-functional gene product as described in US patent application 62/243719, filed October 20, 2015 and 62/309033, filed March 16, 2016.

Genome editing can be accomplished using any method of gene editing available. For example, gene editing can be accomplished through the introduction into a host cell of a polynucleotide modification template (sometimes also referred to as a gene repair oligonucleotide) containing a targeted modification to a gene within the genome of the host cell. The polynucleotide modification template for use in such methods can be either single-stranded or double-stranded. Examples of such methods are generally described, for example, in US Publication No. 2013/0019349.

In some embodiments, gene editing may be facilitated through the induction of a double-stranded break (DSB) in a defined position in the genome near the desired alteration. DSBs can be induced using any DSB-inducing agent available, including, but not limited to, TALENs, meganucleases, zinc finger nucleases, Cas9-gRNA systems (based on bacterial CRISPR-Cas systems), and the like. In some embodiments, the introduction of a DSB can be combined with the introduction of a polynucleotide modification template. Other guided endonucleases and nucleo-protein complexes that find use in the methods disclosed herein include those described in WO 2013/088446.

The process for editing a genomic sequence combining DSB and modification templates generally comprises: introducing into a host cell, a DSB-inducing agent, or a nucleic acid encoding a DSB-inducing agent, that recognizes a target sequence in the chromosomal sequence and is able to induce a DSB in the genomic sequence, and at least one polynucleotide modification template comprising at least one nucleotide alteration when compared to the nucleotide sequence to be edited. The polynucleotide modification template can further comprise nucleotide sequences flanking the at least one nucleotide alteration, in which the flanking sequences are substantially homologous to the chromosomal region flanking the DSB. Genome editing using DSB-inducing agents, such as Cas9-gRNA complexes, has been described, for example in U.S. Patent Application US 2015-0082478 A1, published on March 19, 2015, WO2015/026886 A1, published on February 26, 2015, US application 62/023246, filed on July 07, 2014, and US application 62/036,652, filed on August 13, 2014, all of which are incorporated by reference herein.

The terms "knock-in", "gene knock-in , "gene insertion" and "genetic knock-in" are used interchangeably herein. A knock-in represents the replacement or insertion of a DNA sequence at a specific DNA sequence in cell by targeting with a Cas9 protein (for example by homologous recombination (HR), wherein a suitable donor DNA polynucleotide is also used). Examples of knock-ins are a specific insertion of a heterologous amino acid coding sequence in a coding region of a gene, or a specific insertion of a transcriptional regulatory element in a genetic locus.

Various methods and compositions can be employed to obtain a cell or organism having a polynucleotide of interest inserted in a target site for a Cas9 endonuclease. Such methods can employ homologous recombination (HR) to provide integration of the polynucleotide of Interest at the target site. In one method described herein, a polynucleotide of interest is introduced into the organism cell via a donor DNA construct. As used herein, "donor DNA" is a DNA construct that comprises a polynucleotide of Interest to be inserted into the target site of a Cas9 endonuclease. The donor DNA construct further comprises a first and a second region of homology that flank the polynucleotide of Interest. The first and second regions of homology of the donor DNA share homology to a first and a second genomic region, respectively, present in or flanking the target site of the cell or organism genome.

The donor DNA can be tethered to the guide polynucleotide. Tethered donor DNAs can allow for co-localizing target and donor DNA, useful in genome editing, gene insertion, and targeted genome regulation, and can also be useful in targeting post-mitotic cells where function of endogenous HR machinery is expected to be highly diminished (Mali et al., 2013, Nature Methods Vol. 10 : 957-963).

Episomal DNA molecules can also be ligated into the double-strand break, for example, integration of T-DNAs into chromosomal double-strand breaks (Chilton and Que, (2003) Plant Physiol 133:956-65; Salomon and Puchta, (1998) EMBO J 17:6086-95). Once the sequence around the double-strand breaks is altered, for example, by exonuclease activities involved in the maturation of double-strand breaks, gene conversion pathways can restore the original structure if a homologous sequence is available, such as a homologous chromosome in non-dividing somatic cells, or a sister chromatid after DNA replication (Molinier et al., (2004) Plant Cell 16:342-52). Ectopic and/or epigenic DNA sequences may also serve as a DNA repair template for homologous recombination (Puchta, (1999) Genetics 152:1173-81).

Homology-directed repair (HDR) is a mechanism in cells to repair double-stranded and single stranded DNA breaks. Homology-directed repair includes homologous recombination (HR) and single-strand annealing (SSA) (Lieber. 2010 Annu. Rev. Biochem. 79:181-211). The most common form of HDR is called homologous recombination (HR), which has the longest sequence homology requirements between the donor and acceptor DNA. Other forms of HDR include single-stranded annealing (SSA) and breakage-induced replication, and these require shorter sequence homology relative to HR. Homology-directed repair at nicks (single-stranded breaks) can occur via a mechanism distinct from HDR at double-strand breaks (Davis and Maizels. PNAS (0027-8424), 111 (10), p. E924-E932).

By "homology" is meant DNA sequences that are similar. For example, a "region of homology to a genomic region" that is found on the donor DNA is a region of DNA that has a similar sequence to a given "genomic region" in the cell or organism genome. A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a donor polynucleotide can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bps. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology-Hybridization with Nucleic Acid Probes, (Elsevier, New York).

In one embodiment of the disclosure, the method comprises a method for modifying a target site in the genome of a cell, the method comprising introducing into said cell at least one guide RNA, at least one donor DNA, and at least one Cas9 endonuclease selected from the group consisting of SEQ ID NOs: 11, 12, 13, 14, 15, 50-53 and 74, or a functional fragment or variant thereof, wherein said at least one guide RNA and at least one Cas9 endonuclease can form a complex that is capable of recognizing, binding to, and optionally nicking or cleaving all or part of said target site, wherein said donor DNA comprises a polynucleotide of interest.

As used herein, a "genomic region" is a segment of a chromosome in the genome of a cell that is present on either side of the target site or, alternatively, also comprises a portion of the target site. The genomic region can comprise at least 5-10, 5-15, 5-20, 5-25, 5-30, 5-35, 5-40, 5-45, 5- 50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800. 5-2900, 5-3000, 5-3100 or more bases such that the genomic region has sufficient homology to undergo homologous recombination with the corresponding region of homology.

The structural similarity between a given genomic region and the corresponding region of homology found on the donor DNA can be any degree of sequence identity that allows for homologous recombination to occur. For example, the amount of homology or sequence identity shared by the "region of homology" of the donor DNA and the "genomic region" of the organism genome can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination

The region of homology on the donor DNA can have homology to any sequence flanking the target site. While in some instances the regions of homology share significant sequence homology to the genomic sequence immediately flanking the target site, it is recognized that the regions of homology can be designed to have sufficient homology to regions that may be further 5' or 3' to the target site. The regions of homology can also have homology with a fragment of the target site along with downstream genomic regions

In one embodiment, the first region of homology further comprises a first fragment of the target site and the second region of homology comprises a second fragment of the target site, wherein the first and second fragments are dissimilar.

As used herein, "homologous recombination" includes the exchange of DNA fragments between two DNA molecules at the sites of homology. The frequency of homologous recombination is influenced by a number of factors. Different organisms vary with respect to the amount of homologous recombination and the relative proportion of homologous to non-homologous recombination. Generally, the length of the region of homology affects the frequency of homologous recombination events: the longer the region of homology, the greater the frequency. The length of the homology region needed to observe homologous recombination is also species-variable. In many cases, at least 5 kb of homology has been utilized, but homologous recombination has been observed with as little as 25-50 bp of homology. See, for example, Singer et al., (1982) Cell 31:25-33; Shen and Huang, (1986) Genetics 112:441-57; Watt et al., (1985) Proc. Natl. Acad. Sci. USA 82:4768-72, Sugawara and Haber, (1992) Mol Cell Biol 12:563-75, Rubnitz and Subramani, (1984) Mol Cell Biol 4:2253-8; Ayares et al., (1986) Proc. Natl. Acad. Sci. USA 83:5199-203; Liskay et al., (1987) Genetics 115:161-7.

Alteration of the genome of a prokaryotic and eukaryotic cell or organism cell, for example, through homologous recombination (HR), is a powerful tool for genetic engineering. Homologous recombination has been demonstrated in plants (Halfter et al., (1992) Mol Gen Genet 231:186-93) and insects (Dray and Gloor, 1997, Genetics 147:689-99). Homologous recombination has also been accomplished in other organisms. For example, at least 150-200 bp of homology was required for homologous recombination in the parasitic protozoan Leishmania (Papadopoulou and Dumas, (1997) Nucleic Acids Res 25:4278-86). In the filamentous fungus Aspergillus *nidulans*, gene replacement has been accomplished with as little as 50 bp flanking homology (Chaveroche et al., (2000) Nucleic Acids Res 28:e97). Targeted gene replacement has also been demonstrated in the ciliate *Tetrahymena thermophila* (Gaertig et al., (1994) Nucleic Acids Res 22:5391-8). In mammals, homologous recombination has been most successful in the mouse using pluripotent embryonic stem cell lines (ES) that can be grown in culture, transformed, selected and introduced into a mouse embryo (Watson et al., 1992, Recombinant DNA, 2nd Ed., (Scientific American Books distributed by WH Freeman & Co.).

DNA double-strand breaks appear to be an effective factor to stimulate homologous recombination pathways (Puchta et al., (1995) Plant Mol Biol 28:281-92; Tzfira and White, (2005) Trends Biotechnol 23:567-9; Puchta, (2005) J Exp Bot 56:1-14). Using DNA-breaking agents, a two- to nine-fold increase of homologous recombination was observed between artificially constructed homologous DNA repeats in plants (Puchta et al., (1995) Plant Mol Biol 28:281-92). In maize protoplasts, experiments with linear DNA molecules demonstrated enhanced homologous recombination between plasmids (Lyznik et al., (1991) Mol Gen Genet 230:209-18).

Further uses for guide RNA/Cas9 endonuclease systems have been described (See U.S. Patent Application US 2015-0082478 A1, published on March 19, 2015, WO2015/026886 A1, published on February 26, 2015, US 2015-0059010 A1, published on February 26, 2015, US application 62/023246, filed on July 07, 2014, and US application 62/036,652, filed on August 13, 2014, all of which are incorporated by reference herein) and include but are not limited to modifying or replacing nucleotide sequences of interest (such as a regulatory elements), insertion of polynucleotides of interest, gene knock-out, gene-knock in, modification of splicing sites and/or introducing alternate splicing sites, modifications of nucleotide sequences encoding a protein of interest, amino acid and/or protein fusions, and gene silencing by expressing an inverted repeat into a gene of interest.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide a guide polynucleotide/Cas9 endonuclease complex to a unique DNA target site.

Polynucleotides of interest and/or traits can be stacked together in a complex trait locus as described in WO2012/129373,published March 14, 2013,and in PCT/US13/22891, published January 24, 2013, both hereby incorporated by reference. The guide polynucleotide/Cas9 endonuclease system described herein provides for an efficient system to generate double-strand breaks and allows for traits to be stacked in a complex trait locus.

A guide polynucleotide/Cas9 system as described herein, mediating gene targeting, can be used in methods for directing transgene insertion and / or for producing complex transgenic trait loci comprising multiple transgenes in a fashion similar as disclosed in WO2012/129373, published March 14, 2013where instead of using a double-strand break inducing agent to introduce a gene of interest, a guide polynucleotide/Cas9 system as disclosed herein is used. A complex trait locus includes a genomic locus that has multiple transgenes genetically linked to each other. By inserting independent transgenes within 0.1, 0.2, 0.3, 0.4, 0.5 , 1.0, 2, or even 5 centimorgans (cM) from each other, the transgenes can be bred as a single genetic locus (see, for example, U.S. patent application 13/427,138) or PCT application PCT/US2012/030061. After selecting a plant comprising a transgene, plants containing (at least) one transgenes can be crossed to form an F1 that contains both transgenes. In progeny from these F1 (F2 or BC1) 1/500 progeny would have the two different transgenes recombined onto the same chromosome. The complex locus can then be bred as single genetic locus with both transgene traits. This process can be repeated to stack as many traits as desired.

The Cas9 endonuclease described herein can be expressed and purified by methods known in the art (such as those described in Example 2 of US patent applications 62/162,377 filed May 15, 2015, incorporated herein by reference).

Any one component of the guide polynucleotide/Cas9 endonuclease complex, the guide polynucleotide/Cas9 endonuclease complex itself, as well as the polynucleotide modification template(s) and/or donor DNA(s), can be introduced into a cell by any method known in the art.

"Introducing" is intended to mean presenting to the organism, such as a cell or organism, the polynucleotide or polypeptide or polynucleotide-protein complex, in such a manner that the component(s) gains access to the interior of a cell of the organism or to the cell itself. The methods and compositions do not depend on a particular method for introducing a sequence into an organism or cell, only that the polynucleotide or polypeptide gains access to the interior of at least one cell of the organism. Introducing includes reference to the incorporation of a nucleic acid into a eukaryotic or prokaryotic cell where the nucleic acid may be incorporated into the genome of the cell, and includes reference to the transient (direct) provision of a nucleic acid, protein or polynucleotide-protein complex (PGEN, RGEN) to the cell.

Methods for introducing polynucleotides or polypeptides or a polynucleotide-protein complex into cells or organisms are known in the art including, but not limited to, microinjection, electroporation, stable transformation methods, transient transformation methods, ballistic particle acceleration (particle bombardment), whiskers mediated transformation, *Agrobacterium*-mediated transformation, direct gene transfer, viral-mediated introduction, transfection, transduction, cell-penetrating peptides, mesoporous silica nanoparticle (MSN)-mediated direct protein delivery, topical applications, sexual crossing , sexual breeding, and any combination thereof.

For example, the guide polynucleotide (guide RNA, crNucleotide + tracrNucleotide, guide DNA and/or guide RNA-DNA molecule) can be introduced into a cell directly (transiently) as a single stranded or double stranded polynucleotide molecule. The guide RNA can also be introduced into a cell indirectly by introducing a recombinant DNA molecule comprising a heterologous nucleic acid fragment encoding the guide RNA, operably linked to a specific promoter that is capable of transcribing the guide RNA (crRNA+tracrRNA molecules) in said cell. The specific promoter can be, but is not limited to, a RNA polymerase III promoter, which allow for transcription of RNA with precisely defined, unmodified, 5'- and 3'- ends (Ma et al., 2014, Mol. Ther. Nucleic Acids 3:e161; DiCarlo et al., 2013, Nucleic Acids Res. 41: 4336-4343; WO2015026887, published on February 26, 2015). Any promoter capable of transcribing the guide RNA in a cell can be used and includes a heat shock /heat inducible promoter operably linked to a nucleotide sequence encoding the guide RNA.

The Cas9 endonuclease described herein can be introduced into a cell by directly introducing the Cas9 protein itself (referred to as direct delivery of Cas9 endonuclease), the mRNA encoding the Cas9 protein, and/ or the guide polynucleotide/Cas9 endonuclease complex itself (also referred to as ribonucleotide complex or RNP complex), using any method known in the art. The Cas9 endonuclease can also be introduced into a cell indirectly by introducing a recombinant DNA molecule that encodes the Cas9 endonuclease. The Cas9 endonuclease can be introduced into a cell transiently or can be incorporated into the genome of the host cell using any method known in the art. Uptake of the Cas9 endonuclease and/or the guided polynucleotide into the cell can be facilitated with a Cell Penetrating Peptide (CPP) as described in PCT/US2015/058760, filed November 03, 2015. Any promoter capable of expressing the Cas9 endonuclease in a cell can be used and includes a heat shock /heat inducible promoter operably linked to a nucleotide sequence encoding the Cas9 endonuclease.

Direct delivery of a polynucleotide modification template into plant cells can be achieved through particle mediated delivery, and any other direct method of delivery, such as but not limiting to, polyethylene glycol (PEG)-mediated transfection to protoplasts, whiskers mediated transformation, electroporation, particle bombardment, cell-penetrating peptides, or mesoporous silica nanoparticle (MSN)-mediated direct protein delivery can be successfully used for delivering a polynucleotide modification template in eukaryotic cells, such as plant cells.

The donor DNA can be introduced by any means known in the art. The donor DNA may be provided by any transformation method known in the art including, for example, Agrobacterium-mediated transformation or biolistic particle bombardment. The donor DNA may be present transiently in the cell or it could be introduced via a viral replicon. In the presence of the Cas9 endonuclease and the target site, the donor DNA is inserted into the transformed plant's genome.

Direct delivery of any one of the guided Cas9 system components can be accompanied by direct delivery (co-delivery) of other mRNAs that can promote the enrichment and/or visualization of cells receiving the guide polynucleotide/Cas9 endonuclease complex components. For example, direct co-delivery of the guide polynucleotide/Cas9 endonuclease components (and/or guide polynucleotide/Cas9 endonuclease complex itself) together with mRNA encoding phenotypic markers (such as but not limiting to transcriptional activators such as CRC (Bruce et al. 2000 The Plant Cell 12:65-79) can enable the selection and enrichment of cells without the use of an exogenous selectable marker by restoring function to a non-functional gene product as described in PCT/US16/57272 filed October 17, 2016 and PCT/US16/57279, filed October 17, 2016, both incorporated herein by reference.

Protocols for introducing polynucleotides, polypeptides or polynucleotide-protein complexes (PGEN, RGEN) into eukaryotic cells, such as plants or plant cells are known and include microinjection (Crossway et al., (1986) Biotechniques 4:320-34 and U.S. Patent No. 6,300,543), meristem transformation (U.S. Patent No. 5,736,369), electroporation (Riggs et al., (1986) Proc. Natl. Acad. Sci. USA 83:5602-6, *Agrobacterium*-mediated transformation (U.S. Patent Nos. 5,563,055 and 5,981,840), whiskers mediated transformation (Ainley et al. 2013, Plant Biotechnology Journal 11:1126-1134; Shaheen A. and M. Arshad 2011 Properties and Applications of Silicon Carbide (2011), 345-358 Editor(s): Gerhardt, Rosario. Publisher: InTech, Rijeka, Croatia. CODEN: 69PQBP; ISBN: 978-953-307-201-2), direct gene transfer (Paszkowski et al., (1984) EMBO J 3:2717-22), and ballistic particle acceleration (U.S. Patent Nos. 4,945,050; 5,879,918; 5,886,244; 5,932,782; Tomes et al., (1995) "Direct DNA Transfer into Intact Plant Cells via Microprojectile Bombardment" in Plant Cell, Tissue, and Organ Culture: Fundamental Methods, ed. Gamborg & Phillips (Springer-Verlag, Berlin); McCabe et al., (1988) Biotechnology 6:923-6; Weissinger et al., (1988) Ann Rev Genet 22:421-77; Sanford et al., (1987) Particulate Science and Technology 5:27-37 (onion); Christou et al., (1988) Plant Physiol 87:671-4 (soybean); Finer and McMullen, (1991) In Vitro Cell Dev Biol 27P:175-82 (soybean); Singh et al., (1998) Theor Appl Genet 96:319-24 (soybean); Datta et al., (1990) Biotechnology 8:736-40 (rice); Klein et al., (1988) Proc. Natl. Acad. Sci. USA 85:4305-9 (maize); Klein et al., (1988) Biotechnology 6:559-63 (maize); U.S. Patent Nos. 5,240,855; 5,322,783 and 5,324,646; Klein et al., (1988) Plant Physiol 91:440-4 (maize); Fromm et al., (1990) Biotechnology 8:833-9 (maize); Hooykaas-Van Slogteren et al., (1984) Nature 311:763-4; U.S. Patent No. 5,736,369 (cereals); Bytebier et al., (1987) Proc. Natl. Acad. Sci. USA 84:5345-9 (*Liliaceae*); De Wet et al., (1985) in The Experimental Manipulation of Ovule Tissues, ed. Chapman et al., (Longman, New York), pp. 197-209 (pollen); Kaeppler et al., (1990) Plant Cell Rep 9:415-8) and Kaeppler et al., (1992) Theor Appl Genet 84:560-6 (whisker-mediated transformation); D'Halluin et al., (1992) Plant Cell 4:1495-505 (electroporation); Li et al., (1993) Plant Cell Rep 12:250-5; Christou and Ford (1995) Annals Botany 75:407-13 (rice) and Osjoda et al., (1996) Nat Biotechnol 14:745-50 (maize via *Agrobacterium tumefaciens*)*.*

Alternatively, polynucleotides may be introduced into plant or plant cells by contacting cells or organisms with a virus or viral nucleic acids. Generally, such methods involve incorporating a polynucleotide within a viral DNA or RNA molecule. In some examples a polypeptide of interest may be initially synthesized as part of a viral polyprotein, which is later processed by proteolysis *in vivo* or *in vitro* to produce the desired recombinant protein. Methods for introducing polynucleotides into plants and expressing a protein encoded therein, involving viral DNA or RNA molecules, are known, see, for example, U.S. Patent Nos. 5,889,191, 5,889,190, 5,866,785, 5,589,367 and 5,316,931.

The polynucleotide or recombinant DNA construct can be provided to or introduced into a prokaryotic and eukaryotic cell or organism using a variety of transient transformation methods. Such transient transformation methods include, but are not limited to, the introduction of the polynucleotide construct directly into the plant.

Nucleic acids and proteins can be provided to a cell by any method including methods using molecules to facilitate the uptake of anyone or all components of a guided Cas9 system (protein and/or nucleic acids), such as cell-penetrating peptides and nanocariers. See also US20110035836 Nanocarier based plant transfection and transduction, and EP 2821486 A1 Method of introducing nucleic acid into plant cells, incorporated herein by reference.

Other methods of introducing polynucleotides into a prokaryotic and eukaryotic cell or organism or plant part can be used, including plastid transformation methods, and the methods for introducing polynucleotides into tissues from seedlings or mature seeds.

Stable transformation is intended to mean that the nucleotide construct introduced into an organism integrates into a genome of the organism and is capable of being inherited by the progeny thereof. Transient transformation is intended to mean that a polynucleotide is introduced into the organism and does not integrate into a genome of the organism or a polypeptide is introduced into an organism. Transient transformation indicates that the introduced composition is only temporarily expressed or present in the organism.

Polynucleotides of interest are further described herein and include polynucleotides reflective of the commercial markets and interests of those involved in the development of the crop. Crops and markets of interest change, and as developing nations open up world markets, new crops and technologies will emerge also. In addition, as our understanding of agronomic traits and characteristics such as yield and heterosis increase, the choice of genes for genetic engineering will change accordingly.

Polynucleotides of interest include, but are not limited to, polynucleotides encoding important traits for agronomics, herbicide-resistance, insecticidal resistance, disease resistance, nematode resistance, herbicide resistance, microbial resistance, fungal resistance, viral resistance, fertility or sterility, grain characteristics, and commercial products.

General categories of polynucleotides of interest include, for example, genes of interest involved in information, such as zinc fingers, those involved in communication, such as kinases, and those involved in housekeeping, such as heat shock proteins. More specific polynucleotides of interest include, but are not limited to, genes involved in crop yield, grain quality, crop nutrient content, starch and carbohydrate quality and quantity as well as those affecting kernel size, sucrose loading, protein quality and quantity, nitrogen fixation and/or utilization, fatty acid and oil composition, genes encoding proteins conferring resistance to abiotic stress (such as drought, nitrogen, temperature, salinity, toxic metals or trace elements, or those conferring resistance to toxins such as pesticides and herbicides), genes encoding proteins conferring resistance to biotic stress (such as attacks by fungi, viruses, bacteria, insects, and nematodes, and development of diseases associated with these organisms).

Agronomically important traits such as oil, starch, and protein content can be genetically altered in addition to using traditional breeding methods. Modifications include increasing content of oleic acid, saturated and unsaturated oils, increasing levels of lysine and sulfur, providing essential amino acids, and also modification of starch. Hordothionin protein modifications are described in U.S. Patent Nos. 5,703,049, 5,885,801, 5,885,802, and 5,990,389, herein incorporated by reference.

Polynucleotides of interest include, any nucleotide sequence encoding a protein or polypeptide that improves desirability of crops. Polynucleotide sequences of interest may encode proteins involved in providing disease or pest resistance. By "disease resistance" or "pest resistance" is intended that the plants avoid the harmful symptoms that are the outcome of the plant-pathogen interactions. Pest resistance genes may encode resistance to pests that have great yield drag such as rootworm, cutworm, European Corn Borer, and the like. Disease resistance and insect resistance genes such as lysozymes or cecropins for antibacterial protection, or proteins such as defensins, glucanases or chitinases for antifungal protection, or *Bacillus thuringiensis* endotoxins, protease inhibitors, collagenases, lectins, or glycosidases for controlling nematodes or insects are all examples of useful gene products. Genes encoding disease resistance traits include detoxification genes, such as against fumonisin (U.S. Patent No. 5,792,931); avirulence (avr) and disease resistance (R) genes (Jones et al. (1994) Science 266:789; Martin et al. (1993) Science 262:1432; and Mindrinos et al. (1994) Cell 78:1089); and the like. Insect resistance genes may encode resistance to pests that have great yield drag such as rootworm, cutworm, European Corn Borer, and the like. Such genes include, for example, *Bacillus thuringiensis* toxic protein genes (U.S. Patent Nos. 5,366,892; 5,747,450; 5,736,514; 5,723,756; 5,593,881; and Geiser et al. (1986) Gene 48:109); and the like.

An "herbicide resistance protein" or a protein resulting from expression of an "herbicide resistance-encoding nucleic acid molecule" includes proteins that confer upon a cell the ability to tolerate a higher concentration of an herbicide than cells that do not express the protein, or to tolerate a certain concentration of an herbicide for a longer period of time than cells that do not express the protein. Herbicide resistance traits may be introduced into plants by genes coding for resistance to herbicides that act to inhibit the action of acetolactate synthase (ALS, also referred to as acetohydroxyacid synthase, AHAS), in particular the sulfonylurea-type (UK: sulphonylurea) herbicides, genes coding for resistance to herbicides that act to inhibit the action of glutamine synthase, such as phosphinothricin or basta (e.g., the bar gene), glyphosate (e.g., the EPSP synthase gene and the GAT gene), HPPD inhibitors (e.g, the HPPD gene) or other such genes known in the art. See, for example, US Patent Nos. 7,626,077, 5,310,667, 5,866,775, 6,225,114, 6,248,876, 7,169,970, 6,867,293, and US Provisional Application No. 61/401,456, each of which is herein incorporated by reference. The bar gene encodes resistance to the herbicide basta, the *nptII* gene encodes resistance to the antibiotics kanamycin and geneticin, and the ALS-gene mutants encode resistance to the herbicide chlorsulfuron.

Furthermore, it is recognized that the polynucleotide of interest may also comprise antisense sequences complementary to at least a portion of the messenger RNA (mRNA) for a targeted gene sequence of interest. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, 80%, or 85% sequence identity to the corresponding antisense sequences may be used. Furthermore, portions of the antisense nucleotides may be used to disrupt the expression of the target gene. Generally, sequences of at least 50 nucleotides, 100 nucleotides, 200 nucleotides, or greater may be used.

In addition, the polynucleotide of interest may also be used in the sense orientation to suppress the expression of endogenous genes in plants. Methods for suppressing gene expression in plants using polynucleotides in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, generally greater than about 65% sequence identity, about 85% sequence identity, or greater than about 95% sequence identity. See, U.S. Patent Nos. 5,283,184 and 5,034,323; herein incorporated by reference.

The polynucleotide of interest can also be a phenotypic marker. A phenotypic marker is screenable or a selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers include, but are not limited to, DNA segments that comprise restriction enzyme sites; DNA segments that encode products which provide resistance against otherwise toxic compounds including antibiotics, such as, spectinomycin, ampicillin, kanamycin, tetracycline, Basta, neomycin phosphotransferase II (NEO) and hygromycin phosphotransferase (HPT)); DNA segments that encode products which are otherwise lacking in the recipient cell (e.g., tRNA genes, auxotrophic markers); DNA segments that encode products which can be readily identified (e.g., phenotypic markers such as β-galactosidase, GUS; fluorescent proteins such as green fluorescent protein (GFP), cyan (CFP), yellow (YFP), red (RFP), and cell surface proteins); the generation of new primer sites for PCR (e.g., the juxtaposition of two DNA sequence not previously juxtaposed), the inclusion of DNA sequences not acted upon or acted upon by a restriction endonuclease or other DNA modifying enzyme, chemical, etc.; and, the inclusion of a DNA sequences required for a specific modification (e.g., methylation) that allows its identification.

Additional selectable markers include genes that confer resistance to herbicidal compounds, such as sulphonylureas, glufosinate ammonium, bromoxynil, imidazolinones, and 2,4-dichlorophenoxyacetate (2,4-D). See for example, Acetolactase synthase (ALS) for resistance to sulfonylureas, imidazolinones, triazolopyrimidine sulfonamides, pyrimidinylsalicylates and sulphonylaminocarbonyl-triazolinones Shaner and Singh, 1997, Herbicide Activity: Toxicol Biochem Mol Biol 69-110); glyphosate resistant 5-enolpyruvylshikimate-3-phosphate (EPSPS)(Saroha et al. 1998, J. Plant Biochemistry & Biotechnology Vol 7:65-72);

Polynucleotides of interest includes genes that can be stacked or used in combination with other traits, such as but not limited to herbicide resistance or any other trait described herein. Polynucleotides of interest and/or traits can be stacked together in a complex trait locus as described in US-2013-0263324-A1, published 03 Oct 2013 and in PCT/US13/22891, published January 24, 2013, both applications are hereby incorporated by reference.

A polypeptide of interest includes any protein or polypeptide that is encoded by a polynucleotide of interest described herein.

Further provided are methods for identifying at least one plant cell, comprising in its genome, a polynucleotide of interest integrated at the target site. A variety of methods are available for identifying those plant cells with insertion into the genome at or near to the target site. Such methods can be viewed as directly analyzing a target sequence to detect any change in the target sequence, including but not limited to PCR methods, sequencing methods, nuclease digestion, Southern blots, and any combination thereof. See, for example, US Patent Application 12/147,834, herein incorporated by reference to the extent necessary for the methods described herein. The method also comprises recovering a plant from the plant cell comprising a polynucleotide of Interest integrated into its genome. The plant may be sterile or fertile. It is recognized that any polynucleotide of interest can be provided, integrated into the plant genome at the target site, and expressed in a plant.

As used herein, "nucleic acid" means a polynucleotide and includes a single or a double-stranded polymer of deoxyribonucleotide or ribonucleotide bases. Nucleic acids may also include fragments and modified nucleotides. Thus, the terms "polynucleotide", "nucleic acid sequence", "nucleotide sequence" and "nucleic acid fragment" are used interchangeably to denote a polymer of RNA and/or DNA and/or RNA-DNA that is single- or double-stranded, optionally containing synthetic, non-natural, or altered nucleotide bases. Nucleotides (usually found in their 5'-monophosphate form) are referred to by their single letter designation as follows: "A" for adenosine or deoxyadenosine (for RNA or DNA, respectively), "C" for cytosine or deoxycytosine, "G" for guanosine or deoxyguanosine, "U" for uridine, "T" for deoxythymidine, "R" for purines (A or G), "Y" for pyrimidines (C or T), "K" for G or T, "H" for A or C or T, "I" for inosine, and "N" for any nucleotide.

"Open reading frame" is abbreviated ORF.

The terms "fragment that is functionally equivalent" and "functionally equivalent fragment" are used interchangeably herein. These terms refer to a portion or subsequence of an isolated nucleic acid fragment or polypeptide in which the ability to alter gene expression or produce a certain phenotype is retained whether or not the fragment encodes an active enzyme. For example, the fragment can be used in the design of genes to produce the desired phenotype in a modified plant. Genes can be designed for use in suppression by linking a nucleic acid fragment, whether or not it encodes an active enzyme, in the sense or antisense orientation relative to a plant promoter sequence.

The term "conserved domain" or "motif" means a set of amino acids conserved at specific positions along an aligned sequence of evolutionarily related proteins. While amino acids at other positions can vary between homologous proteins, amino acids that are highly conserved at specific positions indicate amino acids that are essential to the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

Polynucleotide and polypeptide sequences, variants thereof, and the structural relationships of these sequences can be described by the terms "homology", "homologous", "substantially identical", "substantially similar" and "corresponding substantially" which are used interchangeably herein. These refer to polypeptide or nucleic acid sequences wherein changes in one or more amino acids or nucleotide bases do not affect the function of the molecule, such as the ability to mediate gene expression or to produce a certain phenotype. These terms also refer to modification(s) of nucleic acid sequences that do not substantially alter the functional properties of the resulting nucleic acid relative to the initial, unmodified nucleic acid. These modifications include deletion, substitution, and/or insertion of one or more nucleotides in the nucleic acid fragment.

Substantially similar nucleic acid sequences encompassed may be defined by their ability to hybridize (under moderately stringent conditions, e.g., 0.5X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions.

The term "selectively hybridizes" includes reference to hybridization, under stringent hybridization conditions, of a nucleic acid sequence to a specified nucleic acid target sequence to a detectably greater degree (e.g., at least 2-fold over background) than its hybridization to non-target nucleic acid sequences and to the substantial exclusion of non-target nucleic acids. Selectively hybridizing sequences typically have about at least 80% sequence identity, or 90% sequence identity, up to and including 100% sequence identity (i.e., fully complementary) with each other.

The term "stringent conditions" or "stringent hybridization conditions" includes reference to conditions under which a probe will selectively hybridize to its target sequence in an *in vitro* hybridization assay. Stringent conditions are sequence-dependent and will be different in different circumstances. By controlling the stringency of the hybridization and/or washing conditions, target sequences can be identified which are 100% complementary to the probe (homologous probing). Alternatively, stringency conditions can be adjusted to allow some mismatching in sequences so that lower degrees of similarity are detected (heterologous probing). Generally, a probe is less than about 1000 nucleotides in length, optionally less than 500 nucleotides in length.

Typically, stringent conditions will be those in which the salt concentration is less than about 1.5 M Na ion, typically about 0.01 to 1.0 M Na ion concentration (or other salt(s)) at pH 7.0 to 8.3, and at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C.

"Sequence identity" or "identity" in the context of nucleic acid or polypeptide sequences refers to the nucleic acid bases or amino acid residues in two sequences that are the same when aligned for maximum correspondence over a specified comparison window.

The term "percentage of sequence identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. Useful examples of percent sequence identities include, but are not limited to, 50%, 55%, 60%, 65%, 70%, 75%, 80%, 85%, 90% or 95%, or any integer percentage from 50% to 100%. These identities can be determined using any of the programs described herein.

Sequence alignments and percent identity or similarity calculations may be determined using a variety of comparison methods designed to detect homologous sequences including, but not limited to, the MegAlign™ program of the LASERGENE bioinformatics computing suite (DNASTAR Inc., Madison, WI). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

The "Clustal V method of alignment" corresponds to the alignment method labeled Clustal V (described by Higgins and Sharp, (1989) CABIOS 5:151-153; Higgins et al., (1992) Comput Appl Biosci 8:189-191) and found in the MegAlign™ program of the LASERGENE bioinformatics computing suite (DNASTAR Inc., Madison, WI). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a "percent identity" by viewing the "sequence distances" table in the same program.

The "Clustal W method of alignment" corresponds to the alignment method labeled Clustal W (described by Higgins and Sharp, (1989) CABIOS 5:151-153; Higgins et al., (1992) Comput Appl Biosci 8:189-191) and found in the MegAlign™ v6.1 program of the LASERGENE bioinformatics computing suite (DNASTAR Inc., Madison, WI). Default parameters for multiple alignment (GAP PENALTY=10, GAP LENGTH PENALTY=0.2, Delay Divergen Seqs (%)=30, DNA Transition Weight=0.5, Protein Weight Matrix=Gonnet Series, DNA Weight Matrix=IUB). After alignment of the sequences using the Clustal W program, it is possible to obtain a "percent identity" by viewing the "sequence distances" table in the same program.

Unless otherwise stated, sequence identity/similarity values provided herein refer to the value obtained using GAP Version 10 (GCG, Accelrys, San Diego, CA) using the following parameters: % identity and % similarity for a nucleotide sequence using a gap creation penalty weight of 50 and a gap length extension penalty weight of 3, and the nwsgapdna.cmp scoring matrix; % identity and % similarity for an amino acid sequence using a GAP creation penalty weight of 8 and a gap length extension penalty of 2, and the BLOSUM62 scoring matrix (Henikoff and Henikoff, (1989) Proc. Natl. Acad. Sci. USA 89:10915). GAP uses the algorithm of Needleman and Wunsch, (1970) J Mol Biol 48:443-53, to find an alignment of two complete sequences that maximizes the number of matches and minimizes the number of gaps. GAP considers all possible alignments and gap positions and creates the alignment with the largest number of matched bases and the fewest gaps, using a gap creation penalty and a gap extension penalty in units of matched bases.

"BLAST" is a searching algorithm provided by the National Center for Biotechnology Information (NCBI) used to find regions of similarity between biological sequences. The program compares nucleotide or protein sequences to sequence databases and calculates the statistical significance of matches to identify sequences having sufficient similarity to a query sequence such that the similarity would not be predicted to have occurred randomly. BLAST reports the identified sequences and their local alignment to the query sequence.

It is well understood by one skilled in the art that many levels of sequence identity are useful in identifying polypeptides from other species or modified naturally or synthetically wherein such polypeptides have the same or similar function or activity. Useful examples of percent identities include, but are not limited to, 50%, 55%, 60%, 65%, 70%, 75%, 80%, 85%, 90% or 95%, or any integer percentage from 50% to 100%. Indeed, any integer amino acid identity from 50% to 100% may be useful in describing the present disclosure, such as 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

"Gene" includes a nucleic acid fragment that expresses a functional molecule such as, but not limited to, a specific protein, including regulatory sequences preceding (5' non-coding sequences) and following (3' non-coding sequences) the coding sequence. "Native gene" refers to a gene as found in nature with its own regulatory sequences.

A "mutated gene" is a gene that has been altered through human intervention. Such a "mutated gene" has a sequence that differs from the sequence of the corresponding non-mutated gene by at least one nucleotide addition, deletion, or substitution. In certain embodiments of the disclosure, the mutated gene comprises an alteration that results from a guide polynucleotide/Cas9 endonuclease system as disclosed herein. A mutated plant is a plant comprising a mutated gene.

As used herein, a "targeted mutation" is a mutation in a gene (referred to as the target gene), including a native gene, that was made by altering a target sequence within the target gene using any method known to one skilled in the art, including a method involving a guided Cas9 endonuclease system as disclosed herein.

A guide polynucleotide/Cas9 endonuclease induced targeted mutation can occur in a nucleotide sequence that is located within or outside a genomic target site that is recognized and cleaved by the Cas9 endonuclease.

The term "genome" as it applies to a prokaryotic and eukaryotic cell or organism cells encompasses not only chromosomal DNA found within the nucleus, but organelle DNA found within subcellular components (e.g., mitochondria, or plastid) of the cell.

An "allele" is one of several alternative forms of a gene occupying a given locus on a chromosome. When all the alleles present at a given locus on a chromosome are the same, that plant is homozygous at that locus. If the alleles present at a given locus on a chromosome differ, that plant is heterozygous at that locus.

"Coding sequence" refers to a polynucleotide sequence which codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences include, but are not limited to, promoters, translation leader sequences, 5' untranslated sequences, 3' untranslated sequences, introns, polyadenylation target sequences, RNA processing sites, effector binding sites, and stem-loop structures.

A "codon-modified gene" or "codon-preferred gene" or "codon-optimized gene" is a gene having its frequency of codon usage designed to mimic the frequency of preferred codon usage of the host cell.

Methods are available in the art for synthesizing plant-preferred genes. See, for example, U.S. Patent Nos. 5,380,831, and 5,436,391, and Murray et al. (1989) Nucleic Acids Res. 17:477-498, herein incorporated by reference. Additional sequence modifications are known to enhance gene expression in a plant host. These include, for example, elimination of: one or more sequences encoding spurious polyadenylation signals, one or more exon-intron splice site signals, one or more transposon-like repeats, and other such well-characterized sequences that may be deleterious to gene expression. The G-C content of the sequence may be adjusted to levels average for a given plant host, as calculated by reference to known genes expressed in the host plant cell. When possible, the sequence is modified to avoid one or more predicted hairpin secondary mRNA structures. Thus, "a plant-optimized nucleotide sequence" of the present disclosure comprises one or more of such sequence modifications.

A "promoter" is a region of DNA involved in recognition and binding of RNA polymerase and other proteins to initiate transcription. The promoter sequence consists of proximal and more distal upstream elements, the latter elements often referred to as enhancers. An "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, and/or comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters".

It has been shown that certain promoters are able to direct RNA synthesis at a higher rate than others. These are called "strong promoters". Certain other promoters have been shown to direct RNA synthesis at higher levels only in particular types of cells or tissues and are often referred to as "tissue specific promoters", or "tissue-preferred promoters" if the promoters direct RNA synthesis preferably in certain tissues but also in other tissues at reduced levels.

A plant promoter includes a promoter capable of initiating transcription in a plant cell. For a review of plant promoters, see, Potenza et al., 2004, In Vitro Cell Dev Biol 40:1-22; Porto et al., 2014, Molecular Biotechnology (2014), 56(1), 38-49.

Constitutive promoters include, for example, the core CaMV 35S promoter (Odell et al., (1985) Nature 313:810-2); rice actin (McElroy et al., (1990) Plant Cell 2:163-71); ubiquitin (Christensen et al., (1989) Plant MolBiol 12:619-32; ALS promoter (U.S. Patent No. 5,659,026) and the like.

Tissue-preferred promoters can be utilized to target enhanced expression within a particular plant tissue. Tissue-preferred promoters include, for example, WO2013/103367 published on 11 July 2013, Kawamata et al., (1997) Plant Cell Physiol 38:792-803; Hansen et al., (1997) Mol Gen Genet 254:337-43; Russell et al., (1997) Transgenic Res 6:157-68; Rinehart et al., (1996) Plant Physiol 112:1331-41; Van Camp et al., (1996) Plant Physiol 112:525-35; Canevascini et al., (1996) Plant Physiol 112:513-524; Lam, (1994) Results Probl Cell Differ 20:181-96; and Guevara-Garcia et al., (1993) Plant J 4:495-505. Leaf-preferred promoters include, for example, Yamamoto et al., (1997) Plant J 12:255-65; Kwon et al., (1994) Plant Physiol 105:357-67; Yamamoto et al., (1994) Plant Cell Physiol 35:773-8*;* Gotor et al., (1993) Plant J 3:509-18; Orozco et al., (1993) Plant MolBiol 23:1129-38; Matsuoka et al., (1993) Proc. Natl. Acad. Sci. USA 90:9586-90; Simpson et al., (1958) EMBO J 4:2723-9; Timko et al., (1988) Nature 318:57-8. Root-preferred promoters include, for example, Hire et al., (1992) Plant Mol Biol 20:207-18 (soybean root-specific glutamine synthase gene); Miao et al., (1991) Plant Cell 3:11-22 (cytosolic glutamine synthase (GS)); Keller and Baumgartner, (1991) Plant Cell 3:1051-61 (root-specific control element in the GRP 1.8 gene of French bean); Sanger et al., (1990) Plant Mol Biol 14:433-43 (root-specific promoter of *A. tumefaciens* mannopine synthase (MAS)); Bogusz et al., (1990) Plant Cell 2:633-41 (root-specific promoters isolated from *Parasponia andersonii* and *Trema tomentosa*); Leach and Aoyagi, (1991) Plant Sci 79:69-76 (*A*. *rhizogenes* rolC and rolD root-inducing genes); Teeri et al., (1989) EMBO J 8:343-50 (*Agrobacterium* wound-induced TR1' and TR2' genes); VfENOD-GRP3 gene promoter (Kuster et al., (1995) Plant Mol Biol 29:759-72); and rolB promoter (Capana et al., (1994) Plant Mol Biol 25:681-91 ; phaseolin gene (Murai et al., (1983) Science 23:476-82; Sengopta-Gopalen et al., (1988) Proc. Natl. Acad. Sci. USA 82:3320-4). See also, U.S. Patent Nos. 5,837,876; 5,750,386; 5,633,363; 5,459,252; 5,401,836; 5,110,732 and 5,023,179.

Seed-preferred promoters include both seed-specific promoters active during seed development, as well as seed-germinating promoters active during seed germination. See, Thompson et al., (1989) BioEssays 10:108. Seed-preferred promoters include, but are not limited to, Cim1 (cytokinin-induced message); cZ19B1 (maize 19 kDa zein); and milps (myo-inositol-1-phosphate synthase); (WO00/11177; and U.S. Patent 6,225,529). For dicots, seed-preferred promoters include, but are not limited to, bean β-phaseolin, napin, β-conglycinin, soybean lectin, cruciferin, and the like. For monocots, seed-preferred promoters include, but are not limited to, maize 15 kDa zein, 22 kDa zein, 27 kDa gamma zein, waxy, shrunken 1, shrunken 2, globulin 1, oleosin, and nuc1. See also, WO00/12733, where seed-preferred promoters from *END1* and *END2* genes are disclosed.

The term "inducible promoter" refers to a promoter that selectively express a coding sequence or functional RNA in response to the presence of an endogenous or exogenous stimulus, for example by chemical compounds (chemical inducers) or in response to environmental, hormonal, chemical, and/or developmental signals. Inducible or regulated promoters include, for example, promoters induced or regulated by light, heat, stress, flooding or drought, salt stress, osmotic stress, phytohormones, wounding, or chemicals such as ethanol, abscisic acid (ABA), jasmonate, salicylic acid, or safeners.

Chemical inducible (regulated) promoters can be used to modulate the expression of a gene in a prokaryotic and eukaryotic cell or organism through the application of an exogenous chemical regulator. The promoter may be a chemical-inducible promoter, where application of the chemical induces gene expression, or a chemical-repressible promoter, where application of the chemical represses gene expression. Chemical-inducible promoters include, but are not limited to, the maize In2-2 promoter, activated by benzene sulfonamide herbicide safeners (De Veylder et al., (1997) Plant Cell Physiol 38:568-77), the maize GST promoter (GST-II-27, WO93/01294), activated by hydrophobic electrophilic compounds used as pre-emergent herbicides, and the tobacco PR-1a promoter (Ono et al., (2004) Biosci Biotechnol Biochem 68:803-7) activated by salicylic acid. Other chemical-regulated promoters include steroid-responsive promoters (see, for example, the glucocorticoid-inducible promoter (Schena et al., (1991) Proc. Natl. Acad. Sci. USA 88:10421-5; McNellis et al., (1998) Plant J 14:247-257); tetracycline-inducible and tetracycline-repressible promoters (Gatz et al., (1991) Mol Gen Genet 227:229-37; U.S. Patent Nos. 5,814,618 and 5,789,156).

Pathogen inducible promoters induced following infection by a pathogen include, but are not limited to those regulating expression of PR proteins, SAR proteins, beta-1,3-glucanase, chitinase, *etc.*

A stress-inducible promoter includes the RD29A promoter (Kasuga et al. (1999) Nature Biotechnol. 17:287-91). One of ordinary skill in the art is familiar with protocols for simulating stress conditions such as drought, osmotic stress, salt stress and temperature stress and for evaluating stress tolerance of plants that have been subjected to simulated or naturally-occurring stress conditions.

Another example of an inducible promoter useful in plant cells, is the ZmCAS1 promoter, described in US patent application, US 2013-0312137A1, published on November 21, 2013, incorporated by reference herein.

New promoters of various types useful in plant cells are constantly being discovered; numerous examples may be found in the compilation by Okamuro and Goldberg, (1989) In The Biochemistry of Plants, Vol. 115, Stumpf and Conn, eds (New York, NY: Academic Press), pp. 1-82.

"Translation leader sequence" refers to a polynucleotide sequence located between the promoter sequence of a gene and the coding sequence. The translation leader sequence is present in the mRNA upstream of the translation start sequence. The translation leader sequence may affect processing of the primary transcript to mRNA, mRNA stability or translation efficiency. Examples of translation leader sequences have been described (e.g., Turner and Foster, (1995) Mol Biotechnol 3:225-236).

"3' non-coding sequences", "transcription terminator" or "termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) Plant Cell 1:671-680.

"RNA transcript" refers to the product resulting from RNA polymerase-catalyzed transcription of a DNA sequence. When the RNA transcript is a perfect complimentary copy of the DNA sequence, it is referred to as the primary transcript or pre-mRNA. A RNA transcript is referred to as the mature RNA or mRNA when it is a RNA sequence derived from post-transcriptional processing of the primary transcript pre-mRNA. "Messenger RNA" or "mRNA" refers to the RNA that is without introns and that can be translated into protein by the cell. "cDNA" refers to a DNA that is complementary to, and synthesized from, an mRNA template using the enzyme reverse transcriptase. The cDNA can be single-stranded or converted into double-stranded form using the Klenow fragment of DNA polymerase I. "Sense" RNA refers to RNA transcript that includes the mRNA and can be translated into protein within a cell or *in vitro.* "Antisense RNA" refers to an RNA transcript that is complementary to all or part of a target primary transcript or mRNA, and that blocks the expression of a target gene (see, e.g., U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., the coding sequence is under the transcriptional control of the promoter). Coding sequences can be operably linked to regulatory sequences in a sense or antisense orientation. In another example, the complementary RNA regions can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook et al., Molecular Cloning: A Laboratory Manual; Cold Spring Harbor Laboratory: Cold Spring Harbor, NY (1989). Transformation methods are well known to those skilled in the art and are described *infra*.

The term "recombinant" refers to an artificial combination of two otherwise separated segments of sequence, e.g., by chemical synthesis, or manipulation of isolated segments of nucleic acids by genetic engineering techniques.

The terms "plasmid", "vector" and "cassette" refer to a linear or circular extra chromosomal element often carrying genes that are not part of the central metabolism of the cell, and usually in the form of double-stranded DNA. Such elements may be autonomously replicating sequences, genome integrating sequences, phage, or nucleotide sequences, in linear or circular form, of a single- or double-stranded DNA or RNA, derived from any source, in which a number of nucleotide sequences have been joined or recombined into a unique construction which is capable of introducing a polynucleotide of interest into a cell. "Transformation cassette" refers to a specific vector containing a gene and having elements in addition to the gene that facilitates transformation of a particular host cell. "Expression cassette" refers to a specific vector containing a gene and having elements in addition to the gene that allow for expression of that gene in a host.

The terms "recombinant DNA molecule", "recombinant DNA construct", "expression construct", "construct", and "recombinant construct" are used interchangeably herein. A recombinant DNA construct comprises an artificial combination of nucleic acid sequences, e.g., regulatory and coding sequences that are not all found together in nature. For example, a recombinant DNA construct may comprise regulatory sequences and coding sequences that are derived from different sources, or regulatory sequences and coding sequences derived from the same source, but arranged in a manner different than that found in nature. Such a construct may be used by itself or may be used in conjunction with a vector. If a vector is used, then the choice of vector is dependent upon the method that will be used to introduce the vector into the host cells as is well known to those skilled in the art. For example, a plasmid vector can be used. The skilled artisan is well aware of the genetic elements that must be present on the vector in order to successfully transform, select and propagate host cells. The skilled artisan will also recognize that different independent transformation events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

"Mature" protein refers to a post-translationally processed polypeptide (i.e., one from which any pre- or propeptides present in the primary translation product have been removed). "Precursor" protein refers to the primary product of translation of mRNA (i.e., with pre- and propeptides still present). Pre- and propeptides may be but are not limited to intracellular localization signals.

The presently disclosed guide polynucleotides, Cas9 endonucleases, polynucleotide modification templates, donor DNAs, guide polynucleotide/Cas9 endonuclease systems and any one combination thereof, can be introduced into a cell.

Cells include, but are not limited to, human, non-human, animal, bacterial, fungal, insect, yeast, non-conventional yeast, and plant cells as well as plants and seeds produced by the methods described herein.

Any plant or plant part can be used, including monocot and dicot plants or plant part.

Examples of monocot plants that can be used include, but are not limited to, corn (Zea *mays*), rice (*Oryza sativa*), rye (*Secale cereale*), sorghum (*Sorghum bicolor, Sorghum vulgare*), millet (e.g., pearl millet (*Pennisetum glaucum*), proso millet (*Panicum miliaceum*), foxtail millet (*Setaria italica*), finger millet (*Eleusine coracana*))*,* wheat (*Triticum* species, *Triticum aestivum, Triticum monococcum*), sugarcane (*Saccharum spp*.), oats (*Avena*), barley (*Hordeum*), switchgrass (*Panicum virgatum*), pineapple (*Ananas comosus*), banana (*Musa spp*.), palm, ornamentals, turfgrasses, and other grasses.

The term "dicotyledonous " or "dicot" refers to the subclass of angiosperm plants also knows as "dicotyledoneae" and includes reference to whole plants, plant organs (e.g., leaves, stems, roots, etc.), seeds, plant cells, and progeny of the same. Examples of dicot plants that can be used include, but are not limited to, soybean (*Glycine max*), *Brassica* species (Canola) (*Brassica napus, B. campestris, Brassica rapa, Brassica. juncea*), *alfalfa* (*Medicago sativa*),), alfalfa (*Medicago sativa*)*,* tobacco (*Nicotiana tabacum*), *Arabidopsis* (*Arabidopsis thaliana*), sunflower (*Helianthus annuus*), cotton (*Gossypium arboreum*, *Gossypium barbadense*), and peanut (*Arachis hypogaea*), tomato (*Solanum lycopersicum*)*,* potato (*Solanum tuberosum.*

Plant that can be used include safflower (Carthamus tinctorius), sweet potato (Ipomoea batatus), cassava (Manihot esculenta), coffee (Coffea spp.), coconut (Cocos nucifera), citrus trees (Citrus spp.), cocoa (Theobroma cacao), tea (Camellia sinensis), banana (Musa spp.), avocado (Persea americana), fig (Ficus casica), guava (Psidium guajava), mango (Mangifera indica), olive (Olea europaea), papaya (Carica papaya), cashew (Anacardium occidentale), macadamia (Macadamia integrifolia), almond (Prunus amygdalus), sugar beets (Beta vulgaris), vegetables, ornamentals, and conifers.

Vegetables include tomatoes (Lycopersicon esculentum), lettuce (e.g., Lactuca sativa), green beans (Phaseolus vulgaris), lima beans (Phaseolus limensis), peas (Lathyrus spp.), and members of the genus Cucumis such as cucumber (C. sativus), cantaloupe (C. cantalupensis), and musk melon (C. melo). Ornamentals include azalea (Rhododendron spp.), hydrangea (Macrophylla hydrangea), hibiscus (Hibiscus rosasanensis), roses (Rosa spp.), tulips (Tulipa spp.), daffodils (Narcissus spp.), petunias (Petunia hybrida), carnation (Dianthus caryophyllus), poinsettia (Euphorbia pulcherrima), and chrysanthemum.

Conifers that may be employed in practicing the present invention include, for example, pines such as loblolly pine (Pinus taeda), slash pine (Pinus elliotii), ponderosa pine (Pinus ponderosa), lodgepole pine (Pinus contorta), and Monterey pine (Pinus radiata); Douglas fir (Pseudotsuga menziesii); Western hemlock (Tsuga canadensis); Sitka spruce (Picea glauca); redwood (Sequoia sempervirens); true firs such as silver fir (Abies amabilis) and balsam fir (Abies balsamea); and cedars such as Western red cedar (Thuja plicata) and Alaska yellow cedar (Chamaecyparis nootkatensis).

The term "plant" includes whole plants, plant organs, plant tissues, seeds, plant cells, seeds and progeny of the same. Plant cells include, without limitation, cells from seeds, suspension cultures, embryos, meristematic regions, callus tissue, leaves, roots, shoots, gametophytes, sporophytes, pollen and microspores. Plant parts include differentiated and undifferentiated tissues including, but not limited to roots, stems, shoots, leaves, pollens, seeds, tumor tissue and various forms of cells and culture (e.g., single cells, protoplasts, embryos, and callus tissue). The plant tissue may be in plant or in a plant organ, tissue or cell culture. The term "plant organ" refers to plant tissue or a group of tissues that constitute a morphologically and functionally distinct part of a plant. The term "genome" refers to the entire complement of genetic material (genes and non-coding sequences) that is present in each cell of an organism, or virus or organelle; and/or a complete set of chromosomes inherited as a (haploid) unit from one parent. "Progeny" comprises any subsequent generation of a plant.

As used herein, the term "plant part" refers to plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like, as well as the parts themselves. Grain is intended to mean the mature seed produced by commercial growers for purposes other than growing or reproducing the species. Progeny, variants, and mutants of the regenerated plants are also included within the scope of the invention, provided that these parts comprise the introduced polynucleotides.

A transgenic plant includes, for example, a plant which comprises within its genome a heterologous polynucleotide introduced by a transformation step. The heterologous polynucleotide can be stably integrated within the genome such that the polynucleotide is passed on to successive generations. The heterologous polynucleotide may be integrated into the genome alone or as part of a recombinant DNA construct. A transgenic plant can also comprise more than one heterologous polynucleotide within its genome. Each heterologous polynucleotide may confer a different trait to the transgenic plant. A heterologous polynucleotide can include a sequence that originates from a foreign species, or, if from the same species, can be substantially modified from its native form. Transgenic can include any cell, cell line, callus, tissue, plant part or plant, the genotype of which has been altered by the presence of heterologous nucleic acid including those transgenics initially so altered as well as those created by sexual crosses or asexual propagation from the initial transgenic. The alterations of the genome (chromosomal or extra-chromosomal) by conventional plant breeding methods, by the genome editing procedure described herein that does not result in an insertion of a foreign polynucleotide, or by naturally occurring events such as random cross-fertilization, non-recombinant viral infection, non-recombinant bacterial transformation, non-recombinant transposition, or spontaneous mutation are not intended to be regarded as transgenic.

In certain embodiments of the disclosure, a fertile plant is a plant that produces viable male and female gametes and is self-fertile. Such a self-fertile plant can produce a progeny plant without the contribution from any other plant of a gamete and the genetic material contained therein. Other embodiments of the disclosure can involve the use of a plant that is not self-fertile because the plant does not produce male gametes, or female gametes, or both, that are viable or otherwise capable of fertilization. As used herein, a "male sterile plant" is a plant that does not produce male gametes that are viable or otherwise capable of fertilization. As used herein, a "female sterile plant" is a plant that does not produce female gametes that are viable or otherwise capable of fertilization. It is recognized that male-sterile and female-sterile plants can be female-fertile and male- fertile, respectively. It is further recognized that a male fertile (but female sterile) plant can produce viable progeny when crossed with a female fertile plant and that a female fertile (but male sterile) plant can produce viable progeny when crossed with a male fertile plant.

The term "non-conventional yeast" herein refers to any yeast that is not a *Saccharomyces* (e.g., *S*. *cerevisiae*) or *Schizosaccharomyces* yeast species. Non-conventional yeast are described in Non-Conventional Yeasts in Genetics, Biochemistry and Biotechnology: Practical Protocols (K. Wolf, K.D. Breunig, G. Barth, Eds., Springer-Verlag, Berlin, Germany, 2003), which is incorporated herein by reference. Non-conventional yeast in certain embodiments may additionally (or alternatively) be yeast that favor non-homologous end-joining (NHEJ) DNA repair processes over repair processes mediated by homologous recombination (HR). Definition of a non-conventional yeast along these lines - preference of NHEJ over HR - is further disclosed by Chen et al. (PLoS ONE 8:e57952), which is incorporated herein by reference. Preferred non-conventional yeast herein are those of the genus *Yarrowia* (e.g., *Yarrowia lipolytica*)*.* The term "yeast" herein refers to fungal species that predominantly exist in unicellular form. Yeast can alternative be referred to as "yeast cells" herein. (see also US provisional application 62/036,652, filed on August 13, 2014, which is incorporated by reference herein.

The term "crossed" or "cross" or "crossing" in the context of this disclosure means the fusion of gametes via pollination to produce progeny (i.e., cells, seeds, or plants). The term encompasses both sexual crosses (the pollination of one plant by another) and selfing (self-pollination, i.e., when the pollen and ovule (or microspores and megaspores) are from the same plant or genetically identical plants).

The term "introgression" refers to the transmission of a desired allele of a genetic locus from one genetic background to another. For example, introgression of a desired allele at a specified locus can be transmitted to at least one progeny plant via a sexual cross between two parent plants, where at least one of the parent plants has the desired allele within its genome. Alternatively, for example, transmission of an allele can occur by recombination between two donor genomes, e.g., in a fused protoplast, where at least one of the donor protoplasts has the desired allele in its genome. The desired allele can be, e.g., a transgene, a modified (mutated or edited) native allele, or a selected allele of a marker or QTL.

A "centimorgan" (cM) or "map unit" is the distance between two linked genes, markers, target sites, loci, or any pair thereof, wherein 1% of the products of meiosis are recombinant. Thus, a centimorgan is equivalent to a distance equal to a 1% average recombination frequency between the two linked genes, markers, target sites, loci, or any pair thereof.

The present disclosure finds use in the breeding of plants comprising one or more introduced traits.

Maize plants (Zea *mays* L.) can be bred by both self-pollination and cross-pollination techniques. Maize has male flowers, located on the tassel, and female flowers, located on the ear, on the same plant. It can self-pollinate ("selfing") or cross pollinate. Natural pollination occurs in maize when wind blows pollen from the tassels to the silks that protrude from the tops of the incipient ears. Pollination may be readily controlled by techniques known to those of skill in the art. The development of maize hybrids requires the development of homozygous inbred lines, the crossing of these lines, and the evaluation of the crosses. Pedigree breeding and recurrent selections are two of the breeding methods used to develop inbred lines from populations. Breeding programs combine desirable traits from two or more inbred lines or various broad-based sources into breeding pools from which new inbred lines are developed by selfing and selection of desired phenotypes. A hybrid maize variety is the cross of two such inbred lines, each of which may have one or more desirable characteristics lacked by the other or which complement the other. The new inbreds are crossed with other inbred lines and the hybrids from these crosses are evaluated to determine which have commercial potential. The hybrid progeny of the first generation is designated F1. The F1 hybrid is more vigorous than its inbred parents. This hybrid vigor, or heterosis, can be manifested in many ways, including increased vegetative growth and increased yield.

Hybrid maize seed can be produced by a male sterility system incorporating manual detasseling. To produce hybrid seed, the male tassel is removed from the growing female inbred parent, which can be planted in various alternating row patterns with the male inbred parent. Consequently, providing that there is sufficient isolation from sources of foreign maize pollen, the ears of the female inbred will be fertilized only with pollen from the male inbred. The resulting seed is therefore hybrid (F1) and will form hybrid plants.

Field variation impacting plant development can result in plants tasseling after manual detasseling of the female parent is completed. Or, a female inbred plant tassel may not be completely removed during the detasseling process. In any event, the result is that the female plant will successfully shed pollen and some female plants will be self-pollinated. This will result in seed of the female inbred being harvested along with the hybrid seed which is normally produced. Female inbred seed does not exhibit heterosis and therefore is not as productive as F1 seed. In addition, the presence of female inbred seed can represent a germplasm security risk for the company producing the hybrid.

Alternatively, the female inbred can be mechanically detasseled by machine. Mechanical detasseling is approximately as reliable as hand detasseling, but is faster and less costly. However, most detasseling machines produce more damage to the plants than hand detasseling. Thus, no form of detasseling is presently entirely satisfactory, and a need continues to exist for alternatives which further reduce production costs and to eliminate self-pollination of the female parent in the production of hybrid seed.

Mutations that cause male sterility in plants have the potential to be useful in methods for hybrid seed production for crop plants such as maize and can lower production costs by eliminating the need for the labor-intensive removal of male flowers (also known as de-tasseling) from the maternal parent plants used as a hybrid parent. Examples of genes used in such ways include male fertility genes such as MS26 (see for example U.S. Patents 7,098,388, 7,517,975, 7,612,251), MS45 (see for example U.S. Patents 5,478,369, 6,265,640) or MSCA1 (see for example U.S. Patent 7,919,676).

Mutations that cause male sterility in maize have been produced by a variety of methods such as X-rays or UV-irradiations, chemical treatments, or transposable element insertions (ms23, ms25, ms26, ms32) (Chaubal et al. (2000) Am J Bot 87:1193-1201). Conditional regulation of fertility genes through fertility/sterility "molecular switches" could enhance the options for designing new male-sterility systems for crop improvement (Unger et al. (2002) Transgenic Res 11:455-465).

Chromosomal intervals that correlate with a phenotype or trait of interest can be identified. A variety of methods well known in the art are available for identifying chromosomal intervals. The boundaries of such chromosomal intervals are drawn to encompass markers that will be linked to the gene controlling the trait of interest. In other words, the chromosomal interval is drawn such that any marker that lies within that interval (including the terminal markers that define the boundaries of the interval) can be used as a marker for northern leaf blight resistance. In one embodiment, the chromosomal interval comprises at least one QTL, and furthermore, may indeed comprise more than one QTL. Close proximity of multiple QTLs in the same interval may obfuscate the correlation of a particular marker with a particular QTL, as one marker may demonstrate linkage to more than one QTL. Conversely, e.g., if two markers in close proximity show co-segregation with the desired phenotypic trait, it is sometimes unclear if each of those markers identifies the same QTL or two different QTL. The term "quantitative trait locus" or "QTL" refers to a region of DNA that is associated with the differential expression of a quantitative phenotypic trait in at least one genetic background, e.g., in at least one breeding population. The region of the QTL encompasses or is closely linked to the gene or genes that affect the trait in question. An "allele of a QTL" can comprise multiple genes or other genetic factors within a contiguous genomic region or linkage group, such as a haplotype. An allele of a QTL can denote a haplotype within a specified window wherein said window is a contiguous genomic region that can be defined, and tracked, with a set of one or more polymorphic markers. A haplotype can be defined by the unique fingerprint of alleles at each marker within the specified window.

A variety of methods are available to identify those cells having an altered genome at or near a target site without using a screenable marker phenotype. Such methods can be viewed as directly analyzing a target sequence to detect any change in the target sequence, including but not limited to PCR methods, sequencing methods, nuclease digestion, Southern blots, and any combination thereof.

Proteins may be altered in various ways including amino acid substitutions, deletions, truncations, and insertions. Methods for such manipulations are generally known. For example, amino acid sequence variants of the protein(s) can be prepared by mutations in the DNA. Methods for mutagenesis and nucleotide sequence alterations include, for example, Kunkel, (1985) Proc. Natl. Acad. Sci. USA 82:488-92; Kunkel et al., (1987) Meth Enzymol 154:367-82; U.S. Patent No. 4,873,192; Walker and Gaastra, eds. (1983) Techniques in Molecular Biology (MacMillan Publishing Company, New York) and the references cited therein. Guidance regarding amino acid substitutions not likely to affect biological activity of the protein is found, for example, in the model of Dayhoff et al., (1978) Atlas of Protein Sequence and Structure (Natl Biomed Res Found, Washington, D.C.). Conservative substitutions, such as exchanging one amino acid with another having similar properties, may be preferable. Conservative deletions, insertions, and amino acid substitutions are not expected to produce radical changes in the characteristics of the protein, and the effect of any substitution, deletion, insertion, or combination thereof can be evaluated by routine screening assays. Assays for double-strand- break-inducing activity are known and generally measure the overall activity and specificity of the agent on DNA substrates containing target sites.

Standard DNA isolation, purification, molecular cloning, vector construction, and verification/characterization methods are well established, see, for example Sambrook et al., (1989) Molecular Cloning: A Laboratory Manual, (Cold Spring Harbor Laboratory Press, NY). Vectors and constructs include circular plasmids, and linear polynucleotides, comprising a polynucleotide of interest and optionally other components including linkers, adapters, regulatory or analysis. In some examples a recognition site and/or target site can be contained within an intron, coding sequence, 5' UTRs, 3' UTRs, and/or regulatory regions.

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s) and "kb" means kilobase(s).

Non-limiting examples of compositions and methods disclosed herein are as follows:
1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 41, 42, 43, 44, 45, 66, 67, 80, a functional fragment of SEQ ID NOs: 41, 42, 43, 44, 45, 66, 67, 80, and a functional variant of SEQ ID NOs: 41, 42, 43, 44, 45, 66, 67, 80.
2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79.
3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77.
4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of SEQ ID NOs : 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79, wherein said chimeric non-naturally occurring crRNA comprises a variable targeting domain capable of hybridizing to said target sequence.
5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77, wherein said chimeric non-naturally occurring crRNA comprises a variable targeting domain capable of hybridizing to said target sequence.
6. A guide RNA/Cas9 endonuclease complex comprising at least one guide RNA and a Cas9 endonuclease, wherein said Cas9 endonuclease is selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA/Cas9 endonuclease complex is capable of recognizing, binding to, and optionally nicking or cleaving all or part of a target sequence.
7. A guide RNA/Cas9 endonuclease complex comprising at least one guide RNA and a Cas9 endonuclease, wherein said Cas9 endonuclease is encoded by a DNA sequence selected from the group consisting of SEQ ID NOs: 6, 7, 8, 9, 10, 46, 47, 48, 49, 73, a functional fragment of SEQ ID NOs: 6, 7, 8, 9, 10, 46, 47, 48, 49, 73, and a functional variant of SEQ ID NOs: 6, 7, 8, 9, 10, 46, 47, 48, 49, 73, wherein said guide RNA/Cas9 endonuclease complex is capable of recognizing, binding to, and optionally nicking or cleaving all or part of a target sequence.
8. The guide RNA/Cas9 endonuclease complex of any of embodiments 6-7 comprising at least one guide RNA of any one of embodiments 1-5.
9. The guide RNA/Cas9 endonuclease complex of any of embodiments 6-7, wherein said target sequence is located in the genome of a cell.
10. A method for modifying a target site in the genome of a cell, the method comprising introducing into said cell at least one guide RNA and at least one Cas9 endonuclease protein selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA and Cas9 endonuclease can form a complex that is capable of recognizing, binding to, and optionally nicking or cleaving all or part of said target site.
11. The method of embodiment 10, further comprising identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).
12. A method for editing a nucleotide sequence in the genome of a cell, the method comprising introducing into said cell a polynucleotide modification template, at least one guide RNA, and at least one Cas9 endonuclease protein selected from the group consisting of SEQ ID NOs: consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.
13. A method for modifying a target site in the genome of a cell, the method comprising introducing into said cell at least one guide RNA, at least one donor DNA, and at least one Cas9 endonuclease selected from the group consisting of SEQ ID NOs: SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said at least one guide RNA and at least one Cas9 endonuclease can form a complex that is capable of recognizing, binding to, and optionally nicking or cleaving all or part of said target site, wherein said donor DNA comprises a polynucleotide of interest.
14. The method of embodiment 13, further comprising identifying at least one cell that has said polynucleotide of interest integrated in or near said target site.
15. The method of any one of embodiments 10-14, wherein the cell is selected from the group consisting of a human, non-human, animal, bacterial, fungal, insect, yeast, non-conventional yeast, and plant cell.
16. The method of embodiment 15, wherein the plant cell is selected from the group consisting of a monocot and dicot cell.
17. The method of embodiment 16, wherein the plant cell is selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, Arabidopsis, and safflower cell.
18. A plant comprising a modified target site, wherein said plant originates from a plant cell comprising a modified target site produced by the method of any of embodiments 10-17.
19. A plant comprising an edited nucleotide, wherein said plant originates from a plant cell comprising an edited nucleotide produced by the method of embodiment 12.

### EXAMPLES

In the following Examples, unless otherwise stated, parts and percentages are by weight and degrees are Celsius. It should be understood that these Examples, while indicating embodiments of the disclosure, are given by way of illustration only. From the above discussion and these Examples, one skilled in the art can make various changes and modifications of the disclosure to adapt it to various usages and conditions. Such modifications are also intended to fall within the scope of the appended claims.

### EXAMPLE 1

### Characterization of new Cas9 endonucleases and cognate guide RNAs

CRISPR-Cas loci from uncharacterized CRISPR-Cas systems were identified by searching internal Pioneer-DuPont databases consisting of microbial genomes as described below. First, multiple sequence alignment of protein sequences from a diverse collection of Cas9 endonucleases was performed using MUSCLE (Edgar R. (2004) Nucleic Acids Res. 32(5): 1792-97). The alignments were examined and curated and were used to build profile hidden Markov models (HMM) for Cas9 subfamilies using HMMER (Eddy S.R. (1998) Bioinformatics 14:755-763; Eddy S.R. (2011) PLoS Comp. Biol., 7:e1002195). The resulting HMM models were then utilized to search protein sequences translated from DNA sequence collections for the presence of cas9-like genes. The resulting genes were further validated as encoding a Cas9 protein by examining the translated amino acid sequence for the presence of HNH and RuvC cleavage domains. Surprisingly, when searching for the presence of other structural components of a Type II CRISPR-Cas system (Makarova et al. (2015) Nat. Rev. Microbiol. 13:722-736; such cas1 gene, cas2 gene, CRISPR array and tracrRNA encoding region) for the novel CRISPR-Loci described herein, no cas1 and cas2 genes were identified in proximity of the cas9 gene. Cas1 and cas2 genes were searched for by examining the protein translations of open-reading-frames (ORFs) ≥ 201 nucleotides within the CRISPR-Cas locus against the NCBI protein database for those matching known Cas1 and Cas2 proteins using the PSI-BLAST program (Altschul, S. F. et al. (2005) FEBS J. 272:5101-5109). The CRISPR array was detected using the PILER-CR program (Edgar R. (2007) BMC Bioinformatics 8:18. Additional CRISPR array repeats not detected by PILER-CR were identified by performing pairwise alignments of the locus with the PILER-CR identified repeats using the blastn program (Altschul, S. F. et al. (1997) Nucleic Acids Res. 25:3389-3402). The tracrRNA encoding region, termed the anti-repeat, was established by searching the locus for regions (distinct from the CRISPR array) with complete to partial homology to the repeats in the CRISPR array. Interestingly, the cas9 genes of the novel CRISPR-Cas loci described herein were flanked on both sides by a CRISPR array (Figures 1-5). In total, 5 DNA regions (Locus 1- Locus 5) were selected (Table 2).

**Table 2. List of sequence for novel CRISPR-Cas loci (Locus 1- Locus 5) identified from Pioneer-Dupont databases.**

| CRISPR-Cas locus name | Genus/species of Origin | SEQ ID NO: |
|---|---|---|
| Locus 1 | Bacillus species | 1 |
| Locus 2 | Bacillus species | 2 |
| Locus 3 | Bacillus species | 3 |
| Locus 4 | Bacillus species | 4 |
| Locus 5 | unknown | 5 |

A schematic of the DNA locus for each novel Cas9 system is depicted in Figures 1-5. The cas9 gene open-reading-frame (cas9 gene ORF), CRISPR array with CRISPR repeats and anti-repeat (the genomic DNA region demonstrating partial homology to the CRISPR array repeat that indicates the location of the encoded tracrRNA) are indicated.

The genomic DNA sequence and length of each cas9 gene ORF and cas9 gene translation (not including the stop codon) are referenced in Table 3 for each system. Note that both Locus 3 and Locus 5 may encode Cas endonuclease proteins that may be comprised of 3 possible lengths being different in length at the 5' end of the ORF or N-terminal end of the protein (Table 3). Table 4 lists the consensus sequence of the CRISPR array repeats from the DNA locus of each system and the sequences of the anti-repeat for each system (as DNA sequence on the same strand as the cas9 gene ORF).

**Table 3. Sequence and length of the cas9 gene ORF and cas9 gene translation from each CRISPR-Cas system identified as described herein.**

| Non-type II Cas9 endonuclease name | *cas9* Gene ORF (SEQ ID NO:) | Length of *cas9* Gene ORF (bp) | Translation of *cas9* Gene ORF (not including the stop codon) (SEQ ID NO) | Length of *cas9* Gene Translation (No. of Amino Acids) |
|---|---|---|---|---|
| Cas-Locus 1 | 6 | 3213 | 11 | 1070 |
| Cas-Locus 2 | 7 | 3213 | 12 | 1070 |
| Cas-Locus 3 | 8, 46, 47 | 3207, 3213, 3225 | 13, 50, 51 | 1068, 1070, 1074 |
| Cas-Locus 4 | 9 | 3207 | 14 | 1068 |
| Cas-Locus 5 | 10, 48, 49 | 3207, 3213, 3225 | 15, 52, 53 | 1068, 1070, 1074 |

**Table 4. CRISPR repeat consensus and anti-repeat (putative tracrRNA coding region) for the CRISPR-Cas systems described herein.**

| CRISPR-Cas locus name | CRISPR repeat consensus (SEQ ID NO) | CRISPR repeat length | CRISPR Array Transcriptional Direction | Anti-Repeat Consensus (SEQ ID NO) | Anti-Repeat direction |
|---|---|---|---|---|---|
| Locus 1 | 16 | 36 | Anti-sense | 21 | Sense |
| Locus 2 | 17 | 36 | Anti-sense | 22 | Sense |
| Locus 3 | 18 | 36 | Anti-sense | 23 | Sense |
| Locus 4 | 19 | 36 | Anti-sense | 24 | Sense |
| Locus 5 | 20 | 36 | Anti-sense | 25 | Sense |

The possible transcriptional directions of the putative tracrRNAs for each new system were considered by examining the secondary structures and possible termination signals present in a RNA version of the sense and anti-sense genomic DNA sequences surrounding the anti-repeat (as described in US patent applications 62/162,377 filed May 15, 2015, 62/162,353 filed May 15, 2015 and 62/196,535 filed July 24, 2015, all three applications incorporated in their entirety herein by reference). Based on the hairpin-like secondary structures and termination signals present for each system, the transcriptional direction of the tracrRNA for all the CRISPR-Cas systems can be deduced. Because the anti-repeat in the tracrRNA can hybridize to the crRNA derived from the CRISPR array to form a duplexed RNA capable of guiding the Cas9 endonuclease to cleave invading DNA the transcriptional direction of the CRISPR array may also be determined based on the direction of tracrRNA transcription (since double-stranded RNA hybridizes with 5' to 3' directionality). The transcriptional directions of both the tracrRNA and CRISPR array were deduced for each system as described above and are listed in Table 4 and depicted in Figures 1-5. Based on the likely transcriptional direction of the tracrRNA and CRISPR array, single guide RNAs (sgRNAs, SEQ ID NOs: 41-45) were designed and are shown in Table 5.

**Table 5. Examples of sgRNAs (SEQ ID NOs: 41-45) and its components (VT, crRNA repeat, loop, anti-repeat and 3'tracrRNA) for each new diverse CRISPR-Cas endonuclease described herein.**

| Cas9 endonuclease name | Variable targeting domain (VT) | crRNA repeat (SEQ ID NO) | Loop | Anti-Repeat (SEQ ID NO) | 3'tracrRNA (SEQ ID NO) | Single guide RNA (sgRNA) SEQ ID NO: |
|---|---|---|---|---|---|---|
| Cas-Locus 1 | N_{20 (*)} | 26 | N_{4 (**)} | 31 | 36 | 41 |
| Cas-Locus 2 | N_{20 (*)} | 27 | N_{4 (**)} | 32 | 37 | 42 |
| Cas-Locus 3 | N_{20 (*)} | 28 | N_{4 (**)} | 33 | 38 | 43 |
| Cas-Locus 4 | N_{20 (*)} | 29 | N_{4 (**)} | 34 | 39 | 44 |
| Cas-Locus 5 | N_{20 (*)} | 30 | N_{4 (**)} | 35 | 40 | 45 |

| | | | | | | |
|---|---|---|---|---|---|---|
| N_{20 (*)} indicates a series of 20 nucleotides as one example of a sgRNA variable targeting domain. As described herein, the variable targeting domain of a sgRNA can vary for example, but not limiting from at least 12 to 30 nucleotides. N_{4 (**)} indicates a loop of 4 nucleotides such as but not limiting to GAAA. As described herein, the length of the loop can vary from at least 3 nucleotides to 100 nucleotides. | | | | | | |

Rapid in vitro methods to characterize the protospacer adjacent motif (PAM) specificity of Type II Cas9 proteins have been described (see US patent applications 62/162,377 filed May 15, 2015, 62/162,353 filed May 15, 2015 and 62/196,535 filed July 24, 2015, incorporated in their entirety herein by reference) and can be used to characterize the PAM preference of the novel CRISPR-Cas systems described herein.

The single guide RNAs described herein (Table 5) can be complexed with the respective purified Cas9 protein (for example SEQ ID NO: 41 -Table 5- can be complexed with the Cas-Locus 1 endonuclease protein of SEQ ID NO: 11 - Table 3) and assayed for their ability to support cleavage of a randomized PAM plasmid DNA library (as described in Example 7 of WO2016/186946, published on November 24, 2016, incorporated herein by reference. If the sgRNA does not support cleavage activity, new guide RNA designs (either sgRNA or duplexed crRNA and tracrRNA; in both possible transcriptional directions of the CRISPR array and anti-repeat region) will be tested for their ability to support cleavage.

Once a guide RNA that supports Cas9 cleavage has been established, the PAM specificity of each Cas9 endonuclease can be assayed (as described in Examples 4, 8, 14 and 15 of WO2016/186946, published on November 24, 2016, incorporated herein by reference). PAM preferences which extend past the randomized PAM region may also be examined (as described in Example 11 of US patent applications 62/162,377, filed May 15, 2015). After PAM preferences have been determined, the sgRNAs may be further refined for maximal activity or cellular transcription by either increasing or decreasing the tracrRNA 3' end tail length, increasing or decreasing crRNA repeat and tracrRNA anti-repeat length, modifying the 4 nt self-folding loop or altering the sequence composition.

The guide RNA solutions provided in Table 5 supported target recognition and cleavage for all of the non-Type II Cas9s examined (Cas-Locus 1 (SEQ ID NO: 11), Cas-Locus 2 (SEQ ID NO: 12), Cas-Locus 3 (SEQ ID NO: 51) and Cas-Locus 5 (SEQ ID NO: 15)). Digestion of randomized PAM libraries followed by the capture and analysis of the PAM sequences which supported cleavage activity as described previously (see Examples 4, 8, 14 and 15 of WO2016/186946, published on November 24, 2016, incorporated herein by reference) yielded the PAM recognition profiles shown in Tables 6-9.

Taken together, the non-Type II Cas9 proteins combined with the guide polynucleotide solutions listed in Table 5 were capable of programmable RNA directed DNA target recognition and cleavage. Interestingly, Cas-Locus 3 and Cas-Locus 5 exhibited a strong preference for only a single PAM nucleotide, a G at position 5.

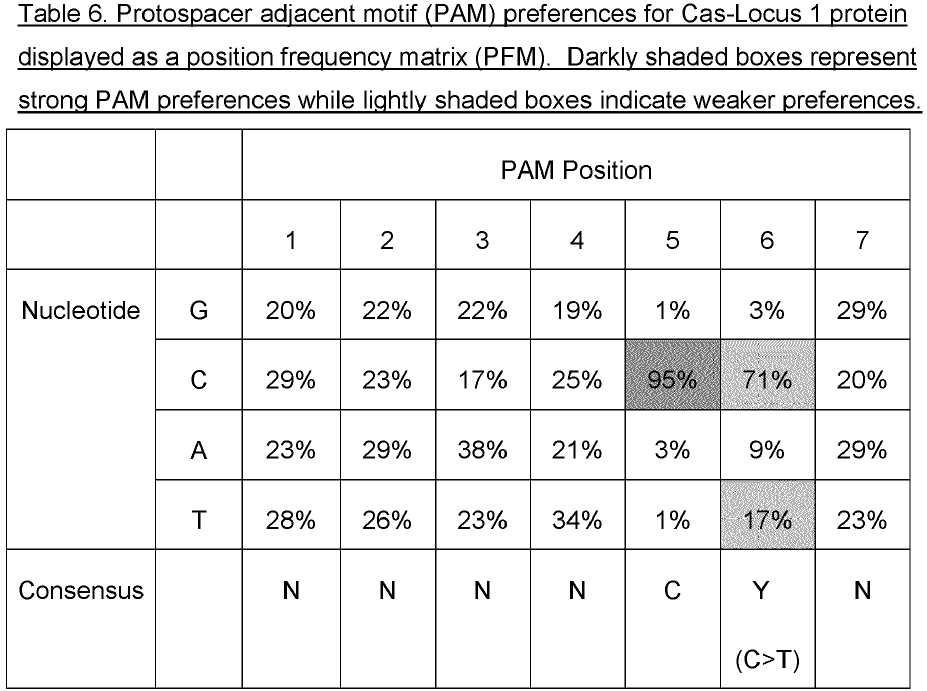

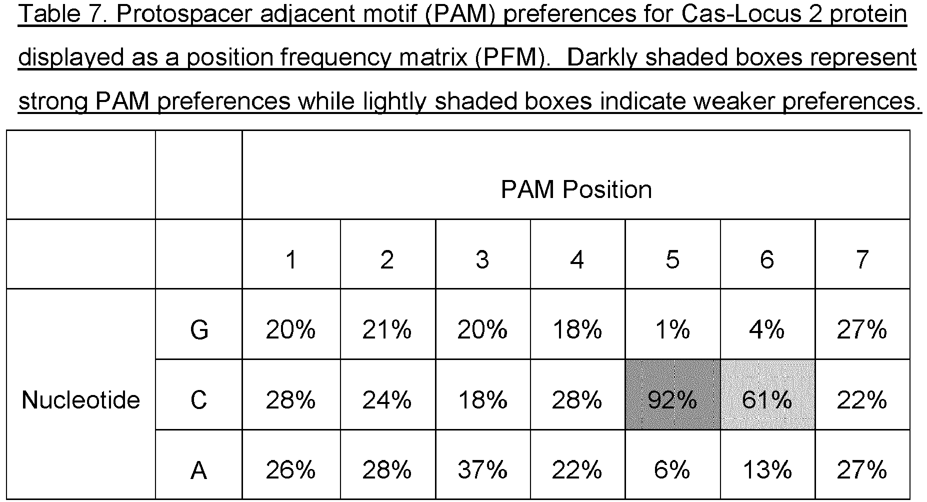

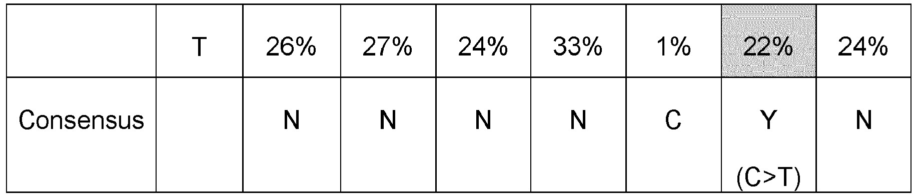

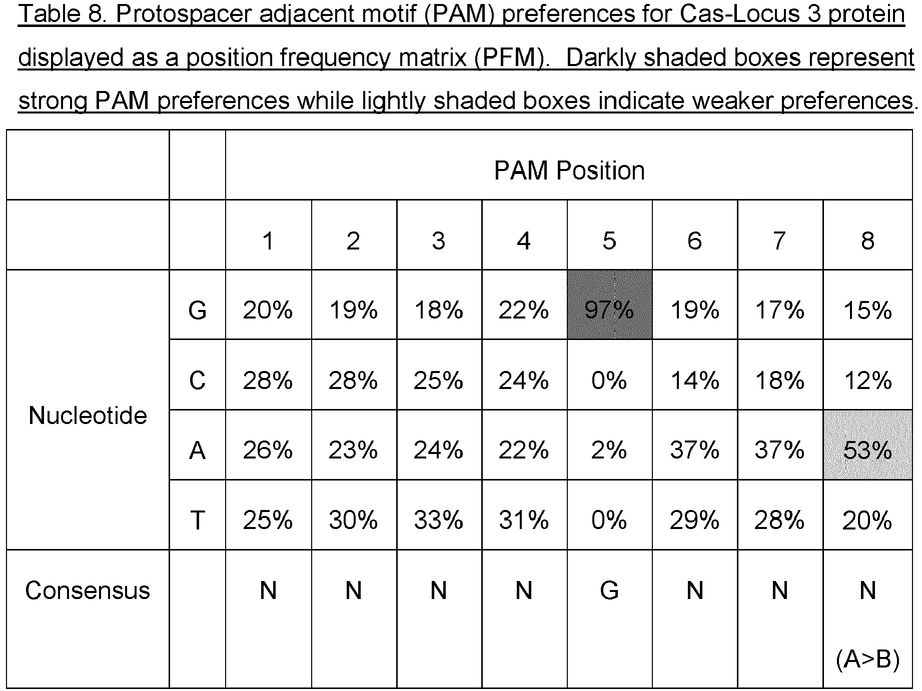

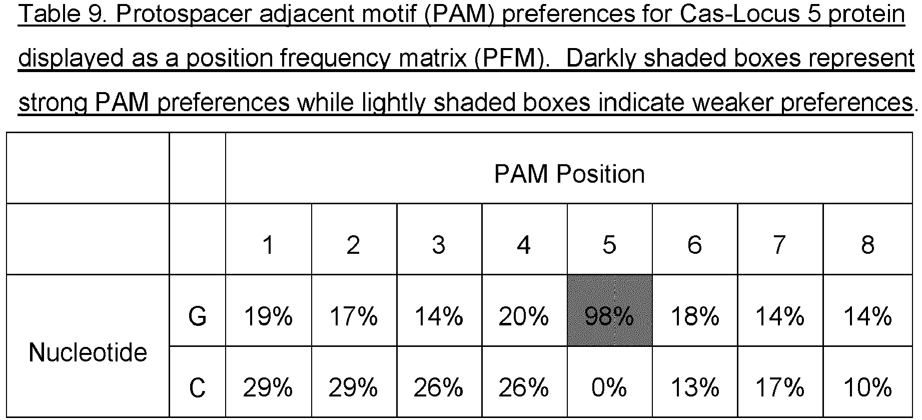

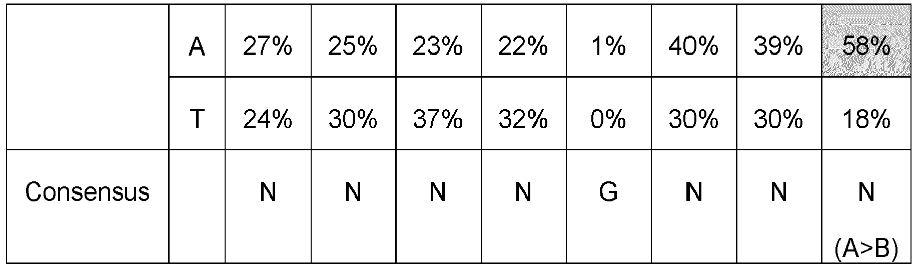

### EXAMPLE 2

### Maize optimized non-Type II Cas9 endonucleases and guide polynucleotide expression cassettes for genome modification in maize plants

To confer efficient expression in maize cells, the non-Type II Cas-Locus 3 endonuclease gene from *Bacillus* (SEQ ID NO: 47) was maize codon optimized per standard techniques known in the art and the potato ST-LS1 intron 2 (SEQ ID NO: 54) was introduced in order to eliminate its expression in *E*.*coli* and *Agrobacterium*. To facilitate nuclear localization of the maize optimized Cas-Locus 3 endonuclease protein in maize cells, Simian virus 40 (SV40) monopartite (MAPKKKRKV, SEQ ID NO: 55) and Agrobacterium tumefaciens bipartite VirD2 T-DNA border endonuclease (KRPRDRHDGELGGRKRAR, SEQ ID NO: 56) nuclear localization signals were incorporated at the amino and carboxyl-termini of the Cas9 open reading frame, respectively. The resulting nucleotide sequences, encoding the maize optimized Cas-Locus 3 endonuclease and nuclear localization signals (SEQ ID NO: 57), were then operably linked to a maize ubiquitin promoter (SEQ ID NO: 58), maize ubiquitin 5 prime UTR (SEQ ID NO: 59), maize ubiquitin intron 1 (SEQ ID NO: 60) and suitable terminator by standard molecular biological techniques. The key components of the resulting maize optimized Cas-Locus 3 expression cassette are illustrated in Figure 6 resulting in SEQ ID NO: 61.

The Cas-Locus 3 endonuclease is directed by two small RNAs (CRISPR RNA (crRNA) and trans-activating crRNA (tracrRNA)) or a synthetic engineered single guide RNA (sgRNA) to cleave double-stranded DNA. To establish the optimal sgRNA solution for Cas-Locus 3 endonuclease, two additional versions of the computationally predicted sgRNA in Table 5 (SEQ ID NO: 43) were also generated (Table 10) (SEQ ID NOs: 66 and 67). The resulting guide RNAs were comprised of a ∼20 nt sequence that serves to direct Cas-Locus 3 cleavage by base pairing with one strand of the DNA target site (Cas-Locus 3 variable targeting domain) and a sequence that aids recognition by Cas-Locus 3 (referred to as Cas-Locus 3 recognition domain). To transcribe the sgRNAs necessary for directing Cas-Locus 3 endonuclease cleavage activity in maize cells, a U6 polymerase III promoter (SEQ ID NO: 68) and terminator (TTTTTTTT) were isolated from maize and operably fused to the ends of DNA sequences encoding the various sgRNA solutions for Cas-Locus 3. To promote optimal transcription of the guide RNA from the maize U6 polymerase III promoter, a G nucleotide was added to the 5 prime end of the sequence to be transcribed. The key components of the resulting maize optimized Cas-Locus 3 guide RNA transcriptional cassettes generated here are illustrated in Figure 7.

**Table 10. Two single guide RNA (sgRNA) variations (SEQ ID NOs: 66 and 67) for testing with Cas-Locus 3 endonuclease in maize cells. The components (VT, crRNA repeat, loop, anti-repeat and 3'tracrRNA) for each are as shown.**

| Cas9 endonuclease name | Variable targeting domain (VT) | crRNA repeat (SEQ ID NO) | Loop | Anti-Repeat (SEQ ID NO) | 3'tracrRNA (SEQ ID NO) | Single guide RNA (sgRNA) SEQ ID NO: |
|---|---|---|---|---|---|---|
| Cas-Locus 3 | N_{20 (*)} | 62 | N_{4 (**)} | 64 | 38 | 66 |
| Cas-Locus 3 | N_{20 (*)} | 63 | N_{4 (**)} | 65 | 38 | 67 |

### EXAMPLE 3

### The maize optimized Cas-Locus 3 endonuclease and guide polynucleotide system may be used to direct cleavage of maize chromosomal DNA

To test whether the maize optimized Cas-Locus 3 endonuclease (SEQ ID NO: 57) and guide polynucleotide system described in Examples 1 and 2 could be used to recognize, cleave, and mutate maize chromosomal DNA, three different genomic target sequences (Table 11) were targeted for cleavage and examined by deep sequencing for the presence of mutations indicative of cleavage.

**Table 11. Cas-Locus 3 genomic target sequences**

| Location | Target Site Designation | Target Site | | Target Site SEQ ID NO: | |
|---|---|---|---|---|---|
| | | ∼20 nt 5 Prime of PAM | PAM | | |
| Chr1: 51.81 cM | MS26 | GGTCAGGGACACCATAT GAAT | GAAAGAAA | | 69 |
| Chr2: 28.45 cM | LIGULELE SS | GATCCCACATAGGTGATG AGCT | AAGAGTAA | | 70 |
| Chr9: 119.15cM | MS45 | GACCACACGAATCTGGC CTTGA | CCAGGATA | | 71 |

Target sites with an appropriate protospacer adjacent motif (PAM) (NNNNGNNA) for Cas-Locus 3 and the ∼20 nts 5 prime of the PAM were selected (Table 11). The ∼20 nt DNA sequence 5 prime of the PAM for each target was then separately introduced into the variable targeting domain of the maize optimized guide RNA transcriptional cassettes described in Example 2 per standard molecular biology techniques so that upon transcription each cassette produced a guide RNA that forms a guide RNA/Cas-Locus 3 endonuclease complex that directs the Cas-Locus 3 endonuclease to its target site. Each of the resulting guide RNA transcriptional cassettes were then separately co-delivered with the maize optimized Cas-Locus 3 endonuclease expression cassette described in Example 2 by biolistic transformation into Hi-Type II 10-day-old immature maize embryos (IMEs) in the presence of BBM and WUS2 genes as described in Svitashev *et al.* (2015). Since particle gun transformation can be highly variable, a visual marker DNA expression cassette encoding a yellow fluorescent protein was also co-delivered with the Cas-Locus 3 and small RNA encoding vectors to aid in the selection of evenly transformed IMEs and each treatment was performed in triplicate. After 2 days, the 20-30 most evenly transformed IMEs were harvested based on their fluorescence. Total genomic DNA was extracted and the region surrounding the intended target site was PCR amplified with Phusion® HighFidelity PCR Master Mix (New England Biolabs, M0531L) adding on the sequences necessary for amplicon-specific barcodes and Illumnia sequencing using "tailed" primers through two rounds of PCR and deep sequenced similar to that described in Karvelis *et al.* (2015) (Methods Section: *In planta* mutation detection). The resulting reads were examined for the presence of mutations at the expected site of cleavage by comparison to control experiments where the small RNA transcriptional cassette was omitted from the transformation as described in Karvelis *et al.* (2015) except the 24 most prevalent mutation types were used to calculate the overall mutation frequency.

Surprisingly, all 3 sites targeted for cleavage with the guided polynucleotide/Cas-Locus 3 system described herein exhibited the presence of mutations in the form of small insertions or deletions (indels) indicative of chromosomal cleavage and repair (Table 12). The single guide RNA (sgRNA) solution resulting in the highest frequency of indel mutations, SEQ ID NO: 66, is listed in Table 10.

Taken together, these data indicate that non-Type II endonuclease and guide polynucleotide system described herein may be used to target maize chromosomal DNA for cleavage and be utilized as a tool to bind, nick, or cleave plant chromosomal DNA for the purposes of plant genomic and gene manipulation.

**Table 12. Frequency of indels at 3 maize sites two days after transformation with the maize optimized Cas-Locus 3 and guide polynucleotide system (SEQ ID NO: 66).**

| Endonuclease | Maize Target | Average Mutation Frequency | Standard Error Measurement |
|---|---|---|---|
| Cas-Locus 3 | MS26 (SEQ ID NO: 69) | 0.42% | 0.14% |
| Cas-Locus 3 | LIGULELESS (SEQ ID NO: 70) | 0.24% | 0.12% |
| Cas-Locus 3 | MS45 (SEQ ID NO: 71) | 0.29% | 0.05% |

### EXAMPLE 4

### Characterization of a new Cas9 endonucleases (Cas-Locus 42) and cognate guide RNAs

CRISPR-Cas loci from uncharacterized CRISPR-Cas systems were identified by searching internal Pioneer-DuPont databases consisting of microbial genomes as described below. First, multiple sequence alignment of protein sequences from a diverse collection of Cas9 endonucleases was performed using MUSCLE (Edgar R. (2004) Nucleic Acids Res. 32(5): 1792-97). The alignments were examined and curated and were used to build profile hidden Markov models (HMM) for Cas9 subfamilies using HMMER (Eddy S.R. (1998) Bioinformatics 14:755-763; Eddy S.R. (2011) PLoS Comp. Biol., 7:e1002195). The resulting HMM models were then utilized to search protein sequences translated from DNA sequence collections for the presence of cas9-like genes. The resulting genes were further validated as encoding a Cas9 protein by examining the translated amino acid sequence for the presence of HNH and RuvC cleavage domains. Surprisingly, when searching for the presence of other structural components of a Type II CRISPR-Cas system (Makarova et al. (2015) Nat. Rev. Microbiol. 13:722-736; such as cas1 gene, cas2 gene, CRISPR array and tracrRNA encoding region) for the novel CRISPR-Locus described herein, no cas1 and cas2 genes were identified in proximity of the cas9 gene. Cas1 and cas2 genes were searched for by examining the protein translations of open-reading-frames (ORFs) ≥ 201 nucleotides within the CRISPR-Cas locus against the NCBI protein database for those matching known Cas1 and Cas2 proteins using the PSI-BLAST program (Altschul, S. F. et al. (2005) FEBS J. 272:5101-5109). The CRISPR array was detected using the PILER-CR program (Edgar R. (2007) BMC Bioinformatics 8:18. Additional CRISPR array repeats not detected by PILER-CR were identified by performing pairwise alignments of the locus with the PILER-CR identified repeats using the blastn program (Altschul, S. F. et al. (1997) Nucleic Acids Res. 25:3389-3402). The tracrRNA encoding region, termed the anti-repeat, was established by searching the locus for regions (distinct from the CRISPR array) with complete to partial homology to the repeats in the CRISPR array. Interestingly, the cas9 gene of the novel CRISPR-Cas Locus (Locus 42) was flanked on both sides by one or more CRISPR arrays (Figure 8 and Table 13).

**Table 13. Novel CRISPR-Cas Locus (Locus 42) identified from Pioneer-Dupont databases.**

| CRISPR-Cas locus name | Genus/species of Origin | SEQ ID NO: |
|---|---|---|
| Locus 42 | Bacillus species | 72 |

A schematic of the DNA locus for the novel Cas9 system is depicted in Figure 8. The *cas9* gene open-reading-frame (*cas9* gene ORF), CRISPR array with CRISPR repeats and anti-repeat (the genomic DNA region demonstrating partial homology to the CRISPR array repeat that indicates the location of the encoded tracrRNA) are indicated.

The genomic DNA sequence and length of the *cas9* gene ORF and *cas9* gene translation (not including the stop codon) are referenced in Table 14. Table 15 lists the consensus sequence of the CRISPR array repeats from the DNA locus and the sequences of the anti-repeat (as DNA sequence on the same strand as the cas9 gene ORF).

**Table 14. Sequence and length of the cas9 gene ORF and cas9 gene translation from the novel CRISPR-Cas system identified as described herein.**

| Cas9 endonuclease name | *cas9* Gene ORF (SEQ ID NO) | Length of *cas9* Gene ORF (bp) | Translation of *cas9* Gene ORF (not including the stop codon) (SEQ ID NO) | Length of *cas9* Gene Translation (No. of Amino Acids) |
|---|---|---|---|---|
| Cas-Locus 42 | 73 | 3204 | 74 | 1068 |

**Table 15. CRISPR repeat consensus and anti-repeat (putative tracrRNA encoding region) for the CRISPR-Cas system described herein.**

| CRISPR-Cas locus name | CRISPR repeat consensus (SEQ ID NO) | CRISPR repeat length | CRISPR Array Transcriptional Direction | Anti-Repeat (SEQ ID NO) | Anti-Repeat direction |
|---|---|---|---|---|---|
| Locus 42 | 75 | 36 | Anti-sense | 76 | Sense |

The possible transcriptional directions of the putative tracrRNAs for the new system were considered by examining the secondary structures and possible termination signals present in a RNA version of the sense and anti-sense genomic DNA sequences surrounding the anti-repeat (as described in PCT/US16/32028 filed May 12, 2016, and PCT/US16/32073 filed May 12, 2016, both applications incorporated in their entirety herein by reference). Based on the hairpin-like secondary structures and termination signals present for the system, the transcriptional direction of the tracrRNA for the CRISPR-Cas system can be deduced. Because the anti-repeat in the tracrRNA can hybridize to the crRNA derived from the CRISPR array to form a duplexed RNA capable of guiding the Cas9 endonuclease to cleave invading DNA the transcriptional direction of the CRISPR array may also be determined based on the direction of tracrRNA transcription (since double-stranded RNA hybridizes with 5' to 3' directionality). The transcriptional directions of both the tracrRNA and CRISPR array were deduced for the system as described above and are listed in Table 15 and depicted in Figure 8. Based on the likely transcriptional direction of the tracrRNA and CRISPR array, a single guide RNA (sgRNA, SEQ ID NO: 80) was designed and is shown in Table 16.

**Table 16. Example of a sgRNA (SEQ ID NO: 80) and its components (VT, crRNA repeat, loop, anti-repeat and 3' tracrRNA) for the new diverse CRISPR-Cas endonuclease described herein.**

| Cas endonuclease name | Variable targeting domain (VT) | crRNA repeat (SEQ ID NO) | Loop | Anti-Repeat (SEQ ID NO) | 3'tracrRNA (SEQ ID NO) | Single guide RNA (sgRNA) SEQ ID NO: |
|---|---|---|---|---|---|---|
| Cas-Locus 42 | N_{20 (*)} | 77 | N_{4 (**)} | 78 | 79 | 80 |

| | | | | | | |
|---|---|---|---|---|---|---|
| N_{20 (*)} indicates a series of 20 nucleotides as one example of a sgRNA variable targeting domain. As described herein, the variable targeting domain of a sgRNA can vary for example, but not limiting from at least 12 to 30 nucleotides. N_{4 (**)} indicates a loop of 4 nucleotides such as but not limiting to GAAA. As described herein, the length of the loop can vary from at least 3 nucleotides to 100 nucleotides. | | | | | | |

Rapid in vitro methods to characterize the protospacer adjacent motif (PAM) specificity of Type II Cas9 proteins have been described (see WO2016/186946, published on November 24, 2016 and WO2016/186953, published on November 24, 2016, both incorporated in their entirety herein by reference) and can be used to characterize the PAM preference of the novel CRISPR-Cas system described herein.

The single guide RNA described herein (Table 16) can be complexed with the respective purified Cas9 protein (for example SEQ ID NO: 80 - can be complexed with the Cas9 endonuclease protein of SEQ ID NO: 74) and assayed for its ability to support cleavage of a randomized PAM plasmid DNA library (as described in Example 7 WO2016/186946, published on November 24, 2016, incorporated herein by reference. If the sgRNA does not support cleavage activity, new guide RNA designs (either sgRNA or duplexed crRNA and tracrRNA; in both possible transcriptional directions of the CRISPR array and anti-repeat region) will be tested for their ability to support cleavage.

Once a guide RNA that supports Cas9 cleavage has been established, the PAM specificity of each Cas9 endonuclease can be assayed (as described in Examples 4, 8, 14 and 15 of WO2016/186946, published on November 24, 2016, incorporated herein by reference. PAM preferences which extend past the randomized PAM region may also be examined (as described in Example 11 of WO2016/186946, published on November 24, 2016, incorporated herein by reference. After PAM preferences have been determined, the sgRNAs may be further refined for maximal activity or cellular transcription by either increasing or decreasing the tracrRNA 3' end tail length, increasing or decreasing crRNA repeat and tracrRNA anti-repeat length, modifying the 4 nt self-folding loop or altering the sequence composition.

The guide RNA solution provided in Table 16 supported target recognition and cleavage for the non-Type II Cas9s examined (Cas-Locus 42). Digestion of randomized PAM libraries followed by the capture and analysis of the PAM sequences which supported cleavage activity as described previously (see Examples 4, 8, 14 and 15 of WO2016/186946, published on November 24, 2016, incorporated herein by reference) yielded the PAM recognition profile shown in Table 17.

Taken together, the non-Type II Cas9 protein (Cas-Locus 42) combined with the guide polynucleotide solution listed in Table 5 were capable of programmable RNA directed DNA target recognition and cleavage.

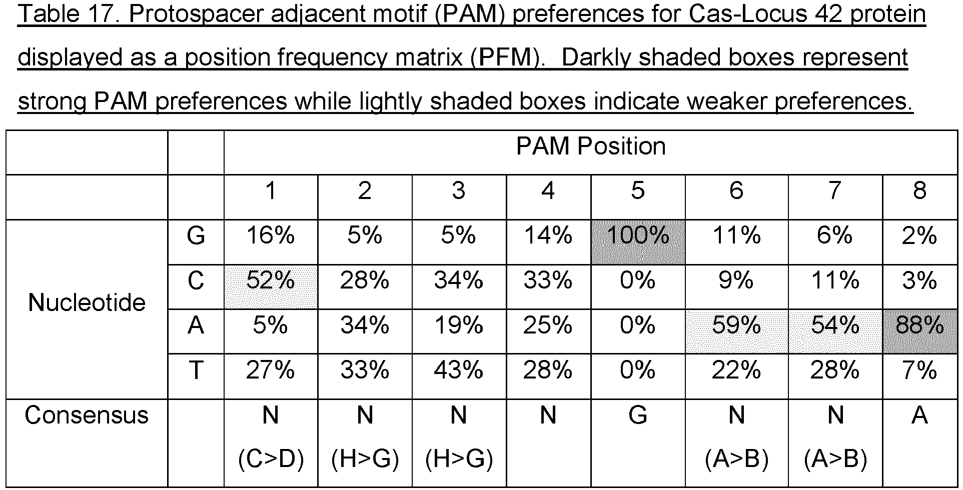

### EXAMPLE 5

### Transformation of Maize Immature Embryos

Transformation can be accomplished by various methods known to be effective in plants, including particle-mediated delivery, *Agrobacterium*-mediated transformation, PEG-mediated delivery, and electroporation.

### a. Particle-mediated delivery

Transformation of maize immature embryos using particle delivery is performed as follows. Media recipes follow below.

The ears are husked and surface sterilized in 30% Clorox bleach plus 0.5% Micro detergent for 20 minutes, and rinsed two times with sterile water. The immature embryos are isolated and placed embryo axis side down (scutellum side up), 25 embryos per plate, on 560Y medium for 4 hours and then aligned within the 2.5-cm target zone in preparation for bombardment. Alternatively, isolated embryos are placed on 560L (Initiation medium) and placed in the dark at temperatures ranging from 26°C to 37°C for 8 to 24 hours prior to placing on 560Y for 4 hours at 26°C prior to bombardment as described above.

Plasmids containing the double strand brake inducing agent and donor DNA are constructed using standard molecular biology techniques and co-bombarded with plasmids containing the developmental genes ODP2 (AP2 domain transcription factor ODP2 (Ovule development protein 2); US20090328252 A1) and Wushel (US2011/0167516).

The plasmids and DNA of interest are precipitated onto 0.6 µm (average diameter) gold pellets using a water-soluble cationic lipid transfection reagent as follows. DNA solution is prepared on ice using 1 µg of plasmid DNA and optionally other constructs for co-bombardment such as 50 ng (0.5 µl) of each plasmid containing the developmental genes ODP2 (AP2 domain transcription factor ODP2 (Ovule development protein 2); US20090328252 A1) and Wushel. To the pre-mixed DNA, 20 µl of prepared gold particles (15 mg/ml) and 5 µl of a water-soluble cationic lipid transfection reagent is added in water and mixed carefully. Gold particles are pelleted in a microfuge at 10,000 rpm for 1 min and supernatant is removed. The resulting pellet is carefully rinsed with 100 ml of 100% EtOH without resuspending the pellet and the EtOH rinse is carefully removed. 105 µl of 100% EtOH is added and the particles are resuspended by brief sonication. Then, 10 µl is spotted onto the center of each macrocarrier and allowed to dry about 2 minutes before bombardment.

Alternatively, the plasmids and DNA of interest are precipitated onto 1.1 µm (average diameter) tungsten pellets using a calcium chloride (CaCl₂) precipitation procedure by mixing 100 µl prepared tungsten particles in water, 10 µl (1 µg) DNA in Tris EDTA buffer (1 µg total DNA), 100 µl 2.5 M CaC12, and 10 µl 0.1 M spermidine. Each reagent is added sequentially to the tungsten particle suspension, with mixing. The final mixture is sonicated briefly and allowed to incubate under constant vortexing for 10 minutes. After the precipitation period, the tubes are centrifuged briefly, liquid is removed, and the particles are washed with 500 ml 100% ethanol, followed by a 30 second centrifugation. Again, the liquid is removed, and 105 µl of 100% ethanol is added to the final tungsten particle pellet. For particle gun bombardment, the tungsten/DNA particles are briefly sonicated. 10 µl of the tungsten/DNA particles is spotted onto the center of each macrocarrier, after which the spotted particles are allowed to dry about 2 minutes before bombardment.

The sample plates are bombarded at level #4 with a Biorad Helium Gun. All samples receive a single shot at 450 PSI, with a total of ten aliquots taken from each tube of prepared particles/DNA.

Following bombardment, the embryos are incubated on 560P (maintenance medium) for 12 to 48 hours at temperatures ranging from 26C to 37C, and then placed at 26C. After 5 to 7 days the embryos are transferred to 560R selection medium containing 3 mg/liter Bialaphos, and subcultured every 2 weeks at 26C. After approximately 10 weeks of selection, selection-resistant callus clones are transferred to 288J medium to initiate plant regeneration. Following somatic embryo maturation (2-4 weeks), well-developed somatic embryos are transferred to medium for germination and transferred to a lighted culture room. Approximately 7-10 days later, developing plantlets are transferred to 272V hormone-free medium in tubes for 7-10 days until plantlets are well established. Plants are then transferred to inserts in flats (equivalent to a 2.5" pot) containing potting soil and grown for 1 week in a growth chamber, subsequently grown an additional 1-2 weeks in the greenhouse, then transferred to Classic 600 pots (1.6 gallon) and grown to maturity. Plants are monitored and scored for transformation efficiency, and/or modification of regenerative capabilities.

Initiation medium (560L) comprises 4.0 g/l N6 basal salts (SIGMA C-1416), 1.0 ml/l Eriksson's Vitamin Mix (1000X SIGMA-1511), 0.5 mg/l thiamine HCI, 20.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).

Maintenance medium (560P) comprises 4.0 g/l N6 basal salts (SIGMA C-1416), 1.0 ml/l Eriksson's Vitamin Mix (1000X SIGMA-1511), 0.5 mg/l thiamine HCI, 30.0 g/l sucrose, 2.0 mg/l 2,4-D, and 0.69 g/l L-proline (brought to volume with D-I H2O following adjustment to pH 5.8 with KOH); 3.0 g/l Gelrite (added after bringing to volume with D-I H2O); and 0.85 mg/l silver nitrate (added after sterilizing the medium and cooling to room temperature).

Bombardment medium (560Y) comprises 4.0 g/l N6 basal salts (SIGMA C-1416), 1.0 ml/l Eriksson's Vitamin Mix (1000X SIGMA-1511), 0.5 mg/l thiamine HCI, 120.0 g/l sucrose, 1.0 mg/l 2,4-D, and 2.88 g/l L-proline (brought to volume with D-I H2O following adjustment to pH 5.8 with KOH); 2.0 g/l Gelrite (added after bringing to volume with D-I H2O); and 8.5 mg/l silver nitrate (added after sterilizing the medium and cooling to room temperature).
Selection medium (560R) comprises 4.0 g/l N6 basal salts (SIGMA C-1416), 1.0 ml/l Eriksson's Vitamin Mix (1000X SIGMA-1511), 0.5 mg/l thiamine HCI, 30.0 g/l sucrose, and 2.0 mg/l 2,4-D (brought to volume with D-I H2O following adjustment to pH 5.8 with KOH); 3.0 g/l Gelrite (added after bringing to volume with D-I H2O); and 0.85 mg/l silver nitrate and 3.0 mg/l bialaphos (both added after sterilizing the medium and cooling to room temperature).

Plant regeneration medium (288J) comprises 4.3 g/l MS salts (GIBCO 11117-074), 5.0 ml/l MS vitamins stock solution (0.100 g nicotinic acid, 0.02 g/l thiamine HCL, 0.10 g/l pyridoxine HCL, and 0.40 g/l glycine brought to volume with polished D-I H2O) (Murashige and Skoog (1962) Physiol. Plant. 15:473), 100 mg/l myo-inositol, 0.5 mg/l zeatin, 60 g/l sucrose, and 1.0 ml/l of 0.1 mM abscisic acid (brought to volume with polished D-I H2O after adjusting to pH 5.6); 3.0 g/l Gelrite (added after bringing to volume with D-I H2O); and 1.0 mg/l indoleacetic acid and 3.0 mg/l bialaphos (added after sterilizing the medium and cooling to 60°C). Hormone-free medium (272V) comprises 4.3 g/l MS salts (GIBCO 11117-074), 5.0 ml/l MS vitamins stock solution (0.100 g/l nicotinic acid, 0.02 g/l thiamine HCL, 0.10 g/l pyridoxine HCL, and 0.40 g/l glycine brought to volume with polished D-I H2O), 0.1 g/l myo-inositol, and 40.0 g/l sucrose (brought to volume with polished D-I H2O after adjusting pH to 5.6); and 6 g/l bacto-agar (added after bringing to volume with polished D-I H2O), sterilized and cooled to 60°C.

### b. Agrobacterium-mediated transformation

Agrobacterium-mediated transformation was performed essentially as described in Djukanovic et al. (2006) Plant Biotech J 4:345-57. Briefly, 10-12 day old immature embryos (0.8 -2.5 mm in size) were dissected from sterilized kernels and placed into liquid medium (4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 68.5 g/L sucrose, 36.0 g/L glucose, pH 5.2). After embryo collection, the medium was replaced with 1 ml *Agrobacterium* at a concentration of 0.35-0.45 OD550. Maize embryos were incubated with *Agrobacterium* for 5 min at room temperature, then the mixture was poured onto a media plate containing 4.0 g/L N6 Basal Salts (Sigma C-1416), 1.0 ml/L Eriksson's Vitamin Mix (Sigma E-1511), 1.0 mg/L thiamine HCI, 1.5 mg/L 2, 4-D, 0.690 g/L L-proline, 30.0 g/L sucrose, 0.85 mg/L silver nitrate, 0.1 nM acetosyringone, and 3.0 g/L Gelrite, pH 5.8. Embryos were incubated axis down, in the dark for 3 days at 20°C, then incubated 4 days in the dark at 28°C, then transferred onto new media plates containing 4.0 g/L N6 Basal Salts (Sigma C-1416), 1.0 ml/L Eriksson's Vitamin Mix (Sigma E-1511), 1.0 mg/L thiamine HCl, 1.5 mg/L 2, 4-D, 0.69 g/L L-proline, 30.0 g/L sucrose, 0.5 g/L MES buffer, 0.85 mg/L silver nitrate, 3.0 mg/L Bialaphos, 100 mg/L carbenicillin, and 6.0 g/L agar, pH 5.8. Embryos were subcultured every three weeks until transgenic events were identified. Somatic embryogenesis was induced by transferring a small amount of tissue onto regeneration medium (4.3 g/L MS salts (Gibco 11117), 5.0 ml/L MS Vitamins Stock Solution, 100 mg/L myo-inositol, 0.1 µM ABA, 1 mg/L IAA, 0.5 mg/L zeatin, 60.0 g/L sucrose, 1.5 mg/L Bialaphos, 100 mg/L carbenicillin, 3.0 g/L Gelrite, pH 5.6) and incubation in the dark for two weeks at 28°C. All material with visible shoots and roots were transferred onto media containing 4.3 g/L MS salts (Gibco 11117), 5.0 ml/L MS Vitamins Stock Solution, 100 mg/L myo-inositol, 40.0 g/L sucrose, 1.5 g/L Gelrite, pH 5.6, and incubated under artificial light at 28°C. One week later, plantlets were moved into glass tubes containing the same medium and grown until they were sampled and/or transplanted into soil.

### EXAMPLE 6

### Transient Expression of BBM Enhances Transformation

Parameters of the transformation protocol can be modified to ensure that the BBM activity is transient. One such method involves precipitating the BBM-containing plasmid in a manner that allows for transcription and expression, but precludes subsequent release of the DNA, for example, by using the chemical PEI. In one example, the BBM plasmid is precipitated onto gold particles with PEI, while the transgenic expression cassette (UBI::moPAT∼GFPm::PinII; moPAT is the maize optimized PAT gene) to be integrated is precipitated onto gold particles using the standard calcium chloride method.

Briefly, gold particles were coated with PEI as follows. First, the gold particles were washed. Thirty-five mg of gold particles, 1.0 in average diameter (A.S.I. #162-0010), were weighed out in a microcentrifuge tube, and 1.2 ml absolute EtOH was added and vortexed for one minute. The tube was incubated for 15 minutes at room temperature and then centrifuged at high speed using a microfuge for 15 minutes at 4oC. The supernatant was discarded and a fresh 1.2 ml aliquot of ethanol (EtOH) was added, vortexed for one minute, centrifuged for one minute, and the supernatant again discarded (this is repeated twice). A fresh 1.2 ml aliquot of EtOH was added, and this suspension (gold particles in EtOH) was stored at -20oC for weeks. To coat particles with polyethylimine (PEI; Sigma #P3143), 250 µl of the washed gold particle/EtOH mix was centrifuged and the EtOH discarded. The particles were washed once in 100 µl ddH2O to remove residual ethanol, 250 µl of 0.25 mM PEI was added, followed by a pulse-sonication to suspend the particles and then the tube was plunged into a dry ice/EtOH bath to flash-freeze the suspension, which was then lyophilized overnight. At this point, dry, coated particles could be stored at -80oC for at least 3 weeks. Before use, the particles were rinsed 3 times with 250 µl aliquots of 2.5 mM HEPES buffer, pH 7.1, with 1x pulse-sonication, and then a quick vortex before each centrifugation. The particles were then suspended in a final volume of 250 µl HEPES buffer. A 25 µl aliquot of the particles was added to fresh tubes before attaching DNA. To attach uncoated DNA, the particles were pulse-sonicated, then 1 µg of DNA (in 5 µl water) was added, followed by mixing by pipetting up and down a few times with a Pipetteman and incubated for 10 minutes. The particles were spun briefly (i.e. 10 seconds), the supernatant removed, and 60 µl EtOH added. The particles with PEI-precipitated DNA-1 were washed twice in 60 µl of EtOH. The particles were centrifuged, the supernatant discarded, and the particles were resuspended in 45 µl water. To attach the second DNA (DNA-2), precipitation using a water-soluble cationic lipid transfection reagent was used. The 45 µl of particles/DNA-1 suspension was briefly sonicated, and then 5 µl of 100 ng/µl of DNA-2 and 2.5 µl of the water-soluble cationic lipid transfection reagent were added. The solution was placed on a rotary shaker for 10 minutes, centrifuged at 10,000g for 1 minute. The supernatant was removed, and the particles resuspended in 60 µl of EtOH. The solution was spotted onto macrocarriers and the gold particles onto which DNA-1 and DNA-2 had been sequentially attached were delivered into scutellar cells of 10 DAP Hi-II immature embryos using a standard protocol for the PDS-1000. For this experiment, the DNA-1 plasmid contained a UBI::RFP::pinII expression cassette, and DNA-2 contained a UBI::CFP::pinII expression cassette. Two days after bombardment, transient expression of both the CFP and RFP fluorescent markers was observed as numerous red & blue cells on the surface of the immature embryo. The embryos were then placed on non-selective culture medium and allowed to grow for 3 weeks before scoring for stable colonies. After this 3-week period, 10 multicellular, stably-expressing blue colonies were observed, in comparison to only one red colony. This demonstrated that PEI-precipitation could be used to effectively introduce DNA for transient expression while dramatically reducing integration of the PEI-introduced DNA and thus reducing the recovery of RFP-expressing transgenic events. In this manner, PEI-precipitation can be used to deliver transient expression of BBM and/or WUS2.

For example, the particles are first coated with UBI::BBM::pinll using PEI, then coated with UBI::moPAT∼YFP using a water-soluble cationic lipid transfection reagent, and then bombarded into scutellar cells on the surface of immature embryos. PEI-mediated precipitation results in a high frequency of transiently expressing cells on the surface of the immature embryo and extremely low frequencies of recovery of stable transformants Thus, it is expected that the PEI-precipitated BBM cassette expresses transiently and stimulates a burst of embryogenic growth on the bombarded surface of the tissue (i.e. the scutellar surface), but this plasmid will not integrate. The PAT∼GFP plasmid released from the Ca++/gold particles is expected to integrate and express the selectable marker at a frequency that results in substantially improved recovery of transgenic events. As a control treatment, PEI-precipitated particles containing a UBI::GUS::pinII (instead of BBM) are mixed with the PAT∼GFP/Ca++ particles. Immature embryos from both treatments are moved onto culture medium containing 3mg/l bialaphos. After 6-8 weeks, it is expected that GFP+, bialaphos-resistant calli will be observed in the PEI/BBM treatment at a much higher frequency relative to the control treatment (PEI/GUS).

As an alternative method, the BBM plasmid is precipitated onto gold particles with PEI, and then introduced into scutellar cells on the surface of immature embryos, and subsequent transient expression of the BBM gene elicits a rapid proliferation of embryogenic growth. During this period of induced growth, the explants are treated with Agrobacterium using standard methods for maize (see Example 1), with T-DNA delivery into the cell introducing a transgenic expression cassette such as UBI::moPAT∼GFPm::pinII. After co-cultivation, explants are allowed to recover on normal culture medium, and then are moved onto culture medium containing 3 mg/l bialaphos. After 6-8 weeks, it is expected that GFP+, bialaphos-resistant calli will be observed in the PEI/BBM treatment at a much higher frequency relative to the control treatment (PEI/GUS).
It may be desirable to "kick start" callus growth by transiently expressing the BBM and/or WUS2 polynucleotide products. This can be done by delivering BBM and WUS2 5'-capped polyadenylated RNA, expression cassettes containing BBM and WUS2 DNA, or BBM and/or WUS2 proteins. All of these molecules can be delivered using a biolistics particle gun. For example 5'-capped polyadenylated BBM and/or WUS2 RNA can easily be made in vitro using Ambion's mMessage mMachine kit. RNA is co-delivered along with DNA containing a polynucleotide of interest and a marker used for selection/screening such as Ubi::moPAT∼GFPm::PinII. It is expected that the cells receiving the RNA will immediately begin dividing more rapidly and a large portion of these will have integrated the agronomic gene. These events can further be validated as being transgenic clonal colonies because they will also express the PAT∼GFP fusion protein (and thus will display green fluorescence under appropriate illumination). Plants regenerated from these embryos can then be screened for the presence of the polynucleotide of interest

### EXAMPLE 7

### Identification of amino acid domains of novel Cas9 systems of the present disclosure.

Multiple functional domains and conserved elements were determined for each of the novel Cas9 endonuclease protein of the present disclosure. Table 18 shows the domain location of the HNH, RuvC-I, RuvC-II, RuvC-III, REC1, REC1', Bridge Helix (BH) and PAM interacting (PI) domains along the amino acid sequence of each Cas9 endonuclease.

The novel Cas9 endonucleases of the present disclosure comprised an HNH domain, an RuvC domain that included three subdomains (RuvC-I, Ruvc-II and RuvC-II), a Brige Helicx domain a PAM interacting domain and DNA/RNA recognition regions including REC1 and REC1'. The REC1 binds to repeat::anti-repeat RNA duplex of the guide RNA while REC1' mainly interacts with targetDNA::guide RNA hybrid duplex.

**Table 18. Location of HNH, RuvC-I, RuvC-II, RuvC-III, REC1, REC1',, Bridge Helix (BH) and PAM interacting (PI) domains of novel Cas9 endonucleases of the present disclosure relative to their respective amino acid sequence.**

| Cas9 | Length | RuvC-I | BH | REC1 | REC1' | RuvC-II | HNH | RuvC-III | PI |
|---|---|---|---|---|---|---|---|---|---|
| Cas-Locus-1 | 1070 | 1-53 | 54-81 | 82-245 | 246-473 | 474-519 | 520-683 | 684-828 | 829-1070 |
| (SEQ ID NO: 11) | | | | | | | | | |
| Locus-2 | 1070 | 1-53 | 54-93 | 93-245 | 246-473 | 474-519 | 520-683 | 684-828 | 829-1070 |
| SEQ ID NO: 12) | | | | | | | | | |
| Locus-3 | 1068 | 1-41 | 42-81 | 82-231 | 232-460 | 461-505 | 506-660 | 661-808 | 809-1068 |
| SEQ ID NO: 13) | | | | | | | | | |
| Locus-4 | 1068 | 1-41 | 42-81 | 82-231 | 232-460 | 461-505 | 506-660 | 661-808 | 809-1068 |
| SEQ ID NO: 14) | | | | | | | | | |
| Locus-5 | 1068 | 1-41 | 42-81 | 82-231 | 232-460 | 461-505 | 506-660 | 661-808 | 809-1068 |
| SEQ ID NO: 15) | | | | | | | | | |
| Locus-42 | 1068 | 1-41 | 42-81 | 82-231 | 232-460 | 461-505 | 506-660 | 661-808 | 808-1068 |
| SEQ ID NO: 74) | | | | | | | | | |

| | | | | | | | | | |
|---|---|---|---|---|---|---|---|---|---|
| Length refers to the total amino acids of each Cas9 endonuclease protein. | | | | | | | | | |

The number range shown for each Cas9 endonuclease domain (RuvC-I, Bridge Helix (BH), REC1, REC1', RuvC-II, HNH, RuvC-III and PAM interacting (PI)) indicates the location of the first amino acid and last amino acid of that domain relative to the amino acid sequence of its respective Cas9 endonuclease. For example, the RuvCI domain of Cas-Locus-1 (1-53) comprises 53 amino acids spanning from the first amino acid (amino acid 1) to the 53^{rd} amino acid of the Cas9 endonuclease (Cas-Locus1) of SEQ ID NO: 11. None indicates that no REC2 domain is present in said Cas9 endonuclease.

### ASPECTS OF THE INVENTION

1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of SEQ ID NOs: 41, 42, 43, 44, 45, 66, 67, 80, a functional fragment of SEQ ID NOs: 41, 42, 43, 44, 45, 66, 67, 80, and a functional variant of SEQ ID NOs: 41, 42, 43, 44, 45, 66, 67, 80.
2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of SEQ ID NOs: 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79.
3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77.
4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of SEQ ID NOs : 31, 32, 33, 34, 35, 36, 37, 38, 39, 40, 78, 79, a functional fragment of SEQ ID NOs: 31, 32, 33, 34, 35, 36, 37, 38, 39, 40, 78, 79, and a functional variant of SEQ ID NOs: 31, 32, 33, 34, 35, 36, 37, 38, 39, 40, 78, 79, wherein said chimeric non-naturally occurring crRNA comprises a variable targeting domain capable of hybridizing to said target sequence.
5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of SEQ ID NOs: 26, 27, 28, 29, 30, 62, 63, 77, a functional fragment of SEQ ID NOs: 26, 27, 28, 29, 30, 62, 63, 77, and a functional variant of SEQ ID NOs: 26, 27, 28, 29, 30, 62, 63, 77, wherein said chimeric non-naturally occurring crRNA comprises a variable targeting domain capable of hybridizing to said target sequence.
6. A guide RNA/Cas9 endonuclease complex comprising at least one guide RNA and a Cas9 endonuclease, wherein said Cas9 endonuclease is selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA/Cas9 endonuclease complex is capable of recognizing, binding to, and optionally nicking or cleaving all or part of a target sequence.
7. A guide RNA/Cas9 endonuclease complex comprising at least one guide RNA and a Cas9 endonuclease, wherein said Cas9 endonuclease is encoded by a DNA sequence selected from the group consisting of SEQ ID NOs: 6, 7, 8, 9, 10, 46, 47, 48, 49, 73, a functional fragment of SEQ ID NOs: 6, 7, 8, 9, 10, 46, 47, 48, 49, 73, and a functional variant of SEQ ID NOs: 6, 7, 8, 9, 10, 46, 47, 48, 49, 73, wherein said guide RNA/Cas9 endonuclease complex is capable of recognizing, binding to, and optionally nicking or cleaving all or part of a target sequence.
8. The guide RNA/Cas9 endonuclease complex of any of aspects 6-7 comprising at least one guide RNA of any one of aspects 1-5.
9. The guide RNA/Cas9 endonuclease complex of any of aspects 6-7, wherein said target sequence is located in the genome of a cell.
10. A method for modifying a target site in the genome of a cell, the method comprising introducing into said cell at least one guide RNA and at least one Cas9 endonuclease protein selected from the group consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said guide RNA and Cas9 endonuclease can form a complex that is capable of recognizing, binding to, and optionally nicking or cleaving all or part of said target site.
11. The method of aspect 10, further comprising identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii).
12. A method for editing a nucleotide sequence in the genome of a cell, the method comprising introducing into said cell a polynucleotide modification template, at least one guide RNA, and at least one Cas9 endonuclease protein selected from the group consisting of SEQ ID NOs: consisting of SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said polynucleotide modification template comprises at least one nucleotide modification of said nucleotide sequence, wherein said guide RNA and Cas9 endonuclease can form a complex that is capable of recognizing, binding to, and optionally nicking or cleaving all or part of said target site.
13. A method for modifying a target site in the genome of a cell, the method comprising introducing into said cell at least one guide RNA, at least one donor DNA, and at least one Cas9 endonuclease selected from the group consisting of SEQ ID NOs: SEQ ID NOs: 11, 12, 13, 14, 15, 50, 51, 52, 53, 74, a functional fragment of SEQ ID NOs: 11, 12, 13, 14, 15, 50, 51, 52, 53, 74, and a functional variant of SEQ ID NOs: 11, 12, 13, 14, 15, 50, 51, 52, 53, 74, wherein said at least one guide RNA and at least one Cas9 endonuclease can form a complex that is capable of recognizing, binding to, and optionally nicking or cleaving all or part of said target site, wherein said donor DNA comprises a polynucleotide of interest.
14. The method of aspect 13, further comprising identifying at least one cell that has said polynucleotide of interest integrated in or near said target site.
15. The method of any one of aspects 10-14, wherein the cell is selected from the group consisting of a human, non-human, animal, bacterial, fungal, insect, yeast, non-conventional yeast, and plant cell.
16. The method of aspect 15, wherein the plant cell is selected from the group consisting of a monocot and dicot cell.
17. The method of aspect 16, wherein the plant cell is selected from the group consisting of maize, rice, sorghum, rye, barley, wheat, millet, oats, sugarcane, turfgrass, or switchgrass, soybean, canola, alfalfa, sunflower, cotton, tobacco, peanut, potato, tobacco, Arabidopsis, and safflower cell.
18. A plant comprising a modified target site, wherein said plant originates from a plant cell comprising a modified target site produced by the method of any of aspects 10-17.
19. A plant comprising an edited nucleotide, wherein said plant originates from a plant cell comprising an edited nucleotide produced by the method of aspect 12.

## Claims

1. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA is selected from the group consisting of:
(a) SEQ ID NOs: 41 and 42; and
(b) a sequence sharing at least 90% sequence identity with SEQ ID NO: 41 or 42 and retaining the ability to function as a guide RNA.

2. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said tracrRNA comprises a nucleotide sequence selected from the group consisting of:
(a) SEQ ID NOs: 31, 32, 36, and 37; and
(b) a sequence sharing at least 90% sequence identity with SEQ ID NO: 31, 32, 36, or 37 and retaining the ability to function as a tracrRNA.

3. A single guide RNA capable of forming a guide RNA/Cas9 endonuclease complex, wherein said guide RNA/Cas9 endonuclease complex can recognize, bind to, and optionally nick or cleave a target sequence, wherein said single guide RNA comprises a chimeric non-naturally occurring crRNA linked to a tracrRNA, wherein said chimeric non-naturally occurring crRNA comprises a nucleotide sequence selected from the group consisting of:
(a) SEQ ID NOs: 26 and 27; and
(b) a sequence sharing at least 90% sequence identity with SEQ ID NO: 26 or 27 and retaining the ability to function as a crRNA.

4. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a variable targeting domain capable of hybridizing to said target sequence, wherein said tracrRNA comprises a nucleotide sequence selected from the group consisting of:
(a) SEQ ID NOs: 31, 32, 36, and 37; and
(b) a sequence sharing at least 90% sequence identity with SEQ ID NO: 31, 32, 36, or 37 and retaining the ability to function as a tracrRNA;
wherein said chimeric non-naturally occurring crRNA comprises a variable targeting domain capable of hybridizing to said target sequence.

5. A guide RNA capable of forming a guide RNA/Cas9 endonuclease complex, wherein said guide RNA/Cas9 endonuclease complex can recognize, bind to, and optionally nick or cleave a target sequence, wherein said guide RNA is a duplex molecule comprising a chimeric non-naturally occurring crRNA and a tracrRNA, wherein said chimeric non-naturally occurring crRNA comprises a nucleotide sequence selected from the group consisting of:
(a) SEQ ID NOs: 26 and 27; and
(b) a sequence sharing at least 90% sequence identity with SEQ ID NO: 26 or 27 and retaining the ability to function as a crRNA;
wherein said chimeric non-naturally occurring crRNA comprises a variable targeting domain capable of hybridizing to said target sequence.

6. A guide RNA/Cas9 endonuclease complex comprising at least one guide RNA and a Cas9 endonuclease, wherein the RNA/Cas9 endonuclease complex comprises a guide RNA of any one of claims 1 to 5:
(a) wherein said Cas9 endonuclease is selected from the group consisting of:
(i) SEQ ID NOs: 11 and 12; and
(ii) a sequence sharing at least 90% sequence identity with SEQ ID NO: 11 or 12 and retaining the ability to recognise, bind to and optionally nick or cleave a target site; or
(b) wherein said Cas9 endonuclease is encoded by a DNA sequence selected from the group consisting of SEQ ID NOs: 6 and 7;
wherein said guide RNA/Cas9 endonuclease complex is capable of recognizing, binding to, and optionally nicking or cleaving all or part of a target sequence.

7. The guide RNA/Cas9 endonuclease complex of claim 6, wherein said target sequence is located in the genome of a cell.

8. A method for modifying a target site in the genome of a cell, the method comprising introducing into said cell:
(a) at least one guide RNA of any one of claims 1 to 5; and
(b) at least one Cas9 endonuclease protein selected from the group consisting of:
(i) SEQ ID NOs: 11 and 12; and
(ii) a sequence sharing at least 90% sequence identity with SEQ ID NO: 11 or 12 and retaining the ability to recognise, bind to and optionally nick or cleave a target site;
wherein said guide RNA and Cas9 endonuclease can form a complex that is capable of recognizing, binding to, and optionally nicking or cleaving all or part of said target site; and
wherein the cell is selected from the group consisting of a bacterial, fungal, insect, yeast, non-conventional yeast, and plant cell.

9. The method of claim 8, further comprising:
(a) identifying at least one cell that has a modification at said target, wherein the modification at said target site is selected from the group consisting of (i) a replacement of at least one nucleotide, (ii) a deletion of at least one nucleotide, (iii) an insertion of at least one nucleotide, and (iv) any combination of (i) - (iii); or
(b) introducing into said cell a polynucleotide modification template, wherein said polynucleotide modification template comprises at least one nucleotide modification as compared to said nucleotide sequence, wherein said guide RNA and Cas9 endonuclease can form a complex that is capable of recognizing, binding to, and optionally nicking or cleaving all or part of said target site.

10. The method of claim 8, further comprising introducing into said cell at least one donor DNA, wherein said donor DNA comprises a polynucleotide of interest.

11. The method of claim 10, further comprising identifying at least one cell that has said polynucleotide of interest integrated in or near said target site.

12. The method of any one of claims 8 to 11, wherein the plant cell is selected from the group consisting of a monocot and dicot cell;
preferably wherein the plant cell is selected from the group consisting of maize, rice, sorghum, rye, barley, wheat, millet, oats, sugarcane, turfgrass, switchgrass, soybean, canola, alfalfa, sunflower, cotton, peanut, potato, tobacco, Arabidopsis, and safflower cell.

13. A cell comprising:
(a) at least one Cas9 endonuclease comprising SEQ ID NO: 11 or 12 or sharing at least 90% sequence identity with SEQ ID NO: 11 or 12; and
(b) at least one guide RNA comprising SEQ ID NO: 41, 42, 31, 32, 36, 37, 26, or 27 or sharing at least 90% sequence identity with SEQ ID NO: 41, 42, 31, 32, 36, 37, 26, or 27;
preferably wherein the cell is selected from the group consisting of a human, non-human, animal, bacterial, fungal, insect, yeast, non-conventional yeast, and plant cell.

14. An *ex vivo* method for modifying a target site in the genome of a cell, the method comprising introducing into said cell:
(a) at least one guide RNA of any one of claims 1 to 5; and
(b) at least one Cas9 endonuclease protein selected from the group consisting of:
(i) SEQ ID NOs: 11 and 12; and
(ii) a sequence sharing at least 90% sequence identity with SEQ ID NO: 11 or 12 and retaining the ability to recognise, bind to and optionally nick or cleave a target site;
wherein said guide RNA and Cas9 endonuclease can form a complex that is capable of recognizing, binding to, and optionally nicking or cleaving all or part of said target site.

15. At least:
(a) one guide RNA of any one of claims 1 to 5; and
(b) one Cas9 endonuclease protein selected from the group consisting of:
(i) SEQ ID NOs: 11 and 12; and
(ii) a sequence sharing at least 90% sequence identity with SEQ ID NO: 11 or 12 and retaining the ability to recognise, bind to and optionally nick or cleave a target site;
wherein said guide RNA and Cas9 endonuclease can form a complex that is capable of recognizing, binding to, and optionally nicking or cleaving all or part of said target site;
for use in a method of treatment of the human or animal body, said treatment comprising modifying a target site in the genome of a cell;
wherein the cell is selected from a human and an animal cell.
